(19) Europäisches Patentamt
European Patent Office
Office européen des brevets

(11) EP 0 918 059 B1

(12) **EUROPÄISCHE PATENTSCHRIFT**

(45) Veröffentlichungstag und Bekanntmachung des Hinweises auf die Patenterteilung:
**25.06.2003 Patentblatt 2003/26**

(21) Anmeldenummer: **98121670.8**

(22) Anmeldetag: **13.11.1998**

(51) Int Cl.[7]: **C07K 5/097**, C07K 5/078, C07K 5/023, C07K 5/117, C07D 233/78, A61K 38/05, A61K 38/06, A61K 38/07

(54) **Substituierte Imidazolidinderivate, ihre Herstellung, ihre Verwendung und sie enthaltende pharmazeutische Präparate**

Substituted imidazoline derivatives, their preparation, their use, and pharmaceutical compositions containing them

Dérivés d'imidazoline substitués, leur préparation, leur application et compositions pharmaceutiques les contenant

(84) Benannte Vertragsstaaten:
**AT BE CH CY DE DK ES FI FR GB GR IE IT LI LU NL PT SE**
Benannte Erstreckungsstaaten:
**RO SI**

(30) Priorität: **19.11.1997 DE 19751251**

(43) Veröffentlichungstag der Anmeldung:
**26.05.1999 Patentblatt 1999/21**

(73) Patentinhaber: **Aventis Pharma Deutschland GmbH**
**65929 Frankfurt am Main (DE)**

(72) Erfinder:
- **Wehner, Volkmar Dr.**
  **97657 Sandberg (DE)**
- **Stilz, Hans Ulrich Dr.**
  **65929 Frankfurt (DE)**
- **Schmidt, Wolfgang Dr.**
  **65929 Frankfurt (DE)**
- **Seiffge, Dirk Dr.**
  **55246 Mainz-Kostheim (DE)**

(56) Entgegenhaltungen:
**EP-A- 0 449 079**
**EP-A- 0 530 505**
**EP-A- 0 566 919**
**EP-A- 0 580 008**
**EP-A- 0 584 694**
**EP-A- 0 796 855**
**EP-A- 0 842 943**
**EP-A- 0 842 944**
**EP-A- 0 842 945**
**WO-A-93/13798**
**WO-A-93/15764**
**WO-A-93/18057**
**WO-A-94/21607**
**WO-A-95/14008**
**WO-A-96/00581**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 918 059 B1

## Beschreibung

Die vorliegende Erfindung betrifft substituierte Imidazolidinderivate der Formel I,

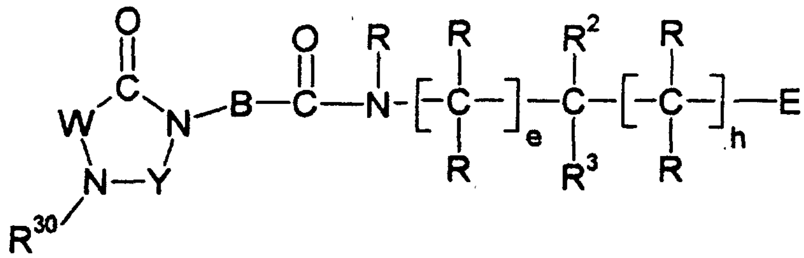

(I)

in der B, E, W, Y, R, $R^2$, $R^3$, $R^{30}$, e und h die unten angegebenen Bedeutungen haben. Die Verbindungen der Formel I sind wertvolle Arzneimittelwirkstoffe, die sich zum Beispiel zur Therapie und Prophylaxe von Entzündungserkrankungen, beispielsweise der rheumatoiden Arthritis, oder von allergischen Erkrankungen eignen. Die Verbindungen der Formel I sind Inhibitoren der Adhäsion und Migration von Leukozyten und/oder Antagonisten des zur Gruppe der Integrine gehörenden Adhäsionsrezeptors VLA-4. Sie eignen sich generell zur Therapie oder Prophylaxe von Krankheiten, die durch ein unerwünschtes Ausmaß an Leukozytenadhäsion und/oder Leukozytenmigration verursacht werden oder damit verbunden sind oder bei denen Zell-Zell- oder Zell-Matrix-Interaktionen eine Rolle spielen, die auf Wechselwirkungen von VLA-4-Rezeptoren mit ihren Liganden beruhen. Die Erfindung betrifft weiterhin Verfahren zur Herstellung der Verbindungen der Formel I, ihre Verwendung, insbesondere als Arzneimittelwirkstoffe, und pharmazeutische Präparate, die Verbindungen der Formel I enthalten.

Die Integrine sind eine Gruppe von Adhäsionsrezeptoren, die bei Zell-Zellbindenden und Zell-Extrazelluläre Matrix-bindenden Prozessen eine wesentliche Rolle spielen. Sie weisen eine aß-heterodimere Struktur auf und zeigen eine weite zelluläre Verbreitung und ein hohes Maß an evolutiver Konservierung. Zu den Integrinen gehört zum Beispiel der Fibrinogen-Rezeptor auf Thrombozyten, der vor allem mit der RGD-Sequenz des Fibrinogens interagiert, oder der Vitronectin-Rezeptor auf Osteoclasten, der vor allem mit der RGD-Sequenz des Vitronectins oder des Osteopontins interagiert. Man teilt die Integrine in drei Großgruppen ein, die β2-Unterfamilie mit den Vertretern LFA-1, Mac-1 und p150/95, die insbesondere für Zell-Zell-Interaktionen des Immunsystems verantwortlich sind, und die Unterfamilien β1 und β3, deren Vertreter hauptsächlich die Zellanheftung an Komponenten der extrazellulären Matrix vermitteln (Ruoslahti, Annu. Rev. Biochem. 1988, 57, 375). Die Integrine der β1-Unterfamilie, auch VLA-Proteine (very late (activation) antigen) genannt, umfassen mindestens sechs Rezeptoren, die spezifisch mit Fibronektin, Kollagen und/oder Laminin als Liganden interagieren. Innerhalb der VLA-Familie ist das Integrin VLA-4 (α4β1) insofern untypisch, als es hauptsächlich auf lymphoide und myeloide Zellen begrenzt ist und bei diesen verantwortlich ist für Zell-Zell-Interaktionen mit einer Vielzahl von anderen Zellen. VLA-4 vermittelt zum Beispiel die Interaktion von T- und B-Lymphozyten mit dem Heparin II-Bindungsfragment von humanem Plasmafibronektin (FN). Die Bindung von VLA-4 mit dem Heparin II-Bindungsfragment des Plasmafibronektins beruht vor allem auf einer Interaktion mit einer LDVP-Sequenz. Im Unterschied zum Fibrinogen- oder Vitronectin-Rezeptor ist VLA-4 kein typisches RGD-bindendes Integrin (Kilger und Holzmann, J. Mol. Meth. 1995, 73, 347).

Die im Blut zirkulierenden Leukozyten zeigen normalerweise nur eine geringe Affinität zu den vaskulären endothelialen Zellen, die die Blutgefäße auskleiden. Zytokine, die von entzündetem Gewebe abgegeben werden, bewirken die Aktivierung von Endothelzellen und damit die Expression einer Vielzahl von Zelloberflächenantigenen. Diese umfassen zum Beispiel die Adhäsionsmoleküle ELAM-1 (endothelial cell adhesion molecule-1; auch als E-Selektin bezeichnet), das unter anderem Neutrophile bindet, ICAM-1 (intercellular adhesion molecule-1), das mit LFA-1 (leucocyte function-associated antigen 1) auf Leukozyten interagiert, und VCAM-1 (vascular cell adhesion molecule-1), das verschiedene Leukozyten, unter anderem Lymphozyten, bindet (Osborn et al., Cell 1989, 59, 1203). VCAM-1 ist, wie ICAM-1, ein Mitglied der Immunglobulin-Gen-Überfamilie. Identifiziert wurde VCAM-1 (zuerst bekannt als INCAM-110) als ein Adhäsionsmolekül, daß auf endothelialen Zellen durch Entzündungs-Zytokine wie TNF und IL-1 und Lipopolysaccharide (LPS) induziert wird. Elices et al. (Cell 1990, 60, 577) zeigten, daß VLA-4 und VCAM-1 ein Rezeptor-Ligand-Paar bilden, das die Anheftung von Lymphozyten an aktiviertes Endothel vermittelt. Die Bindung von VCAM-1 an VLA-4 erfolgt dabei nicht durch eine Interaktion des VLA-4 mit einer RGD-Sequenz, eine solche ist im VCAM-1-nicht enthalten (Bergelson et al., Current Biology 1995, 5, 615). VLA-4 tritt aber auch auf anderen Leukozyten auf, und über den VCAM-1/VLA-4-Adhäsionsmechanismus wird auch die Anheftung von anderen Leukozyten als Lymphozyten vermittelt. VLA-4 repräsentiert somit ein einzelnes Beispiel eines β1-Integrin-Rezeptors, der über die Liganden VCAM-1 bzw. Fibronektin sowohl bei Zell-Zell-Interaktionen als auch bei Zell-Extrazellulärer Matrix-Interaktionen eine

wesentliche Rolle spielt.

Die Zytokin-induzierten Adhäsionsmoleküle spielen eine wichtige Rolle bei der Rekrutierung von Leukozyten in extravaskuläre Gewebebereiche. Leukozyten werden in entzündliche Gewebebereiche durch Zelladhäsionsmoleküle rekrutiert, die auf der Oberfläche von endothelialen Zellen exprimiert werden und als Liganden für Leukozyten-Zelloberflächen-Proteine oder -Proteinkomplexe (Rezeptoren) dienen (die Begriffe Ligand und Rezeptor können auch vice versa verwendet werden). Leukozyten aus dem Blut müssen zunächst an endotheliale Zellen anheften, bevor sie in das Synovium auswandern können. Da VCAM-1 an Zellen bindet, die das Integrin VLA-4 ($\alpha 4\beta 1$) tragen, wie Eosinophile, T- und B-Lymphozyten, Monozyten oder Neutrophile, kommt ihm und dem VCAM-1/VLA-4-Mechanismus die Funktion zu, derartige Zellen aus dem Blutstrom in Infektionsgebiete und Entzündungsherde zu rekrutieren (Elices et al., Cell 1990, 60, 577; Osborn, Cell 1990, 62, 3; Issekutz et al., J. Exp. Med. 1996, 183, 2175).

Der VCAM-1/VLA-4-Adhäsionsmechanismus wurde mit einer Reihe von physiologischen und pathologischen Prozessen in Verbindung gebracht. VCAM-1 wird außer von Zytokin-induziertem Endothel unter anderem noch von den folgenden Zellen exprimiert: Myoblasten, lymphoiden dendritischen Zellen und Gewebsmakrophagen, rheumatoidem Synovium, Zytokin-stimulierten Neuralzellen, parietalen Epithelzellen der Bowmans Kapsel, dem renalen Tubularepithel, entzündetem Gewebe bei Herz- und Nieren-Transplantat-Abstoßung und von Intestinalgewebe bei Graft versus host-Krankheit. VCAM-1 findet man auch exprimiert auf solchen Gewebearealen des arteriellen Endotheliums, die frühen arteriosklerotischen Plaques eines Kaninchenmodells entsprechen. Zusätzlich wird VCAM-1 auf follikulären dendritischen Zellen von humanen Lymphknoten exprimiert und findet sich auf Stromazellen des Knochenmarks, zum Beispiel in der Maus. Letzterer Befund weist auf eine Funktion von VCAM-1 in der B-Zell-Entwicklung hin. VLA-4 wird, außer auf Zellen haematopoetischen Ursprunges, auch zum Beispiel auf Melanoma-Zellinien gefunden, und der VCAM-1/VLA-4-Adhäsionsmechanismus wird mit der Metastasierung von solchen Tumoren in Verbindung gebracht (Rice et al., Science 1989, 246, 1303).

Die hauptsächliche Form, in der VCAM-1 in vivo auf endothelialen Zellen vorkommt und die die dominante Form in vivo ist, wird als VCAM-7D bezeichnet und trägt sieben Immunglobulin-Domänen. Die Domänen 4, 5 und 6 ähneln in ihren Aminosäuresequenzen den Domänen 1, 2 und 3. Die vierte Domäne ist bei einer weiteren, aus sechs Domänen bestehenden Form, hier als VCAM-6D bezeichnet, durch alternatives Splicing entfernt. Auch VCAM-6D kann VLA-4-exprimierende Zellen binden.

Weitere Angaben zu VLA-4, VCAM-1, Integrinen und Adhäsionsproteinen finden sich zum Beispiel in den Artikeln von Kilger und Holzmann, J. Mol. Meth. 1995, 73, 347; Elices, Cell Adhesion in Human Disease, Wiley, Chichester 1995, S. 79; Kuijpers, Springer Semin. Immunopathol. 1995, 16, 379.

Aufgrund der Rolle des VCAM-1/VLA-4-Mechanismus bei Zelladhäsionsprozessen, die von Bedeutung zum Beispiel bei Infektionen, Entzündungen oder Atherosklerose sind, wurde versucht, durch Eingriffe in diese Adhäsionsprozesse Krankheiten zu bekämpfen, insbesondere zum Beispiel Entzündungen (Osbom et al., Cell 1989, 59, 1203). Eine Methode hierzu ist die Verwendung von monoklonalen Antikörpern, die gegen VLA-4 gerichtet sind. Derartige monoklonale Antikörper (mAK), die als VLA-4-Antagonisten die Interaktion zwischen VCAM-1 und VLA-4 blockieren, sind bekannt. So inhibieren zum Beispiel die anti-VLA-4 mAK HP2/1 und HP1/3 die Anheftung von VLA-4 exprimierenden Ramos-Zellen (B-Zell-ähnlichen Zellen) an humane Nabelschnurendothelzellen und an VCAM-1-transfizierte COS-Zellen. Ebenso inhibiert der anti-VCAM-1 mAK 4B9 die Adhäsion von Ramos-Zellen, Jurkat-Zellen (T-Zell-ähnlichen Zellen) und HL60-Zellen (Granulozyten-ähnlichen Zellen) an COS-Zellen transfiziert mit genetischen Konstrukten, die veranlassen, daß VCAM-6D und VCAM-7D exprimiert werden. In vitro-Daten mit Antikörpern, die gegen die $\alpha 4$-Untereinheit von VLA-4 gerichtet sind, zeigen, daß die Anheftung von Lymphozyten an synoviale Endothelzellen blockiert wird, eine Adhäsion, die bei der rheumatoiden Arthritis eine Rolle spielt (van Dinther-Janssen et al., J. Immunol. 1991, 147, 4207).

In vivo-Versuche haben gezeigt, daß eine experimentelle autoimmune Enzephalomyelitis durch anti-$\alpha 4$ mAK gehemmt werden kann. Die Wanderung von Leukozyten in einen Entzündungsherd wird ebenfalls durch einen monoklonalen Antikörper gegen die $\alpha 4$-Kette von VLA-4 blockiert. Die Beeinflussung des VLA-4-abhängigen Adhäsionsmechanismus mit Antikörpern wurde auch in einem Asthma-Modell untersucht, um die Rolle von VLA-4 bei der Rekrutierung von Leukozyten in entzündetes Lungengewebe zu untersuchen (WO-A-93/13798). Die Gabe von anti-VLA-4-Antikörpern inhibierte die Spätphasenreaktion und die Atemwegsüberreaktion in allergischen Schafen.

Der VLA-4 abhängige Zelladhäsionsmechanismus wurde ebenfalls in einem Primatenmodell der inflammatory bowel disease (IBD) untersucht. In diesem Modell, das der ulcerativen Colitis im Menschen entspricht, ergab die Gabe von anti-VLA-4-Antikörpem eine signifikante Reduktion der akuten Entzündung.

Darüber hinaus konnte gezeigt werden, daß die VLA-4-abhängige Zelladhäsion bei den folgenden klinischen Konditionen einschließlich der folgenden chronischen entzündlichen Prozesse eine Rolle spielt: Rheumatoide Arthritis (Cronstein und Weismann, Arthritis Rheum. 1993, 36, 147; Elices et al., J. Clin. Invest. 1994, 93, 405), Diabetes mellitus (Yang et al., Proc. Natl. Acad. Sci. USA 1993, 90, 10494), systemischer Lupus erythematosus (Takeuchi et al., J. Clin. Invest. 1993, 92, 3008), Allergien vom verzögerten Typ (Typ IV-Allergie) (Elices et al., Clin. Exp. Rheumatol. 1993, 11, S77), multiple Sklerose (Yednock et al., Nature 1992, 356, 63), Malaria (Ockenhouse et al., J. Exp. Med. 1992, 176,

1183), Arteriosklerose (O'Brien et al., J. Clin. Invest. 1993, 92, 945), Transplantation (Isobe et al., Transplantation Proceedings 1994, 26, 867-868), verschiedene Malignitäten, zum Beispiel Melanom (Renkonen et al., Am. J. Pathol. 1992, 140, 763), Lymphom (Freedman et al., Blood 1992, 79, 206) und andere (Albelda et al., J. Cell Biol. 1991, 114, 1059).

Eine VLA-4-Blockierung durch geeignete Antagonisten bietet danach effektive therapeutische Möglichkeiten, insbesondere zum Beispiel verschiedene entzündliche Konditionen einschließlich Asthma und IBD zu behandeln. Die besondere Relevanz von VLA-4-Antagonisten für die Behandlung der rheumatoiden Arthritis ergibt sich dabei, wie bereits gesagt, aus der Tatsache, daß Leukozyten aus dem Blut zunächst an endotheliale Zellen anheften müssen, ehe sie in das Synovium auswandern können, und daß bei dieser Anheftung der VLA-4-Rezeptor eine Rolle spielt. Darauf, daß durch Entzündungsagenzien auf endothelialen Zellen VCAM-1 induziert wird (Osbom, Cell 1990, 62, 3; Stoolman, Cell 1989, 56, 907), und auf die Rekrutierung verschiedener Leukozyten in Infektionsgebiete und Entzündungsherde wurde bereits oben eingegangen. T-Zellen adherieren dabei an aktiviertes Endothel hauptsächlich über die LFA-1/ICAM-1- und VLA-4/VCAM-1-Adhäsionsmechanismen (Springer, Cell 1994, 76, 301). Auf den meisten synovialen T-Zellen ist die Bindungskapazität von VLA-4 für VCAM-1 bei der rheumatoiden Arthritis erhöht (Postigo et al., J. Clin. Invest. 1992, 89, 1445). Zusätzlich wurde eine verstärkte Anheftung von synovialen T-Zellen an Fibronektin beobachtet (Laffon et al., J. Clin. Invest. 1991, 88, 546; Morales-Ducret et al., J. Immunol. 1992, 149, 1424). VLA-4 ist also hochreguliert sowohl im Rahmen seiner Expression als auch hinsichtlich seiner Funktion auf T-Lymphozyten der rheumatoiden Synovialmembran. Die Blockierung der Bindung von VLA-4 an seine physiologischen Liganden VCAM-1 und Fibronektin ermöglicht eine effektive Verhinderung oder Linderung von artikulären Entzündungsprozessen. Dies wird auch durch Experimente mit dem Antikörper HP2/1 an Lewis-Ratten mit Adjuvanz-Arthritis bestätigt, bei denen eine effektive Krankheitsprävention beobachtet wurde (Barbadillo et al., Springer Semin. Immunopathol. 1995, 16, 427). VLA-4 stellt also ein wichtiges therapeutisches Zielmolekül dar.

Die oben erwähnten VLA-4-Antikörper und der Einsatz von Antikörpern als VLA-4-Antagonisten sind in den Patentanmeldungen WO-A-93/13798, WO-A-93/15764, WO-A-94/16094, WO-A-94/17828 und WO-A-95/19790 beschrieben. In den Patentanmeldungen WO-A-94/15958, WO-A-95/15973, WO-A-96/00581, WO-A-96/06108 und WO-A-96/20216 werden peptidische Verbindungen als VLA-4-Antagonisten beschrieben. Der Einsatz von Antikörpern und peptidischen Verbindungen als Arzneimitteln ist aber mit Nachteilen behaftet, zum Beispiel mangelnder oraler Verfügbarkeit, leichter Abbaubarkeit oder immunoger Wirkung bei längerfristiger Anwendung, und es besteht somit Bedarf nach VLA-4-Antagonisten mit einem günstigen Eigenschaftsprofil für einen Einsatz in der Therapie und Prophylaxe.

In der WO-A-95/14008, der WO-A-94/21607 (US-A-5 658 935), der WO-A-93/18057, der EP-A-449 079 (US-A-5 686 421), der EP-A-530 505 (US-A-5 389 614), der EP-A-566 919 (US-A-5 397 796), der EP-A-580 008 (US-A-5 424 293) und der EP-A-584 694 (US-A-5 554 594) sind substituierte 5-Ring-Heterocyclen beschrieben, die am N-terminalen Ende des Moleküls eine Amino-, Amidino- oder Guanidinofunktion aufweisen und die thrombozytenaggregationshemmende Wirkungen zeigen. In der EP-A-796 855 sind weitere Heterocyclen beschrieben, die Inhibitoren der Knochenresorption sind. In der EP-A-842 943, EP-A-842 945 und EP-A-842 944 (deutsche Patentanmeldungen 19647380.2, 19647381.0 und 19647382.9) wird beschrieben, daß Verbindungen aus diesen Reihen und weitere Verbindungen überraschenderweise auch die Leukozytenadhäsion hemmen und VLA-4-Antagonisten sind. Weitere Untersuchungen zeigten, daß auch die Verbindungen der vorliegenden Anmeldung starke Hemmstoffe der Leukozytenadhäsion und/oder VLA-4-Antagonisten sind.

Die vorliegende Erfindung betrifft Verbindungen der Formel I,

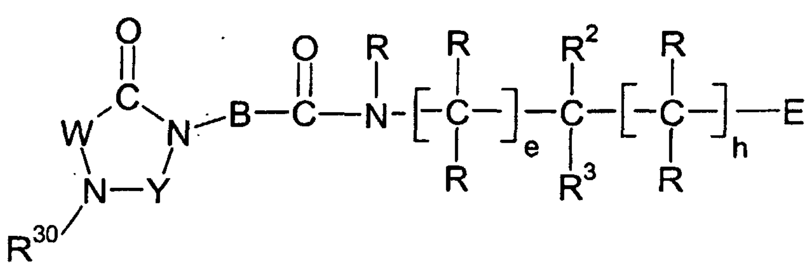

(I)

worin

W für einen zweiwertigen Rest aus der Reihe $R^1$-A-C($R^{13}$) und

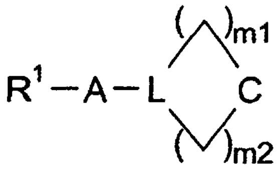

steht, worin die Ringsysteme

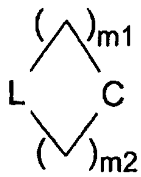

ein oder zwei gleiche oder verschiedene Heteroatome aus der Reihe N und O enthalten können, gesättigt oder einfach ungesättigt sein können und durch 1 oder 2 gleiche oder verschiedene Substituenten $R^{13}$ und/oder durch ein oder zwei doppelt gebundene Sauerstoffatome substituiert sein können, und worin L für C ($R^{13}$) oder N steht und worin m1 und m2 unabhängig voneinander für eine der Zahlen 0, 1, 2, 3 und 4 stehen, die Summe m1 + m2 aber für eine der Zahlen 1, 2, 3 und 4 steht;

Y für eine Carbonylgruppe oder Thiocarbonylgruppe steht;

A für eine direkte Bindung, einen der zweiwertigen Reste ($C_1$-$C_6$)-Alkylen, (C5-$C_6$)-Cycloalkylen, Phenylen, Phenylen-($C_1$-$C_4$)-alkyl oder für einen zweiwertigen Rest eines 5-gliedrigen oder 6-gliedrigen gesättigten oder ungesättigten Heterocyclus, der ein oder zwei Stickstoffatome enthalten kann und einfach oder zweifach durch ($C_1$-$C_6$)-Alkyl oder doppelt gebundenen Sauerstoff oder Schwefel substituiert sein kann, steht, wobei in dem Rest Phenylenalkyl der Rest $R^1$ an die Phenylengruppe gebunden ist;

B für einen zweiwertigen Methylenrest oder Ethylenrest steht, wobei der Methylenrest und der Ethylenrest unsubstituiert sind oder substituiert sind durch einen oder zwei gleiche oder verschiedene Reste aus der Reihe ($C_1$-$C_8$)-Alkyl, ($C_2$-$C_8$)-Alkenyl, ($C_2$-$C_8$)-Alkinyl, ($C_3$-$C_6$)-Cycloalkyl, ($C_3$-$C_6$)-Cycloalkyl-($C_1$-$C_6$)-alkyl, gegebenenfalls substituiertes ($C_6$-$C_{10}$)-Aryl, im Arylrest gegebenenfalls substituiertes ($C_6$-$C_{10}$)-Aryl-($C_1$-$C_6$)-alkyl, gegebenenfalls substituiertes Heteroaryl, im Heteroarylrest gegebenenfalls substituiertes Heteroaryl-($C_1$-$C_6$)-alkyl;

E für $R^{10}CO$, HO-$CH_2$ oder $R^8CO$-O-$CH_2$ steht;

R für Wasserstoff oder ($C_1$-$C_8$)-Alkyl steht, wobei alle Reste R unabhängig voneinander sind und die Reste R gleich oder verschieden sein können;

$R^1$ für Wasserstoff, ($C_1$-$C_{10}$)-Alkyl, das gegebenenfalls durch Fluor einfach oder mehrfach substituiert sein kann, im Arylrest gegebenenfalls substituiertes $R^{21}$-(($C_6$-$C_{10}$)-Aryl), im Arylrest gegebenenfalls substituiertes ($R^{21}$-(($C_6$-$C_{10}$)-Aryl))-($C_1$-$C_6$)-alkyl, den Rest Het-, Het-($C_1$-$C_6$)-alkyl oder für einen der Reste X-NH-C(=NH)-$R^{20}$-, $X^1$-NH-$R^{20}$-, $R^{22}N(R^{21})$-C(O)-, O= und S= steht;

x für Wasserstoff, ($C_1$-$C_6$)-Alkyl, ($C_1$-$C_6$)-Alkylcarbonyl, ($C_1$-$C_6$)-Alkoxycarbonyl, ($C_1$-$C_8$)-Alkylcarbonyloxy-($C_1$-$C_6$)-alkoxycarbonyl, gegebenenfalls substituiertes ($C_6$-$C_{10}$)-Arylcarbonyl, gegebenenfalls substituiertes ($C_6$-$C_{10}$)-Aryloxycarbonyl, ($C_6$-$C_{14}$)-Aryl-($C_1$-$C_6$)-alkoxycarbonyl, das im Arylrest auch substituiert sein kann, Hydroxy, ($C_1$-$C_6$)-Alkoxy oder Amino steht;

$X^1$ eine der Bedeutungen von X hat oder für R'-NH-C(=N-R") steht, worin R' und R" unabhängig voneinander die Bedeutungen von X haben;

$R^2$ für Wasserstoff oder ($C_1$-$C_8$)-Alkyl steht;

$R^3$ für Wasserstoff, ($C_1$-$C_8$)-Alkyl, das gegebenenfalls durch 1 bis 6 Fluoratome substituiert sein kann, gegebenenfalls substituiertes ($C_6$-$C_{12}$)-Aryl, im Arylrest gegebenenfalls substituiertes ($C_6$-$C_{12}$)-Aryl-($C_1$-$C_6$)-alkyl, gegebenenfalls substituiertes Heteroaryl, im Heteroarylrest gegebenenfalls substituiertes Heteroaryl-($C_1$-$C_6$)-alkyl, ($C_3$-$C_8$)-Cycloalkyl, ($C_3$-$C_8$)-Cycloalkyl-($C_1$-$C_6$)-alkyl, ($C_6$-$C_{12}$)-Bicycloalkyl, ($C_6$-$C_{12}$)-Bicycloalkyl-($C_1$-$C_6$)-alkyl, ($C_6$-$C_{12}$)-Tricycloalkyl, ($C_6$-$C_{12}$)-Tricycloalkyl-($C_1$-$C_6$)-alkyl, ($C_2$-$C_8$)-Alkenyl, ($C_2$-$C_8$)-Alkinyl, $R^{11}NH$, $COOR^{21}$, $CON(CH_3)R^4$, $CONHR^4$, $CON(CH_3)R^{15}$ oder $CONHR^{15}$ steht;

$R^4$ für ($C_1$-$C_8$)-Alkyl steht, das unsubstituiert ist oder einfach oder zweifach substituiert ist durch gleiche oder

verschiedene Reste aus der Reihe Hydroxy, $(C_1-C_8)$-Alkoxy, $R^5$, gegebenenfalls substituiertes $(C_3-C_8)$-Cycloalkyl, Hydroxycarbonyl, Aminocarbonyl, $(C_6-C_{10})$-Aryl-$(C_1-C_4)$-alkoxycarbonyl, das im Arylrest auch substituiert sein kann, $(C_1-C_6)$-Alkoxycarbonyl, $R^6$-CO, $R^7$-CO, Tetrazolyl, Trifluormethyl;

$R^5$ für gegebenenfalls substituiertes $(C_6-C_{12})$-Aryl, im Arylrest gegebenenfalls substituiertes $(C_6-C_{12})$-Aryl-$(C_1-C_8)$-alkyl oder einen Rest eines gegebenenfalls substituierten monocyclischen oder bicyclischen, 5-gliedrigen bis 12-gliedrigen heterocyclischen Ringes, der aromatisch, teilweise gesättigt oder vollständig gesättigt sein kann und der ein, zwei oder drei gleiche oder verschiedene Heteroatome aus der Reihe Stickstoff, Sauerstoff und Schwefel enthalten kann, steht;

$R^6$ für den Rest einer natürlichen oder unnatürlichen Aminosäure, Iminosäure, gegebenenfalls N-$(C_1-C_8)$-alkylierten oder N-($(C_6-C_{12})$-Aryl-$(C_1-C_8)$-alkylierten) Azaaminosäure, die im Arylrest auch substituiert sein kann, oder den Rest eines Dipeptids oder Tripeptids steht, sowie für deren Ester und Amide, worin freie funktionelle Gruppen durch in der Peptidchemie übliche Schutzgruppen geschützt sein können und worin die Stickstoffatome in den Amidbindungen in der Gruppe $R^6$-CO einen Rest R als Substituenten tragen können;

$R^7$ für den Rest eines über ein Stickstoffatom gebundenen 5-gliedrigen bis 7-gliedrigen, gesättigten monocyclischen oder bicyclischen Heterocyclus steht, der ein, zwei, drei oder vier gleiche oder verschiedene zusätzliche Ring-Heteroatome aus der Reihe Sauerstoff, Stickstoff und Schwefel enthalten kann und der an Kohlenstoffatomen und an zusätzlichen Ring-Stickstoffatomen gegebenenfalls substituiert sein kann, worin zusätzliche Ring-Stickstoffatome gleiche oder verschiedene Reste aus der Reihe Wasserstoff, $R^h$, HCO, $R^h$CO, $R^h$O-CO, HO-CO-$(C_1-C_4)$-Alkyl und $R^h$O-CO-$(C_1-C_4)$-Alkyl als Substituenten tragen können und $R^h$ für $(C_1-C_6)$-Alkyl, $(C_3-C_8)$-Cycloalkyl, $(C_3-C_8)$-Cycloalkyl-$(C_1-C_8)$-alkyl, gegebenenfalls substituiertes $(C_6-C_{10})$-Aryl oder im Arylrest gegebenenfalls substituiertes $(C_6-C_{10})$-Aryl-$(C_1-C_4)$-alkyl steht;

$R^8$ für Wasserstoff, $(C_1-C_6)$-Alkyl oder im Phenylrest gegebenenfalls substituiertes Phenyl-$(C_1-C_4)$-alkyl steht;

$R^{10}$ für Hydroxy, $(C_1-C_8)$-Alkoxy, $(C_6-C_{10})$-Aryl-$(C_1-C_6)$-alkoxy, das im Arylrest auch substituiert sein kann, gegebenenfalls substituiertes $(C_6-C_{10})$-Aryloxy, $(C_1-C_6)$-Alkylcarbonyloxy-$(C_1-C_6)$-alkoxy, $(C_1-C_6)$-Alkoxycarbonyloxy-$(C_1-C_6)$-alkoxy, Amino, Mono- oder Di-($(C_1-C_6)$-alkyl)-amino, Aminocarbonyl-$(C_1-C_6)$-alkoxy oder (Mono- oder Di-($(C_1-C_6)$-alkyl)-amino)-carbonyl-$(C_1-C_6)$-alkoxy steht;

$R^{11}$ für Wasserstoff, $R^{12a}$, $R^{12a}$-CO, $R^{12a}$-O-CO, $R^{12b}$-CO oder $R^{12a}$-$S(O)_2$ steht;

$R^{12a}$ für $(C_1-C_{10})$-Alkyl, $(C_2-C_8)$-Alkenyl, $(C_2-C_8)$-Alkinyl, $(C_5-C_{10})$-Cycloalkyl, $(C_5-C_{10})$-Cycloalkyl-$(C_1-C_8)$-alkyl, gegebenenfalls substituiertes $(C_6-C_{14})$-Aryl, im Arylrest gegebenenfalls substituiertes $(C_6-C_{14})$-Aryl-$(C_1-C_8)$-alkyl, gegebenenfalls substituiertes Heteroaryl, im Heteroarylrest gegebenenfalls substituiertes Heteroaryl-$(C_1-C_8)$-alkyl oder den Rest $R^{15}$ steht;

$R^{12b}$ für Amino, Di-($(C_1-C_{10})$-alkyl)-amino oder $R^{12a}$-NH steht;

$R^{13}$ für Wasserstoff oder $(C_1-C_6)$-Alkyl steht;

$R^{15}$ für $R^{16}$-$(C_1-C_6)$-alkyl oder für $R^{16}$ steht;

$R^{16}$ für einen 6-gliedrigen bis 14-gliedrigen bicyclischen oder tricyclischen Rest steht, der gesättigt oder teilweise ungesättigt ist und der auch ein, zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe $(C_1-C_4)$-Alkyl und Oxo substituiert sein kann;

$R^{20}$ für eine direkte Bindung oder $(C_1-C_2)$-Alkylen steht;

$R^{21}$ für Wasserstoff, $(C_1-C_6)$-Alkyl, $(C_3-C_6)$-Cycloalkyl, $(C_3-C_6)$-Cycloalkyl-$(C_1-C_4)$-alkyl, gegebenenfalls substituiertes $(C_6-C_{10})$-Aryl, im Arylrest gegebenenfalls substituiertes $(C_6-C_{10})$-Aryl-$(C_1-C_4)$-alkyl, den Rest Het- oder Het-$(C_1-C_4)$-alkyl steht, wobei Alkylreste durch Fluor einfach oder mehrfach substituiert sein können und die Reste $R^{21}$ bei mehrfachem Auftreten gleich oder verschieden sein können;

$R^{22}$ für einen der Reste $R^{21}$-, $R^{21}N(R^{21})$- oder $R^{21}N(R^{21})$-$C(=N(R^{21}))$- steht;

$R^{30}$ für einen der Reste $R^{32}(R)N$-CO-N(R)-$R^{31}$, $R^{32}(R)N$-CS-N(R)-$R^{31}$, $R^3$-CO-N(R)-$R^{31}$ oder $R^{32}(R)N$-CO-$R^{31}$ steht, wobei $R^{30}$ nicht für $R^{32}$-CO-N(R)-$R^{31}$ stehen kann, wenn gleichzeitig W für $R^1$-A-C($R^{13}$) steht, A für eine direkte Bindung steht und $R^1$ und $R^{13}$ für Wasserstoff stehen;

$R^{31}$ für den zweiwertigen Rest -$R^{33}$-$R^{34}$-$R^{35}$-$R^{36}$- steht, wobei $R^{36}$ an das Stickstoffatom im Imidazolidinring in der Formel I gebunden ist;

$R^{32}$ für Wasserstoff, $(C_1-C_6)$-Alkyl, das gegebenenfalls durch 1 bis 6 Fluoratome substituiert sein kann, $(C_2-C_6)$-Alkenyl, $(C_2-C_6)$-Alkinyl, $(C_5-C_6)$-Cycloalkyl, $(C_5-C_6)$-Cycloalkyl-$(C_1-C_6)$-alkyl, gegebenenfalls substituiertes $(C_6-C_{10})$-Aryl, im Arylrest gegebenenfalls substituiertes $(C_6-C_{10})$-Aryl-$(C_1-C_6)$-alkyl, gegebenenfalls substituiertes Heteroaryl oder im Heteroarylrest gegebenenfalls substituiertes Heteroaryl-$(C_1-C_6)$-alkyl steht;

$R^{33}$ für eine direkte Bindung oder einen zweiwertigen $(C_1-C_4)$-Alkylenrest steht;

$R^{34}$ für einen zweiwertigen Rest aus der Reihe $(C_1-C_6)$-Alkylen, $(C_5-C_6)$-Cycloalkylen, gegebenenfalls substituiertes $(C_6-C_{10})$-Arylen und gegebenenfalls substituiertes Heteroarylen steht;

$R^{35}$ für eine direkte Bindung oder einen zweiwertigen $(C_1-C_4)$-Alkylenrest steht;
$R^{36}$ für eine direkte Bindung, die Gruppe -CO- oder die Gruppe $-S(O)_n-$ steht;
Het für einen Rest eines monocyclischen oder polycyclischen, 5-gliedrigen bis 12-gliedrigen, aromatischen oder nicht aromatischen Ringes steht, der 1 oder 2 gleiche oder verschiedene Heteroatome aus der Reihe N und O als Ringglieder enthält und gegebenenfalls durch einen oder mehrere, gleiche oder verschiedene Substituenten substituiert sein kann;
e und h unabhängig voneinander für 0 oder 1 stehen;
n für 1 oder 2 steht;

in allen ihren stereoisomeren Formen und Mischungen davon in allen Verhältnissen, und ihre physiologisch verträglichen Salze.

Können Reste oder Substituenten mehrfach in den Verbindungen der Formel I auftreten, so können sie alle unabhängig voneinander die angegebenen Bedeutungen haben und gleich oder verschieden sein. In zusammengesetzen Resten, zum Beispiel Arylalkyl, geht die freie Bindung, über die der Rest gebunden ist, von der am rechten Ende des Namens angegebenen Komponente aus, im Falle des Arylalkylrestes also von der Alkylgruppe, an die dann als Substituent eine Arylgruppe gebunden ist.

Alkylreste können geradkettig oder verzweigt sein. Dies gilt auch, wenn sie Substituenten tragen oder als Substituenten anderer Reste auftreten, beispielsweise in Alkoxyresten, Alkoxycarbonylresten oder Arylalkylresten. Beispiele für geeignete Alkylreste sind Methyl, Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Pentadecyl, n-Hexadecyl, n-Heptadecyl, n-Octadecyl, Isopropyl, Isobutyl, Isopentyl, Isohexyl, 3-Methylpentyl, Neopentyl, Neohexyl, 2,3,5-Trimethylhexyl, sec-Butyl, tert-Butyl, tert-Pentyl. Bevorzugte Alkylreste sind Methyl, Ethyl, n-Propyl, Isopropyl, n-Butyl, Isobutyl, sec-Butyl, tert-Butyl, n-Pentyl, Isopentyl, n-Hexyl und Isohexyl. Sind Alkylreste durch Fluoratome substituiert, so können sie, soweit nicht anders angegeben, beispielsweise 1, 2, 3, 4, 5, 6 oder 7 Fluoratome enthalten. Beispielsweise kann in einem fluorsubstituierten Alkylrest eine Methylgruppe als Trifluormethylgruppe vorliegen.

Alkylenreste (= Alkandiylreste), das heißt zweiwertige, von einem Alkan abgeleitete Reste, können ebenfalls geradkettig oder verzweigt sein. Sie können über beliebige Positionen gebunden sein. Beispiele für Alkylenreste sind die den vorstehend genannten einwertigen Resten entsprechenden zweiwertigen Reste, zum Beispiel Methylen, Ethylen (= 1,2-Ethylen oder 1,1-Ethylen), Trimethylen (= 1,3-Propylen), Tetramethylen (= 1,4-Butylen), Pentamethylen, Hexamethylen oder durch Alkylreste substituiertes Methylen oder Ethylen. Beispiele für substituiertes Methylen sind Methylengruppen, die durch eine Methylgruppe, eine Ethylgruppe, eine n-Propylgruppe, eine Isopropylgruppe, eine n-Butylgruppe, eine Isobutylgruppe, eine tert-Butylgruppe, eine n-Pentylgruppe, eine Isopentylgruppe oder eine n-Hexylgruppe substituiert sind. Substituiertes Ethylen kann sowohl an dem einem Kohlenstoffatom als auch an dem anderen Kohlenstoffatom oder auch an beiden Kohlenstoffatomen substituiert sein kann.

Auch Alkenylreste und Alkenylenreste (= Alkendiylreste) sowie Alkinylreste können geradkettig oder verzweigt sein. Beispiele für Alkenylreste sind Vinyl, 1-Propenyl, Allyl, Butenyl, 2-Methyl-1-propenyl, 2-Methyl-2-propenyl, 3-Methyl-2-butenyl, für Alkenylenreste Vinylen, Propenylen, Butenylen, für Alkinylreste Ethinyl, 1-Propinyl, Propargyl.

Cycloalkylreste sind insbesondere Cyclopropyl, Cyclobutyl, Cyclopentyl, Cyclohexyl, Cycloheptyl, Cyclooctyl, Cyclononyl, Cyclodecyl, Cycloundecyl und Cyclododecyl, die aber auch durch beispielsweise durch $(C_1-C_4)$-Alkyl substituiert sein können. Beispiele für substituierte Cycloalkylreste sind 4-Methylcyclohexyl und 2,3-Dimethylcyclopentyl. Diese Erläuterungen zu den einwertigen Cycloalkylresten gelten entsprechend für Cycloalkylenreste (= Cycloalkandiylreste), das heißt zweiwertige, von Cycloalkanen abgeleitete Reste. Cycloalkylenreste können über beliebige Positionen gebunden sein.

Bicycloalkylreste, Tricycloalkylreste und die für $R^{16}$ stehenden 6-gliedrigen bis 14-gliedrigen bicyclischen und tricyclischen Reste werden formal durch Abstraktion eines Wasserstoffatoms aus Bicyclen bzw. Tricyclen erhalten. Die zugrunde liegenden Bicyclen und Tricyclen können als Ringglieder nur Kohlenstoffatome enthalten, es kann sich also um Bicycloalkane oder Tricycloalkane handeln, sie können im Falle der für $R^{16}$ stehenden Reste aber auch ein bis vier gleiche oder verschiedene Heteroatome aus der Reihe Stickstoff, Sauerstoff und Schwefel enthalten, es kann sich also um Aza-, Oxa- und Thia-bicycloalkane und -tricycloalkane handeln. Sind Heteroatome enthalten, so sind bevorzugt ein oder zwei Heteroatome, insbesondere Stickstoffatome oder Sauerstoffatome, enthalten. Die Heteroatome können beliebige Positionen im bicyclischen bzw. tricyclischen Gerüst einnehmen, sie können sich in den Brücken oder im Falle von Stickstoffatomen auch an den Brückenköpfen befinden. Sowohl die Bicycloalkane und Tricycloalkane als auch ihre Hetero-Analoga können vollständig gesättigt sein oder eine oder mehrere Doppelbindungen enthalten. Bevorzugt enthalten sie eine oder zwei Doppelbindungen oder sind insbesondere vollständig gesättigt. Sowohl die Bicycloalkane und Tricycloalkane als auch die Hetero-Analoga und sowohl die gesättigten als auch die ungesättigten Vertreter können unsubstituiert sein oder in beliebigen geeigneten Positionen durch eine oder mehrere Oxogruppen und/oder eine oder mehrere gleiche oder verschiedene $(C_1-C_4)$-Alkylgruppen, zum Beispiel Methylgruppen oder Isopropylgruppen, bevorzugt Methylgruppen, substituiert sein. Die freie Bindung des bicyclischen oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo-Position oder einer endo-Position.

Beispiele für Grundkörper bicyclischer Ringsysteme, von denen sich ein bicyclischer Rest ableiten kann, sind das Norbornan (= Bicyclo[2.2.1]heptan), das Bicyclo[2.2.2]octan und das Bicyclo[3.2.1]octan, Beispiele für Heteroatome enthaltende, ungesättigte oder substituierte Systeme sind das 7-Azabicylo[2.2.1]heptan, das Bicyclo[2.2.2.]oct-5-en und der Campher (= 1,7,7-Trimethyl-2-oxobicyclo[2.2.1]heptan).

Beispiele für Systeme, von denen sich ein tricyclischer Rest ableiten kann, sind das Twistan (= Tricyclo [4.4.0.0$^{3,8}$]decan), das Adamantan (= Tricyclo[3.3.1.1$^{3,7}$]decan), das Noradamantan (= Tricyclo[3.3.1.0$^{3,7}$]nonan), das Tricyclo[2.2.1.0$^{2,6}$]heptan, das Tricyclo[5.3.2.0$^{4,9}$]dodecan, das Tricyclo[5.4.0.0$^{2,9}$]undecan oder das Tricyclo [5.5.1.0$^{3,11}$]tridecan.

Bevorzugt leiten sich bicyclische oder tricyclische Reste von verbrückten Bicyclen bzw. Tricyclen ab, also von Systemen, in denen Ringe zwei oder mehr als zwei Atome gemeinsam haben. Bevorzugt sind, soweit nicht anders angegeben, weiterhin auch bicyclische oder tricyclische Reste mit 6 bis 18 Ringgliedern, besonders bevorzugt solche mit 6 bis 14 Ringgliedern, ganz besonders bevorzugt solche mit 7 bis 12 Ringgliedern.

Im einzelnen sind besonders bevorzugte bicyclische oder tricyclische Reste, die zum Beispiel für eine Bicycloalkylgruppe oder für eine Tricycloalkylgruppe stehen können, der 2-Norbornylrest, sowohl derjenige mit der freien Bindung in der exo-Position als auch derjenige mit der freien Bindung in der endo-Position, der 2-Bicyclo[3.2.1]octylrest, der Adamantylrest, sowohl der 1-Adamantylrest als auch der 2-Adamantylrest, der Homoadamantylrest und der Noradamantylrest, zum Beispiel der 3-Noradamantylrest. Darüber hinaus bevorzugt sind der 1-Adamantylrest und der 2-Adamantylrest.

Die vorstehenden Erläuterungen zu den einwertigen Bicycloalkylresten und Tricycloalkylresten gelten entsprechend für die zweiwertigen Bicycloalkylenreste und Tricycloalkylenreste (= Bicycloalkandiylreste und Tricycloalkandiylreste).

($C_6$-$C_{14}$)-Arylgruppen sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl, ($C_6$-$C_{10}$)-Arylgruppen sind beispielsweise 1-Naphthyl, 2-Naphthyl und Phenyl. Biphenylylreste, Naphthylreste und insbesondere Phenylreste sind bevorzugte Arylreste. Arylreste, insbesondere Phenylreste, können unsubstituiert sein oder einfach oder mehrfach, zum Beispiel einfach, zweifach, dreifach oder vierfach, durch gleiche oder verschiedene Reste substituiert sein. Substituierte Arylreste, insbesondere Phenylreste, sind bevorzugt substituiert durch Reste aus der Reihe ($C_1$-$C_8$)-Alkyl, insbesondere ($C_1$-$C_4$)-Alkyl wie Methyl; ($C_1$-$C_8$)-Alkoxy, insbesondere ($C_1$-$C_4$)-Alkoxy wie Methoxy; ($C_1$-$C_8$)-Alkoxy, insbesondere ($C_1$-$C_4$)-Alkoxy, das durch ein oder mehrere Fluoratome, zum Beispiel 1, 2, 3, 4 oder 5 Fluoratome, substituiert ist, wie Trifluormethoxy; Halogen; Nitro; Amino; Trifluormethyl; Hydroxy; Hydroxy-($C_1$-$C_4$)-alkyl wie zum Beispiel Hydroxymethyl oder 1-Hydroxyethyl oder 2-Hydroxyethyl; Methylendioxy; Ethylendioxy; Formyl; Acetyl; Cyan; Hydroxycarbonyl; Aminocarbonyl; ($C_1$-$C_4$)-Alkoxycarbonyl; Phenyl; Phenoxy; Benzyl; Benzyloxy; Tetrazolyl. Entsprechendes gilt beispielsweise für substituierte Arylreste in Gruppen wie Arylalkyl, Arylcarbonyl, etc. Arylalkylreste sind zum Beispiel 1- und 2-Naphthylmethyl, 2-, 3- und 4-Biphenylylmethyl und 9-Fluorenylmethyl und insbesondere Benzyl, die alle auch substituiert sein können. Substituierte Arylalkylreste sind beispielsweise Benzylreste und Naphthylmethylreste, die im Arylteil durch einen oder mehrere ($C_1$-$C_8$)-Alkylreste, insbesondere ($C_1$-$C_4$)-Alkylreste, substituiert sind, zum Beispiel 2-, 3- und 4-Methylbenzyl, 4-Isobutylbenzyl, 4-tert-Butylbenzyl, 4-Octylbenzyl, 3,5-Dimethylbenzyl, Pentamethylbenzyl, 2-, 3-, 4-, 5-, 6-, 7- und 8-Methyl-1-naphthylmethyl, 1-, 3-, 4-, 5-, 6-, 7- und 8-Methyl-2-naphthylmethyl; Benzylreste und Naphthylmethylreste, die im Arylteil durch einen oder mehrere ($C_1$-$C_8$)-Alkoxyreste, insbesondere ($C_1$-$C_4$)-Alkoxyreste, substituiert sind, zum Beispiel 4-Methoxybenzyl, 4-Neopentyloxybenzyl, 3,5-Dimethoxybenzyl, 2,3,4-Trimethoxybenzyl; 3,4-Methylendioxybenzyl; Trifluormethoxybenzylreste; Nitrobenzylreste, zum Beispiel 2-, 3- und 4-Nitrobenzyl; Halobenzylreste, zum Beispiel 2-, 3- und 4-Chlor- und 2-, 3-, und 4-Fluorbenzyl, 3,4-Dichlorbenzyl, Pentafluorbenzyl; Trifluormethylbenzylreste, zum Beispiel 3- und 4-Trifluormethylbenzyl oder 3,5-Bistrifluormethylbenzyl. Substituierte Arylalkylreste können aber auch voneinander verschiedene Substituenten enthalten. In den Verbindungen der Formel I können aber im allgemeinen nicht mehr als zwei Nitrogruppen im Molekül vorhanden sein.

In monosubstituierten Phenylresten kann sich der Substituent in der 2-Position, der 3-Position oder der 4-Position befinden. Zweifach substituiertes Phenyl kann in der 2,3-Position, der 2,4-Position, der 2,5-Position, der 2,6-Position, der 3,4-Position oder der 3,5-Position substituiert sein. In dreifach substituierten Phenylresten können sich die Substituenten in der 2,3,4-Position, der 2,3,5-Position, der 2,4,5-Position, der 2,4,6-Position, der 2,3,6-Position oder der 3,4,5-Position befinden.

Die vorstehenden Erläuterungen zu den einwertigen Arylresten gelten entsprechend für zweiwertige Arylenreste, das heißt zweiwertige, von Aromaten abgeleitete Reste. Arylenreste können über beliebige Positionen verknüpft sein. Ein Beispiel für Arylenreste sind Phenylenreste, die beispielsweise als 1,4-Phenylen oder als 1,3-Phenylen vorliegen können.

Phenylen-alkyl ist insbesondere Phenylenmethyl ($-C_6H_4-CH_2-$) oder Phenylenethyl (zum Beispiel

$-C_6H_4-CH_2-CH_2-$).

Heteroaryl steht für einen Rest eines monocyclischen oder polycyclischen aromatischen Systems mit 5 bis 14 Ringgliedern, das 1, 2, 3, 4 oder 5 Heteroatome als Ringglieder enthält. Beispiele für Heteroatome sind N, O und S. Sind mehrere Heteroatome enthalten, können diese gleich oder verschieden sein. Heteroarylreste können ebenfalls unsubstituiert oder einfach oder mehrfach, zum Beispiel einfach, zweifach oder dreifach, substituiert sein durch gleiche oder verschiedene Reste aus der Reihe ($C_1$-$C_8$)-Alkyl, insbesondere ($C_1$-$C_4$)-Alkyl, ($C_1$-$C_8$)-Alkoxy, insbesondere ($C_1$-$C_4$)-Alkoxy, ($C_1$-$C_8$)-Alkoxy, insbesondere ($C_1$-$C_4$)-Alkoxy, das durch ein oder mehrere, zum Beispiel 1, 2, 3, 4 oder 5, Fluoratome, substituiert ist, Halogen, Nitro, Amino, Trifluormethyl, Hydroxy, Hydroxy-($C_1$-$C_4$)-alkyl wie zum Beispiel Hydroxymethyl oder 1-Hydroxyethyl oder 2-Hydroxyethyl, Methylendioxy, Ethylendioxy, Formyl, Acetyl, Cyan, Hydroxycarbonyl, Aminocarbonyl, ($C_1$-$C_4$)-Alkoxycarbonyl, Phenyl, Phenoxy, Benzyl, Benzyloxy, Tetrazolyl. Bevorzugt steht Heteroaryl für einen monocyclischen oder bicyclischen aromatischen Rest, der 1, 2, 3 oder 4, insbesondere 1, 2 oder 3, gleiche oder verschiedene Heteroatome aus der Reihe N, O und S enthält und der durch 1, 2, 3 oder 4, insbesondere 1 bis 3, gleiche oder verschiedene Substituenten aus der Reihe ($C_1$-$C_6$)-Alkyl, ($C_1$-$C_6$)-Alkoxy, Fluor, Chlor, Nitro, Amino, Trifluormethyl, Hydroxy, Hydroxy-($C_1$-$C_4$)-alkyl, ($C_1$-$C_4$)-Alkoxycarbonyl, Phenyl, Phenoxy, Benzyloxy und Benzyl substituiert sein kann. Besonders bevorzugt steht Heteroaryl für einen monocyclischen oder bicyclischen aromatischen Rest mit 5 bis 10 Ringgliedern, insbesondere für einen 5-gliedrigen bis 6-gliedrigen monocyclischen aromatischen Rest, der 1, 2 oder 3, insbesondere 1 oder 2, gleiche oder verschiedene Heteroatome aus der Reihe N, O und S enthält und durch 1 oder 2 gleiche oder verschiedene Substituenten aus der Reihe ($C_1$-$C_4$)-Alkyl, ($C_1$-$C_4$)-Alkoxy, Phenyl, Phenoxy, Benzyloxy und Benzyl substituiert sein kann.

Heterocyclen, die für monocyclische oder bicyclische 5-gliedrige bis 12-gliedrige heterocyclische Ringe stehen, können aromatisch oder teilweise gesättigt oder vollständig gesättigt sein. Sie können unsubstituiert sein oder an einem oder mehreren Kohlenstoffatomen oder an einem oder mehreren Stickstoffatomen durch gleiche oder verschiedene Substituenten substituiert sein, wie dies für den Rest Heteroaryl angegeben ist. Insbesondere kann der heterocyclische Ring einfach oder mehrfach, zum Beispiel einfach, zweifach, dreifach oder vierfach, an Kohlenstoffatomen durch gleiche oder verschiedene Reste aus der Reihe ($C_1$-$C_8$)-Alkyl, zum Beispiel ($C_1$-$C_4$)-Alkyl, ($C_1$-$C_8$)-Alkoxy, zum Beispiel ($C_1$-$C_4$)-Alkoxy wie Methoxy, Phenyl-($C_1$-$C_4$)-alkoxy, zum Beispiel Benzyloxy, Hydroxy, Oxo, Halogen, Nitro, Amino oder Trifluormethyl substituiert sein, und/oder es können Ring-Stickstoffatome in heterocyclischen Ringen wie auch in Heteroarylresten durch ($C_1$-$C_8$)-Alkyl, zum Beispiel ($C_1$-$C_4$)-Alkyl wie Methyl oder Ethyl, durch gegebenenfalls substituiertes Phenyl oder Phenyl-($C_1$-$C_4$)-alkyl, zum Beispiel Benzyl, substituiert sein.

Die Gruppe Het umfaßt zum einen aromatische Heterocyclen und damit auch die für Heteroaryl stehenden Gruppen, soweit diese hinsichtlich der Zahl der Ringglieder und Heteroatome unter die Definition von Het fallen. Het umfaßt aber zusätzlich auch nicht aromatische Heterocyclen, die vollständig gesättigt sind oder die eine oder mehrere Doppelbindungen im Ringsystem enthalten. Het kann an Stickstoffatomen und/oder Kohlenstoffatomen durch einen oder mehrere, zum Beispiel 1, 2, 3 oder 4, gleiche oder verschiedene Substituenten substituiert sein, beispielsweise durch ($C_1$-$C_8$)-Alkyl, insbesondere ($C_1$-$C_4$)-Alkyl, ($C_3$-$C_{12}$)-Cycloalkyl, ($C_3$-$C_{12}$)-Cycloalkyl-($C_1$-$C_8$)-alkyl, gegebenenfalls substituiertes ($C_6$-$C_{14}$)-Aryl, im Arylrest gegebenenfalls substituiertes ($C_6$-$C_{14}$)-Aryl-($C_1$-$C_8$)-alkyl, Heteroaryl, Heteroaryl-($C_1$-$C_8$)-alkyl, ($C_1$-$C_8$)-Alkoxy, insbesondere ($C_1$-$C_4$)-Alkoxy, gegebenenfalls substituiertes Phenoxy, Benzyloxy, Halogen, Nitro, Amino, ($C_1$-$C_8$)-Alkylamino, Di-(($C_1$-$C_8$)-Alkyl)-amino, Trifluormethyl, Hydroxy, Methylendioxy, Ethylendioxy, Cyan, Hydroxycarbonyl, Aminocarbonyl, ($C_1$-$C_4$)-Alkoxycarbonyl und allgemein durch Estergruppen, Acylgruppen, Oxo, Thioxo, wobei Alkylreste durch Fluor einfach oder mehrfach substituiert sein können.

Beispiele für Grundkörper von Heterocyclen, die einem Heteroarylrest, dem Rest Het, dem Rest eines monocyclischen oder bicyclischen 5-gliedrigen bis 12-gliedrigen heterocyclischen Ringes, dem zweiwertigen Rest eines 5-gliedrigen oder 6-gliedrigen Heterocyclus, dem für $R^7$ stehenden heterocyclischen Rest oder einem für $R^{16}$ stehenden heterocyclischen Rest zugrunde liegen können, sind, soweit sie im Einzelfall unter die jeweilige Definition fallen, Pyrrol, Furan, Thiophen, Imidazol, Pyrazol, Oxazol, Isoxazol, Thiazol, Isothiazol, Tetrazol, Pyridin, Pyrazin, Pyrimidin, Indol, Isoindol, Indazol, Phthalazin, Chinolin, Isochinolin, Chinoxalin, Chinazolin, Cinnolin, β-Carbolin und benz-anellierte, cyclopenta-, cyclohexa- oder cyclohepta-anellierte Derivate dieser Heterocyclen.

Stickstoffheterocyclen können auch als N-Oxide vorliegen oder als Quartärsalze.

Reste, die für Heteroaryl oder den Rest eines monocyclische oder bicyclischen 5-gliedrige bis 12-gliedrigen heterocyclischen Rings stehen können, sind beispielsweise 2- oder 3-Pyrrolyl, Phenylpyrrolyl, zum Beispiel 4- oder 5-Phenyl-2-pyrrolyl, 2- oder 3-Furyl, 2- oder 3-Thienyl, 4-Imidazolyl, Methylimidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3 - oder 4-Isochinolyl, 1-Oxo-1,2-dihydro-3-isochinolyl, 2-Chinoxalinyl, 2-Benzofuranyl, 2-Benzothienyl, 2-Benzoxazolyl oder 2-Benzothiazolyl oder, als Reste von teilweie gesättigten oder vollständig gesättigten heterocyclischen Ringen, beispielsweise auch Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl, Benzodioxolanyl.

Die Erläuterungen zu Heteroarylresten gelten entsprechend für die zweiwertigen Heteroarylenreste, das heißt die zweiwertigen, von Heteroaromaten abgeleiteten Reste.

Für den Rest $R^7$ stehende heterocyclische Reste können an den Kohlenstoffatomen und/oder an zusätzlichen Ring-Stickstoffatomen unsubstituiert oder einfach oder mehrfach, zum Beispiel zweifach, dreifach, vierfach oder fünffach, durch gleiche oder verschiedene Substituenten substituiert sein. Kohlenstoffatome können zum Beispiel durch ($C_1$-$C_8$)-Alkyl, insbesondere ($C_1$-$C_4$)-Alkyl, ($C_1$-$C_8$)-Alkoxy, insbesondere ($C_1$-$C_4$)-Alkoxy, Halogen, Nitro, Amino, Trifluormethyl, Hydroxy, Oxo, Cyan, Hydroxycarbonyl, Aminocarbonyl, ($C_1$-$C_4$)-Alkoxycarbonyl, Phenyl, Phenoxy, Benzyl, Benzyloxy, Tetrazolyl substituiert sein, insbesondere durch ($C_1$-$C_4$)-Alkyl, zum Beispiel Methyl, Ethyl oder tert-Butyl, ($C_1$-$C_4$)-Alkoxy, zum Beispiel Methoxy, Hydroxy, Oxo, Phenyl, Phenoxy, Benzyl, Benzyloxy. Schwefelatome können zum Sulfoxid oder zum Sulfon oxidiert sein. Beispiele für den Rest Het sind 1-Pyrrolidinyl, 1-Piperidinyl, 1-Piperazinyl, 4-substituiertes 1-Piperazinyl, 4-Morpholinyl, 4-Thiomorpholinyl, 1-Oxo-4-thiomorpholinyl, 1,1-Dioxo-4-thiomorpholinyl, Perhydroazepin-1-yl, 2,6-Dimethyl-1-piperidinyl, 3,3-Dimethyl-4-morpholinyl, 4-Isopropyl-2,2,6,6-tetramethyl-1-piperazinyl, 4-Acetyl-1-piperazinyl, 4-Ethoxycarbonyl-1-piperazinyl. Halogen steht für Fluor, Chlor, Brom oder Iod, insbesondere für Fluor oder Chlor.

Der Substituent an einem für B stehenden substituierten Methylenrest oder Ethylenrest kann zum einen Cyclus enthalten, wenn es sich um einen Substituenten aus der Reihe ($C_3$-$C_6$)-Cycloalkyl, ($C_3$-$C_6$)-Cycloalkyl-($C_1$-$C_6$)-alkyl, gegebenenfalls substituiertes ($C_6$-$C_{10}$)-Aryl, im Arylrest gegebenenfalls substituiertes ($C_6$-$C_{10}$)-Aryl-($C_1$-$C_6$)-alkyl, gegebenenfalls substituiertes Heteroaryl und im Heteroarylrest gegebenenfalls substituiertes Heteroaryl-($C_1$-$C_6$) handelt. Zum anderen kann der Substituent an einem für B stehenden substituierten Methylenrest oder Ethylenrest acyclisch sein, wenn es sich um einen Substituenten aus der Reihe ($C_1$-$C_8$)-Alkyl, ($C_2$-$C_8$)-Alkenyl und ($C_2$-$C_8$)-Alkinyl handelt. Die acyclischen Substituenten können 2, 3, 4, 5, 6, 7 oder 8 Kohlenstoffatome und im Falle eines gesättigten Alkylrestes auch 1 Kohlenstoffatom enthalten. Im Falle der Alkenylreste und Alkinylreste kann sich die Doppelbindung oder Dreifachbindung in einer beliebigen Position befinden und im Falle der Doppelbindung cis-Konfiguration oder trans-Konfiguration aufweisen. Wie oben erläutert, können diese Alkylreste, Alkenylreste und Alkinylreste geradkettig oder verzweigt sein.

Als Beispiele für Substituenten, die der für B stehende Methylenrest oder Ethylenrest tragen kann, seien insbesondere genannt Methyl, Ethyl, n-Propyl, n-Butyl, n-Pentyl, n-Hexyl, n-Heptyl, n-Octyl, Isopropyl, Isobutyl, Isopentyl, Isohexyl, sec-Butyl, tert-Butyl, tert-Pentyl, Neopentyl, Neohexyl, 3-Methylpentyl, 2-Ethylbutyl, Vinyl, Allyl, 1-Propenyl, 2-Butenyl, 3-Butenyl, 3-Methyl-2-butenyl, Ethinyl, 1-Propinyl, 2-Propinyl, 6-Hexinyl, Phenyl, Benzyl, 1-Phenylethyl, 2-Phenylethyl, 3-Phenylpropyl, 4-Biphenylylmethyl, Cyclopropyl, Cyclopropylmethyl, Cyclobutyl, Cyclobutylmethyl, Cyclopentyl, Cyclopentylmethyl, Cyclohexyl, Cyclohexylmethyl, 2-Cyclohexylethyl, 2-Pyridyl, 3-Pyridyl, 4-Pyridyl, 2-Pyridylmethyl, 3-Pyridylmethyl, 4-Pyridylmethyl, 2-(4-Pyridyl)ethyl, 2-Furylmethyl, 3-Furylmethyl, 2-Thienylmethyl, 3-Thienylmethyl oder 2-(3-Indolyl)ethyl.

Der für $R^6$ stehende Rest einer Aminosäure, Iminosäure oder Azaaminosäure oder eines Dipeptids oder Tripeptids wird wie in der Peptidchemie üblich aus der entsprechenden Aminosäure, Iminosäure oder Azaaminosäure oder dem Dipeptid oder Tripeptid erhalten, indem von der N-terminalen Aminogruppe oder von der Iminogruppe formal ein Wasserstoffatom entfernt wird. Über die so entstehende freie Bindung an der Aminogruppe oder der Iminogruppe ist diese Gruppe dann peptidartig durch eine Amidbindung mit der CO-Gruppe in der Gruppe $R^6$-CO verknüpft.

Die natürlichen und unnatürlichen Aminosäuren können in allen stereochemischen Formen vorliegen, beispielsweise in der D-Form, der L-Form oder in Form einer Mischung von Stereoisomeren, zum Beispiel in Form eines Racemats. Bevorzugte Aminosäuren sind $\alpha$-Aminosäuren und $\beta$-Aminosäuren, besonders bevorzugt sind $\alpha$-Aminosäuren. Als in Betracht kommende Aminosäuren seien beispielsweise genannt (vgl. Houben-Weyl, Methoden der organischen Chemie, Band 15/1 und 15/2, Georg Thieme Verlag, Stuttgart, 1974):

Aad, Abu, $\gamma$Abu, ABz, 2ABz, $\varepsilon$Aca, Ach, Acp, Adpd, Ahb, Aib, $\beta$Aib, Ala, $\beta$Ala, $\Delta$Ala, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, $\beta$Lys, $\Delta$Lys, Met, Mim, Min, nArg, Nle, Nva, Oly, Orn, Pan, Pec, Pen, Phe, Phg, Pic, Pro, $\Delta$Pro, Pse, Pya, Pyr, Pza, Qin, Ros, Sar, Sec, Sem, Ser, Thi, $\beta$Thi, Thr, Thy, Thx, Tia, Tle, Tly, Trp, Trta, Tyr, Val, tert-Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-phenylaminoessigsäure, 2-(p-Chlorphenyl)-aminoessigsäure.

Steht $R^6$ für den Rest einer natürlichen oder unnatürlichen $\alpha$-Aminosäure, so kann dieser Rest beispielsweise der Formel -N(R)-CH(SC)-CO-AG entsprechen, in der CO-AG für die Säuregruppe der Aminosäure oder ein Derivat davon, zum Beispiel eine Estergruppe, eine Amidgruppe oder eine einen Peptidrest enthaltende Gruppe, steht und SC für die Seitenkette der $\alpha$-Aminosäure steht, also zum Beispiel für einen der Substituenten, die in der $\alpha$-Position der vorstehend aufgelisteten $\alpha$-Aminosäuren enthalten sind. Beispiele für Seitenketten sind Alkylreste, zum Beispiel die Methylgruppe im Alanin oder die Isopropylgruppe im Valin, der Benzylrest im Phenylalanin, der Phenylrest im Phenylglycin, der 4-Aminobutylrest im Lysin oder die Hydroxycarbonylmethylgruppe in der Asparaginsäure. Solche

Seitenketten und damit die Aminosäuren können außer durch ihre chemische Struktur zum Beispiel auch aufgrund ihrer physikochemischen Eigenschaften zu einer Gruppe zusammengefaßt werden, beispielsweise können lipophile Seitenketten von hydrophilen Seitenketten, die polare Gruppen enthalten, unterschieden werden. Beispiele für lipophile Seitenketten, die in für $R^6$ stehenden Aminosäuren enthalten sein können, sind Alkylreste, Arylalkylreste oder Arylreste. Entsprechendes gilt für Aminosäuren, die Teil eines für $R^6$ stehenden Restes eines Dipeptids oder Tripeptids sind.

Azaaminosäuren sind natürliche oder unnatürliche Aminosäuren, in denen eine CH-Einheit durch ein Stickstoffatom ersetzt ist, beispielsweise in $\alpha$-Aminosäuren der Zentralbaustein

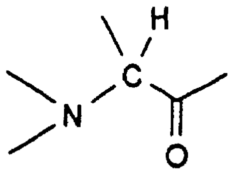

durch

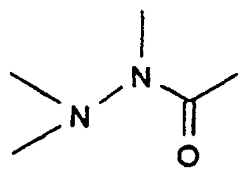

ersetzt ist.

Als Reste von Iminosäuren kommen insbesondere Reste von Heterocyclen aus der folgenden Gruppe in Betracht: Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro(bicyclo[2.2.2]octan)-2,3-pyrrolidin-5-carbonsäure; 2-Azatricyclo[4.3.0.1$^{6,9}$]decan-3-cabonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure, Hydroxypyrrolidin-2-carbonsäure, die alle gegebenenfalls substituiert sein können (siehe folgende Formeln):

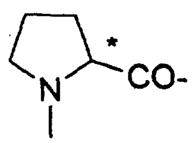 ; 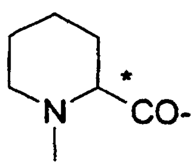 ; 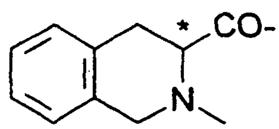 ; 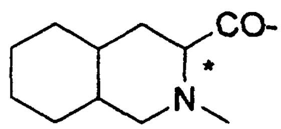 ;

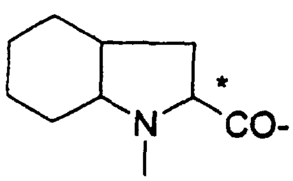 ; 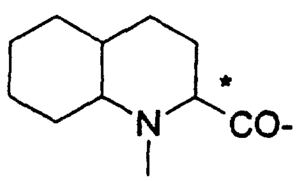 ; 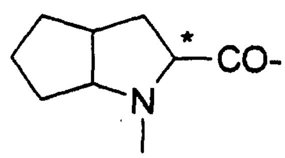 ;

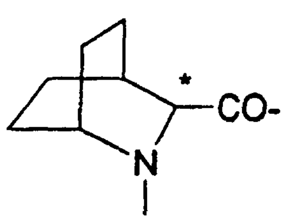 ; 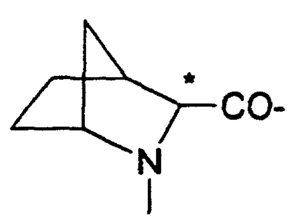 ; 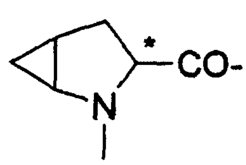 ;

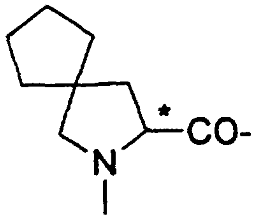 ; 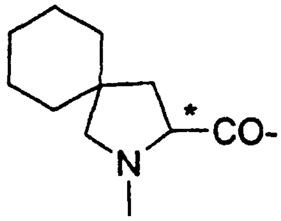 ; 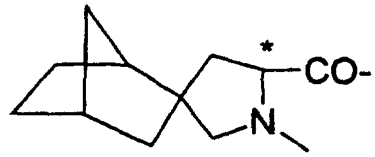 ;

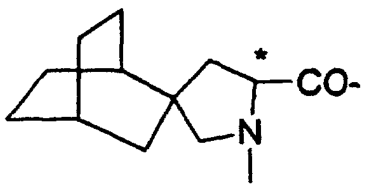 ; 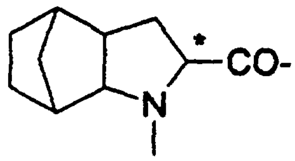 ; 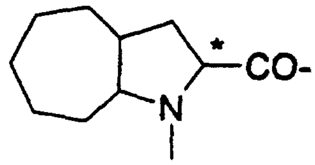 ;

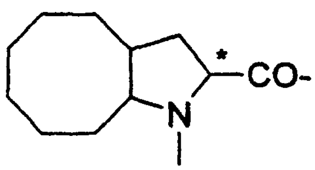 ; 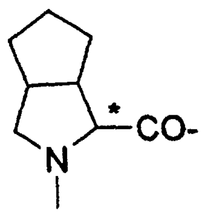 ; 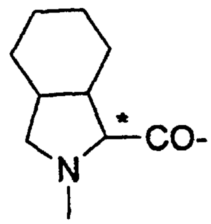 ; 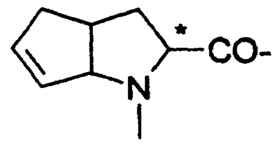 ;

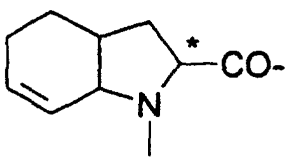 ; 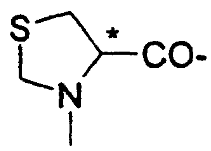 ; 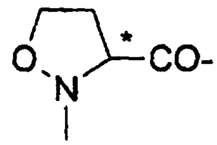 ; 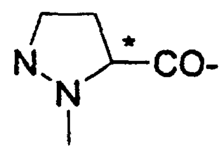 ;

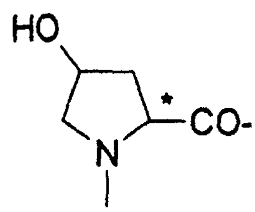 .

Die den obigen Resten zugrundeliegenden Heterocyclen sind beispielsweise bekannt aus US-A-4,344,949; US-A 4,374,847; US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873; EP-A 271,865 und EP-A 344,682.

Dipeptide und Tripeptide können als Bausteine natürliche oder unnatürliche Aminosäuren, Iminosäuren sowie Azaaminosäuren enthalten. Ferner können die natürlichen oder unnatürlichen Aminosäuren, Iminosäuren, Azaaminosäuren, Dipeptide und Tripeptide auch in Form von Derivaten der Carbonsäuregruppe vorliegen, zum Beispiel als Ester oder Amide, wie zum Beispiel als Methylester, Ethylester, n-Propylester, Isopropylester, Isobutylester, tert-Butylester, Benzylester, unsubstituiertes Amid, Methylamid, Ethylamid, Semicarbazid oder ω-Amino-($C_2$-$C_8$)-alkylamid.

Funktionelle Gruppen in Resten von Aminosäuren, Iminosäuren, Azaaminosäuren, Dipeptiden und Tripeptiden sowie in anderen Teilen der Verbindungen der Formel I können geschützt vorliegen. Geeignete Schutzgruppen wie zum Beispiel Urethanschutzgruppen, Carboxylschutzgruppen und Seitenkettenschutzgruppen sind bei Hubbuch, Kontakte (Merck) 1979, Nr. 3, Seiten 14 bis 23, und bei Büllesbach, Kontakte (Merck) 1980, Nr. 1, Seiten 23 bis 35, beschrieben. Insbesondere seien genannt: Aloc, Pyoc, Fmoc, Tcboc, Z, Boc, Ddz, Bpoc, Adoc, Msc, Moc, $Z(NO_2)$, $Z(Hal_n)$, Bobz, Iboc, Adpoc, Mboc, Acm, tert-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze. Von Verbindungen der Formel I, welche saure Gruppen, zum Beispiel Carbonsäuregruppen enthalten, sind solche Salze beispielsweise Alkalimetallsalze oder Erdalkalimetallsalze, wie zum Beispiel Natriumsalze, Kaliumsalze, Magnesiumsalze und Calciumsalze, oder Ammoniumsalze wie zum Beispiel Salze mit physiologisch verträglichen quartären Ammoniumionen und Säureadditionssalze mit Ammoniak und physiologisch verträglichen organischen Aminen, wie zum Beispiel Triethylamin, Ethanolamin, Tris-(2-hydroxyethyl)-amin, $\alpha,\alpha,\alpha$-Tris-(hydroxymethyl)-methylamin oder mit Aminosäuren, insbesondere basischen Aminosäuren.

Verbindungen der Formel I, welche basische Gruppen, zum Beispiel eine Aminogruppe, Amidinogruppe oder Guanidinogruppe enthalten, bilden Salze mit anorganischen Säuren, wie zum Beispiel Salzsäure, Schwefelsäure oder Phosphorsäure, und mit organischen Carbonsäuren oder Sulfonsäuren, wie zum Beispiel Essigsäure, Citronensäure, Benzoesäure, Maleinsäure, Fumarsäure, Weinsäure, Methansulfonsäure oder p-Toluolsulfonsäure. Verbindungen, die sowohl saure Gruppen als auch basische Gruppen enthalten, können auch in Form von inneren Salzen oder Betainen vorliegen, die ebenso von der vorliegenden Erfindung umfaßt werden.

Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen.

Die Verbindungen der Formel I können in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren der Verbindungen der Formel I, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Ebenso sind Diastereomere in diastereomerenreiner Form und in Form von Mischungen in allen Verhältnissen Gegenstand der Erfindung. Bei Vorliegen einer cis/trans-Isomerie sind sowohl die cis-Form als auch die trans-Form und Mischungen dieser Formen in allen Verhältnissen Gegenstand der Erfindung. Die Herstellung von einzelnen Stereoisomeren kann gewünschtenfalls durch Verwendung von stereochemisch einheitlichen Ausgangssubstanzen bei der Synthese, durch stereoselektive Synthese oder durch Auftrennung eines Gemisches nach üblichen Methoden, zum Beispiel durch Chromatographie oder Kristallisation, erfolgen, im Fall von Enantiomeren zum Beispiel durch Chromatographie an chiralen Phasen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe einer Ausgangssubstanz oder eines Zwischenprodukts im Verlaufe der Synthese.

Die erfindungsgemäßen Verbindungen der Formel I können darüber hinaus bewegliche Wasserstoffatome enthalten, also in verschiedenen tautomeren Formen vorliegen. Auch alle Tautomeren der Verbindungen der Formel I sind Gegenstand der vorliegenden Erfindung. Die vorliegende Erfindung umfaßt weiterhin Derivate von Verbindungen der Formel I, zum Beispiel Solvate wie Hydrate und Addukte mit Alkoholen, Ester, Prodrugs und andere physiologisch verträgliche Derivate von Verbindungen der Formel I, sowie aktive Metabolite von Verbindungen der Formel I. Gegenstand der Erfindung sind insbesondere Prodrugs der Verbindungen der Formel I, die unter physiologischen Bedingungen in Verbindungen der Formel I umgewandelt werden. Geeignete Prodrugs für die Verbindungen der Formel I, also chemisch modifizierte Derivate der Verbindungen der Formel I mit in gewünschter Weise verbesserten Eigenschaften, sind dem Fachmann bekannt. Nähere Angaben zu Prodrugs finden sich zum Beispiel in Fleisher et al., Advanced Drug Delivery Reviews 19 (1996) 115-130; Design of Prodrugs, H. Bundgaard, Ed., Elsevier, 1985; H. Bundgaard, Drugs of the Future 16 (1991) 443; Saulnier et al., Bioorg. Med. Chem. Lett. 4 (1994) 1985; Safadi et al., Pharmaceutical Res. 10 (1993) 1350. Als Prodrugs für die Verbindungen der Formel I kommen speziell in Betracht Ester-Prodrugs von Carbonsäuregruppen, Amid-Prodrugs von Carbonsäuregruppen und Alkohol-Prodrugs von Carbonsäuregruppen sowie Acyl-Prodrugs und Carbamat-Prodrugs von acylierbaren stickstoffhaltigen Gruppen wie Aminogruppen, Amidinogruppen und Guanidinogruppen. In den Acyl-Prodrugs oder Carbamat-Prodrugs ist ein an einem Stickstoffatom befindliches Wasserstoffatom durch eine Acylgruppe oder Carbamatgruppe ersetzt. Als Acylgruppen und Carbamatgruppen für die Acyl-Prodrugs und Carbamat-Prodrugs kommen beispielsweise die Gruppen $R^p$-CO und $R^{pa}$O-CO in Betracht, in denen $R^p$ für Wasserstoff, $(C_1-C_{18})$-Alkyl, $(C_3-C_{12})$-Cycloalkyl, $(C_3-C_{12})$-Cycloalkyl-$(C_1-C_8)$-alkyl, $(C_6-C_{14})$-Aryl, $(C_6-C_{14})$-Aryl-$(C_1-C_8)$-alkyl, Heteroaryl oder Heteroaryl-$(C_1-C_8)$-alkyl steht und $R^{pa}$ die für $R^p$ angegebenen Bedeutungen mit Ausnahme von Wasserstoff hat.

Die einzelnen Strukturelemente in der Formel I haben bevorzugt beispielsweise die folgenden Bedeutungen, die sie unabhängig voneinander haben können. Mehrfach auftretende Reste können die Bedeutungen unabhängig voneinander haben und können gleich oder verschieden sein.

In Verbindungen der Formel I, in denen W für den zweiwertigen Rest

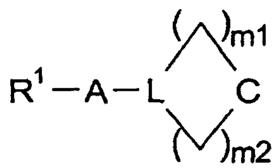

steht, steht die Summe m1 + m2 bevorzugt für eine der Zahlen 1, 3 und 4. Bevorzugt steht W für den zweiwertigen Rest $R^1$-A-C($R^{13}$), worin $R^{13}$ die oben angegebenen Bedeutungen hat, aber verschieden von Wasserstoff ist. Spezielle derartige Gruppen W sind beispielsweise die zweiwertigen Reste Di-(($C_1$-$C_4$)-alkyl)methylen, (($C_1$-$C_4$)-alkyl))$_2$C<, Dimethylmethylen $(CH_3)_2C$< und (Methyl)(phenyl)methylen $(CH_3)(C_6H_5)C$<.

Steht W für den Rest

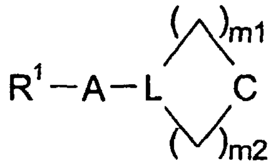 ,

so bilden eine Reihe von derartigen Gruppen die gegebenenfalls wie angegeben substituierten carbocylischen Gruppen der Formel $(CH_2)_{m3}C$<, in der die Zahl m3 der über die endständigen Gruppen an das Spirokohlenstoffatom C< gebundenen Polymethylenkette für 2, 3, 4 oder 5 steht. Spezielle derartige Gruppen W sind beispielsweise die zweiwertigen Reste 1,1-Cyclopropyliden (= Dimethylenmethylen), 1,1-Cyclopentyliden (= Tetramethylenmethylen) und 1,1-Cyclohexyliden (= Pentamethylenmethylen), das heißt die Reste

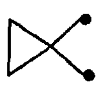 , 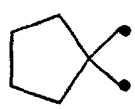 und 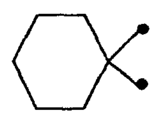 ,

in denen die freien Bindungen durch die Striche mit einem Punkt am Ende symbolisiert sind, wobei die vom 5-Ring und vom 6-Ring abgeleiteten Reste jeweils ein doppelt gebundenes Sauerstoffatom als Substituenten tragen können. Insgesamt bilden Verbindungen der Formel I, in der W eine andere Bedeutung als $CH_2$ hat, eine Gruppe von bevorzugten Verbindungen.

Y steht bevorzugt für eine Carbonylgruppe.

Ein für A stehender zweiwertiger Rest ($C_1$-$C_6$)-Alkylen ist bevorzugt ein ($C_1$-$C_4$)-Alkylenrest, ein für A stehender zweiwertiger Rest Phenylen-($C_1$-$C_4$)-alkyl bevorzugt ein Phenylen-($C_1$-$C_2$)-alkylrest. Bevorzugt steht A für eine direkte Bindung oder einen der zweiwertigen Reste ($C_1$-$C_4$)-Alkylen, Phenylen und Phenylen-($C_1$-$C_2$)-alkyl. Steht W für den Rest $R^1$-A-C($R^{13}$), so wird eine Reihe von bevorzugten Resten $R^1$-Agebildet von den Resten ($C_1$-$C_4$)-Alkyl, gegebenenfalls substituiertes Phenyl und im Phenylrest gegebenenfalls substituiertes Phenyl-($C_1$-$C_2$)-alkyl, insbesondere von den Resten ($C_1$-$C_4$)-Alkyl und gegebenenfalls substituiertes Phenyl.

B steht bevorzugt für einen substituierten Methylenrest oder Ethylenrest, insbesondere für einen substituierten Methylenrest, der einen oder zwei gleiche oder verschiedene Reste aus der Reihe ($C_1$-$C_8$)-Alkyl, ($C_2$-$C_8$)-Alkenyl ($C_2$-$C_8$)-Alkinyl, ($C_3$-$C_6$)-Cycloalkyl, ($C_3$-$C_6$)-Cycloalkyl-($C_1$-$C_6$)-alkyl, gegebenenfalls substituiertes ($C_6$-$C_{10}$)-Aryl, im Arylrest gegebenenfalls substituiertes ($C_6$-$C_{10}$)-Aryl-($C_1$-$C_6$)-alkyl, gegebenenfalls substituiertes Heteroaryl, im Heteroarylrest gegebenenfalls substituiertes Heteroaryl-($C_1$-$C_6$)-alkyl trägt. Ein ($C_1$-$C_8$)-Alkylsubstituent an einem für B stehenden Methylenrest oder Ethylenrest ist bevorzugt ein ($C_1$-$C_6$)-Alkylsubstituent. Ist ein für B stehendender stehender Methylenrest oder Ethylenrest substituiert, so ist er bevorzugt einfach substituiert. Ist ein für B stehender Methylenrest oder Ethylenrest substituiert, so ist er bevorzugt substituiert durch einen oder zwei gleiche oder verschiedene Reste,

insbesondere einen Rest, aus der Reihe $(C_1-C_8)$-Alkyl, insbesondere $(C_1-C_6)$-Alkyl, das heißt geradkettiges oder verzweigtes Alkyl mit 1, 2, 3, 4, 5, 6, 7 oder 8 Kohlenstoffatomen, und $(C_3-C_6)$-Cycloalkyl-$(C_1-C_2)$-alkyl.

Die Reste R stehen bevorzugt unabhängig voneinander für Wasserstoff, Methyl oder Ethyl.

$R^2$ steht bevorzugt für Wasserstoff oder $(C_1-C_6)$-Alkyl, besonders bevorzugt für Wasserstoff, Methyl oder Ethyl.

$R^3$ steht bevorzugt für Wasserstoff, $(C_1-C_8)$-Alkyl, das gegebenenfalls durch 1 bis 6 Fluoratome substituiert sein kann, gegebenenfalls substituiertes $(C_6-C_{10})$-Aryl, im Arylrest gegebenenfalls substituiertes $(C_6-C_{10})$-Aryl-$(C_1-C_4)$-alkyl, gegebenenfalls substituiertes Heteroaryl, im Heteroarylrest gegebenenfalls substituiertes Heteroaryl-$(C_1-C_4)$-alkyl, $(C_3-C_8)$-Cycloalkyl, $(C_3-C_8)$-Cycloalkyl-$(C_1-C_4)$-alkyl, $(C_6-C_{12})$-Bicycloalkyl, $(C_6-C_{12})$-Bicycloalkyl-$(C_1-C_4)$-alkyl, $(C_6-C_{12})$-Tricycloalkyl, $(C_6-C_{12})$-Tricycloalkyl-$(C_1-C_4)$-alkyl, $R^{11}NH$, $COOR^{21}$, $CON(CH_3)R^4$, $CONHR^4$, $CON(CH_3)R^{15}$ oder $CONHR^{15}$. Besonders bevorzugt steht $R^3$ für Wasserstoff, $(C_1-C_8)$-Alkyl, das gegebenenfalls durch 1 bis 6 Fluoratome substituiert sein kann, gegebenenfalls substituiertes $(C_6-C_{10})$-Aryl, im Arylrest gegebenenfalls substituiertes $(C_6-C_{10})$-Aryl-$(C_1-C_4)$-alkyl, gegebenenfalls substituiertes Heteroaryl, im Heteroarylrest gegebenenfalls substituiertes Heteroaryl-$(C_1-C_4)$-alkyl, $(C_3-C_8)$-Cycloalkyl, $(C_3-C_8)$-Cycloalkyl-$(C_1-C_4)$-alkyl, $R^{11}NH$, $COOR^{21}$, $CON(CH_3)R^4$, $CONHR^4$, $CON(CH_3)R^{15}$ oder $CONHR^{15}$. Speziell bevorzugt steht $R^3$ beispielsweise für $(C_1-C_8)$-Alkyl, insbesondere $(C_1-C_4)$-Alkyl, zum Beispiel Methyl, das gegebenenfalls durch 1 bis 6 Fluoratome substituiert sein kann, $(C_6-C_{10})$-Aryl, insbesondere Phenyl, das unsubstituiert oder substituiert sein kann, oder $CONHR^4$.

Ein für $R^4$ stehender unsubstituierter oder substituierter $(C_1-C_8)$-Alkylrest ist bevorzugt ein $(C_1-C_6)$-Alkylrest. Bevorzugt ist es, wenn einer der Substituenten in der für $R^4$ stehenden Alkylgruppe in der 1-Position der Alkylgruppe gebunden ist, also an dasjenige Kohlenstoffatom der Alkylgruppe, an das auch das Stickstoffatom in der Gruppe $CONHR^4$ oder in der Gruppe $CON(CH_3)R^4$ gebunden ist, und wenn dieser Substituent in der 1-Position einer der Reste Hydroxycarbonyl, Aminocarbonyl, $(C_6-C_{10})$-Aryl-$(C_1-C_4)$-alkoxycarbonyl, das im Arylrest auch substituiert sein kann, $R^6$-CO, $R^7$-CO, $(C_1-C_6)$-Alkoxycarbonyl oder Tetrazolyl ist. In diesem bevorzugten Fall steht der Rest $-NHR^4$ bzw. der Rest $-N(CH_3)R^4$ dann für den Rest einer $\alpha$-Aminosäure bzw. einer N-Methyl-$\alpha$-aminosäure oder eines Derivates davon, wobei formal der Rest der Aminosäure durch Abstraktion eines Wasserstoffatoms von der Aminogruppe der Aminosäure erhalten wird (ist der Substituent in der 1-Position die Gruppe $R^6$-CO, so steht der Rest $-NHR^4$ bzw. der Rest $-N(CH_3)R^4$ entsprechend für den Rest eines Dipeptids, Tripeptids oder Tetrapeptids). Speziell bevorzugte $\alpha$-Aminosäuren sind dabei solche mit einer lipophilen Seitenkette, zum Beispiel Phenylglycin, Phenylalanin, Valin, Leucin, Isoleucin und Homologe davon, sowie Derivate dieser Aminosäuren wie Ester, Amide oder die Derivate, in denen die Carbonsäuregruppe in den Rest $R^6$-CO oder $R^7$-CO überführt ist.

$R^5$ steht bevorzugt für gegebenenfalls substituiertes Phenyl.

$R^{11}$ steht bevorzugt für $R^{12a}$, $R^{12a}$-CO, $R^{12a}$-O-CO oder $R^{12a}-S(O)_2$.

Ein für $R^{13}$ stehender Alkylrest ist bevorzugt ein Methylrest. Bevorzugt steht $R^{13}$ für $(C_1-C_6)$-Alkyl, besonders bevorzugt für $(C_1-C_4)$-Alkyl, insbesondere für Methyl.

$R^{15}$ steht bevorzugt für $R^{16}-C_1$-Alkyl oder $R^{16}$.

$R^{20}$ steht bevorzugt für eine direkte Bindung oder einen Methylenrest oder Ethylenrest (1,2-Ethylen), besonders bevorzugt für eine direkte Bindung oder einen Methylenrest.

$R^{21}$ steht bevorzugt für Wasserstoff, $(C_1-C_6)$-Alkyl, $(C_3-C_6)$-Cycloalkyl, $(C_3-C_6)$-Cycloalkyl-$(C_1-C_4)$-alkyl, gegebenenfalls substituiertes $(C_6-C_{10})$-Aryl oder im Arylrest gegebenenfalls substituiertes $(C_6-C_{10})$-Aryl-$(C_1-C_4)$-alkyl, wobei Alkylreste durch Fluor einfach oder mehrfach substituiert sein können. $R^{21}$ steht ganz besonders bevorzugt für Wasserstoff, $(C_1-C_6)$-Alkyl, $(C_3-C_6)$-Cycloalkyl, $(C_3-C_6)$-Cycloalkyl-$(C_1-C_2)$-alkyl, gegebenenfalls substituiertes $(C_6-C_{10})$-Aryl oder im Arylrest gegebenenfalls substituiertes $(C_6-C_{10})$-Aryl-$(C_1-C_2)$-alkyl, wobei Alkylreste durch Fluor einfach oder mehrfach substituiert sein können, wobei die Reste $R^{21}$ bei mehrfachem Auftreten unabhängig voneinander sind und gleich oder verschieden sein können.

$R^{30}$ steht bevorzugt für $R^{32}(R)N-CO-N(R)-R^{31}$ oder $R^{32}(R)N-CS-N(R)-R^{31}$, besonders bevorzugt für $R^{32}(R)N-CO-N(R)-R^{31}$, insbesondere für $R^{32}NH-CO-NH-R^{31}$.

$R^{32}$ steht bevorzugt für Wasserstoff, $(C_1-C_6)$-Alkyl, das gegebenenfalls durch 1 bis 6 Fluoratome substituiert sein kann, $(C_2-C_6)$-Alkenyl, $(C_2-C_6)$-Alkinyl, $(C_5-C_6)$-Cycloalkyl, $(C_5-C_6)$-Cycloalkyl-$(C_1-C_4)$-alkyl, gegebenenfalls substituiertes $(C_6-C_{10})$-Aryl, im Arylrest gegebenenfalls substituiertes $(C_6-C_{10})$-Aryl-$(C_1-C_4)$-alkyl, gegebenenfalls substituiertes Heteroaryl oder im Heteroarylrest gegebenenfalls substituiertes Heteroaryl-$(C_1-C_4)$-alkyl. Ein speziell bevorzugter, für $R^{32}$ stehender Rest ist gegebenenfalls substituiertes $(C_6-C_{10})$-Aryl, insbesondere unsubstituiertes Phenyl oder Phenyl, das durch einen oder mehrere gleiche oder verschiedene der oben angegebenen Substituenten an Aromaten substituiert ist.

$R^{33}$ steht bevorzugt für eine direkte Bindung oder einen zweiwertigen $(C_1-C_2)$-Alkylenrest, besonders bevorzugt für eine direkte Bindung.

$R^{34}$ steht bevorzugt für einen zweiwertigen Rest aus der Reihe $(C_1-C_6)$-Alkylen, gegebenenfalls substituiertes $(C_6-C_{10})$-Arylen und gegebenenfalls substituiertes Heteroarylen, besonders bevorzugt für einen zweiwertigen Rest aus der Reihe $(C_1-C_4)$-Alkylen und gegebenenfalls substituiertes $(C_6-C_{10})$-Arylen.

$R^{35}$ steht bevorzugt für eine direkte Bindung oder einen zweiwertigen $(C_1-C_2)$-Alkylenrest, insbesondere eine direkte Bindung oder Methylen oder Ethylen (1,2-Ethylen), besonders bevorzugt für $(C_1-C_2)$-Alkylen (Methylen oder Ethylen).

$R^{36}$ steht bevorzugt für eine direkte Bindung.

$R^{31}$ steht bevorzugt für einen zweiwertigen Rest $-R^{33}-R^{34}-R^{35}-R^{36}-$, in dem einer oder mehrere der Reste $R^{33}$, $R^{34}$, $R^{35}$ und $R^{36}$ bevorzugte Bedeutungen haben. Besonders bevorzugt steht $R^{31}$ für einen zweiwertigen Rest aus der Reihe $(C_1-C_8)$-Alkylen, $(C_5-C_6)$-Cycloalkylen, $(C_5-C_6)$-Cycloalkylen-$(C_1-C_4)$-alkyl, gegebenenfalls substituiertes $(C_6-C_{10})$-Arylen, im Arylenrest gegebenenfalls substituiertes $(C_6-C_{10})$-Arylen-$(C_1-C_4)$-alkyl, gegebenenfalls substituiertes Heteroarylen, im Heteroarylenrest gegebenenfalls substituiertes Heteroarylen-$(C_1-C_4)$-alkyl, $(C_1-C_8)$-Alkylen-CO, gegebenenfalls substituiertes $(C_6-C_{10})$-Arylen-CO, im Arylenrest gegebenenfalls substituiertes $(C_6-C_{10})$-Arylen-$(C_1-C_4)$-alkyl-CO, gegebenenfalls substituiertes Heteroarylen-CO, im Heteroarylenrest gegebenenfalls substituiertes Heteroarylen-$(C_1-C_4)$-alkyl-CO, gegebenenfalls substituiertes $(C_6-C_{10})$-Arylen-$S(O)_n$, im Arylenrest gegebenenfalls substituiertes $(C_6-C_{10})$-Arylen-$(C_1-C_4)$-alkyl-$S(O)_n$, gegebenenfalls substituiertes Heteroarylen-$S(O)_n$ und im Heteroarylenrest gegebenenfalls substituiertes Heteroarylen-$(C_1-C_4)$-alkyl-$S(O)_n$, worin n für 1 oder 2 steht, und wobei die CO-Gruppe und die $S(O)_n$-Gruppe an das Stickstoffatom im Imidazolidinring in der Formel I gebunden sind und im Falle der Reste Cycloalkylenalkyl, Arylenalkyl und Heteroarylenalkyl die Alkylgruppe an das Stickstoffatom im Imidazolidinring in der Formel I gebunden ist. Ganz besonders bevorzugt steht $R^{31}$ für einen zweiwertigen Rest aus der Reihe $(C_1-C_6)$-Alkylen, gegebenenfalls substituiertes $(C_6-C_{10})$-Arylen und im Arylrest gegebenenfalls substituiertes $(C_6-C_{10})$-Arylen-$(C_1-C_4)$-alkyl, wobei im Falle des Arylenalkylrestes die Alkylgruppe an das Stickstoffatom im Imidazolidinring in der Formel I gebunden ist. Darüber hinaus bevorzugt steht $R^{31}$ für einen zweiwertigen Rest aus der Reihe $(C_1-C_6)$-Alkylen und im Arylrest gegebenenfalls substituiertes $(C_6-C_{10})$-Arylen-$(C_1-C_4)$-alkyl, insbesondere $(C_6-C_{10})$ -Arylen-$(C_1-C_2)$-alkyl, wobei im Falle des Arylenalkylrestes die Alkylgruppe an das Stickstoffatom im Imidazolidinring in der Formel I gebunden ist. Speziell bevorzugt steht $R^{31}$ für den zweiwertigen Rest Phenylen-methyl ($-C_6H_4-CH_2-$), insbesondere den Rest -(1,4-Phenylen)-methyl-, in dem die Methylgruppe an das Stickstoffatom im Imidazolidinring in der Formel I gebunden ist.

Steht $R^3$ für Wasserstoff oder einen der Reste $(C_1-C_8)$-Alkyl, gegebenenfalls substituiertes $(C_6-C_{12})$-Aryl, im Arylrest gegebenenfalls substituiertes $(C_6-C_{12})$-Aryl-$(C_1-C_6)$-alkyl, gegebenenfalls substituiertes Heteroaryl, im Heteroarylrest gegebenenfalls substituiertes Heteroaryl-$(C_1-C_6)$-alkyl, $(C_3-C_8)$-Cycloalkyl, $(C_3-C_8)$-Cycloalkyl-$(C_1-C_6)$-alkyl, $(C_6-C_{12})$-Bicycloalkyl, $(C_6-C_{12})$-Bicycloalkyl-$(C_1-C_6)$-alkyl, $(C_6-C_{12})$-Tricycloalkyl, $(C_6-C_{12})$-Tricycloalkyl-$(C_1-C_6)$-alkyl, $(C_2-C_8)$-Alkenyl, $(C_2-C_8)$-Alkinyl, $COOR^{21}$, $CON(CH_3)R^4$, $CONHR^4$, $CON(CH_3)R^{15}$ oder $CONHR^{15}$, so steht bevorzugt e für 0 und h für 1. Steht $R^3$ für $R^{11}NH$, so steht bevorzugt e für 1 und h für 0. Verbindungen der Formel I, in der e für 0 steht und h für 1 steht, bilden eine bevorzugte Gruppe von Verbindungen. In diesen bevorzugten Verbindungen steht besonders bevorzugt die Gruppe $-NR-[C(R)(R)]_e-C(R^2)(R^3)-[C(R)(R)]_h-E$ in der Formel I für die Gruppe $-NH-CH(R^3)-CH_2-E$.

Bevorzugte Verbindungen der Formel I sind solche Verbindungen, in denen einer oder mehrere der Reste bevorzugte Bedeutungen haben oder eine spezifische der aufgeführten bevorzugten Bedeutungen haben, wobei alle Kombinationen von bevorzugten Bedeutungen von Resten Gegenstand der vorliegenden Erfindung sind.

Bevorzugte Verbindungen der Formel I sind solche Verbindungen, worin

W für den zweiwertigen Rest $R^1-A-C(R^{13})$ steht;

Y für eine Carbonylgruppe steht;

A für eine direkte Bindung, einen der zweiwertigen Reste $(C_1-C_6)$-Alkylen, Phenylen, Phenylen-$(C_1-C_2)$-alkyl oder für einen zweiwertigen Rest eines 5-gliedrigen oder 6-gliedrigen gesättigten oder ungesättigten Heterocyclus, der ein oder zwei Stickstoffatome enthalten kann und einfach oder zweifach durch $(C_1-C_6)$-Alkyl oder doppelt gebundenen Sauerstoff oder Schwefel substituiert sein kann, steht, wobei in dem Rest Phenylenalkyl der Rest $R^1$ an die Phenylengruppe gebunden ist;

B für einen zweiwertigen Methylenrest oder Ethylenrest steht, wobei der Methylenrest und der Ethylenrest unsubstituiert sind oder substituiert sind durch einen Rest aus der Reihe $(C_1-C_8)$-Alkyl, $(C_2-C_8)$-Alkenyl, $(C_2-C_8)$-Alkinyl, $(C_3-C_6)$-Cycloalkyl, $(C_3-C_6)$-Cycloalkyl-$(C_1-C_6)$-alkyl, gegebenenfalls substituiertes $(C_6-C_{10})$-Aryl, im Arylrest gegebenenfalls substituiertes $(C_6-C_{10})$-Aryl-$(C_1-C_6)$-alkyl, gegebenenfalls substituiertes Heteroaryl, im Heteroarylrest gegebenenfalls substituiertes Heteroaryl-$(C_1-C_6)$-alkyl;

E für $R^{10}CO$, $HO-CH_2$ oder $R^8CO-O-CH_2$ steht;

R für Wasserstoff oder $(C_1-C_8)$-Alkyl steht, wobei alle Reste R unabhängig voneinander sind und die Reste R gleich oder verschieden sein können;

$R^1$ für Wasserstoff, $(C_1-C_{10})$-Alkyl, das gegebenenfalls durch Fluor einfach oder mehrfach substituiert sein kann, im Arylrest gegebenenfalls substituiertes $R^{21}-((C_6-C_{10})$-Aryl), im Arylrest gegebenenfalls substituiertes $(R^{21}-((C_6-C_{10})$-Aryl))-$(C_1-C_6)$-alkyl, den Rest Het-, Het-$(C_1-C_4)$-alkyl oder für einen der Reste $X-NH-C(=NH)-R^{20}-$, $X^1-NH-R^{20}-$ und O= steht;

X für Wasserstoff, ($C_1$-$C_6$)-Alkyl, ($C_1$-$C_6$)-Alkylcarbonyl, ($C_1$-$C_6$)-Alkoxycarbonyl, ($C_1$-$C_6$)-Alkylcarbonyloxy-($C_1$-$C_6$)-alkoxycarbonyl, gegebenenfalls substituiertes ($C_6$-$C_{10}$)-Arylcarbonyl, gegebenenfalls substituiertes ($C_6$-$C_{10}$)-Aryloxycarbonyl, ($C_6$-$C_{14}$)-Aryl-($C_1$-$C_6$)-alkoxycarbonyl, das im Arylrest auch substituiert sein kann, Hydroxy, ($C_1$-$C_6$)-Alkoxy oder Amino steht;

$X^1$ eine der Bedeutungen von X hat oder für R'-NH-C(=N-R") steht, worin R' und R" unabhängig voneinander die Bedeutungen von X haben;

$R^2$ für Wasserstoff oder ($C_1$-$C_6$)-Alkyl steht;

$R^3$ für Wasserstoff, ($C_1$-$C_8$)-Alkyl, das gegebenenfalls durch 1 bis 6 Fluoratome substituiert sein kann, gegebenenfalls substituiertes ($C_6$-$C_{10}$)-Aryl, im Arylrest gegebenenfalls substituiertes ($C_6$-$C_{10}$)-Aryl-($C_1$-$C_4$)-alkyl, gegebenenfalls substituiertes Heteroaryl, im Heteroarylrest gegebenenfalls substituiertes Hataroaryl-($C_1$-$C_4$)-alkyl, ($C_3$-$C_8$)-Cycloalkyl, ($C_3$-$C_8$)-Cycloalkyl-($C_1$-$C_4$)-alkyl, ($C_6$-$C_{12}$)-Bicycloalkyl, ($C_6$-$C_{12}$) -Bicycloalkyl-($C_1$-$C_4$)-alkyl, ($C_6$-$C_{12}$)-Tricycloalkyl, ($C_6$-$C_{12}$)-Tricycloalkyl-($C_1$-$C_4$)-alkyl, ($C_2$-$C_8$)-Alkenyl, ($C_2$-$C_8$)-Alkinyl, $R^{11}$NH, $COOR^{21}$, $CON(CH_3)R^4$, $CONHR^4$, $CON(CH_3)R^{15}$ oder $CONHR^{15}$ steht;

$R^4$ für ($C_1$-$C_6$)-Alkyl steht, das unsubstituiert ist oder einfach oder zweifach substituiert ist durch gleiche oder verschiedene Reste aus der Reihe Hydroxy, ($C_1$-$C_8$)-Alkoxy, $R^5$, gegebenenfalls substituiertes ($C_3$-$C_8$)-Cycloalkyl, Hydroxycarbonyl, Aminocarbonyl, ($C_6$-$C_{10}$)-Aryl-($C_1$-$C_4$)-alkoxycarbonyl, das im Arylrest auch substituiert sein kann, ($C_1$-$C_6$)-Alkoxycarbonyl, $R^6$-CO, $R^7$-CO, Tetrazolyl, Trifluormethyl;

$R^5$ für gegebenenfalls substituiertes ($C_6$-$C_{10}$)-Aryl, im Arylrest gegebenenfalls substituiertes ($C_6$-$C_{10}$) -Aryl-($C_1$-$C_4$)-alkyl oder einen Rest eines gegebenenfalls substituierten monocyclischen oder bicyclischen, 5-gliedrigen bis 12-gliedrigen heterocyclischen Ringes, der aromatisch, teilweise gesättigt oder vollständig gesättigt sein kann und der ein, zwei oder drei gleiche oder verschiedene Heteroatome aus der Reihe Stickstoff, Sauerstoff und Schwefel enthalten kann, steht;

$R^6$ für einen Rest einer natürlichen oder unnatürlichen Aminosäure oder den Rest eines Dipeptids oder Tripeptids steht, sowie für deren Ester und Amide, worin freie funktionelle Gruppen durch in der Peptidchemie übliche Schutzgruppen geschützt sein können und worin die Stickstoffatome in den Amidbindungen in der Gruppe $R^6$-CO einen Rest R als Substituenten tragen können;

$R^7$ für den Rest eines über ein Stickstoffatom gebundenen 5-gliedrigen bis 7-gliedrigen, gesättigten monocyclischen Heterocyclus steht, der ein oder zwei gleiche oder verschiedene zusätzliche Ring-Heteroatome aus der Reihe Sauerstoff, Stickstoff und Schwefel enthalten kann und der an Kohlenstoffatomen und an zusätzlichen Ring-Stickstoffatomen gegebenenfalls substituiert sein kann, worin zusätzliche Ring-Stickstoffatome gleiche oder verschiedene Reste aus der Reihe Wasserstoff, $R^h$, HCO, $R^h$CO, $R^h$O-CO, HO-CO-($C_1$-$C_4$) -Alkyl und $R^h$O-CO-($C_1$-$C_4$)-Alkyl als Substituenten tragen können und $R^h$ für ($C_1$-$C_4$)-Alkyl, gegebenenfalls substituiertes ($C_6$-$C_{10}$)-Aryl oder im Arylrest gegebenenfalls substituiertes ($C_6$-$C_{10}$)-Aryl-($C_1$-$C_4$)-alkyl steht;

$R^8$ für Wasserstoff, ($C_1$-$C_6$)-Alkyl oder im Phenylrest gegebenenfalls substituiertes Phenyl-($C_1$-$C_4$)-alkyl steht;

$R^{10}$ für Hydroxy, ($C_1$-$C_8$)-Alkoxy, ($C_6$-$C_{10}$)-Aryl-($C_1$-$C_6$)-alkoxy, das im Arylrest auch substituiert sein kann, gegebenenfalls substituiertes ($C_6$-$C_{10}$)-Aryloxy, ($C_1$-$C_6$)-Alkylcarbonyloxy-($C_1$-$C_6$)-alkoxy, ($C_1$-$C_6$)-Alkoxycarbonyloxy-($C_1$-$C_6$)-alkoxy, Amino, Mono- oder Di-(($C_1$-$C_6$)-alkyl)-amino, Aminocarbonyl-($C_1$-$C_6$)-alkoxy oder (Mono- oder Di-(($C_1$-$C_6$)-alkyl)-amino)-carbonyl-($C_1$-$C_6$)-alkoxy steht;

$R^{11}$ für Wasserstoff, $R^{12a}$, $R^{12a}$-CO, $R^{12a}$-O-CO, $R^{12b}$-CO oder $R^{12a}$-$S(O)_2$ steht;

$R^{12a}$ für ($C_1$-$C_8$)-Alkyl, ($C_2$-$C_8$)-Alkenyl, ($C_2$-$C_8$)-Alkinyl, ($C_5$-$C_6$)-Cycloalkyl, ($C_5$-$C_6$)-Cycloalkyl-($C_1$-$C_4$)-alkyl, gegebenenfalls substituiertes ($C_6$-$C_{10}$)-Aryl, im Arylrest gegebenenfalls substituiertes ($C_6$-$C_{10}$)-Aryl-($C_1$-$C_4$)-alkyl, gegebenenfalls substituiertes Heteroaryl, im Heteroarylrest gegebenenfalls substituiertes Heteroaryl-($C_1$-$C_4$)-alkyl oder den Rest $R^{15}$ steht;

$R^{12b}$ für Amino, Di-(($C_1$-$C_8$)-alkyl)-amino oder $R^{12a}$-NH steht;

$R^{13}$ für Wasserstoff oder ($C_1$-$C_6$)-Alkyl steht;

$R^{15}$ für $R^{16}$-($C_1$-$C_6$)-alkyl oder für $R^{16}$ steht;

$R^{16}$ für einen 6-gliedrigen bis 12-gliedrigen bicyclischen oder tricyclischen Rest steht, der gesättigt oder teilweise ungesättigt ist und der auch ein, zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe ($C_1$-$C_4$)-Alkyl und Oxo substituiert sein kann;

$R^{20}$ für eine direkte Bindung oder Methylen steht;

$R^{21}$ für Wasserstoff, ($C_1$-$C_6$)-Alkyl, gegebenenfalls substituiertes ($C_6$-$C_{10}$)-Aryl, im Arylrest gegebenenfalls substituiertes ($C_6$-$C_{10}$)-Aryl-($C_1$-$C_2$)-alkyl, den Rest Het- oder Het-($C_1$-$C_2$)-alkyl steht, wobei Alkylreste einfach bis vierfach durch Fluor substituiert sein können und die Reste $R^{21}$ bei mehrfachem Auftreten gleich oder verschieden sein können;

$R^{30}$ für einen der Reste $R^{32}$(R)N-CO-N(R)-$R^{31}$ oder $R^{32}$(R)N-CS-N(R)-$R^{31}$ steht;

$R^{31}$ für einen zweiwertigen Rest aus der Reihe ($C_1$-$C_6$)-Alkylen, gegebenenfalls substituiertes ($C_6$-$C_{10}$)-Arylen, im Arylenrest gegebenenfalls substituiertes ($C_6$-$C_{10}$)-Arylen-($C_1$-$C_4$)-alkyl, ($C_5$-$C_6$)-Cycloalkylen, ($C_5$-$C_6$)

-Cycloalkylen-($C_1$-$C_4$)-alkyl, gegebenenfalls substituiertes Heteroarylen oder im Heteroarylenrest gegebenenfalls substituiertes Heteroarylen-($C_1$-$C_4$)-alkyl steht, wobei im Falle des Arylenalkylrestes, des Cycloalkylenalkylrestes und des Heteroarylenalkylrestes die Alkylgruppe an das Stickstoffatom im Imidazolidinring in der Formel I gebunden ist;

$R^{32}$ für Wasserstoff, ($C_1$-$C_6$)-Alkyl, das gegebenenfalls durch 1 bis 6 Fluoratome substituiert sein kann, ($C_2$-$C_6$)-Alkenyl, ($C_2$-$C_6$)-Alkinyl, ($C_5$-$C_6$)-Cycloalkyl, ($C_5$-$C_6$)-Cycloalkyl-($C_1$-$C_4$)-alkyl, gegebenenfalls substituiertes ($C_6$-$C_{10}$)-Aryl, im Arylrest gegebenenfalls substituiertes ($C_6$-$C_{10}$)-Aryl-($C_1$-$C_4$)-alkyl, gegebenenfalls substituiertes Heteroaryl oder im Heteroarylrest gegebenenfalls substituiertes Heteroaryl-($C_1$-$C_4$)-alkyl steht;

Het für einen Rest eines monocyclischen oder polycyclischen, 5-gliedrigen bis 10-gliedrigen, aromatischen oder nicht aromatischen Ringes steht, der 1 oder 2 gleiche oder verschiedene Heteroatome aus der Reihe N und O als Ringglieder enthält und gegebenenfalls durch einen oder mehrere, gleiche oder verschiedene Substituenten substituiert sein kann;

e und h unabhängig voneinander für 0 oder 1 stehen;

in allen ihren stereoisomeren Formen und Mischungen davon in allen Verhältnissen, und ihre physiologisch verträglichen Salze.

Eine Reihe von speziell bevorzugten Verbindungen umfaßt solche Verbindungen der Formel I, worin B für unsubstituiertes Methylen steht oder für Methylen steht, das durch einen ($C_1$-$C_8$)-Alkylrest substituiert ist, in allen ihren stereoisomeren Formen und Mischungen davon in alten Verhältnissen, und ihre physiologisch verträglichen Salze. Besonders speziell bevorzugt in dieser Reihe sind Verbindungen der Formel I, worin B für Methylen steht, das durch einen ($C_1$-$C_8$)-Alkylrest substituiert ist, in allen ihren stereoisomeren Formen und Mischungen davon in allen Verhältnissen, und ihre physiologisch verträglichen Salze.

Eine weitere Reihe von speziell bevorzugten Verbindungen umfaßt solche Verbindungen der Formel I, worin $R^{30}$ für einen Rest aus der Reihe $R^{32}$(R)N-CO-N(R)-$R^{31}$ und $R^{32}$(R)N-CS-N(R)-$R^{31}$ steht und $R^{31}$ für einen zweiwertigen Rest aus der Reihe ($C_1$-$C_6$)-Alkylen und im Arylenrest gegebenenfalls substituiertes ($C_6$-$C_{10}$)-Arylen-($C_1$-$C_4$)-alkyl steht, wobei im Falle des Arylenalkylrestes die Alkylgruppe an das Stickstoffatom im Imidazolidinring in der Formel I gebunden ist, in allen ihren stereoisomeren Formen und Mischungen davon in allen Verhältnissen, und ihre physiologisch verträglichen Salze. In dieser Reihe sind darüber hinaus bevorzugt Verbindungen der Formel I, worin $R^{30}$ für den Rest $R^{32}$NH-CO-NH-$R^{31}$ steht und darin $R^{32}$ für gegebenenfalls substituiertes Phenyl steht und $R^{31}$ für den zweiwertigen Rest 1,4-Phenylen-methyl (das heißt den Rest -(1,4-$C_6H_4$)-$CH_2$-) steht, in dem die Methylgruppe an das Stickstoffatom im Imidazolidinring in der Formel I gebunden ist, in allen ihren stereoisomeren Formen und Mischungen davon in allen Verhältnissen, und ihre physiologisch verträglichen Salze.

Eine weitere Reihe von speziell bevorzugten Verbindungen umfaßt solche Verbindungen der Formel I, worin $R^{13}$ für Wasserstoff oder Methyl steht, in allen ihren stereoisomeren Formen und Mischungen davon in allen Verhältnissen, und ihre physiologisch verträglichen Salze. Besonders speziell bevorzugt sind in dieser Reihe Verbindungen der Formel I, worin die Gruppe $R^1$-A- nicht für Wasserstoff steht und gleichzeitig auch die Gruppe $R^{13}$ nicht für Wasserstoff steht, das heißt Verbindungen, in denen W nicht für $CH_2$ steht, in allen ihren stereoisomeren Formen und Mischungen davon in allen Verhältnissen, und ihre physiologisch verträglichen Salze, wobei es ganz besonders speziell bevorzugt ist, wenn in diesen Verbindungen $R^{13}$ für Methyl steht, wenn also Verbindungen vorliegen, in denen W für den zweiwertigen Rest $R^1$-A-C($CH_3$) steht und darin $R^1$-A- eine andere Bedeutung als Wasserstoff hat.

Eine weitere Reihe von speziell bevorzugten Verbindungen umfaßt solche Verbindungen der Formel I, worin gleichzeitig die Reste $R^{13}$ und $R^1$-A- verschieden von Wasserstoff sind, $R^{30}$ für den Rest $R^{32}$-NH-CO-NH-(1,4-$C_6H_4$)-$CH_2$ steht, in dem die Gruppe -(1,4-$C_6H_4$)- einen über die Positionen 1 und 4 verknüpften Phenylenrest bedeutet, und $R^{32}$ für gegebenenfalls substituiertes Phenyl steht, in allen ihren stereoisomeren Formen und Mischungen davon in allen Verhältnissen, und ihre physiologisch verträglichen Salze.

Eine weitere Reihe von speziell bevorzugten Verbindungen umfaßt solche Verbindungen der Formel I, worin gleichzeitig die Reste $R^{13}$ und $R^1$-A- verschieden von Wasserstoff sind, $R^{30}$ für den Rest $R^{32}$-NH-CO-NH-(1,4-$C_6H_4$)-$CH_2$ steht, $R^{32}$ für gegebenenfalls substituiertes Phenyl steht und B für einen zweiwertigen Methylenrest steht, der unsubstituiert ist oder - in einer bevorzugten Form - durch ($C_1$-$C_6$)-Alkyl oder ($C_3$-$C_6$)-Cycloalkyl-($C_1$-$C_2$)-alkyl substituiert ist, in allen ihren stereoisomeren Formen und Mischungen davon in allen Verhältnissen, und ihre physiologisch verträglichen Salze.

Eine weitere Reihe von speziell bevorzugten Verbindungen umfaßt solche Verbindungen der Formel I, worin gleichzeitig die Reste $R^{13}$ und $R^1$-A- verschieden von Wasserstoff sind, $R^{30}$ für den Rest $R^{32}$-NH-CO-NH-(1,4-$C_6H_4$)-$CH_2$ steht, $R^{32}$ für gegebenenfalls substituiertes Phenyl steht, B für einen zweiwertigen Methylenrest steht, der unsubstituiert ist oder - in einer bevorzugten Form - durch ($C_1$-$C_6$)-Alkyl oder ($C_3$-$C_6$)-Cycloalkyl-($C_1$-$C_2$)-alkyl substituiert ist, und der Rest -N(R)-[C(R)(R)]$_e$-C($R^2$)($R^3$)-[C(R)(R)]$_h$-E in der Formel I für den Rest -NH-CH($R^3$)-$CH_2$-E steht, in allen ihren stereoisomeren Formen und Mischungen davon in allen Verhältnissen, und ihre physiologisch verträglichen

Salze.

Eine weitere Reihe von speziell bevorzugten Verbindungen umfaßt solche Verbindungen der Formel I, worin gleichzeitig W für einen der zweiwertigen Reste 1,1-Cyclopropyliden, 1,1-Cyclopentyliden und 1,1-Cyclohexyliden steht, die oben näher erläutert sind, wobei die vom 5-Ring und vom 6-Ring abgeleiteten Reste jeweils ein doppelt gebundenes Sauerstoffatom als Substituenten tragen können, $R^{30}$ für den Rest $R^{32}$-NH-CO-NH-(1,4-$C_6H_4$)-$CH_2$ steht und $R^{32}$ für gegebenenfalls substituiertes Phenyl steht, in allen ihren stereoisomeren Formen und Mischungen davon in allen Verhältnissen, und ihre physiologisch verträglichen Salze.

Eine weitere Reihe von speziell bevorzugten Verbindungen umfaßt solche Verbindungen der Formel I, worin gleichzeitig W für einen der zweiwertigen Reste 1,1-Cyclopropyliden, 1,1-Cyclopentyliden und 1,1-Cyclohexyliden steht, $R^{30}$ für den Rest $R^{32}$-NH-CO-NH-(1,4-$C_6H_4$)-$CH_2$ steht, $R^{32}$ für gegebenenfalls substituiertes Phenyl steht und B für einen zweiwertigen Methylenrest steht, der unsubstituiert ist oder - in einer bevorzugten Form - durch ($C_1$-$C_6$)-Alkyl oder ($C_3$-$C_6$)-Cycloalkyl-($C_1$-C2)-alkyl substituiert ist, in allen ihren stereoisomeren Formen und Mischungen davon in allen Verhältnissen, und ihre physiologisch verträglichen Salze.

Eine weitere Reihe von speziell bevorzugten Verbindungen umfaßt solche Verbindungen der Formel I, worin gleichzeitig W für einen der zweiwertigen Reste 1,1-Cyclopropyliden, 1,1-Cyclopentyliden und 1,1-Cyclohexyliden steht, $R^{30}$ für den Rest $R^{32}$-NH-CO-NH-(1,4-$C_6H_4$)-$CH_2$ steht, $R^{32}$ für gegebenenfalls substituiertes Phenyl steht, B für einen zweiwertigen Methylenrest steht, der unsubstituiert ist oder - in einer bevorzugten Form - durch ($C_1$-$C_6$)-Alkyl oder ($C_3$-$C_6$)-Cycloalkyl-($C_1$-$C_2$)-alkyl substituiert ist, und der Rest -N(R)-$[C(R)(R)]_e$-C($R^2$)($R^3$)-$[C(R)(R)]_h$-E in der Formel I für den Rest -NH-CH($R^3$)-$CH_2$-E steht, in allen ihren stereoisomeren Formen und Mischungen davon in allen Verhältnissen, und ihre physiologisch verträglichen Salze.

Eine weitere Reihe von speziell bevorzugten Verbindungen umfaßt solche Verbindungen der Formel I, worin in dem Rest -N(R)-$[(R)(R)]_e$-C($R^2$)($R^3$)-$[C(R)(R)]_h$-E, der durch eine Amidbindung mit der Gruppe -B-CO- verknüpft ist, die Kette von Kohlenstoffatomen zwischen der Gruppe N(R) und der ersten an diese Kette gebundenen Gruppe, die eine Säuregruppe wie eine Carbonsäuregruppe oder Tetrazolylgruppe oder ein Derivat davon wie einen Ester oder ein Amid darstellt, zwei oder mehr als zwei Kohlenstoffatome umfaßt, in allen ihren stereoisomeren Formen und Mischungen davon in allen Verhältnissen, und ihre physiologisch verträglichen Salze. Diese erste Säuregruppe (oder das Derivat davon), die ausgehend von der Gruppe N(R) an diese Kette von Kohlenstoffatomen gebunden ist, kann die Gruppe E sein oder kann die Gruppe $R^3$ sein, wenn letztere zum Beispiel für $COOR^{21}$, $CONHR^4$, etc. steht. Besonders speziell bevorzugt sind in dieser Reihe Verbindungen der Formel I, worin in dem Rest -N(R)-$[C(R)(R)]_e$-C($R^2$)($R^3$)-$[C(R)(R)]_h$-E die Kette von Kohlenstoffatomen zwischen der Gruppe N(R) und der ersten an diese Kette gebundenen Gruppe, die eine Säuregruppe oder ein Derivat davon darstellt, genau zwei Kohlenstoffatome umfaßt, in allen ihren stereoisomeren Formen und Mischungen davon in allen Verhältnissen, und ihre physiologisch verträglichen Salze. Derartige besonders speziell bevorzugte Verbindungen der Formel I können zum Beispiel Verbindungen sein, worin e für 1 steht, das heißt Verbindungen, die die Gruppe -N(R)-C(R)(R)-C($R^2$)($R^3$)-$[C(R)(R)]_h$-E enthalten, wobei im Falle dieser Verbindungen h für 1 oder 0 stehen kann und wobei es im Falle dieser Verbindungen bevorzugt ist, wenn $R^3$ für $R^{11}$NH steht und gleichzeitig h für 0 steht, in allen ihren stereoisomeren Formen und Mischungen davon in allen Verhältnissen, und ihre physiologisch verträglichen Salze. Derartige besonders speziell bevorzugte Verbindungen der Formel I können zum Beispiel auch Verbindungen sein, worin e für 0 steht, h für 1 steht und $R^3$ nicht für eine Säuregruppe oder ein Derivat davon steht, in allen ihren stereoisomeren Formen und Mischungen davon in allen Verhältnissen, und ihre physiologisch verträglichen Salze, das heißt Verbindungen, die einen Rest -N(R)-C($R^2$)($R^{3a}$)-C(R)(R)-E enthalten, worin $R^{3a}$ wie $R^3$ definiert ist, aber nicht für eine Carbonsäuregruppe oder ein Derivat davon wie einen Ester oder ein Amid stehen kann. Bevorzugt steht in diesen Verbindungen $R^{3a}$ für Wasserstoff, ($C_1$-$C_8$)-Alkyl,das gegebenenfalls durch 1 bis 6 Fluoratome substituiert sein kann, gegebenenfalls substituiertes ($C_6$-$C_{12}$)-Aryl, im Arylrest gegebenenfalls substituiertes ($C_6$-$C_{14}$)-Aryl-($C_1$-$C_6$)-alkyl, gegebenenfalls substituiertes Heteroaryl, im Heteroarylrest gegebenenfalls substituiertes Heteroaryl-($C_1$-$C_6$)-alkyl, ($C_3$-$C_8$)-Cycloalkyl, ($C_3$-$C_8$)-Cycloalkyl-($C_1$-$C_6$)-alkyl, ($C_6$-$C_{12}$)-Bicycloalkyl, ($C_6$-$C_{12}$)-Bicycloalkyl-($C_1$-$C_6$)-alkyl, ($C_6$-$C_{12}$)-Tricycloalkyl, ($C_6$-$C_{12}$)-Tricycloalkyl-($C_1$-$C_6$)-alkyl, ($C_2$-$C_8$)-Alkenyl oder ($C_2$-$C_8$)-Alkinyl. Besonders bevorzugt steht in diesen Verbindungen $R^{3a}$ für Wasserstoff, ($C_1$-$C_6$)-Alkyl, das gegebenenfalls durch 1 bis 6 Fluoratome substituiert sein kann, gegebenenfalls substituiertes ($C_6$-$C_{10}$)-Aryl, im Arylrest gegebenenfalls substituiertes ($C_6$-$C_{10}$)-Aryl-($C_1$-$C_4$)-alkyl, gegebenenfalls substituiertes Heteroaryl, im Heteroarylrest gegebenenfalls substituiertes Heteroaryl-($C_1$-$C_4$)-alkyl, ($C_5$-$C_6$)-Cycloalkyl, ($C_5$-$C_6$)-Cycloalkyl-($C_1$-$C_4$)-alkyl, ($C_{10}$-$C_{12}$)-Tricycloalkyl oder ($C_{10}$-$C_{12}$)-Tricycloalkyl-($C_1$-$C_4$)-alkyl. Bevorzugt ist es in den Verbindungen dieser Reihe weiterhin, wenn die Gruppe -N(R)- im Rest -N(R)-$[C(R)(R)]_e$-C($R^2$)($R^3$)-$[C(R)(R)]_h$-E für die Gruppe -NH- steht.

Eine weitere Reihe von speziell bevorzugten Verbindungen umfaßt solche Verbindungen der Formel I, worin in dem Rest -N(R)-$[C(R)(R)]_e$-C($R^2$)($R^3$)-$[C(R)(R)]_h$-E die Kette von Kohlenstoffatomen zwischen der Gruppe N(R) und der ersten an diese Kette gebundenen Gruppe, die eine Säuregruppe oder ein Derivat davon darstellt, nur ein Kohlenstoffatom umfaßt, in allen ihren stereoisomeren Formen und Mischungen davon in allen Verhältnissen und ihre physiologisch verträglichen Salze, wobei aber in diesen Verbindungen die erste Säuregruppe oder das Derivat davon, das ausgehend von der Gruppe N(R) an die Kette von Kohlenstoffatomen gebunden ist, die folgende Bedingung erfüllen

muß: a) die erste Säuregruppe oder das Derivat davon ist eine Amidgruppe, die aber in einem Alkylsubstituenten am Amidstickstoff keine an diesen Alkylsubstituenten gebundene Carbonsäuregruppe (oder ein Derivat davon wie eine Estergruppe oder eine Amidgruppe) enthält, oder b) die erste Säuregruppe ist eine freie Säuregruppe (oder ein Salz davon), oder c) die erste Säuregruppe oder das Derivat davon ist eine Estergruppe. Verbindungen dieser Reihe können zum Beispiel Verbindungen der Formel I sein, in der e für 0 steht und $R^3$ für $COOR^{21}$, $CONHR^{15}$ oder $CON(CH_3)R^{15}$, bevorzugt für $CONHR^{15}$, steht und h für 0 oder 1, bevorzugt für 1, steht. Verbindungen dieser Reihe können zum Beispiel auch Verbindungen der Formel I sein, in der e für 0 steht, h für 0 oder 1, bevorzugt für 1, steht und $R^3$ für $CON(CH_3)R^4$ oder $CONHR^4$ steht, worin aber ein für $R^4$ stehender $(C_1-C_8)$-Alkylrest nicht durch eine Carbonsäuregruppe oder ein Derivat davon wie einen Ester oder ein Amid substituiert sein kann, also zum Beispiel Verbindungen, in denen $R^4$ für $(C_1-C_8)$-Alkyl steht, das unsubstituiert ist oder durch einen oder mehrere gleiche oder verschiedene Reste aus der Reihe Hydroxy, $(C_1-C_8)$-Alkoxy, $R^5$, gegebenenfalls substituiertes $(C_3-C_8)$-Cycloalkyl, Tetrazolyl, Trifluormethyl substituiert ist. In den Verbindungen dieser Reihe steht bevorzugt E für eine Säuregruppe oder ein Derivat davon.

Generell sind Verbindungen der Formel I bevorzugt, die an einem oder an mehreren Chiralitätszentren, zum Beispiel bei entsprechender Substitution an dem die Reste $R^2$ und $R^3$ tragenden Kohlenstoffatom und/oder an dem Zentrum W im Imidazolidin-Ring in der Formel I, eine einheitliche Konfiguration aufweisen. Das heißt, Verbindungen sind bevorzugt, die an einem oder mehreren Chiralitätszentren in einheitlicher oder im wesentlichen einheitlicher Konfiguration vorliegen, entweder in der R-Konfiguration oder in der S-Konfiguration, aber nicht als R/S-Gemisch. Die einzelnen Chiralitätszentren in diesen Verbindungen der Formel I können aber unabhängig voneinander die R-Konfiguration oder die S-Konfiguration aufweisen und gleiche oder verschiedene Konfigurationen haben.

Die Verbindungen der Formel I können beispielsweise hergestellt werden durch Fragmentkondensation einer Verbindung der Formel II

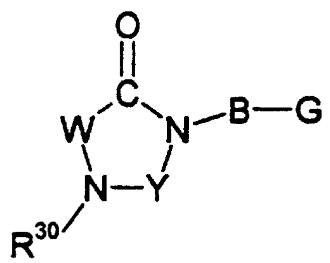

(II)

mit einer Verbindung der Formel III,

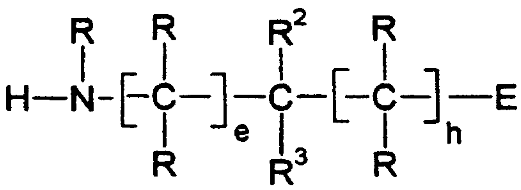

(III)

wobei in den Formeln II und III die Gruppen W, Y, B, E, R, $R^2$, $R^3$, $R^{30}$ sowie e und h wie oben angegeben definiert sind oder auch in diesen Gruppen funktionelle Gruppen in geschützter Form oder in Form von Vorstufen enthalten sein können, und wobei G für Hydroxycarbonyl, $(C_1-C_6)$-Alkoxycarbonyl oder aktivierte Carbonsäurederivate wie Säurechloride oder Aktivester steht. Wenn Verbindungen der Formel I hergestellt werden sollen, in denen zum Beispiel $R^3$ in der Formel I für ein Carbonsäurederivat steht oder ein solche Gruppe enthält, kann in den Verbindungen der Formel III aber beispielsweise auch der Rest $R^3$ zunächst für eine in geschützter Form vorliegende Hydroxycarbonylgruppe stehen oder eine solche enthalten, und dann erst nach der Kondensation der Verbindungen der Formeln II und III in einem oder mehreren weiteren Schritten die gewünschte endgültige Gruppe $R^3$ aufgebaut werden. Vorstufen von funktionellen Gruppen sind Gruppen, die nach den üblichen, dem Fachmann bekannten Syntheseverfahren in die gewünschte funktionelle Gruppe umgewandelt werden können. Beispielsweise kann eine Nitrogruppe durch Reduktion, zum Beispiel durch katalytische Hydrierung, in eine Aminogruppe umgewandelt werden und kann als Vorstufe für eine Aminogruppe oder eine daraus durch weitere Umsetzungen erhältliche Gruppe bezeichnet werden. Eine Cyangruppe, die durch Reduktion in eine Aminomethylgruppe oder durch Hydrolyse in eine Säureamidgruppe oder eine Carbonsäuregruppe umgewandelt werden kann, kann als Vorstufe für diese Gruppen bezeichnet werden. Eine Alkoholgruppe, die zu einer Aldehydgruppe oder einer Ketongruppe oxidiert werden kann, kann als Vorstufe für diese Gruppen bezeichnet werden. Eine Vorstufe für eine Gruppe kann aber auch eine Gruppe sein, aus der in mehreren später durchgeführten Reaktionschritten ein größerer Teil des Zeilmoleküls aufgebaut wird. Beispiele für Schutzgruppen, die vor Durchführung einer Reaktion oder einer Reaktionsfolge in das Molekül eingefügt werden und später wieder abgespalten werden, sind oben genannt.

Zur Kondensation der Verbindungen der Formel II mit denen der Formel III verwendet man vorteilhafterweise die dem Fachmann an sich wohlbekannten Kupplungsmethoden der Peptidchemie (siehe zum Beispiel Houben-Weyl, Methoden der Organischen Chemie, Band 15/1 und 15/2, Georg Thieme Verlag, Stuttgart, 1974). Als Kondensationsmittel bzw. Kupplungsreagenzien kommen zum Beispiel Carbonyldiimidazol, Carbodiimide wie Dicyclohexylcarbodiimid (DCC) oder Diisopropylcarbodiimid, das O-((Cyan(ethoxycarbonyl)methylen)amino)-N,N,N',N'tetramethyluronium-tetrafluoroborat (TOTU) oder Propylphosphonsäureanhydrid (PPA) in Frage.

Die Kondensationen können unter den dem Fachmann wohlbekannten Standardbedingungen durchgeführt werden. Bei der Kondensation ist es in der Regel nötig, daß vorhandene, nicht reagierende Aminogruppen durch reversible Schutzgruppen geschützt werden. Gleiches gilt für nicht an der Reaktion beteiligte Carboxylgruppen, die während der Kondensation bevorzugt als ($C_1$-$C_6$)-Alkylester, Benzylester oder tert-Butylester vorliegen. Ein Aminogruppen-Schutz erübrigt sich, wenn die Aminogruppen noch in Form von Vorstufen, zum Beispiel als Nitrogruppen oder Cyangruppen, vorliegen und erst nach der Kondensation zum Beispiel durch Hydrierung gebildet werden. Nach der Kondensation werden die vorhandenen Schutzgruppen in geeigneter Weise abgespalten. Beispielsweise können $NO_2$-Gruppen (Guanidinoschutz in Aminosäuren), Benzyloxycarbonylgruppen und Benzylgruppen in Benzylestem abhydriert werden. Die Schutzgruppen vom tert-Butyltyp werden sauer abgespalten, während der 9-Fluorenylmethyloxycarbonylrest durch sekundäre Amine entfernt wird. Die Herstellung der Verbindungen der Formel I kann beispielsweise auch erfolgen, indem man die Verbindungen nach üblichen Methoden schrittweise an einer Festphase aufbaut, wobei die einzelnen Strukturelemente des Moleküls in unterschiedlicher Reihenfolge eingeführt werden können.

Verbindungen der Formel II, in der W für $R^1$-A-C($R^{13}$) steht und Y für eine Carbonylgruppe steht, können beispielsweise hergestellt werden, indem man zunächst Verbindungen der Formel IV

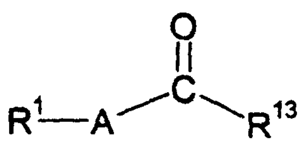

(IV)

in einer Bucherer-Reaktion, zum Beispiel mit Ammoniumcarbonat und Kaliumcyanid, zu Verbindungen der Formel V

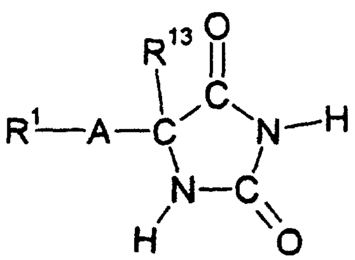

(V)

umsetzt (H. T. Bucherer, V. A. Lieb, J. Prakt. Chem. 141(1934), 5), wobei in den Formeln IV und V die Gruppen $R^1$, $R^{13}$ und A wie oben angegeben definiert sind. Verbindungen der Formel VI,

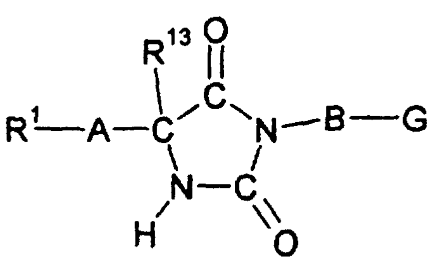

(VI)

in der $R^1$, $R^{13}$, A, B und G wie oben angegeben definiert sind, können dann erhalten werden, indem man die Verbindungen der Formel V beispielsweise zunächst mit einem alkylierenden Reagenz umsetzt, das den Rest -B-G in das Molekül einführt. Die Umsetzung von Verbindungen der Formel VI mit einem zweiten Reagenz der Formel $R^{30}$-LG, in der $R^{30}$ die oben angegebenen Bedeutungen hat und LG eine nucleophil substituierbare Abgangsgruppe, zum Beispiel Halogen, insbesondere Chlor oder Brom, Sulfonyloxy wie Tosyloxy, Methylsulfonyloxy oder Trifluormethylsulfonyloxy, ($C_1$-$C_4$)-Alkoxy, gegebenenfalls substituiertes Phenoxy oder eine heterocyclische Abgangsgruppe wie zum Beispiel Imidazolyl, darstellt, führt dann zu den entsprechenden Verbindungen der Formel II.

Generell kann es je nach den Bedeutungen des Restes $R^{30}$ und anderer Reste auch vorteilhaft sein, nicht den endgültigen Rest $R^{30}$ mittels des Reagenzes $R^{30}$-LG in das Molekül einzuführen, sondern nach Anknüpfung einer Vorstufe der Gruppe $R^{30}$ an den Imidazolidinring den Rest $R^{30}$ am Imidazolidinring aufzubauen. Dies kann zum Beispiel auf der Stufe einer Verbindung der Formel VI bzw. der daraus hergestellten Verbindung der Formel II erfolgen oder auf der Stufe eines anderen Zwischenprodukts der Synthese. Beispielhaft ist diese Vorgehensweise im folgenden an Verbindungen dargestellt, in denen $R^{30}$ für die Harnstoffgruppe $R^{32}$(R)N-CO-N(R)-$R^{31}$ steht. Verbindungen der Formel II, in der $R^{30}$ für $R^{32}$(R)N-CO-N(R)-$R^{31}$ steht, können nach dieser Vorgehensweise beispielsweise hergestellt werden, indem man eine Verbindung der Formel VI zunächst mit einem Reagenz der Formel PG-N(R)-$R^{31}$-LG, in der LG wiederum für eine nucleophil substituierbare Abgangsgruppe steht, zu einer Verbindung der Formel VII

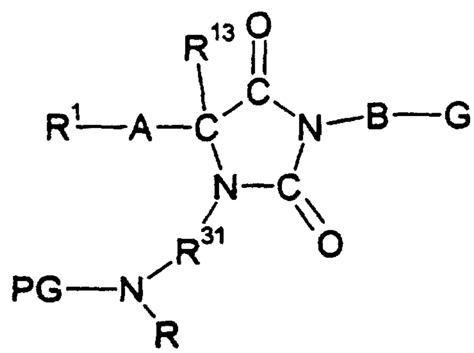

(VII)

umsetzt, wobei PG für eine Amino-Schutzgruppe steht, beispielsweise tert-Butoxycarbonyl oder Benzyloxycarbonyl, und wobei ansonsten die oben angegebenen Bedeutungen gelten. Nach Entfernen der Schutzgruppe PG erhält man durch Umsetzung der entstandenen Aminogruppe HNR- mit beispielsweise einem Isocyanat der Formel $R^{32}$-N=C=O Verbindungen dann der Formel II, in der $R^{30}$ für $R^{32}$NH-CO-N(R)-$R^{31}$ steht. Durch Umsetzung beispielsweise mit einem Carbamoylchlorid der Formel $R^{32}$(R)N-CO-Cl erhält man Verbindungen der Formel II, in der $R^{30}$ für $R^{32}$(R)N-CO-N(R) -$R^{31}$ steht. Entsprechend sind mit Isothiocyanaten und Thiocarbamoylchloriden die analogen Thiohamstoffderivate erhältlich, durch Umsetzung der Aminogruppe mit reaktiven Carbonsäurederivaten, Thiocarbonsäurederivaten, Sulfonsäurederivaten, Sulfinsäurederivaten und Sulfamoylchloriden sind (Thio)Acylamine, Sulfonylamine, Sulfinylamine und Sulfamide erhältlich. Ebenso wie Verbindungen der Formel VII können auch Verbindungen hergestellt und eingesetzt werden, in denen in der Formel VII die Gruppe PG-N(R)- durch eine Gruppe ersetzt ist, die eine Vorstufe für eine Aminogruppe darstellt und die dann in einem weiteren Reaktionsschritt in eine Aminogruppe überführt wird. Beispielsweise kann eine Verbindung der Formel VI zunächst mit einer Nitroverbindung der Formel $O_2N$-$R^{31}$-LG oder einer Cyanverbindung der Formel NC-$R^{31}$-LG zu einer der Verbindung der Formel VII entsprechenden Verbindung umgesetzt werden, dann kann die Nitrogruppe oder die Cyangruppe beispielsweise durch katalytische Hydrierung in die Aminogruppe überführt werden und dann kann die Aminogruppe in die gewünschte Zielgruppe umgewandelt werden, beispielsweise mit einem Isocyanat der Formel $R^{32}$-N=C=O zu einem Harnstoffderivat, in dem $R^{30}$ für $R^{32}$NH-CO-NH-$R^{31}$ steht, oder mit anderen Verbindungen. Gemäß dieser Vorgehensweise können zahlreiche weitere Verbindungen der Formel I aufgebaut werden, wobei die durchzuführenden Reaktionen stets Standardverfahren sind, die dem Fachmann geläufig sind.

Ganz generell können die einzelnen Schritte bei der Herstellung der Verbindungen der Formel I nach oder analog zu bekannten, dem Fachmann geläufigen Methoden durchgeführt werden. Je nach dem Einzelfall kann es hierbei, wie bereits erläutert, bei allen Schritten in der Synthese der Verbindungen der Formel I angebracht sein, funktionelle Gruppen, die zu Nebenreaktionen oder unerwünschten Reaktionen führen könnten, durch eine dem Syntheseproblem angepaßte Schutzgruppenstrategie temporär zu blockieren, was dem Fachmann bekannt ist.

Die erläuterte Vorgehensweise, funktionelle Gruppen nicht direkt in der endgültigen Form in das Molekül einzuführen, sondern zunächst Vorstufen in das Molekül einzuführen und dann auf der Stufe eines Zwischenprodukts die endgültige funktionelle Gruppe aufzubauen, kann, wie bereits erwähnt, entsprechend auch für andere Teile der

Moleküls der Formel I angewandt werden, beispielsweise für die Gruppe $R^1$ oder die Gruppe $R^3$.

Verbindungen der Formel II, in der W für

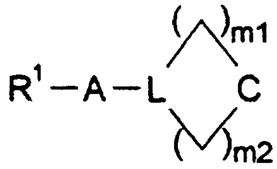

steht und Y für eine Carbonylgruppe steht, können beispielsweise hergestellt werden, indem man Verbindungen der Formel VIII,

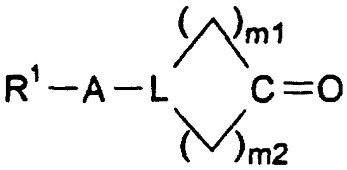

(VIII)

in der $R^1$, A, L, m1 und m2 wie oben angegeben definiert sind, in einer Bucherer-Reaktion wie oben für die Herstellung der Verbindungen der Formel V beschrieben zu Verbindungen der Formel IX

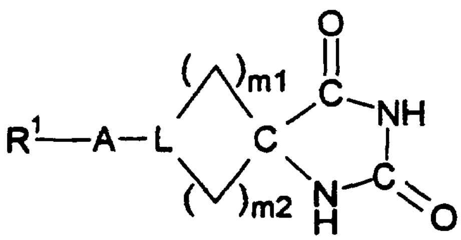

(IX)

umsetzt und diese mit einem Reagenz, das den Rest -B-G in das Molekül einführt, wie oben für die Herstellung der Verbindungen der Formel VI beschrieben in Verbindungen der Formel X

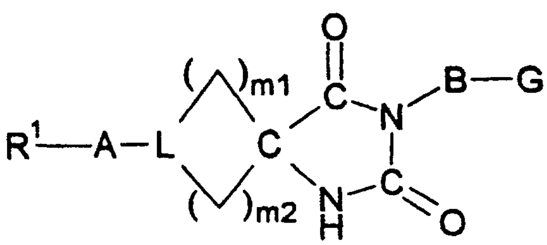

(X)

überführt, wobei in den Verbindungen der Formeln IX und X die Gruppen $R^1$, A, B, G und L sowie m1 und m2 die oben angegebenen Bedeutungen haben. Die Verbindungen der Formel X können dann wiederum, entsprechend den oben beschriebenen Umsetzungen der Verbindungen der Formel VI, mit einem Reagenz der Formel $R^{30}$-LG oder einem Reagenz der Formel PG-N(R)-$R^{31}$-LG umgesetzt werden.

Die Aminoverbindungen der Formel III sind käuflich oder können nach oder analog zu wohlbekannten Standardverfahren aus Ausgangsverbindungen aufgebaut werden, die käuflich sind oder nach oder analog zu Literaturvorschriften erhältlich sind.

Verbindungen der Formel I, in denen W für $R^1$-A-C($R^{13}$) steht, können auch wie folgt erhalten werden: Durch Reaktion von nach Standardverfahren erhältlichen α-Aminosäuren oder N-substituierten α-Aminosäuren oder bevorzugt deren Estern, zum Beispiel der Methylester, Ethylester, tert-Butylester oder Benzylester, beispielsweise von

Verbindungen der Formel XI,

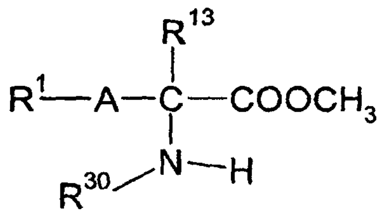

(XI)

worin $R^1$, $R^{13}$, $R^{30}$ und A wie oben angegeben definiert sind, mit einem Isocyanat oder Isothiocyanat beispielsweise der Formel XII,

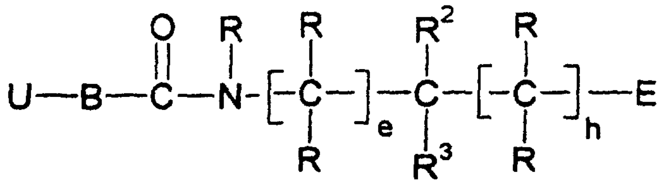

(XII)

worin B, E, R, $R^2$, $R^3$, e und h wie oben angegeben definiert sind und U für Isocyanato oder Isothiocyanato steht, erhält man Harnstoffderivate oder Thioharnstoffderivate beispielsweise der Formel XIII,

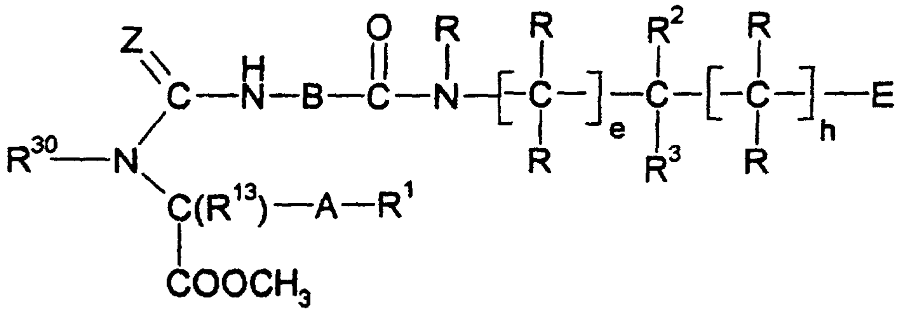

(XIII)

für die die oben angegebenen Definitionen gelten und in der Z für Sauerstoff oder Schwefel steht. Die Verbindungen der Formel XIII können durch Erhitzen mit Säure zu Verbindungen der Formel Ia

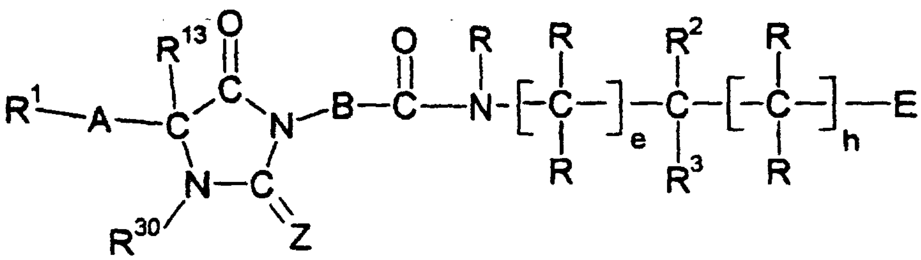

(Ia)

cyclisiert werden, für die die oben angegebenen Bedeutungen gelten. Die Cyclisierung der Verbindungen der Formel XIII zu den Verbindungen der Formel Ia kann auch durch Behandlung mit Basen in inerten Lösungsmittel durchgeführt werden, zum Beispiel durch Behandlung mit Natriumhydrid in einem aprotischen Lösungsmittel wie Dimethylformamid. Während der Cyclisierung können wiederum funktionelle Gruppen in geschützter Form vorliegen.

Verbindungen der Formel I, in denen W für $R^1$-A-C($R^{13}$) steht, können auch erhalten werden, indem man eine Verbindung der Formel XI mit einem Isocyanat oder Isothiocyanat der Formel XIV

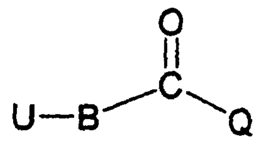

(XIV)

umsetzt, in der B und U wie oben für die Formel XII angegeben definiert sind und Q eine Alkoxygruppe, zum Beispiel eine ($C_1$-$C_4$)-Alkoxygruppe wie Methoxy, Ethoxy oder tert-Butoxy, eine ($C_6$-$C_{14}$)-Aryloxygruppe, zum Beispiel Phenoxy, oder eine ($C_6$-$C_{14}$)-Aryl-($C_1$-$C_4$)-alkoxygruppe, zum Beispiel Benzyloxy, bedeutet. Dabei wird eine Verbindung der Formel XV

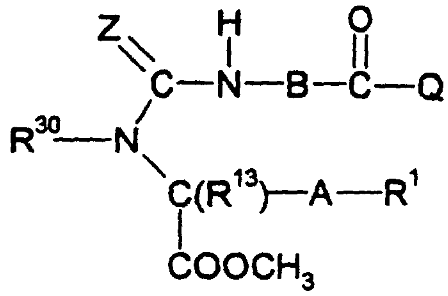

(XV)

erhalten, in der Z für Sauerstoff oder Schwefel steht und A, B, Q, $R^1$, $R^{13}$ und $R^{30}$ wie oben für die Formeln XI und XIV angegeben definiert sind, die dann unter dem Einfluß einer Säure oder einer Base, wie oben für die Cyclisierung der Verbindungen der Formel XIII beschrieben, zu einer Verbindung der Formel XVI,

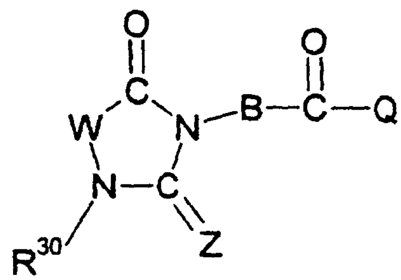

(XVI)

in der W für $R^1$-A-C($R^{13}$) steht und Z, B, Q und $R^{30}$ wie oben angegeben definiert sind, cyclisiert wird. Aus der Verbindung der Formel XVI kann dann beispielsweise durch Hydrolyse der Gruppe CO-Q zur Carbonsäure COOH und nachfolgende Kupplung mit einer Verbindung der Formel III, wie oben für die Kupplung der Verbindungen der Formeln II und III beschrieben, eine Verbindung der Formel Ia erhalten werden. Auch bei diesem Syntheseverfahren können wiederum funktionelle Gruppen in geschützter Form oder in Form von Vorstufen vorliegen.

Eine weitere Methode zur Herstellung von Verbindungen der Formel Ia ist beispielsweise die Umsetzung von Verbindungen der Formel XVII,

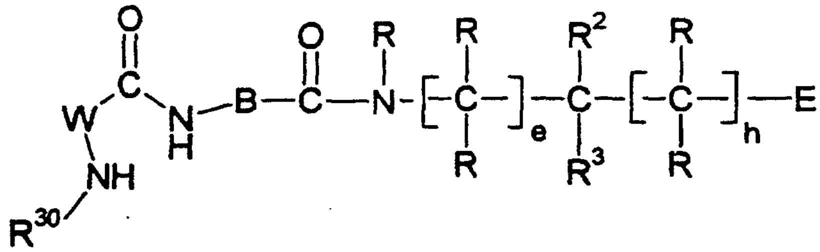

(XVII)

in der W für $R^1$-A-C($R^{13}$) steht und für die ansonsten die oben angegebenen Definitionen gelten, mit Phosgen oder

Thiophosgen oder entsprechenden Äquivalenten (analog S. Goldschmidt und M. Wick, Liebigs Ann. Chem. 575 (1952), 217-231 und C. Tropp, Chem. Ber. 61 (1928), 1431-1439).

Verbindungen der Formel Ia, in denen Z für Sauerstoff steht, können auch hergestellt werden, indem zunächst eine Verbindung der Formel XVIII,

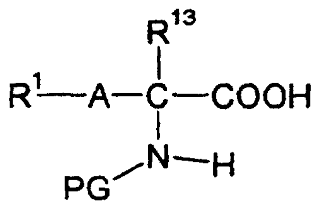

(XVIII)

in der $R^1$, $R^{13}$ und A die oben angegebenen Bedeutungen haben und PG für eine Aminoschutzgruppe wie zum Beispiel für eine Benzyloxycarbonylgruppe steht, mit einer Verbindung der Formel XIX,

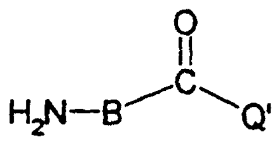

(XIX)

in der B die oben angegebenen Bedeutungen hat und Q' für eine geschützte Carbonsäurehydroxygruppe steht, zum Beispiel für eine Alkoxygruppe wie tert-Butoxy, zu einer Verbindung der Formel XX

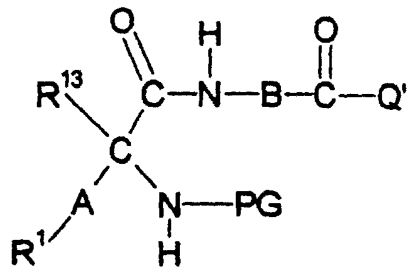

(XX)

gekuppelt wird, in der $R^1$, $R^{13}$, A, B, PG und Q' die oben angegebenen Bedeutungen haben. In der Verbindung der Formel XX kann dann selektiv die Schutzgruppe PG von der Aminogruppe abgespalten werden, zum Beispiel durch Hydrierung im Fall einer Benzyloxycarbonylgruppe, und durch Einführung einer CO-Gruppe ein Ringschluß zu einer Verbindung der Formel XXI,

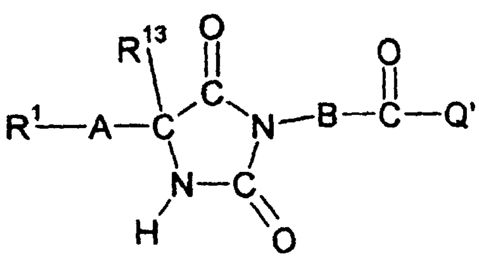

(XXI)

in der $R^1$, $R^{13}$, A, B und Q' die angegebenen Bedeutungen haben, durchgeführt werden. Zur Einführung der Carbonylgruppe kann beispielsweise Phosgen oder ein Phosgenäquivalent Verwendung finden (vergleiche die oben erläuterte Umsetzung der Verbindungen der Formel XVII). Als Zwischenstufe kann bei der Überführung der Verbindung der Formel XX in die der Formel XXI beispielsweise ein Isocyanat auftreten oder gezielt hergestellt werden. Die Überführung der Verbindung der Formel XX in die der Formel XXI kann in einem oder mehreren Schritten erfolgen.

Beispielsweise kann die Cyclisierung nach Einführung der Carbonylgruppe wie die oben beschriebenen Cyclisierungen separat in Gegenwart einer Base wie Natriumhydrid durchgeführt werden. Verbindungen der Formel XX, in der PG für die Benzyloxycarbonylgruppe steht, können auch direkt in Verbindungen der Formel XXI überführt werden, ohne daß zur Einführung der Carbonylgruppe ein Synthesebaustein wie Phosgen eingesetzt wird. Werden Verbindungen der Formel XX, in der PG für Benzyloxycarbonyl steht, mit einer Base wie Natriumhydrid behandelt, so können direkt die Verbindungen der Formel XXI erhalten werden.

In den Verbindungen der Formel XXI kann dann, wie oben für die Verbindungen der Formel VI erläutert, an der NH-Gruppe der Rest $R^{30}$- oder der Rest PG-NR-$R^{31}$eingeführt werden und nach Spaltung der Schutzgruppe CO-Q' zur Carbonsäuregruppe COOH wie oben für die Verbindungen der Formeln VII und II beschrieben die gewünschte Verbindung der Formel Ia (mit Z = Sauerstoff) aufgebaut werden. Auch bei diesem Syntheseverfahren können wiederum funktionelle Gruppen in geschützter Form oder in Form von Vorstufen vorliegen.

Eine im Rest $R^1$ enthaltene Guanidinogruppe kann beispielsweise mit den folgenden Reagenzien aus einer Aminogruppe erhalten werden, die wiederum beispielsweise aus einer Nitrogruppe oder einer Cyangruppe durch Reduktion erhältlich ist

1. O-Methylisoharnstoff (S. Weiss und H. Krommer, Chemiker-Zeitung 98 (1974), 617-618)
2. S-Methylisothioharnstoff (R. F. Borne, M. L. Forrester und I. W. Waters, J. Med. Chem. 20 (1977), 771-776)
3. Nitro-S-methylisothiohamstoff (L. S. Hafner und R. E. Evans, J. Org. Chem. 24 (1959) 57)
4. Formamidinosulfonsäure (K. Kim, Y.-T. Lin und H. S. Mosher, Tetrah. Lett. 29 (1988), 3183-3186)
5. 3,5-Dimethyl-1-pyrazolyl-formamidinium-nitrat (F. L. Scott, D. G. O'Donovan und J. Reilly, J. Amer. Chem. Soc. 75 (1953), 4053-4054)
6. N,N'-Di-tert-butyloxycarbonyl-S-methyl-isothioharnstoff (R. J. Bergeron und J. S. McManis, J. Org. Chem. 52 (1987), 1700-1703)
7. N-Alkoxycarbonyl-, N,N'-Dialkoxycarbonyl-, N-Alkylcarbonyl- und N,N'dialkylcarbonyl-S-methyl-isothiohamstoff (H. Wollweber, H. Kölling, E. Niemers, A. Widdig, P. Andrews, H.-P. Schulz und H. Thomas, Arzneim. Forsch./Drug Res. 34 (1984), 531-542).

Amidine können aus den entsprechenden Cyanverbindungen durch Anlagerung von Alkoholen, zum Beispiel Methanol oder Ethanol, in saurem wasserfreiem Medium, zum Beispiel Dioxan, Methanol oder Ethanol, und anschließende Aminolyse, zum Beispiel Behandlung mit Ammoniak in Alkoholen wie zum Beispiel Isopropanol, Methanol oder Ethanol, hergestellt werden (G. Wagner, P. Richter und Ch. Garbe, Pharmazie 29 (1974), 12-55). Eine weitere Methode, Amidine herzustellen, ist die Anlagerung von Schwefelwasserstoff an die Cyangruppe, gefolgt von einer Methylierung des entstandenen Thioamids und anschließender Umsetzung mit Ammoniak (DDR-Patent Nr. 235 866). Weiterhin kann Hydroxylamin an die Cyangruppe angelagert werden, wobei N-Hydroxyamidine entstehen, die gewünschtenfalls ebenfalls in die Amidine überführt werden können, zum Beispiel durch Hydrierung.

Hinsichtlich der Herstellung der Verbindungen der Formel I wird weiterhin vollinhaltlich Bezug genommen auf die WO-A-95/14008, auf die EP-A-796 855 und die ihr entsprechenden Anmeldungen, sowie auf die WO-A-96/33976. Insbesondere wird auch hinsichtlich der Herstellung der Verbindungen der Formeln V und VI in racemischer Form und in enantiomerenreiner Form Bezug genommen auf die entsprechenden Ausführungen in der WO-A-96/33976, die Bestandteil der vorliegenden Offenbarung sind.

Die Verbindungen der Formel I sind wertvolle Arzneimittelwirkstoffe, die sich beispielsweise für die Therapie und Prophylaxe von Entzündungserkrankungen, allergischen Erkrankungen oder Asthma eignen. Die Verbindungen der Formel I und ihre physiologisch verträglichen Salze und Derivate können erfindungsgemäß am Tier, bevorzugt am Säugetier, und insbesondere am Menschen als Arzneimittel zur Therapie oder Prophylaxe verabreicht werden. Sie können für sich allein, in Mischungen untereinander oder in Form von pharmazeutischen Präparaten verabreicht werden, die eine enterale oder parenterale Anwendung gestatten und die als aktiven Bestandteil eine wirksame Dosis mindestens einer Verbindung der Formel I und/oder ihrer physiologisch verträglichen Salze und Derivate neben üblichen pharmazeutisch einwandfreien Trägerstoffe und/oder Zusatzstoffen enthalten.

Gegenstand der vorliegenden Erfindung sind daher auch die Verbindungen der Formel I und/oder ihre physiologisch verträglichen Salze und Derivate zur Verwendung als Arzneimittel, die Verwendung der Verbindungen der Formel I und/oder ihrer physiologisch verträglichen Salze und Derivate zur Herstellung von Arzneimitteln für die Therapie und Prophylaxe der oben und im folgenden erläuterten Krankheiten, zum Beispiel für die Therapie und Prophylaxe von Entzündungserkrankungen, sowie die Verwendung der Verbindungen der Formel I und/oder ihrer physiologisch verträglichen Salze und Derivate in der Therapie und Prophylaxe dieser Krankheiten. Weiterhin sind Gegenstand der vorliegenden Erfindung pharmazeutische Präparate, die eine wirksame Dosis mindestens einer Verbindung der Formel I und/oder ihrer physiologisch verträglichen Salze und Derivate und einen pharmazeutisch einwandfreien Träger, das heißt übliche pharmazeutisch einwandfreie Trägerstoffe und/oder Zusatzstoffe, enthalten.

Die Arzneimittel können systemisch oder lokal verabreicht werden. Sie können zum Beispiel oral in Form von

Pillen, Tabletten, Filmtabletten, Dragees, Granulaten, Hart- und Weichgelatinekapseln, Pulvern, Lösungen, Sirupen, Emulsionen, Suspensionen oder in anderen Arzneiformen verabreicht werden. Die Verabreichung kann aber auch vaginal oder rektal, zum Beispiel in Form von Suppositorien, oder parenteral oder implantiv, zum Beispiel in Form von Injektionslösungen oder Infusionslösungen, Mikrokapseln oder Rods, oder topisch oder perkutan, zum Beispiel in Form von Salben, Lösungen oder Tinkturen, oder auf anderem Wege, zum Beispiel in Form von Nasalsprays oder Aerosolmischungen, erfolgen. Lösungen können parenteral zum Beispiel intravenös, intramuskulär, subkutan, intraartikulär, intrasynovial oder auf andere Weise verabreicht werden.

Die Herstellung der erfindungsgemäßen pharmazeutischen Präparate erfolgt in an sich bekannter Weise, wobei neben der oder den Verbindungen der Formel I und/oder ihren physiologisch verträglichen Salzen und Derivaten pharmazeutisch inerte anorganische und/oder organische Trägerstoffe verwendet werden können. Für die Herstellung von Pillen, Tabletten, Dragees und Hartgelatinekapseln kann man zum Beispiel Lactose, Maisstärke oder Derivate davon, Talk, Stearinsäure oder deren Salze, etc. verwenden. Trägerstoffe für Weichgelatinekapseln und Suppositorien sind zum Beispiel Fette, Wachse, halbfeste und flüssige Polyole, Polyethylenglykole, natürliche oder gehärtete Öle etc. Als Trägerstoffe für die Herstellung von Lösungen, zum Beispiel Injektionslösungen, oder von Emulsionen oder Sirupen eignen sich zum Beispiel Wasser, Alkohole, Glycerin, Diole, Polyole, Saccharose, Invertzucker, Glukose, pflanzliche Öle etc. Als Trägerstoffe für Mikrokapseln, Implantate oder Rods eignen sich zum Beispiel Mischpolymerisate aus Glykolsäure und Milchsäure. Die pharmazeutischen Präparate enthalten normalerweise etwa 0.5 bis 90 Gew.-% der Verbindungen der Formel I und/oder ihrer physiologisch verträglichen Salze und Derivate.

Die pharmazeutischen Präparate können neben den Wirkstoffen und Trägerstoffen noch Hilfstoffe oder Zusatzstoffe, wie zum Beispiel Füllstoffe, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, Lösungsmittel oder Lösungsvermittler, Mittel zur Erzielung eines Depoteffekts, Salze zur Veränderung des osmotischen Drucks, Überzugsmittel oder Antioxidantien enthalten. Sie können auch zwei oder mehrere Verbindungen der Formel I und/oder deren physiologisch verträgliche Salze und Derivate enthalten.

Ferner können sie neben mindestens einer Verbindung der Formel I und/oder ihren physiologisch verträglichen Salzen und Derivaten noch einen oder mehrere andere therapeutisch oder prophylaktisch wirksame Stoffe enthalten, zum Beispiel Stoffe mit entzündungshemmender Wirkung. Die pharmazeutische Präparate enthalten normalerweise 0.2 bis 500 mg, vorzugsweise 1 bis 100 mg Wirkstoff der Formel I und/oder dessen physiologisch verträgliche Salze und Derivate pro Dosis.

Wenn die Verbindungen der Formel I bzw. sie enthaltende pharmazeutische Zubereitungen als Aerosole verabreicht werden, zum Beispiel als Nasalaerosole oder durch Inhalation, so kann dies beispielsweise unter Verwendung eines Sprays, eines Zerstäubers, eines Pumpzerstäubers, eines Inhalationsgerätes, eines Dosierinhalators oder eines Trockenpulverinhalators erfolgen. Arzneiformen für eine Verabreichung der Verbindungen der Formel I als Aerosol können nach dem Fachmann wohlbekannten Verfahren hergestellt werden. In Betracht kommen für deren Herstellung beispielsweise Lösungen oder Dispersionen der Verbindungen der Formel I in Wasser, Wasser-Alkohol-Gemischen oder geeigneten Kochsalzlösungen unter Verwendung von üblichen Zusatzstoffen, zum Beispiel Benzylalkohol oder anderen geeigneten Konservierungsmitteln, Absorptionsverbesserem zur Erhöhung der Bioverfügbarkeit, Lösungsvermittlern, Dispergiermitteln und anderen, und gegebenenfalls üblichen Treibmitteln, zum Beispiel Fluorchlorkohlenwasserstoffen und/oder Fluorkohlenwasserstoffen.

Die Verbindungen der Formel I haben beispielsweise die Fähigkeit, Zell-Zell-Interaktionsprozesse und Zell-Matrix-Interaktionsprozesse zu inhibieren, bei denen Wechselwirkungen zwischen VLA-4 mit seinen Liganden eine Rolle spielen. Die Wirksamkeit der Verbindungen der Formel I kann zum Beispiel in einem Assay nachgewiesen werden, in dem die Bindung von Zellen, die den VLA-4-Rezeptor aufweisen, zum Beispiel von Leukozyten, an Liganden dieses Rezeptors gemessen wird, zum Beispiel an VCAM-1, das dafür vorteilhafterweise auch gentechnisch hergestellt werden kann. Einzelheiten eines solchen Assay sind weiter unten beschrieben. Insbesondere vermögen die Verbindungen der Formel I die Adhäsion und die Migration von Leukozyten inhibieren, etwa die Anheftung von Leukozyten an endotheliale Zellen, die - wie oben erläutert - über den VCAM-1/VLA-4-Adhäsionsmechanismus gesteuert wird. Außer als Entzündungshemmstoffe eignen sich die Verbindungen der Formel I und ihre physiologisch verträglichen Salze und Derivate daher generell zur Therapie und Prophylaxe von Krankheiten, die auf der Wechselwirkung zwischen dem VLA-4-Rezeptor und seinen Liganden beruhen oder durch eine Hemmung dieser Wechselwirkung beeinflußt werden können, und insbesondere eignen sie sich für die Therapie und Prophylaxe von Krankheiten, die zumindest teilweise durch ein unerwünschtes Ausmaß an Leukozytenadhäsion und/oder Leukozytenmigration verursacht werden oder damit verbunden sind, und zu deren Vorbeugung, Linderung oder Heilung die Adhäsion und/oder Migration von Leukozyten verringert werden soll.

Gegenstand der vorliegenden Erfindung sind daher auch die Verbindungen der Formel I und deren physiologisch verträgliche Salze und Derivate zur Hemmung der Adhäsion und/oder Migration von Leukozyten oder zur Hemmung des VLA-4-Rezeptors und die Verwendung der Verbindungen der Formel I zur Herstellung von Arzneimitteln dafür, also von Arzneimitteln zur Therapie oder Prophylaxe von Krankheiten, bei denen die Leukozytenadhäsion und/oder Leukozytenmigration ein unerwünschtes Ausmaß aufweist, oder zur Therapie oder Prophylaxe von Krankheiten, bei denen VLA-4-abhängige Adhäsionsvorgänge eine Rolle spielen, sowie die Verwendung der Verbindungen der Formel I und/oder ihrer physiologisch verträglichen Salze und Derivate in der Therapie und Prophylaxe derartiger Krankheiten.

Die Verbindungen der Formel I können bei entzündlichen Erscheinungen unterschiedlichster Ursache als Entzündungshemmer eingesetzt werden, um die unerwünschten oder schädigenden Folgen der Entzündung zu verhindern, zu verringern oder zu unterdrücken. Anwendung finden sie beispielsweise zur Therapie oder Prophylaxe der Arthritis, der rheumatoiden Arthritis, der Polyarthritis, der inflammatorischen bowel disease (ulcerativen Colitis), des systemischen Lupus erythematosus, zur Therapie oder Prophylaxe von inflammatorischen Erkrankungen des zentralen Nervensystems, wie zum Beispiel der Multiplen Sklerose, oder zur Therapie oder Prophylaxe von Asthma oder von Allergien, zum Beispiel Allergien vom verzögerten Typ (Typ IV-Allergie). Weiterhin eignen sie sich zur Therapie oder Prophylaxe von cardiovaskulären Erkrankungen, der Arteriosklerose, von Restenosen, von Diabetes, der Schädigung von Organtransplantaten, von Immunerkrankungen, von Autoimmunerkrankungen, von Tumorwachstum oder Tumormetastasierung bei verschiedenen Malignitäten, der Malaria sowie von weiteren Krankheiten, bei denen eine Blockierung des Integrins VLA-4 und/oder eine Beeinflussung der Leukozytenaktivität zur Vorbeugung, Linderung oder Heilung angebracht erscheint.

Die Dosis bei der Anwendung der Verbindungen der Formel I kann innerhalb weiter Grenzen variieren und ist wie üblich in jedem einzelnen Fall den individuellen Gegebenheiten anzupassen, was dem Arzt bekannt ist. Sie hängt beispielsweise von der Art und Schwere der zu behandelnden Krankheit ab, von der eingesetzten Verbindung oder davon, ob ein akuter oder chronischer Krankheitszustand behandelt wird oder Prophylaxe betrieben wird, oder davon, ob neben den Verbindungen der Formel I weitere Wirkstoffe verabreicht werden. Im allgemeinen ist bei der oralen Verabreichung eine Tagesdosis von etwa 0.01 bis 100 mg/kg, vorzugsweise 0.1 bis 10 mg/kg, insbesondere 0.3 bis 2 mg/kg (jeweils pro kg Körpergewicht) bei einem Erwachsenen zur Erzielung wirksamer Ergebnisse angemessen. Bei intravenöser Applikation beträgt die Tagesdosis im allgemeinen etwa 0.01 bis 50 mg/kg, vorzugsweise 0.01 bis 10 mg/kg Körpergewicht. Die Tagesdosis kann, insbesondere bei der Applikation größerer Mengen, in mehrere, zum Beispiel 2, 3, oder 4, Teilverabreichungen aufgeteilt werden. Gegebenenfalls kann es je nach individuellem Verhalten erforderlich werden, von der angegebenen Tagesdosis nach oben oder nach unten abzuweichen.

Außer als Arzneimittelwirkstoffe in der Humanmedizin und der Veterinärmedizin können die Verbindungen der Formel I und ihre Salze und für die betreffende Verwendung geeigneten Derivate weiterhin für diagnostische Zwekke, zum Beispiel bei in vitro-Diagnosen von Zellproben oder Gewebsproben, und als Hilfsmittel oder als wissenschaftliches Tool in biochemischen Untersuchungen eingesetzt werden, bei denen eine VLA-4-Blockierung oder eine Beeinflussung von Zell-Zell- oder Zell-Matrix-Interaktionen angestrebt wird. Weiterhin können die Verbindungen der Formel I und ihre Salze als Zwischenprodukte für die Herstellung anderer Verbindungen dienen, insbesondere anderer Arzneimittelwirkstoffe, die aus Verbindungen der Formel I beispielsweise durch Abwandlung oder Einführung von Resten oder funktionellen Gruppen erhältlich sind, zum Beispiel durch Veresterung, Reduktion, Oxidation oder andere Umwandlungen von funktionellen Gruppen.

Beispiele

Die Produkte wurden über Massenspektren (MS) und/oder NMR-Spektren identifiziert. Basische Verbindungen, die durch Chromatographie unter Verwendung eines Laufmittels gereinigt wurden, das beispielsweise Essigsäure oder Trifluoressigsäure enthielt, und anschließend gefriergetrocknet wurden, oder die mit einer Säure, zum Beispiel mit Trifluoressigsäure, behandelt wurden und zur Aufarbeitung zum Beispiel gefriergetrocknet wurden, enthielten zum Teil je nach Durchführung der Gefriertrocknung oder Aufarbeitung noch die verwendete Säure, sind also teilweise oder vollständig in Form eines Salzes der verwendeten Säure, zum Beispiel in Form des Essigsäuresalzes oder Trifluoressigsäuresalzes, angefallen.

Es bedeuten:

| | |
|---|---|
| MTBE | Methyl-tert-butylether |
| DMF | N,N-Dimethylformamid |
| THF | Tetrahydrofuran |
| DMAP | 4-Dimethylaminopyridin |
| DCC | N,N'-Dicyclohexylcarbodiimid |
| TOTU | O-((Cyan(ethoxycarbonyl)methylen)amino)-N,N,N',N'-tetramethyluroniumtetrafluoroborat |
| HOBT | 1-Hydroxybenzotriazol |
| DIPEA | N,N-Diisopropylethylamin |
| TFA | Trifluoressigsäure |
| DCM | Dichlormethan |

| | | | | | |
|---|---|---|---|---|---|
| Me | Methyl | $CH_3$- | Et | Ethyl | $CH_3$-$CH_2$- |
| nPr | n-Propyl | $CH_3CH_2CH_2$- | iPr | Isopropyl | $(CH_3)_2CH$- |
| nBu | n-Butyl | $CH_3CH_2CH_2CH_2$- | iBu | Isobutyl | $(CH_3)_2CHCH_2$- |
| tBu | tert-Butyl | $(CH_3)_3C$- | Ph | Phenyl | $C_6H_5$- |
| Fmoc | 9-Fluorenylmethoxycarbonyl | | | | |

Die Verbindungen der Beispiele wurden zum Teil nach den allgemeinen Verfahren hergestellt, die im folgenden beschrieben sind und in den Schemata dargestellt sind. Reste in den Formeln in den Schemata, die dieselben Bezeichnungen wie die entsprechenden Reste in der Formel I haben, haben die für die Formel I angegebenen Bedeutungen. Die Bedeutungen anderer Reste sind jeweils angegeben. Die Bedeutung der Reste für eine spezifische Beispielsubstanz und ebenso-die Ausgangsverbindungen, die in die einzelnen Schritte der Synthese einer spezifischen Beispielsubstanz einzusetzen sind, ergeben sich aus der Struktur der Beispielsubstanz.

**A) Allgemeines Verfahren gemäß Schema 1**

Zur Herstellung des Zwischenprodukts der Formel VIa wurde entweder ein in der α-Position durch die Gruppen $R^{13}$ und $R^1$-A- substituierter α-Aminosäurealkylester mit einem Isocyanatocarbonsäure-tert-butylester zum Harnstoff umgesetzt und dieser mit Natriumhydrid cyclisiert (Schritte A und B), oder es wurde ein in der 4-Position durch die Gruppen $R^{13}$ und $R^1$-A- substituiertes Hydantoin mit einem Bromcarbonsäure-tert-butylester alkyliert (Schritt C). Das Zwischenprodukt der

Schema 1

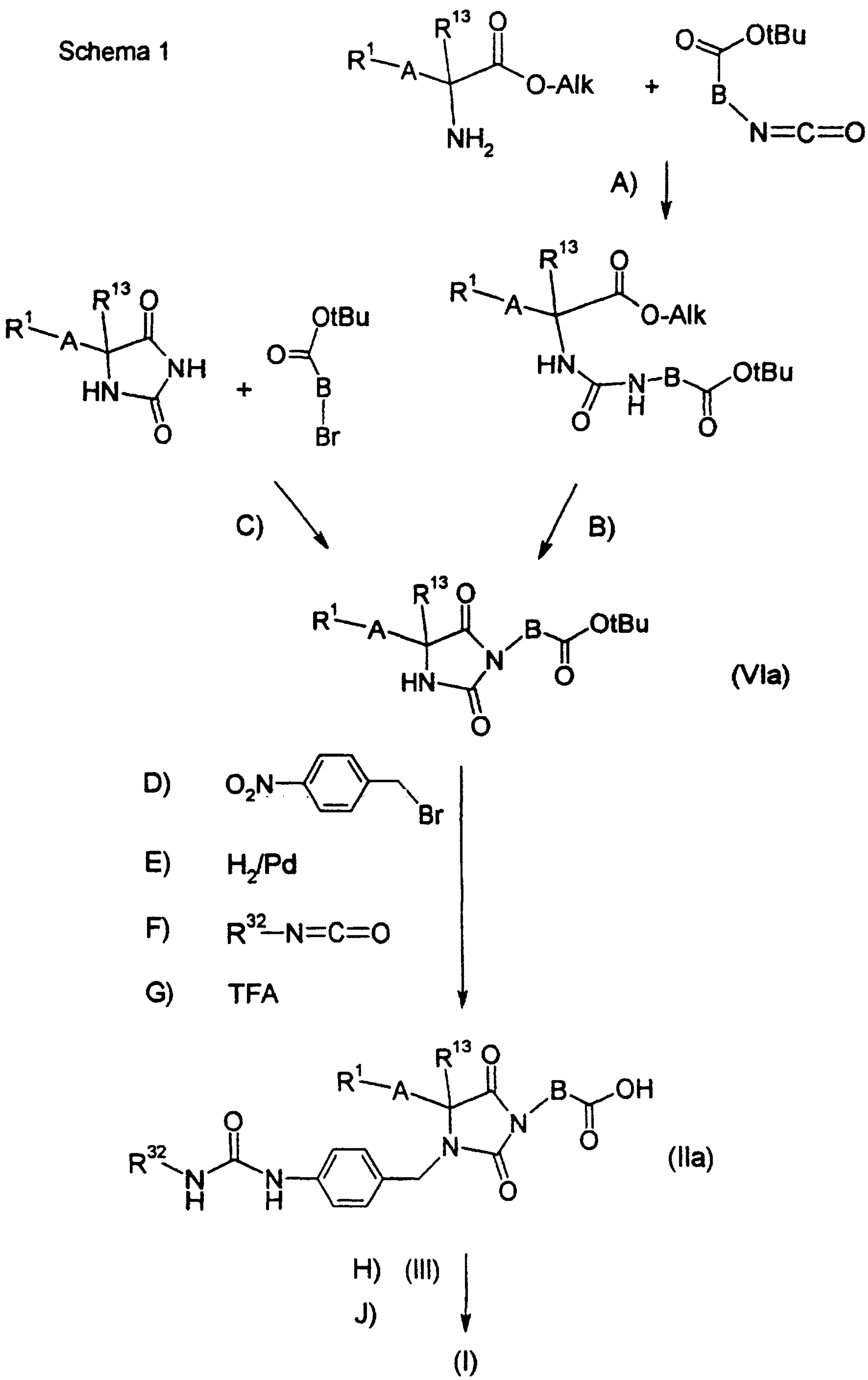


(VIa)

(IIa)

(I)

Formel VIa wurde entweder in situ oder nach vorheriger Isolierung und gegebenenfalls chromatographischer Reinigung mit 4-Nitrobenzylbromid zum 3-(4-Nitrobenzyl)-hydantoinderivat alkyliert (Schritt D). Die Nitrogruppe wurde durch katalytische Hydrierung zur Aminogruppe reduziert (Schritt E), die dann mit einem Isocyanat der Formel $R^{32}$-N=C=O zum Harnstoff umgesetzt wurde (Schritt F). Nach Überführung der tert-Butylestergruppe in die Carbonsäuregruppe mit TFA (Schritt G) wurde das Zwischenprodukt der Formel IIa mit einer Aminoverbindung der Formel III gekuppelt, in der vorhandene Carbonsäuregruppen als Ester geschützt waren (Schritt H). Durch Abspaltung der Esterschutzgruppen wurde schließlich die Verbindung der Formel I erhalten (Schritt J). Alk in Schema 1 steht für Methyl oder Ethyl. Die einzelnen Schritte wurden wie folgt durchgeführt.

Allgemeines Verfahren zur Herstellung von 3-(4-Nitrobenzyl)-hydantoinderivaten; Schritte A, B, D (Methode 1)

Der α-Aminosäurealkylester wurde in DMF gelöst (ca. 2 ml pro mmol Ester) und mit 1 Äquivalent des Isocyanatocarbonsäure-tert-butylesters (hergestellt analog J. S. Nowick et al., J. Org. Chem. 1996, 61, 3929) versetzt. Das Gemisch wurde 12 Stunden bei Raumtemperatur gerührt. Die Lösung des entstandenen Harnstoffes in DMF wurde ohne weitere Reinigung und Aufarbeitung in die weitere Reaktion eingesetzt.

Zur Cyclisierung des Harnstoffs zum Hydantoin wurde die Harnstofflösung auf 0 °C gekühlt und mit 1.2 Äquivalenten (bezogen auf den Harnstoff) Natriumhydrid versetzt. Das Gemisch wurde 15 Minuten bei 0 °C und anschließend 2 Stunden bei Raumtemperatur gerührt. Anschließend wurden 1.1 Äquivalente (bezogen auf den Harnstoff) 4-Nitrobenzylbromid zugegeben und das Gemisch wurde 3 Stunden bei Raumtemperatur gerührt. Bei unvollständigem Umsatz wurden weitere 0.1 Äquivalente Natriumhydrid zugegeben und es wurde weitere 3 Stunden bei Raumtemperatur gerührt. Das Reaktionsgemisch wurde durch Zugabe von Wasser gequencht und das Lösungsmittel am Rotationsverdampfer abgezogen. Der ölige Rückstand wurde in Ethylacetat aufgenommen und die Lösung mit Wasser gewaschen. Die organische Phase wurde über Magnesiumsulfat getrocknet und das Lösungsmittel im Vakuum entfernt. Der Rückstand wurde durch Flash-Chromatographie gereinigt (Hexan/MTBE). Die Produktfraktionen wurden vereinigt.

Allgemeines Verfahren zur Herstellung von 3-(4-Nitrobenzyl)-hydantoinderivaten; Schritte A, B, D (Methode 2)

Die Schritte A und B wurden wie vorstehend im Abschnitt Schritte A, B, D (Methode 1) beschrieben durchgeführt. In der Methode 2 wurde vor der Durchführung von Schritt D die Zwischenstufe der Formel VIa zunächst durch Chromatographie über Kieselgel mit Heptan/MTBE gereinigt. Die Produktfraktionen wurden vereinigt und das Lösungsmittel wurde im Vakuum entfernt. Der Rückstand wurde in DMF gelöst (2.5 ml pro mmol Verbindung der Formel VIa), 1 Äquivalent 4-Nitrobenzylbromid und 1.2 Äquivalente Cäsiumcarbonat wurden zugegeben und das Gemisch wurde ca. 5 Stunden bei Raumtemperatur gerührt und anschließend über Nacht bei Raumtemperatur stehen gelassen. Nach Filtration wurde das Lösungsmittel im Vakuum entfernt und der Rückstand mit Heptan/MTBE über Kieselgel chromatographiert. Die Produktfraktionen wurden eingeengt und in Schritt E eingesetzt.

Allgemeines Verfahren zur Herstellung von 3-(4-Nitrobenzyl)-hydantoinderivaten; Schritte C, D (Methode 1)

Das Hydantoin (16 mmol) wurde in DMF (ca. 7.5 ml pro mmol Hydantoin) gelöst und mit 1.2 Äquivalenten Natriumhydrid versetzt. Das Gemisch wurde 4 Stunden bei Raumtemperatur gerührt. Nach Zugabe von 1.7 Äquivalenten des Bromcarbonsäure-tert-butylesters wurde bei Raumtemperatur über Nacht weitergerührt. Das Lösungsmittel wurde am Rotationsverdampfer entfernt. Der Rückstand wurde durch Flash-Chromatographie gereinigt (Heptan/MTBE). Man erhielt das alkylierte Hydantoin der Formel VIa.

Das alkylierte Hydantoin der Formel VIa wurde in DMF (ca. 4 ml pro mmol Hydantoin) gelöst und mit 1.1 Äquivalenten Natriumhydrid versetzt. Das Gemisch wurde 1 Stunde bei Raumtemperatur gerührt. Nach Zugabe von 1.1 Äquivalenten 4-Nitrobenzylbromid wurde weitere 2-3 Stunden bei Raumtemperatur gerührt. Das Reaktionsgemisch wurde durch Zugabe von Wasser gequencht und das Lösungsmittel am Rotationsverdampfer abgezogen. Der ölige Rückstand wurde in Ethylacetat aufgenommen und mit Wasser gewaschen. Die organische Phase wurde über Magnesiumsulfat getrocknet und das Lösungsmittel im Vakuum entfernt. Der Rückstand wurde durch Flash-Chromatographie gereinigt (Hexan/MTBE). Die Produktfraktionen, die das 3-(4-Nitrobenzyl)-hydantoinderivat enthielten, wurden vereinigt.

Allgemeines Verfahren zur Herstellung von 3-(4-Nitrobenzyl)-hydantoinderivaten; Schritte C, D (Methode 2)

Schritt C wurde wie vorstehend im Abschnitt Schritte C, D (Methode 1) beschrieben durchgeführt. In der Methode 2 wurde in Schritt C die Zwischenstufe der Formel VIa mit 4-Nitrobenzylbromid und Cäsiumcarbonat umgesetzt (analog dem oben beschriebenen Verfahren für die Schritte A, B, D (Methode 2)) und das erhaltene Rohprodukt wie für die Schritte C, D (Methode 1) beschrieben chromatographisch gereinigt.

Allgemeines Verfahren zur katalytischen Reduktion der Nitroverbindungen; Schritt E

Das 3-(4-Nitrobenzyl)-hydantoinderivat wurde in Methanol (ca. 10 ml pro mmol Hydantoinderivat) gelöst und mit Palladium/Kohle in einer Wasserstoffatmosphäre bis zur vollständigen Umsetzung hydriert. Der Katalysator wurde abfitriert und das Lösungsmittel am Rotationsverdampfer entfernt. Man erhielt das 3-(4-Aminobenzyl)-hydantoinderivat.

Allgemeines Verfahren zur Herstellung der Hamstoffe; Schritt F

Das 3-(4-Aminobenzyl)-hydantoinderivat wurde in THF (ca. 4 ml pro mmol Hydantoinderivat) gelöst und mit 1 Äquivalent des Isocyanats der Formel $R^{32}$-N=C=O versetzt. Das Gemisch wurde bis zur vollständigen Umsetzung unter Rückfluß erhitzt. Das Lösungsmittel wurde im Vakuum entfernt. Der Rückstand wurde durch Flash-Chromatographie gereinigt (Hexan/MTBE). Nach Einengen der Produktfraktionen wurde der entsprechende Harnstoff erhalten.

Allgemeines Verfahren zur Überführung der tert-Butylester in die Carbonsäuren; Schritt G

Zur Spaltung der tert-Butylester-Gruppe wurde der in Schritt F erhaltene Harnstoff in TFA (ca. 10 ml pro mmol) 1 Stunde bei Raumtemperatur gerührt. Nach Entfernen der TFA am Rotationsverdampfer wurde der Rückstand gefriergetrocknet. Man erhielt die Carbonsäure der Formel IIa.

Allgemeines Verfahren zur Kupplung der Carbonsäuren mit Aminoverbindungen; Schritt H (Methode 1)

Die Carbonsäure der Formel IIa wurde in DMF (ca. 5 ml pro mmol Carbonsäure) gelöst und mit 1 Äquivalent der zu kuppelnden Aminoverbindung der Formel III, in der gegebenenfalls vorhandene Carbonsäuregruppen als Ester geschützt vorlagen, und mit 1 Äquivalent HOBT versetzt. Das Gemisch wurde auf 0 °C gekühlt, mit 1 Äquivalent DCC versetzt und 1 Stunde bei 0 °C gerührt. Anschließend wurde 4 Stunden bei Raumtemperatur gerührt. Das Gemisch wurde filtriert und das Lösungsmittel im Vakuum entfernt. Reinigung des Rückstandes durch Flash-Chromatographie ergab das Kupplungsprodukt.

Allgemeines Verfahren zur Kupplung der Carbonsäuren mit Aminoverbindungen; Schritt H (Methode 2)

Die Carbonsäure der Formel lla und 1 Äquivalent der zu kuppelnden Aminoverbindung der Formel III wurden in DMF (ca. 5 ml pro mmol Carbonsäure) gelöst. Zu der Lösung wurden nacheinander 1 Äquivalent TOTU und 1 Äquivalent DIPEA gegeben (falls die Aminoverbindung der Formel III als Hydrochlorid eingesetzt wurde, wurden 2 Äquivalente DIPEA zugegeben). Das Gemisch wurde bei Raumtemperatur gerührt. Nach beendeter Reaktion wurde das Lösungsmittel im Vakuum entfernt, der Rückstand in Ethylacetat aufgenommen und die Ethylacetat-Phase nacheinander zweimal mit gesättigter Natriumhydrogencarbonat-Lösung, Kaliumhydrogensulfat/Kaliumsulfat-Lösung und gesättigter Natriumchlorid-Lösung gewaschen. Die Phasen wurden getrennt und die organische Phase wurde über Natriumsulfat getrocknet. Nach Filtration wurde das Lösungsmittel im Vakuum entfernt und der Rückstand durch Chromatographie über Kieselgel gereinigt. In den Fällen, in denen die Verbindung der Formel III eine oder mehrere als tert-Butylester, Methylester oder Ethylester geschützte Carbonsäuregruppe(n) enthielt, wurde entweder zunächst der Ester durch Chromatographie über Kieselgel gereinigt oder es wurden zunächst die Estergruppen gespalten (siehe Schritt J) und anschließend das Endprodukt (die Carbonsäure) gereinigt.

Allgemeines Verfahren zur Spaltung von tert-Butylester-Schutzgruppen; Schritt J (Methode 1)

Zur Spaltung von tert-Butylester-Schutzgruppen wurde das Kupplungsprodukt aus Schritt H in TFA (ca. 10 ml pro mmol) gelöst und 1 Stunde bei Raumtemperatur gerührt. Das Lösungsmittel wurde am Rotationsverdampfer entfernt. Der Rückstand wurde, teilweise nach Zusatz von Essigsäure/Wasser, gefriergetrocknet oder durch Chromatographie gereinigt und anschließend gefriergetrocknet. Man erhielt die entsprechende Säure der Formel I.

Allgemeines Verfahren zur Spaltung von Methylester- und Ethylester-Schutzgruppen; Schritt J (Methode 2)

Zur Spaltung von Methylester- oder Ethylester-Schutzgruppen wurde das Kupplungsprodukt aus Schritt H in Methanol gelöst (ca. 15 ml pro mmol) und die Lösung mit 3 Äquivalenten einer 1 N wäßrigen Lithiumhydroxidlösung versetzt. Das Gemisch wurde über Nacht bei Raumtemperatur stehen gelassen und anschließend mit 1N Salzsäure auf einen pH-Wert von 1 eingestellt. Es wurde Ethylacetat zugegeben, die Phasen wurden getrennt, die organische Phase wurde mit Wasser gewaschen und das Lösungsmittel im Vakuum entfernt. Der Rückstand wurde nach Zusatz

von Essigsäure und Wasser gefriergetrocknet.

**B) Allgemeines Verfahren gemäß Schema 2**

Zur Herstellung des Zwischenproduktes der Formel VIa wurde eine N-Benzyloxycarbonyt-α-aminosäure mit einem Aminosäure-tert-butylester gekuppelt (Schritt K) und das Kupplungsprodukt nach Abspaltung der Benzyloxycarbonylgruppe (= Gruppe Z) durch katalytische Hydrierung (Schritt L) und Einführung einer CO-Gruppe an der erhaltenen freien Aminofunktion zur Verbindung der Formel VIa cyclisiert (Schritt M). Diese wurde analog dem Verfahren gemäß Schema 1 mit 4-Nitrobenzylbromid zum 3-(4-Nitrobenzyl)-hydantoinderivat alkyliert, zur Verbindung der Formel IIa umgesetzt und die Verbindung der Formel IIa durch Kupplung mit einer Aminoverbindung der Formel III, in der Carbonsäuregruppen in geschützter Form als Ester vorlagen, und Abspaltung der Schutzgruppen in die Verbindung der Formel I überführt (Schritte D - J). Die einzelnen Schritte wurden wie folgt durchgeführt.

Allgemeines Verfahren zur Herstellung von 3-(4-Nitrobenzyl)-hydantoinderivaten; Schritte K, L, M, D

Im Schritt K wurden die N-Benzyloxycarbonyl-α-aminosäure und der Aminosäure-tert-butylester wie für das Verfahren gemäß Schema 1, Schritt H (Methode 2) beschrieben gekuppelt. Im Schritt L wurde das Kupplungsprodukt wie für Schema 1, Schritt E beschrieben über Palladium/Kohle hydriert. Anschließend wurde im

Schema 2

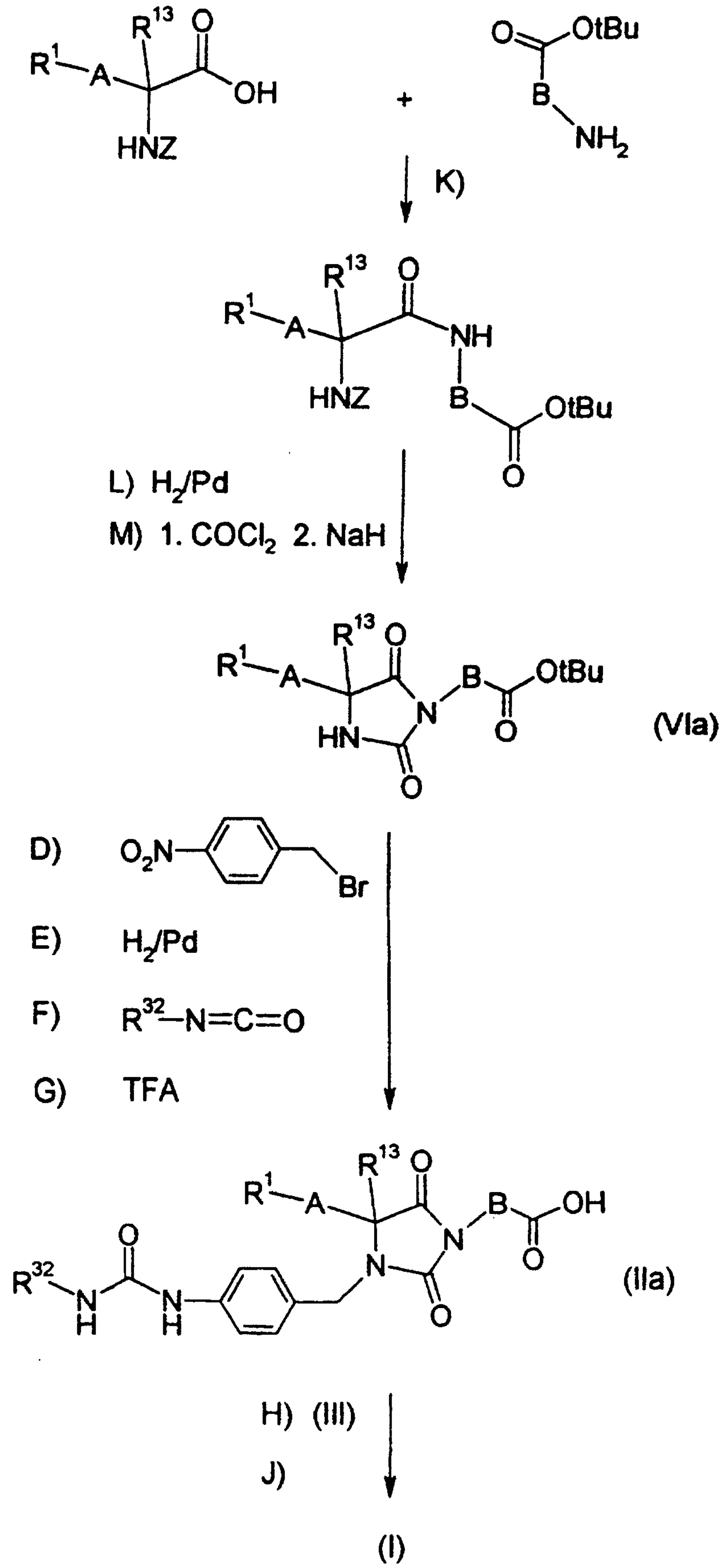

Schritt M zunächst analog J. S. Nowick et al., J. Org. Chem. 1996, 61, 3929 die $H_2N$-Gruppe mit Phosgen in Toluol in das Isocyanat überführt. Das erhaltene Isocyanat wurde in DMF (2.5 ml pro mmol Isocyanat) gelöst. Zu der Lösung wurden bei 0 °C 1.2 Äquivalente Natriumhydrid zugegeben und das Gemisch wurde 1.5 Stunden bei Raumtemperatur gerührt. Das Lösungsmittel wurde im Vakuum entfernt, der Rückstand in Ethylacetat aufgenommen und zweimal mit Wasser gewaschen. Die Phasen wurden getrennt, die Ethylacetat-Phase wurde über Natriumsulfat getrocknet und nach Filtration das Lösungsmittel im Vakuum entfernt. Man erhielt die Verbindung der Formel VIa, die im Schritt D entweder direkt oder nach vorheriger chromatographischer Reinigung nach dem für Schema 1, Schritte C,D (Methode 2) beschriebenen Verfahren mit 4-Nitrobenzylbromid umgesetzt wurde.

Die anschließenden Schritte E, F und G, die im Schritt H unter Verwendung von TOTU durchgeführte Kupplung mit der Verbindung der Formel III und, falls das Kupplungsprodukt aus Schritt H Ester-Schutzgruppen enthielt, der Schritt J wurden analog dem Verfahren gemäß Schema 1, Schritte E, F, G, H (Methode 2) und J durchgeführt.

**C) Allgemeines Verfahren gemäß Schema 3**

Aus einer Verbindung der Formel VIa (Herstellung siehe oben) wurde durch Einführung der N-Boc-geschützten Aminoalkyl-Seitenkette (Schritt N) und anschließende selektive Spaltung der N-Boc-Gruppe (Schritt P) ein Aminoalkylhydantoinderivat hergestellt, das dann analog dem Verfahren gemäß Schema 1 zur Verbindung der Formel IIb umgesetzt wurde (Schritte F, G). Die Verbindung der Formel IIb wurde dann durch Kupplung mit einer Aminoverbindung der Formel III, in der Carbonsäuregruppen in geschützter Form als Ester vorlagen, und Abspaltung der Schutzgruppen in die Verbindung der Formel I überführt (Schritte H, J). Die einzelnen Schritte wurden wie folgt durchgeführt.

Schema 3

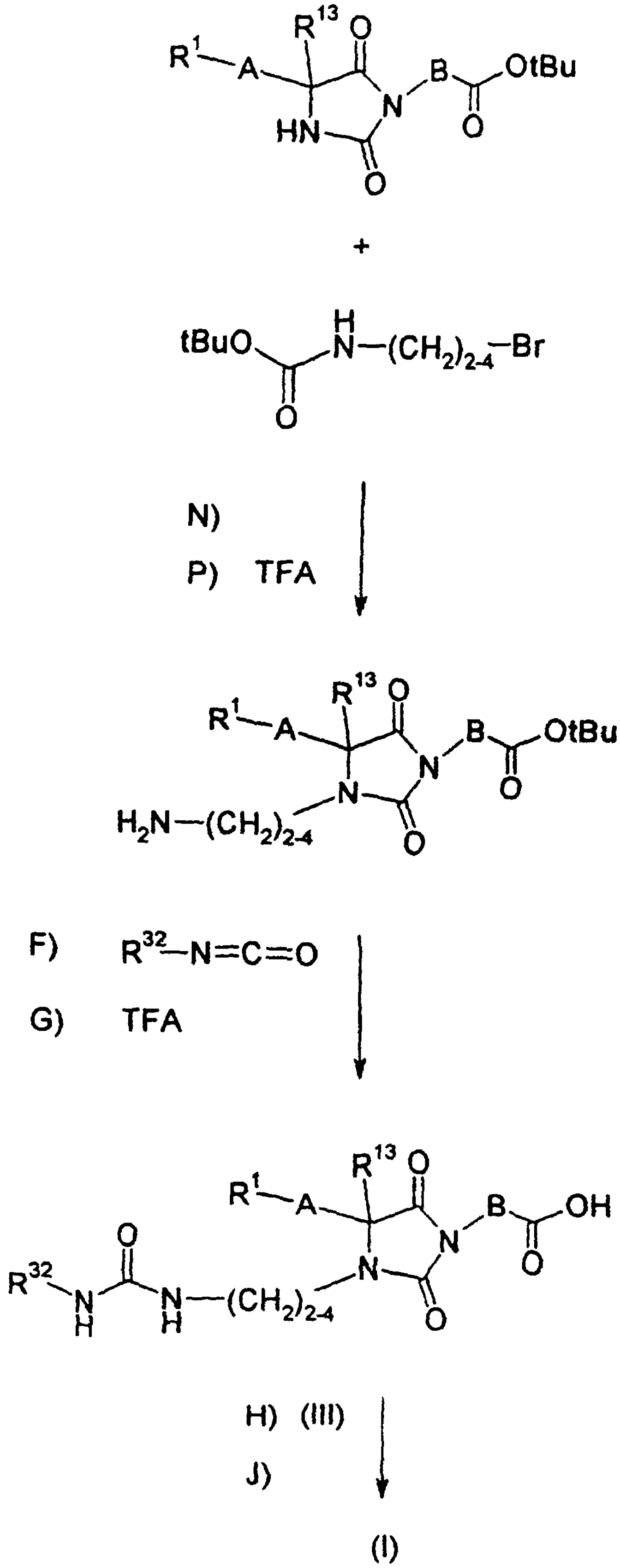


(VIa)

(IIb)

(I)

Allgemeines Verfahren zur Herstellung von 3-(Aminoalkyl)hydantoinderivaten; Schritte N, P

Im Schritt N wurde das Hydantoinderivat der Formel VIa in DMF (ca. 3 ml pro mmol Hydantoinderivat) gelöst, die Lösung mit dem N-Boc-Aminoalkylbromid und 1.05 Äquivalenten Cäsiumcarbonat versetzt und das Gemisch 8-16 Stunden auf 60 °C erhitzt. Das Lösungsmittel wurde im Vakuum entfernt und der Rückstand mit Heptan/MTBE über Kieselgel filtriert. Die Produktfraktionen wurden vereinigt. Nach Entfernen des Lösungsmittels im Vakuum wurde im Schritt P der Rückstand in einem Gemisch aus TFA/DCM (1:1) (ca. 8.5 ml pro mmol) gelöst und nach 4 Minuten in eiskalte Natriumhydrogencarbonatlösung gegossen (ca. 70 ml pro mmol). Die wäßrige Phase wurde zweimal mit DCM extrahiert. Die vereinigten organischen Phasen wurden über Natriumsulfat getrocknet. Nach Filtration und Entfernen des Lösungsmittels im Vakuum erhielt man das 3-(Aminoalkyl)-hydantoinderivat.

Die anschließenden Schritte F, G und H (unter Verwendung von TOTU) und, falls das Kupplungsprodukt aus Schritt H Esterschutzgruppen enthielt, Schritt J wurden wie für Schema 1, Schritte F, G, H (Methode 2) und J beschrieben durchgeführt.

Racemische β-Aminosäuren, die in den vorstehenden beschriebenen Verfahren im Schritt H als Aminoverbindungen der Formel III eingesetzt wurden, wurden wie unten für das Verfahren gemäß Schema 5 beschrieben hergestellt. Enantiomerenreine oder hochangereicherte 3-substituierte 3-Aminopropionsäureester waren kommerziell erhältlich oder wurden analog S. G. Davis et al., Tetrahedron Asymmetry 1991, 2(3), 183-186, hergestellt. Dabei wurde wie folgt vorgegangen.

Allgemeines Verfahren zur Herstellung 3-substituierten 3-Aminopropionsäure-tert-butylestern

Die entsprechende 3-substituierte Acrylsäure (0.1 mol) wurde mit 1.1 Äquivalenten Oxalylchlorid in 100 ml Dichlormethan gelöst. Das Gemisch wurde 4 Stunden bei Raumtemperatur gerührt. Das Lösungsmittel wurde am Rotationsverdampfer entfernt. Der Rückstand wurde in 100 ml tert-Butanol aufgenommen und 2 Stunden bei Raumtemperatur gerührt. Nach beendeter Reaktion wurde das Lösungsmitttel am Rotationsverdampfer entfernt. Der Rückstand wurde in Diethylether gelöst und mit Wasser, Natriumhydrogencarbonatlösung und erneut mit Wasser gewaschen. Die organische Phase wurde über Magnesiumsulfat getrocknet und das Lösungsmittel im Vakuum entfernt. Man erhielt den 3-substituierten Acrylsäure-tert-butylester in einer Ausbeute von > 80 %.

Zur Einführung der Aminogruppe wurden zu einer Lösung von (R)-(+)-N-Benzyl-N-(1-phenyl-ethyl)-amin (60 mmol) in 100 ml THF bei -70 °C über den Zeitraum von 1 Stunde 0.95 Equivalente n-Butyllithium (in n-Hexan) zugetropft. Das Gemisch wurde 1 Stunde bei dieser Temperatur gerührt, dann wurde eine Lösung des 3-substituierten Acrylsäure-tert-butylesters (0.9 Equivalente) in 75 ml THF über den Zeitraum von 1 Stunde zugetropft. Das Gemisch wurde 2 Stunden bei -70 °C gerührt. Nach Entfernen der Kühlung wurden 115 ml 5 %ige Citronensäurelösung zugetropft. Die Lösung wurde 1 Stunde gerührt, mit Essigester versetzt und mit Wasser gewaschen. Die organische Phase wurde mit Natriumhydrogencarbonatlösung und Wasser gewaschen und über Magnesiumsulfat getrocknet. Das Lösungsmitel wurde im Vakuum entfernt. Der Rückstand wurde durch Flash-Chromatographie gereinigt (Heptan/ethylacetat, 9:1). Man erhielt den 3-substituierten 3-(N-Benzyl-N-(1-phenyl-ethyl)-amino)-propionsäure-tert-butylester in einer Ausbeute von ca. 50 % als gelbes Öl. Zur Abspaltung der Benzylgruppe und der Phenylethylgruppe wurde die Substanz (ca. 30 mmol) in 200 ml eines Gemisches aus Ethylacetat und Essigsäure (4:1) gelöst und mit 1.5 g Palladiumhydroxid versetzt. Unter einer Wasserstoffatmosphäre wurde 8 Stunden bei Raumtemperatur hydriert. Der Katalysator wurde abfiltriert und das Filtrat am Rotationsverdampfer eingeengt. Der Rückstand wurde in Ether/Wasser aufgenommen. Die wäßrige Phase wurde mit Natriumhydrogencarbonat neutralisiert und mehrfach mit Ether extrahiert. Die vereinigten organischen Phasen wurden über Magnesiumsulfat getrocknet und vorsichtig am Rotationsverdampfer eingeengt. Man erhielt den 3-substituierten 3-Aminopropionsäure-tert-butylester als dünnflüssiges, leichtflüchtiges Öl in einer Ausbeute von > 50 %.

Analog zu den vorstehend beschriebenen Umsetzungen in Lösung können Reaktionen zur Herstellung der Verbindungen der Formel I auch an fester Phase, das heißt unter Verwendung von harzgebundenen Bausteinen, durchgeführt werden. Es können einzelne oder mehrere Syntheseschritte an der festen Phase durchgeführt werden. Insbesondere können Kupplungen von Verbindungen der Formeln IIa oder IIb anstatt mit Aminoverbindungen der Formel III in Lösung auch mit harzgebundenen Aminoverbindungen der Formel III durchgeführt werden. Verfahren zur Herstellung von Verbindungen der Formel I unter Verwendung von Festphasenreaktionen sind im folgenden beschrieben und in den Schemata 4 und 5 dargestellt.

Die Mengenangaben in den Vorschriften für die Festphasensynthesen beziehen sich immer auf die jeweilige Harzbeladung, die UV-photometrisch nach Abspaltung der Fmoc-Schutzgruppe ermittelt wurde (siehe zum Beispiel "The Combinatorial Chemistry Catalog", Novabiochem).

**D) Allgemeines Verfahren gemäß Schema 4**

Herstellung von Verbindungen der Formel I, die eine Aspararaginsäureeinheit enthalten, durch Festphasensynthese

Zur Anknüpfung an den polymeren Träger wurde ein orthogonal geschützter Asparaginsäurebaustein eingesetzt. Fmoc-Asp(OH)-OAllyl wurde in Gegenwart eines Kupplungsreagenzes mit Wang-Polystyrolharz (Wang-PS) umgesetzt und dann am Harz die Allylester-Schutzgruppe abgespalten (Schritt Q). Der freie C-Terminus wurde dann in Gegenwart eines Kupplungsreagenzes mit einem Aminosäure-tert-butylester umgesetzt (Schritt R). Nach Abspaltung der Fmoc-Schutzgruppe erfolgte dann die Umsetzung am N-Terminus durch Kupplung mit einer Hydantoincarbonsäure, die wie oben beschrieben hergestellt wurde (Schritt S). Nach Abspaltung von Schutzgruppen und Abspaltung vom Harz erhielt man die Verbindung der Formel I (Schritt T). Reste in den Formeln im Schema 4, die dieselben Bezeichnungen wie die entsprechenden Reste in der Formel I haben, haben die für die Formel I angegebenen Bedeutungen. $R^{41}$ entspricht zusammen mit der CH-Gruppe, an die der Rest $R^{41}$ gebunden ist, und mit der an die CH-Gruppe gebundenen Gruppe COOtBu der Gruppe $R^4$ in der Definition der Verbindungen der Formel I, die für Alkyl steht, das durch die in der Definition von $R^4$ angegebenen Substituenten substituiert ist. Die einzelnen Schritte wurden wie folgt durchgeführt.

Herstellung von Fmoc-Asp(OH)-OAllyl

40 g (88.7 mmol) Fmoc-Asp(OtBu)-OAllyl wurden mit 25 ml TFA versetzt und 30 Minuten bei Raumtemperatur gerührt. Das Lösungsmittel wurde am Rotationsverdampfer abgezogen. Der Rückstand wurde im Vakuum getrocknet. Man erhielt Fmoc-Asp(OH)-OAllyl als gelbes Öl in einer Ausbeute von 33.9 g (97 %).
ES(+)-MS: 395.2 $(M+H)^+$

Anknüpfung an den polymeren Träger und Abspaltung der Allylester-Schutzgruppe am polymeren Träger; Schritt Q

40 g Wang-Polystyrolharz (1.1 mmol/g; Bachem) wurden 5 Minuten mit 20 ml DMF bei Raumtemperatur vorgequollen. Nach Zugabe einer Lösung von 26.0 g (1.5

Schema 4

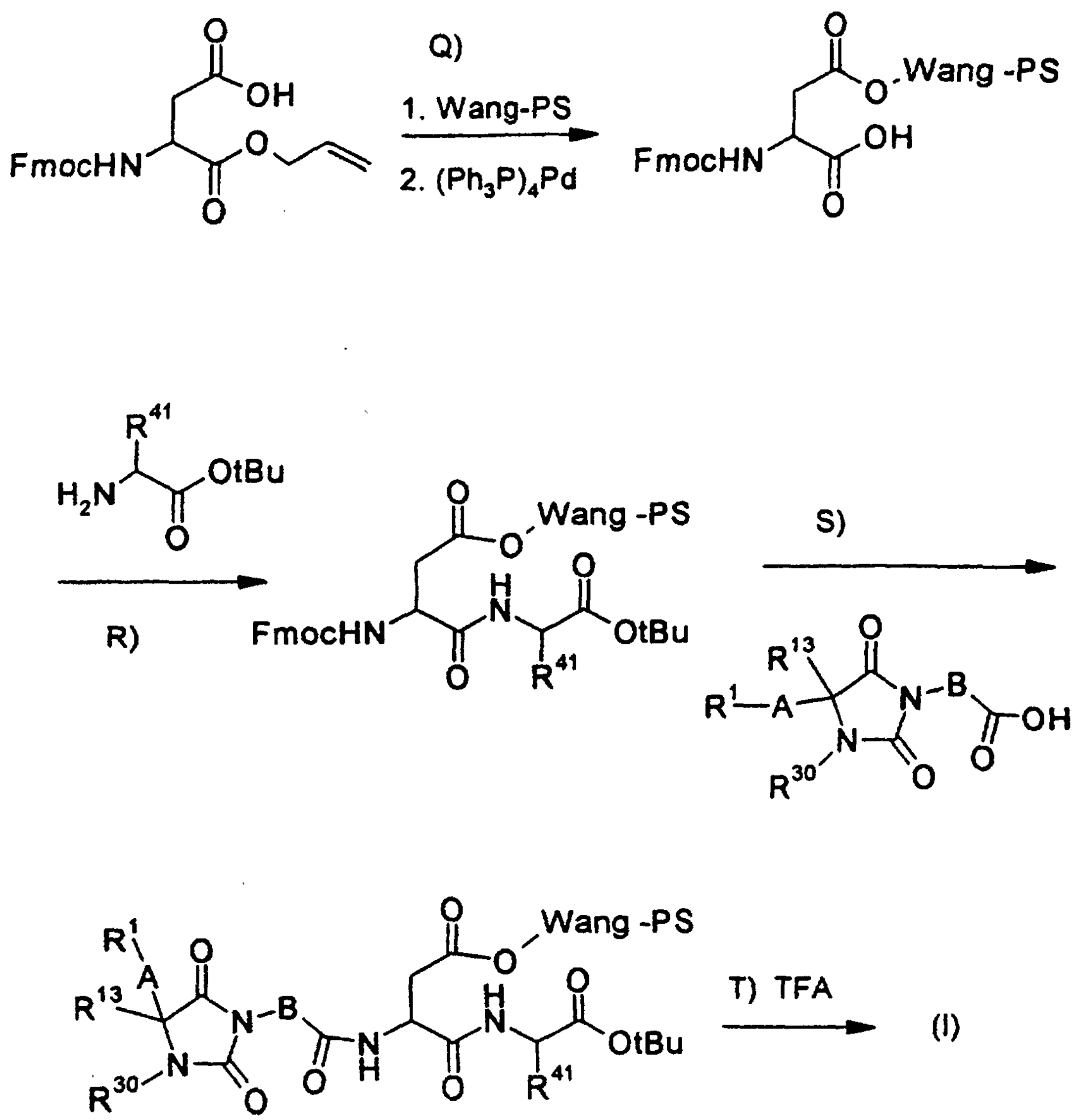


Äquivalente) Fmoc-Asp(OH)-OAllyl, 34.3 g (1.5 Äquivalente) 1-Benzotriazolyloxytripyrrolidinophosphonium-hexafluorophosphat (PyBOP) und 1.5 Äquivalenten DIPEA in 120 ml DMF wurde das Gemisch 10 Stunden bei 40 °C geschüttelt (als Kupplungsreagenz kann mit gleichen Ergebnissen auch TOTU/HOBT eingesetzt werden). Nach beendeter Reaktion wurde die Lösung abgesaugt und das Harz mit DMF gewaschen (5 x 20 ml). Nach Zugabe einer Lösung von Acetanhydrid (10 ml) und DIPEA (1.5 Äquivalente) in 40 ml DMF wurde das Gemisch erneut für 30 Minuten bei Raumtemperatur geschüttelt. Die Lösung wurde abgesaugt und das Harz nacheinander jeweils dreimal mit 40 ml DMF, Methanol und DCM gewaschen. Das Harz wurde anschließend im Vakuum getrocknet. Die Bestimmung der Beladung nach der Fmoc-Methode ergab eine Beladung von 0.6 mmol/g.

Zur Abspaltung der Allylester-Schutzgruppe wurde das Harz unter Argon 5 Minuten in DMF bei Raumtemperatur vorgequollen. Nach Zugabe von Tetrakis(triphenylphosphin)palladium (0.1 Äquivalente) und N-Methylanilin (10 Äquivalente) wurde das Gemisch unter Argon 6 Stunden bei 40 °C geschüttelt. Nach beendeter Reaktion wurde die Lösung abgesaugt und das Harz nacheinander jeweils dreimal mit DMF, Methanol, Toluol und DCM gewaschen und anschließend getrocknet.

Allgemeines Verfahren zur Kupplung mit Aminoverbindungen am polymeren Träger; Schritt R

Das im Schritt Q erhaltene Harz mit freier Carboxylfunktion wurde 5 Minuten in DMF bei Raumtemperatur vorgequollen. Nach Zugabe einer Lösung von HOBT (1.2 Äquivalente), TOTU (1.2 Äquivalente) und DIPEA (1.2 Äquivalente) in DMF wurde das Gemisch 30 Minuten bei Raumtemperatur geschüttelt. Die Aminoverbindung (Aminosäure-tert-butylester) (1.2 Äquivalente) wurde als Lösung in DMF zugegeben. Die Suspension wurde bei Raumtemperatur bis zur vollständigen Umsetzung geschüttelt (HPLC-Kontrolle). Nach beendeter Reaktion wurde die Lösung abgesaugt und das Harz nacheinander jeweils dreimal mit DMF, Methanol, Toluol und DCM gewaschen und anschließend getrocknet.

Allgemeines Verfahren zur Abspaltung der Fmoc-Schutzgruppe am polymeren Träger und Kupplung mit Hydantoincarbonsäuren; Schritt S

Zu 100 mg des in Schritt R erhaltenen Harzes gab man 5 ml einer 20%igen Lösung von Piperidin in DMF und ließ das Gemisch 20 Minuten bei Raumtemperatur schütteln. Das Harz wurde abgesaugt und der Vorgang ein weiteres Mal wiederholt. Das Harz wurde danach mehrfach sorgfältig mit DMF und DCM gewaschen.

Für die Kupplung wurde zum Harz eine Lösung von je 2 Äquivalenten HOBT, TOTU, DIPEA und der Hydantoincarbonsäure in DMF (10 ml/g Harz) gegeben und das Gemisch 12 Stunden bei Raumtemperatur geschüttelt. Das Harz wurde abfiltriert und dreimal mit je 10 ml DMF, einmal mit 10 ml Toluol, einmal mit 10 ml Methanol und dreimal mit 10 ml DCM gewaschen.

Allgemeines Verfahren zur Abspaltung vom Harz; Schritt T

Zu dem in Schritt S erhaltenen Harz wurde ein Gemisch aus TFA und DCM (1:1) gegeben. Die Suspension wurde 1 Stunde geschüttelt. Das Harz wurde abfiltriert und die Lösung im Vakuum eingeengt. Der Rückstand wurde durch Chromatographie an Kieselgel gereinigt (DCM und Ethylacetat).

**E) Allgemeines Verfahren gemäß Schema 5**

Herstellung von Verbindungen der Formel I, die eine β-Aminosäureeinheit enthalten, durch Festphasensynthese

Die eingesetzten racemischen β-Aminosäuren wurden aus den entsprechenden Aldehyden durch Umsetzung mit Malonsäure und Ammoniumacetat hergestellt.

Nach Schutz der Aminofunktion durch Einführung einer Fmoc-Gruppe wurde die Säure mit Tritylchlorid-Polystyrolharz (PS-Trt-Cl) umgesetzt (Schritt U). Gemäß Schema 5, Variante A wurde dann am polymeren Träger die Fmoc-Schutzgruppe abgespalten und anschließend in Gegenwart eine Kupplungsreagenzes mit einer Hydantoincarbonsäure gekuppelt, die wie oben beschrieben hergestellt wurde (Schritt V). Nach Abspaltung vom Harz erhielt man die Verbindung der Formel I (Schritt W).

Gemäß Schema 5, Variante B wurde nach Abspaltung der Fmoc-Schutzgruppe am polymeren Träger in Gegenwart eines Kupplungsreagenzes mit einem Hydantoinbaustein gekuppelt, der an Stelle der in der Verbindung der Formel IIa in Schema 1 enthaltenen Gruppe $R^{32}$-NH-CO-NH die Gruppe Fmoc-NH enthielt (Schritt Y). Dieser Hydantoinbaustein wurde nach dem Verfahren gemäß Schema 1 in Lösung hergestellt, wobei nach der Hydrierung in Schritt E die erhaltene Aminobenzylgruppe in die N-Fmoc-Aminobenzylgruppe überführt wurde. In dem im Schritt Y erhaltenen Kupplungsprodukt am polymeren Träger wurde dann die Fmoc-Schutzgruppe abgespalten. Die erhaltene freie Aminogruppe im Benzylsubstituenten an N-3 des Hydantoins wurde anschließend mit Isocyanaten, Isothiocyanaten oder Carbonsäuren zu Harnstoffen, Thioharnstoffen oder Amiden umgesetzt, oder sie wurde mit einem reaktiven Kohlensäurederivat und Alkoholen oder Aminen zu Carbaminsäureestern oder Harnstoffen umgesetzt (Schritt Z). Nach Abspaltung vom Harz erhielt man schließlich die Verbindung der Formel I (Schritt W). Die einzelnen Schritte wurden wie folgt durchgeführt.

Allgemeines Verfahren zur Herstellung von racemischen β-Aminosäuren der Formel $H_2N-CH(R^3)-CH_2-COOH$

625 mg (6.0 mmol) Malonsäure, 789 mg (10.2 mmol) Ammoniumacetat und 4.0 mmol des jeweiligen Aldehyds der Formel $R^3$-CHO wurden in 10 ml Ethanol suspendiert. Das Gemisch wurde 6 Stunden bei 90 °C gerührt. Der Niederschlag wurde abgesaugt und zweimal mit je 5 ml Ethanol gewaschen.

Schema 5

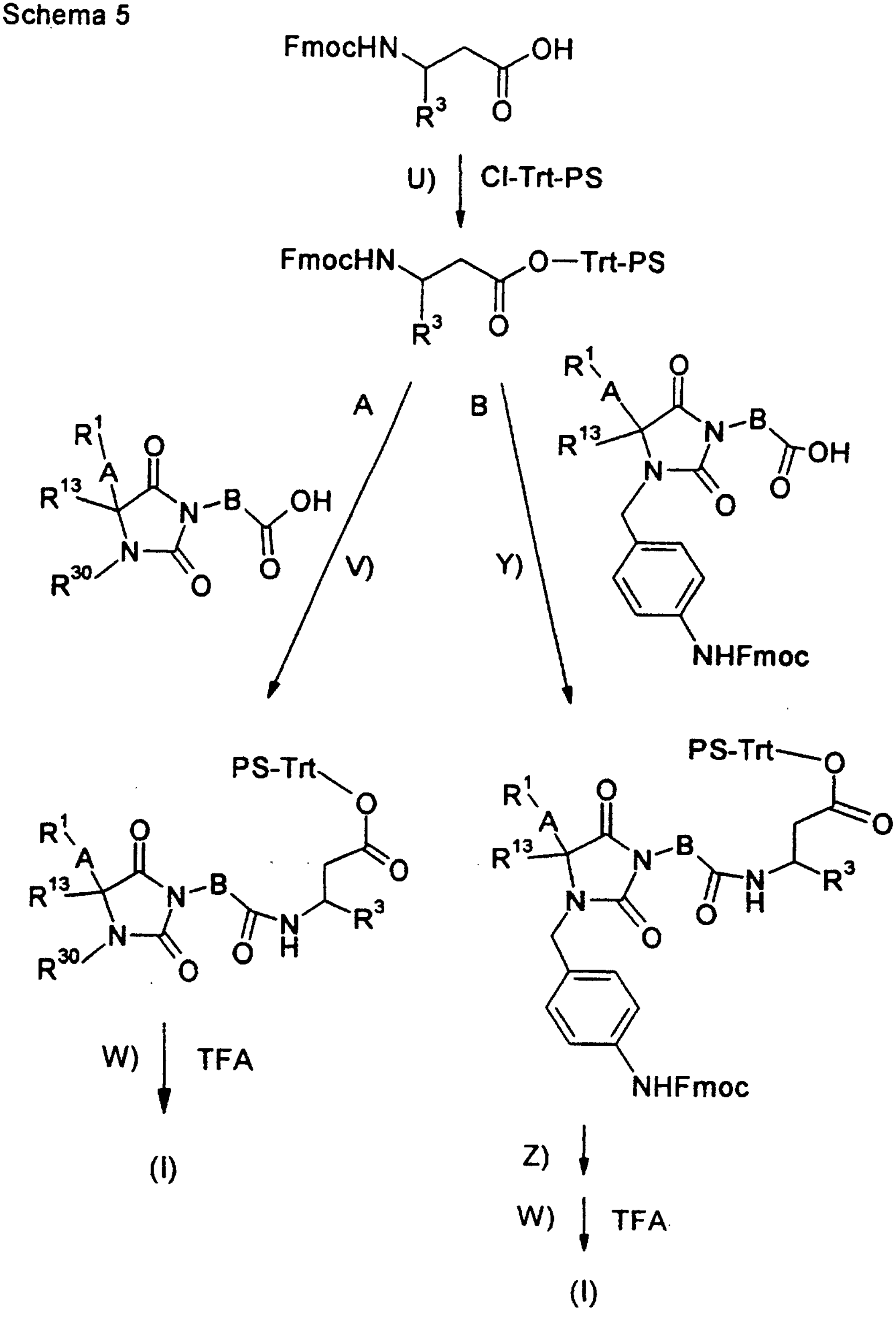


Allgemeines Verfahren zur Einführung der Fmoc-Schutzgruppe in β-Aminosäuren

4.0 mmol der β-Aminosäure und 0.66 g (8.0 mmol) Natriumhydrogencarbonat wurden mit 7 ml Wasser versetzt. Eine Lösung von 1.5 g (4.0 mmol) N-(9-Fluorenylmethoxycarbonyloxy)-succinimid in 15 ml Dioxan wurde zupipettiert und das Gemisch 6 Stunden bei Raumtemperatur gerührt. Dann wurde das Gemisch filtriert und der Rückstand mit 5 ml Ethylacetat nachgewaschen. Der Rückstand wurde in 20 ml 1N Salzsäure aufgenommen und zweimal mit 20

ml Ethylacetat extrahiert. Die vereinigten organischen Phasen wurden über Natriumsulfat getrocknet, filtriert und eingeengt.

Allgemeines Verfahren zur Kupplung der N-Fmoc-β-Aminosäuren an den polymeren Träger; Schritt U

Die Fmoc-geschützen β-Aminosäuren wurden mit Tritylchlorid-Polystyrolharz und 0.5 ml DIPEA in 6 ml DCM suspendiert. Das Gemisch wurde 6 Stunden bei Raumtemperatur geschüttelt. Man gab zu dem Gemisch 1 ml Methanol und schüttelte weitere 30 Minuten bei Raumtemperatur. Das Harz wurde abgesaugt und mehrfach sorgfältig mit DMF und DCM gewaschen. Identität und Reinheit der Verbindungen wurden durch HPLC und MS überprüft. Die Bestimmung der Beladung nach der Fmoc-Methode ergab eine Beladung von 0.2 - 0.3 mmol/g Träger.

Variante A

Allgemeines Verfahren zur Abspaltung der Fmoc-Schutzgruppe am polymeren Träger und zur Kupplung mit Hydantoincarbonsäuren; Schritt V

Man gab zu 100 mg des in Schritt U erhaltenen Harzes 5 ml einer 20%igen Lösung von Piperidin in DMF und ließ das Gemisch 20 Minuten bei Raumtemperatur schütteln. Das Harz wurde abgesaugt und der Vorgang ein weiteres Mal wiederholt.

Danach wurde das Harz mehrfach sorgfältig mit DMF und DCM gewaschen. Dann wurde zu 100 mg des Harzes, das mit der β-Aminosäure beladen war, eine Lösung von 12.2 mg (0.09 mmol) HOBT, 29.5 mg (0.09 mmol) TOTU, 16 μl (0.09 mmol) DIPEA und 0.09 mmol der Hydantoincarbonsäure in 5 ml DMF gegeben und das Gemisch 12 Stunden bei Raumtemperatur geschüttelt. Das Harz wurde abfiltriert und dreimal mit je 10 ml DMF, einmal mit 10 ml Toluol, einmal mit 10 ml Methanol und dreimal mit 10 ml DCM gewaschen.

Allgemeines Verfahren zur Abspaltung vom polymeren Träger; Schritt W

Zur Abspaltung wurde das Harz in 3 ml TFA/DCM suspendiert und 1 Stunde geschüttelt. Das Harz wurde abfiltriert und mit 1 ml DCM gewaschen. Die vereinigten Lösungen wurden im Rotationsverdampfer eingeengt. Der Rückstand wurde in DCM aufgenommen und mit DCM und Ethylacetat über Kieselgel chromatographiert.

Variante B

Allgemeines Verfahren zur Abspaltung der Fmoc-Schutzgruppe am polymeren Träger und zur Kupplung mit N-Fmoc-Aminobenzyl-hydantoincarbonsauren; Schritt Y

Man gab zu 100 mg des in Schritt U erhaltenen Harzes 5 ml einer 20%igen Lösung von Piperidin in DMF und ließ das Gemisch 20 Minuten bei Raumtemperatur schütteln. Das Harz wurde abgesaugt und der Vorgang ein weiteres Mal wiederholt. Danach wurde das Harz mehrfach sorgfältig mit DMF und DCM gewaschen. Zu dem erhaltenen Harz wurde eine Lösung von je 2 Äquivalenten HOBT, TOTU, DIPEA und der N-Fmoc-Aminobenzyl-hydantoincarbonsäure in DMF (10 ml/g Harz) gegeben und das Gemisch 12 Stunden bei Raumtemperatur geschüttelt. Das Harz wurde abfiltriert und dreimal mit je 10 ml DMF, einmal mit 10 ml Toluol, einmal mit 10 ml Methanol und dreimal mit 10 ml DCM gewaschen.

Allgemeines Verfahren zur Abspaltung der Fmoc-Schutzgruppe am polymeren Träger und zur Derivatisierung der Aminogruppe; Schritt Z

Man gab zu 100 mg des mit der N-Fmoc-Aminobenzyl-hydantoincarbonsäure beladenen Harzes 5 ml einer 20%igen Lösung von Piperidin in DMF und ließ das Gemisch 20 Minuten bei Raumtemperatur schütteln. Das Harz wurde abgesaugt und der Vorgang ein weiteres Mal wiederholt. Das Harz wurde danach mehrfach sorgfältig mit DMF und DCM gewaschen. Die erhaltene freie Aminogruppe wurde dann am Harz derivatisiert.

Zur Herstellung von Amiden wurde die entstandene freie Aminogruppe mit Carbonsäuren gekuppelt. Dazu gab man zu 100 mg des mit dem Aminobenzylhydantoin beladenen Harzes eine Lösung von 0.027 mmol HOBT, 0.027 mmol TOTU, 0.027 mmol DIPEA und 0.027 mmol der Carbonsäure in 5 ml DMF und ließ das Gemisch 12 Stunden bei Raumtemperatur schütteln. Das Harz wurde abfiltriert und dreimal mit je 10 ml DMF, einmal mit 10 ml Toluol, einmal mit 10 ml Methanol und dreimal mit 10 ml DCM gewaschen.

Zur Herstellung von Thiohamstoffen wurde die entstandene freie Aminogruppe mit Isothiocyanaten umgesetzt. Dazu gab man zu 100 mg des mit dem Aminobenzylhydantoin beladenen Harzes eine Lösung von 0.027 mmol

des Isothiocyanats und einer katalytischen Menge von 1 mg DMAP in 5 ml DMF und ließ das Gemisch 8 Stunden bei Raumtemperatur schütteln. Das Harz wurde abfiltriert und dreimal mit je 10 ml DMF, einmal mit 10 ml Toluol, einmal mit 10 ml Methanol und dreimal mit 10 ml DCM gewaschen.

Zur Herstellung von Harnstoffen wurde die entstandene freie Aminogruppe mit Isocyanaten umgesetzt. Dazu gab man zu 100 mg des mit dem Aminobenzylhydantoin beladenen Harzes eine Lösung von 0.027 mmol der Isocyanats und einer katalytischen Menge von 1 mg DMAP in 5 ml DMF und ließ das Gemisch 8 Stunden bei Raumtemperatur schütteln. Das Harz wurde abfiltriert und dreimal mit je 10 ml DMF, einmal mit 10 ml Toluol, einmal mit 10 ml Methanol und dreimal mit 10 ml DCM gewaschen.

Zur Herstellung von N,N-disubstituierten Harnstoffen wurde die entstandene freie Aminogruppe zunächst mit Di-(N-succinimidyl)-carbonat und dann mit einem sekundären Amin umgesetzt. Dazu gab man zu 100 mg des mit dem Aminobenzylhydantoin beladenen Harzes einen 10fachen Überschuß Di-(Nsuccinimidyl)-carbonat und DIPEA und schüttelte 5 Stunden bei 40 °C. Die Lösung wurde abgesaugt. Zum Harz wurde ein 10facher Überschuß des Amins in DMF gegeben. Das Gemisch wurde 8 Stunden bei Raumtemperatur geschüttelt. Das Harz wurde abfiltriert und dreimal mit je 10 ml DMF, einmal mit 10 ml Toluol, einmal mit 10 ml Methanol und dreimal mit 10 ml DCM gewaschen.

Die Abspaltung vom polymeren Träger (Schritt W) wurde in der Variante B wie in der Variante A durchgeführt.

**F) Allgemeines Verfahren zur Herstellung von Verbindungen der Formel I, die eine Peptideinheit enthalten, durch Festphasensynthese**

Verbindungen der Formel I, die eine Peptideinheit enthalten, können hergestellt werden, indem zunächst an den polymeren Träger die C-terminale N-Fmoc-α-Aminosäure angeknüpft wird und die Fmoc-Schutzgruppe abgespalten wird. Die freigesetzte Aminofunktion wird dann mit einer weiteren N-Fmoc-Aminosäure gekuppelt und die Fmoc-Schutzgruppe abgespalten. Diese Anknüpfung weiterer Aminosäureeinheiten wird wiederholt, bis die gewünschte Peptideinheit aufgebaut war. Schließlich wird unter Verwendung eines Kupplungsreagenzes eine Hydantoincarbonsäure angeknüpft, das Produkt vom Harz abgespalten und eventuell vorhandene Schutzgruppen abgespalten. Die einzelnen Schritte werden wie folgt durchgeführt.

Allgemeines Verfahren zur Kupplung von N-Fmoc-α-Aminosäuren an den polymeren Träger

Die Fmoc-geschütze α-Aminosäure (1.5 Äquivalente) wird mit Tritylchlorid-Polystyrolharz (1.2 mmol/g) und DIPEA (2 Äquivalente) in DCM (5 ml/g Träger) suspendiert. Das Gemisch wird 6 Stunden bei Raumtemperatur geschüttelt. Man gibt zu dem Gemisch 1 ml Methanol und schüttelt weitere 30 Minuten bei Raumtemperatur. Das Harz wird abgesaugt und mehrfach sorgfältig mit DMF und DCM gewaschen. Identität und Reinheit der Verbindungen werden durch HPLC und MS überprüft.

Allgemeines Verfahren zur Abspaltung der Fmoc-Schutzgruppe am polymeren Träger

Man gibt zu 100 mg des mit der N-Fmoc-α-Aminosäure beladenen Harzes 5 ml einer 20%igen Lösung von Piperidin in DMF und läßt das Gemisch 20 Minuten bei Raumtemperatur schütteln. Das Harz wird abgesaugt und der Vorgang ein weiteres Mal wiederholt. Das Harz wird danach mehrfach sorgfältig mit DMF und DCM gewaschen.

Allgemeines Verfahren zur Kupplung der α-Aminosäuren am polymeren Träger mit N-Fmoc-α-Aminosäuren

Man gibt zu 100 mg des mit der α-Aminosäure beladenen Harzes eine Lösung von 12.2 mg (0.09 mmol) HOBT, 29.5 mg (0.09 mmol) TOTU, 16 µl (0.09 mmol) DIPEA und 0.09 mmol der N-Fmoc-α-Aminosäure in 5 ml DMF und läßt das Gemisch 12 Stunden bei Raumtemperatur schütteln. Das Harz wird abfiltriert und dreimal mit je 10 ml DMF, einmal mit 10 ml Toluol, einmal mit 10 ml Methanol und dreimal mit 10 ml DCM gewaschen.

Zur Einführung weiterer Aminosäuren in die Peptideinheit werden die beiden vorstehenden Schritte (Abspaltung der Fmoc-Schutzgruppe und Kupplung mit einer weiteren N-Fmoc-α-Aminosäure) entsprechend wiederholt.

Allgemeines Verfahren zur Abspaltung der Fmoc-Schutzgruppe am polymeren Träger und zur Kupplung der Peptideinheit am polymeren Träger mit Hydantoincarbonsäuren

Die Fmoc-Gruppe der am Harz aufgebauten Peptideinheit wird wie vorstehend beschrieben abgespalten. Dann gibt man zu 100 mg des mit der Peptideinheit beladenen Harzes eine Lösung von 12.2 mg (0.09 mmol) HOBT, 29.5 mg (0.09 mmol) TOTU, 16 µl (0.09 mmol) DIPEA und 0.09 mmol der Hydantoincarbonsäure in 5 ml DMF und läßt das Gemisch 12 Stunden bei Raumtemperatur schütteln. Das Harz wird abfiltriert und dreimal mit je 10 ml DMF, einmal mit 10 ml Toluol, einmal mit 10 ml Methanol und dreimal mit 10 ml DCM gewaschen.

Allgemeines Verfahren zur Abspaltung vom Harz

Zur Abspaltung der Verbindung vom Harz wird ein Gemisch von TFA und DCM (1:9) zum Harz zugegeben. Die Suspension wird 1 Stunde geschüttelt. Das Harz wird abfiltriert. Die verbleibende Lösung wird im Vakuum eingeengt und der Rückstand durch Kieselgelchromatographie gereinigt.

**G) Allgemeines Verfahren zur Herstellung von unsubstituierten Carbonsäureamiden an der Festphase**

Zur Überführung von Verbindungen der Formel I, die eine Carbonsäuregruppe -COOH enthalten, in die entsprechenden Verbindungen mit einer unsubstituierten Carbonsäureamidgruppe -$CONH_2$ wurde die Carbonsäuregruppe unter Verwendung eines Kupplungsreagenzes an Rink-Amidharz angeknüpft. Die Anknüpfung an die Aminofunktion im Harz wurde analog der Vorschrift für die Anknüpfung von Carbonsäuren an Wang-Harz durchgeführt (siehe Verfahren gemäß Schema 4). Abspaltung mit TFA ergab dann die unsubstituierten Amide.

$$\text{-CO-OH + Rink-Amidharz} \rightarrow \text{-CO-Rink-Amidharz} \rightarrow \text{-CO-NH}_2$$

Im einzelnen wurden 0.5 g der Carbonsäure der Formel I mit 0.35 g TOTU, 0.15 ml DIPEA und 2 g Rink-Amidharz in 10 ml DMF umgesetzt. Die Suspension wurde 1 Stunde bei Raumtemperatur geschüttelt. Das Harz wurde abgesaugt und mit DMF und DCM sorgfältig gewaschen. Anschließend wurde mit 5 ml TFA/DCM (1:1) die Abspaltung vorgenommen. Nach Enfemen des Lösungsmittels wurde der Rückstand gereinigt.

Beispiel 1

((RS)-2-((RS)-4-Phenyl-3-(4-(3-phenylureido)-benzyl)-4-methyl-2,5-dioxoimidazolidin-1-yl)-2-(2-methylpropyl)-acetyl)-L-aspartyl-L-phenylglycin

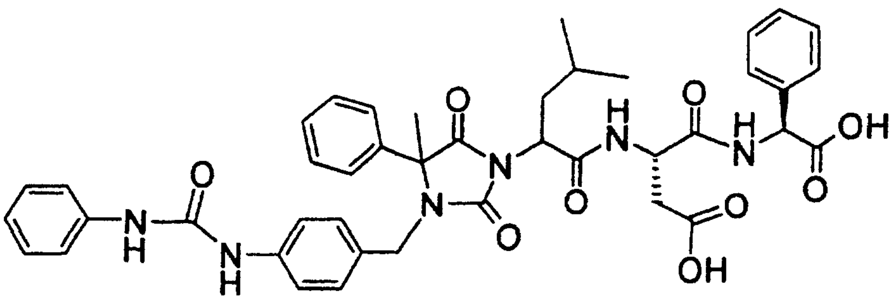

Die Verbindung wurde nach dem Verfahren gemäß Schema 1, Schritte C, D (Methode 1), E, F, G, H (Methode 1), J (Methode 1) hergestellt. Im Schritt H (Ansatzgröße 0.3 mmol) wurde als Aminoverbindung der Formel III H-Asp (OtBu)-Phg-OtBu (Hydrochlorid; Asp = Aspartyl, Phg = Phenylglycyl) eingesetzt. Ausbeute: 52 mg.
ES(+)-MS: 777.9 $(M+H)^+$

Beispiel 2

((RS)-2-((RS)-4-Phenyl-3-(4-(3-(2-methylphenyl)-ureido)-benzyl)-4-methyl-2,5-dioxoimidazolidin-1-yl)-2-(2-methylpropyl)-acetyl)-L-aspartyl-L-phenylglycin

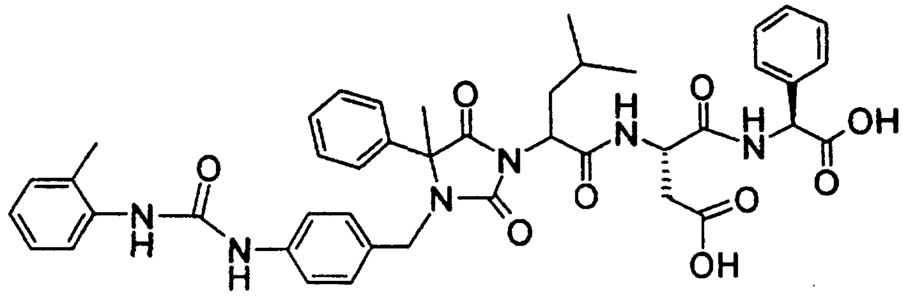

Die Verbindung wurde nach dem Verfahren gemäß Schema 1, Schritte C, D (Methode 1), E, F, G, H (Methode 1), J (Methode 1) hergestellt. Im Schritt H (Ansatzgröße 0.184 mmol) wurde als Aminoverbindung der Formel III H-Asp (OtBu)-Phg-OtBu (Hydrochlorid) eingesetzt. Ausbeute: 59 mg.
ES(+)-MS: 791.9 $(M+H)^+$

Beispiel 3

(S)-3-((RS)-2-((RS)-4-Phenyl-3-(4-(3-(2-methylphenyl)-ureido)-benzyl)-4-methyl-2,5-dioxoimidazolidin-1-yl)-2-(2-methylpropyl)-acetylamino)-3-(3,4-methylendioxyphenyl)-propionsäure

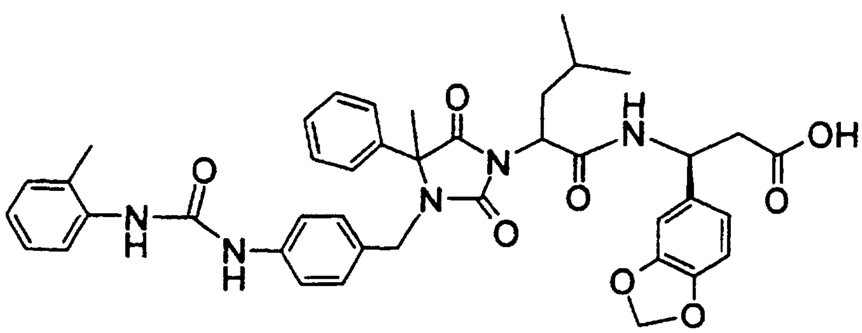

Die Verbindung wurde nach dem Verfahren gemäß Schema 1, Schritte C, D (Methode 1), E, F, G, H (Methode 1), J (Methode 1) hergestellt. Im Schritt H (Ansatzgröße 0.184 mmol) wurde als Aminoverbindung der Formel III der (S)-3-Amino-3-(3,4-methylendioxyphenyl)-propionsäure-tert-butylester eingesetzt. Ausbeute: 92 mg.
ES(+)-MS: 734.9 $(M+H)^+$

Beispiel 4

(R)-3-((RS)-2-((RS)-4-Phenyl-3-(4-(3-(2-methylphenyl)-ureido)-benzyl)-4-methyl-2,5-dioxoimidazolidin-1-yl)-2-(2-methylpropyl)-acetylamino)-3-methyl-propionsäure

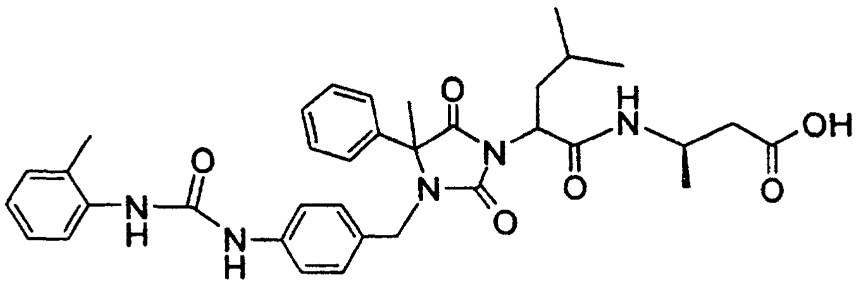

Die Verbindung wurde nach dem Verfahren gemäß Schema 1, Schritte C, D (Methode 1), E, F, G, H (Methode 1), J (Methode 1) hergestellt. Im Schritt H (Ansatzgröße 0.184 mmol) wurde als Aminoverbindung der Formel III der (R)-3-Amino-3-methyl-propionsäure-tert-butylester eingesetzt. Ausbeute: 109 mg.
ES(+)-MS: 628.4 $(M+H)^+$

Beispiel 5

(S)-3-((S)-2-(4,4-Dimethyl-3-(4-(3-phenylureido)-benzyl)-2,5-dioxoimidazolidin-1-yl)-2-(2-methylpropyl)-acetylamino)-3-(3,4-methylendioxy-phenyl)-propionsäure

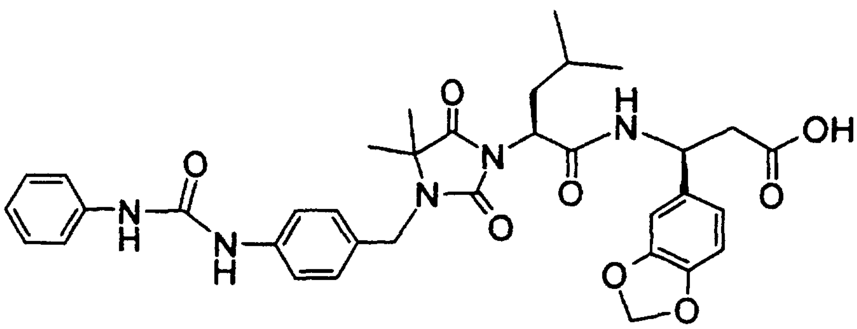

Die Verbindung wurde nach dem Verfahren gemäß Schema 1, Schritte A, B, D (Methode 1), E, F, G, H (Methode 1), J (Methode 1) hergestellt. Im Schritt H (Ansatzgröße 2.6 mmol) wurde als Aminoverbindung der Formel III der (S)-3-Amino-3-(3,4-methylendioxyphenyl)-propionsäure-tert-butylester eingesetzt. Ausbeute: 284 mg.
ES(+)-MS: 658.7 $(M+H)^+$

Beispiel 6

(R)-3-((S)-2-(4,4-Dimethyl-3-(4-(3-phenylureido)-benzyl)-2,5-dioxoimidazolidin-1-yl)-2-(2-methylpropyl)-acetylamino)-3-methyl-propionsäure

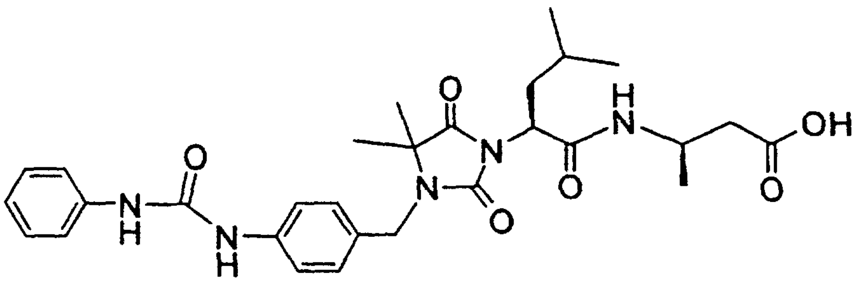

Die Verbindung wurde nach dem Verfahren gemäß Schema 1, Schritte A, B, D (Methode 1), E, F, G, H (Methode 1), J (Methode 1) hergestellt. Im Schritt H (Ansatzgröße 2.6 mmol) wurde als Aminoverbindung der Formel III der (R)-3-Amino-3-methyl-propionsäure-tert-butylester eingesetzt. Ausbeute: 451 mg.
ES(+)-MS: 552.6 $(M+H)^+$

Die Verbindung des Beispiels 6 wurde auch nach dem Verfahren gemäß Schema 1, Schritte A, B, D (Methode 2), E, F, G, H (Methode 1), J (Methode 1) hergestellt.

Die Verbindung des Beispiels 6 wurde auch nach dem Verfahren gemäß Schema 2 hergestellt.

Beispiel 7

(S)-3-((S)-2-(4,4-Dimethyl-3-(4-(3-(2-methylphenyl)-ureido)-benzyl)-2,5-dioxoimidazolidin-1-yl)-2-(2-methylpropyl)-acetylamino)-3-(3,4-methylendioxyphenyl)-propionsäure

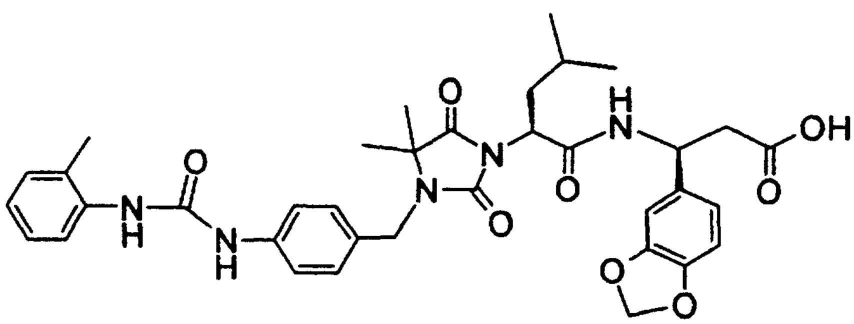

Die Verbindung wurde nach dem Verfahren gemäß Schema 1, Schritte A, B, D (Methode 1), E, F, G, H (Methode 1), J (Methode 1) hergestellt. Im Schritt H (Ansatzgröße 2.3 mmol) wurde als Aminoverbindung der Formel III der (S)-3-Amino-3-(3,4-methylendioxyphenyl)-propionsäure-tert-butylester eingesetzt. Ausbeute: 453 mg.
ES(+)-MS: 672.7 $(M+H)^+$

Beispiel 8

(R)-3-((S)-2-(4,4-Dimethyl-3-(4-(3-(2-methylphenyl)-ureido)-benzyl)-2,5-dioxoimidazolidin-1-yl)-2-(2-methylpropyl)-acetylamino)-3-methyl-propionsäure

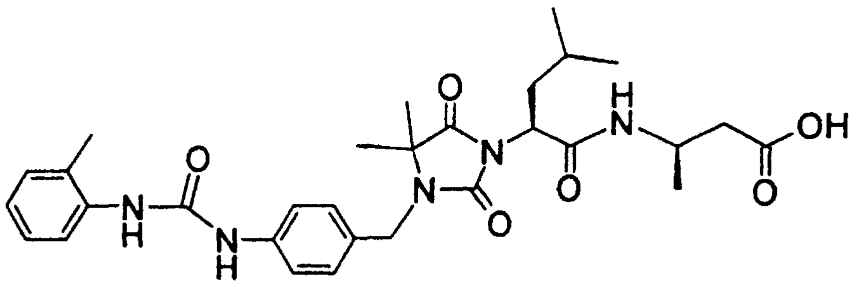

Die Verbindung wurde nach dem Verfahren gemäß Schema 1, Schritte A, B, D (Methode 1), E, F, G, H (Methode 1), J (Methode 1) hergestellt. Im Schritt H (Ansatzgröße 2.3 mmol) wurde als Aminoverbindung der Formel III der (R)-3-Amino-3-methyl-propionsäure-tert-butylester eingesetzt. Ausbeute: 420 mg.
ES(+)-MS: 566.7 $(M+H)^+$

Beispiel 9

(R)-3-(2-(4,4-Dimethyl-3-(4-(3-(2-methylphenyl)-ureido)-benzyl)-2,5-dioxoimidazolidin-1-yl)-acetylamino)-3-methyl-propionsäure

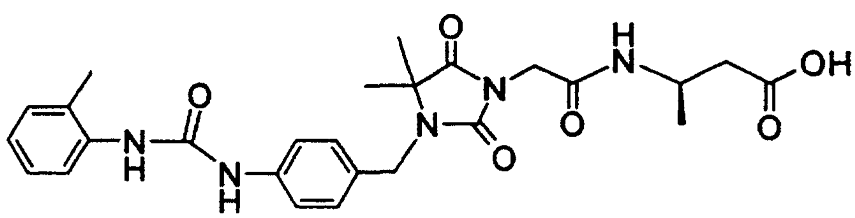

Die Verbindung wurde nach dem Verfahren gemäß Schema 1, Schritte C, D (Methode 1), E, F, G, H (Methode 2), J (Methode 1) hergestellt. Im Schritt H (Ansatzgröße 1.5 mmol) wurde als Aminoverbindung der Formel III der.(R)-3-Amino-3-methyl-propionsäure-tert-butylester eingesetzt. Ausbeute: 440 mg.
ES(+)-MS: 510.6 $(M+H)^+$

Beispiel 10

2-((S)-2-(4,4-Dimethyl-3-(4-(3-(2-methylphenyl)-ureido)-benzyl)-2,5-dioxoimidazolidin-1-yl)-2-(2-methylpropyl)-acetylamino)-essigsäure

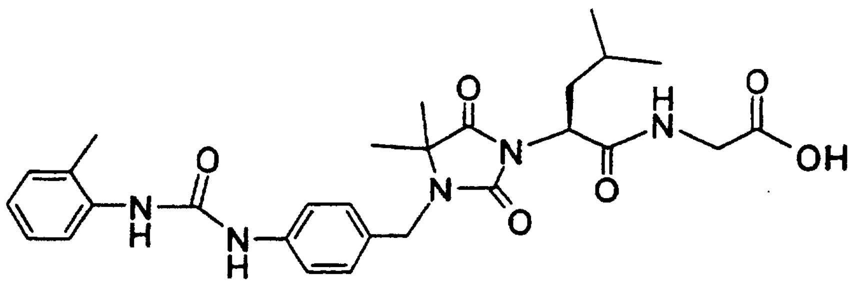

Die Verbindung wurde nach dem Verfahren gemäß Schema 1, Schritte A, B, D (Methode 1), E, F, G, H (Methode 2), J (Methode 2) hergestellt. Im Schritt H (Ansatzgröße 0.21 mmol) wurde als Aminoverbindung der Formel III Glycinmethylester eingesetzt. Ausbeute: 26 mg.
ES(+)-MS: 538.4 $(M+H)^+$

Beispiel 11

(S)-3-(2-(4,4-Dimethyl-3-(4-(3-(2-methylphenyl)-ureldo)-benzyl)-2,5-dioxoimidazolidin-1-yl)-acetylamino)-3-phenylpropionsäure

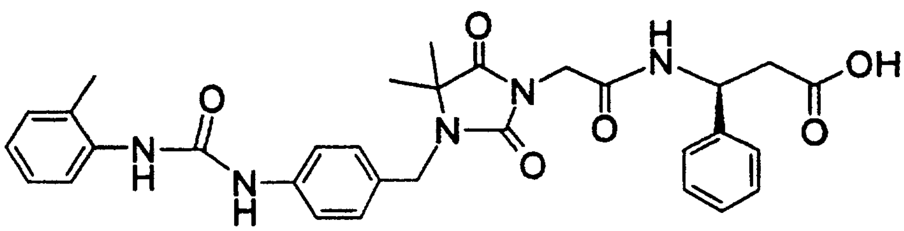

Die Verbindung wurde nach dem Verfahren gemäß Schema 1, Schritte C, D (Methode 1), E, F, G, H (Methode 2), J (Methode 2) hergestellt. Im Schritt H (Ansatzgröße 1.41 mmol) wurde als Aminoverbindung der Formel III der (S)-3-Amino-3-phenyl-propionsäure-ethylester eingesetzt. Ausbeute: 534 mg.
ES(+)-MS: 572.4 $(M+H)^+$
Die Verbindung des Beispiels 11 wurde auch nach dem Verfahren gemäß Schema 1, Schritte C, D (Methode 2), E, F, G, H (Methode 2), J (Methode 2) hergestellt.

Beispiel 12

(R)-3-((S)-2-(4,4-Dimethyl-3-(2-(3-(2-methylphenyl)-ureido)-ethyl)-2,5-dioxoimidazolidin-1-yl)-2-(2-methylpropyl)-acetylamino)-3-methyl-propionsäure

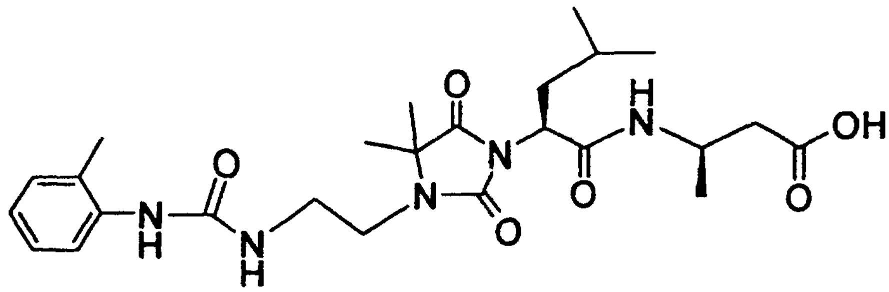

Die Verbindung wurde nach dem Verfahren gemäß Schema 3 hergestellt (Schritt J nach Methode 1). Die Herstellung der Verbindung der Formel VIa erfolgte gemäß Schema 1, Schritte A, B. Im Schritt H (Ansatzgröße 0.19 mmol) wurde als Aminoverbindung der Formel III der (R)-3-Amino-3-methyl-propionsäure-tertbutylester eingesetzt.
Ausbeute: 58 mg.
ES(+)-MS: 504.4 $(M+H)^+$

Beispiel 13

(R)-3-((S)-2-(4,4-Dimethyl-3-(3-(3-(2-methylphenyl)-ureido)-propyl)-2,5-dioxoimidazolldin-1-yl)-2-(2-methylpropyl)-acetylamino)-3-methyl-propionsäure

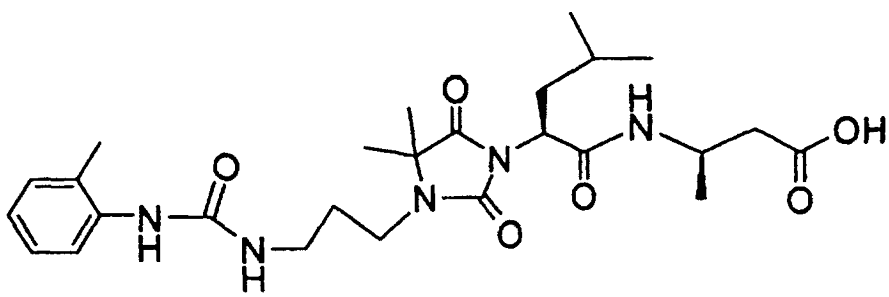

Die Verbindung wurde nach dem Verfahren gemäß Schema 3 hergestellt (Schritt J nach Methode 1). Die Herstellung der Verbindung der Formel VIa erfolgte gemäß Schema 1, Schritte A, B. Im Schritt H (Ansatzgröße 0.25 mmol) wurde als Aminoverbindung der Formel III der (R)-3-Amino-3-methyl-propionsäure-tertbutylester eingesetzt.
Ausbeute: 54 mg.
ES(+)-MS: 518.4 $(M+H)^+$

Beispiel 14

(R)-3-(2-(4,4-Dimethyl-3-(4-(3-(2-fluorphenyl)-ureido)-benzyl)-2,5-dioxoimidazolidin-1-yl)-acetylamino)-3-methyl-propionsäure

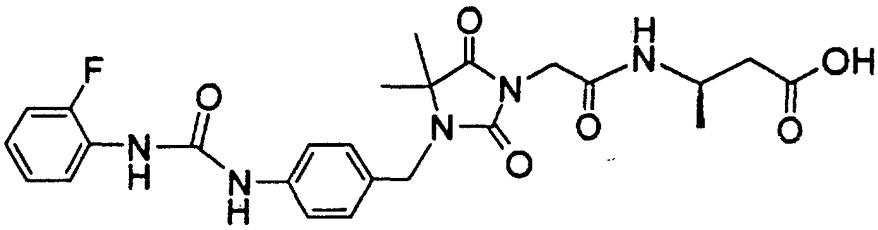

Die Verbindung wurde nach dem Verfahren gemäß Schema 1, Schritte C, D (Methode 1), E, F, G, H (Methode 2), J (Methode 1) hergestellt. Im Schritt H (Ansatzgröße 1.94 mmol) wurde als Aminoverbindung der Formel III der (R)-3-Amino-3-methyl-propionsäure-tert-butylester eingesetzt. Ausbeute: 414 mg.
ES(+)-MS: 514.3 $(M+H)^+$

Beispiel 15

3-(2-(4,4-Dimethyl-3-(4-(3-(2-methylphenyl)-ureido)-benzyl)-2,5-dioxoimidazolidin-1-yl)-acetylamino)-propionsäure

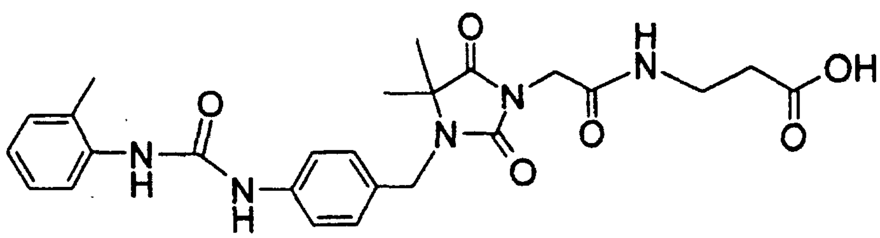

Die Verbindung wurde nach dem Verfahren gemäß Schema 1, Schritte C, D (Methode 1), E, F, G, H (Methode 2), J (Methode 2) hergestellt. Im Schritt H (Ansatzgröße 0.47 mmol) wurde als Aminoverbindung der Formel III der 3-Aminopropionsäure-methylester eingesetzt. Ausbeute: 136 mg.
ES(+)-MS: 496.2 $(M+H)^+$

Beispiel 16

3-((S)-2-(4,4-Dimethyl-3-(4-(3-(2-methylphenyl)-ureido)-benzyl)-2,5-dioxoimidazolidin-1-yl)-2-(2-methylpropyl)-acetylamino)-propionsäure

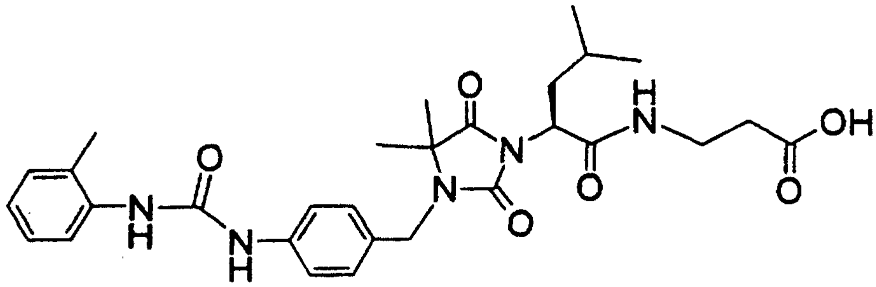

Die Verbindung wurde nach dem Verfahren gemäß Schema 1, Schritte A, B, D (Methode 1), E, F, G, H (Methode 1), J (Methode 2) hergestellt. Im Schritt H (Ansatzgröße 0.21 mmol) wurde als Aminoverbindung der Formel III der 3-Aminopropionsäure-methylester eingesetzt. Ausbeute: 23 mg.
ES(+)-MS: 552.3 $(M+H)^+$

Beispiel 17

(S)-3-((S)-2-(4,4-Dimethyl-3-(4-(3-(2-methylphenyl)-ureido)-benzyl)-2,5-dioxoimidazolidin-1-yl)-2-(2-methylpropyl)-acetylamino)-3-phenyl-propionsäure

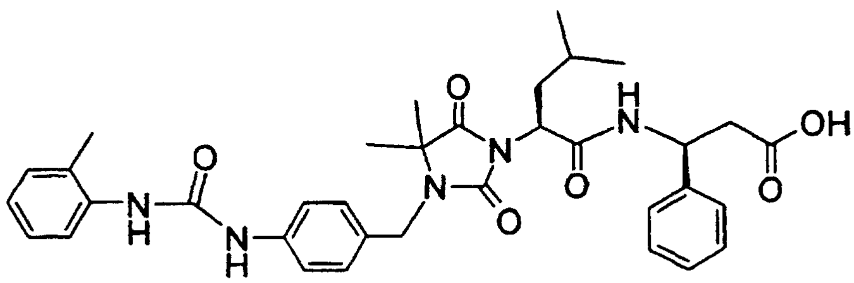

Die Verbindung wurde nach dem Verfahren gemäß Schema 1, Schritte A, B, D (Methode 1), E, F, G, H (Methode 2), J (Methode 2) hergestellt. Im Schritt H (Ansatzgröße 0.208 mmol) wurde als Aminoverbindung der Formel III der (S)-3-Amino-3-phenyl-propionsäure-ethylester eingesetzt. Ausbeute: 66 mg.
ES(+)-MS: 628.4 $(M+H)^+$

Beispiel 18

3-(2-(4,4-Dimethyl-3-(4-(3-(2-fluorphenyl)-ureido)-benzyl)-2,5-dioxoimidazolidin-1-yl)-acetylamino)-propionsäure

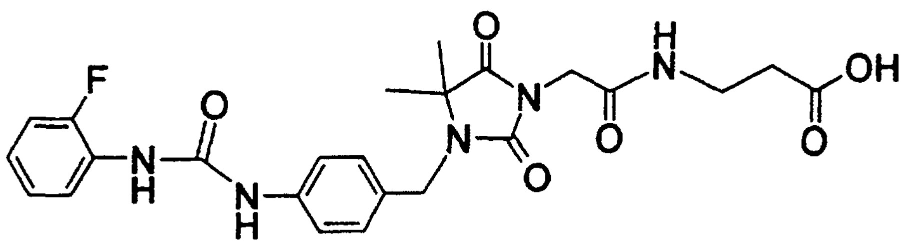

Die Verbindung wurde nach dem Verfahren gemäß Schema 1, Schritte C, D (Methode 1), E, F, G, H (Methode 2), J (Methode 2) hergestellt. Im Schritt H (Ansatzgröße 1.94 mmol) wurde als Aminoverbindung der Formel III 3-Aminopropionsäure-ethylester-hydrochlorid eingesetzt. Ausbeute: 368 mg.
ES(+)-MS: 500.2 $(M+H)^+$

Beispiel 19

(S)-3-((S)-2-(4,4-Dimethyl-3-(4-(3-phenylureido)-benzyl)-2,5-dioxoimidazolidin-1-yl)-2-(2-methylpropyl)-acetylamino)-3-phenyl-propionsäure

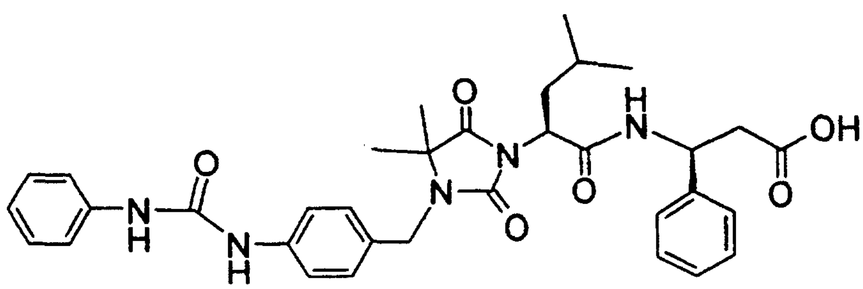

Die Verbindung wurde nach dem Verfahren gemäß Schema 1, Schritte A, B, D (Methode 1), E, F, G, H (Methode 2), J (Methode 2) hergestellt. Im Schritt H (Ansatzgröße 4.11 mmol) wurde als Aminoverbindung der Formel III der (S)-3-Amino-3-phenyl-propionsäure-ethylester eingesetzt. Ausbeute: 1 g.
ES(+)-MS: 614.3 $(M+H)^+$

Beispiel 20

(2-(3-(4-(3-(2-Methylphenyl)-ureido)-benzyl)-2,5-dioxoimidazolidin-1-yl)-acetyl)-N-methyl-L-aspartyl-(2-adamantyl-amid)

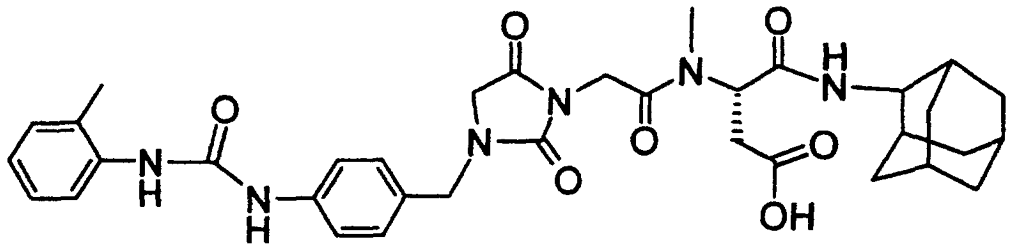

Die Verbindung wurde nach dem Verfahren gemäß Schema 1, Schritte C, D (Methode 1), E, F, G, H (Methode 2), J (Methode 1) hergestellt. Im Schritt H (Ansatzgröße 1.26 mmol) wurde als Aminoverbindung der Formel III N-Methyl-L-asparaginsäure-(2-adamantylamid)-tert-butylester-hydrochlorid eingesetzt. Ausbeute: 617 mg.
ES(+)-MS: 659.4 $(M+H)^+$

Beispiel 21

(2-(3-(4-(3-(2-Methylphenyl)-ureido)-benzyl)-2,5-dioxoimidazolidin-1-yl)-acetyl)-L-aspartyl-(2-adamantylamid)

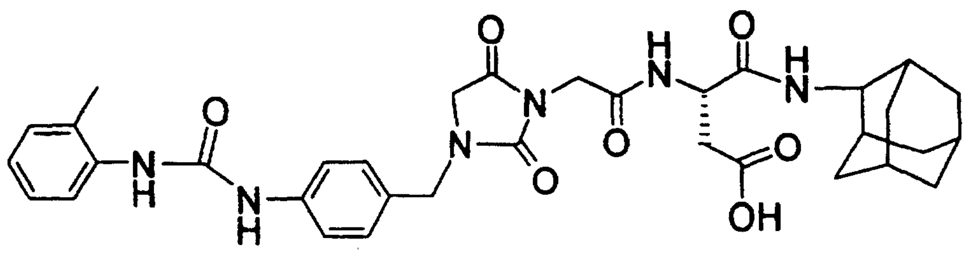

Die Verbindung wurde nach dem Verfahren gemäß Schema 1, Schritte C, D (Methode 1), E, F, G, H (Methode 2), J (Methode 1) hergestellt. Im Schritt H (Ansatzgröße 0.882 mmol) wurde als Aminoverbindung der Formel III L-Asparaginsäure-(2-adamantylamid)-tert-butylester eingesetzt. Ausbeute: 470 mg.
ES(+)-MS: 645.4 $(M+H)^+$

Beispiel 22

(2-(4,4-Dimethyl-3-(4-(3-(2-methylphenyl)-ureido)-benzyl)-2,5-dioxoimidazolidin-1-yl)-acetyl)-N-methyl-L-aspartyl-(2-adamantylamid)

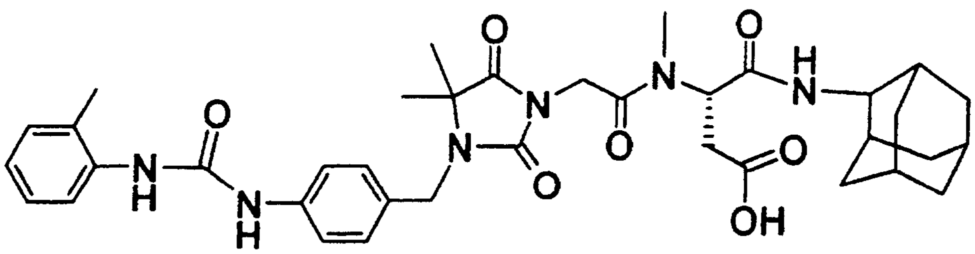

Die Verbindung wurde nach dem Verfahren gemäß Schema 1, Schritte C, D (Methode 1), E, F, G, H (Methode 2), J (Methode 1) hergestellt. Im Schritt H (Ansatzgröße 0.942 mmol) wurde als Aminoverbindung der Formel III N-Methyl-L-asparaginsäure-(2-adamantylamid)-tert-butylester-hydrochlorid eingesetzt.
Ausbeute: 535 mg.
ES(+)-MS: 687.4 $(M+H)^+$

Beispiel 23

(2-(4,4-Dimethyl-3-(4-(3-phenylureido)-benzyl)-2,5-dioxoimidazolidin-1-yl)-acetyl)-N-methyl-L-aspartyl-(2-adamantylamid)

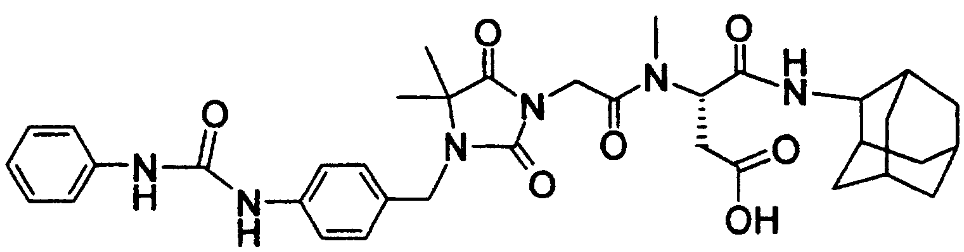

Die Verbindung wurde nach dem Verfahren gemäß Schema 1, Schritte C, D (Methode 1), E, F, G, H (Methode 2), J (Methode 1) hergestellt. Im Schritt H (Ansatzgröße 1.41 mmol) wurde als Aminoverbindung der Formel III N-Methyl-L-asparaginsäure-(2-adamantylamid)-tert-butylester-hydrochlorid eingesetzt.
Ausbeute: 599 mg.
ES(+)-MS: 673.4 $(M+H)^+$

Beispiel 24

(2-(4,4-Dimethyl-3-(4-(3-phenylureido)-benzyl)-2,5-dioxoimidazolidin-1-yl)-acetyl)-L-aspartyl-(2-adamantylamid)

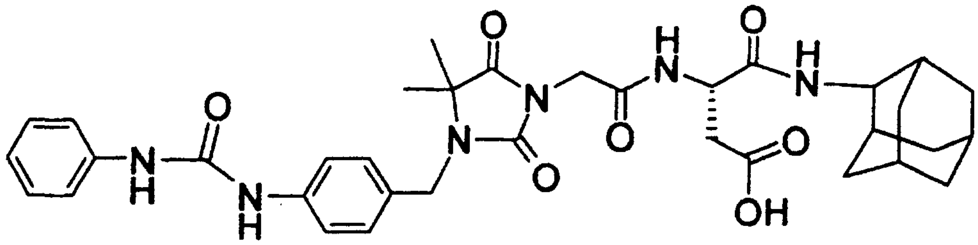

Die Verbindung wurde nach dem Verfahren gemäß Schema 1, Schritte C, D (Methode 1), E, F, G, H (Methode 2), J (Methode 1) hergestellt. Im Schritt H (Ansatzgröße 0.974 mmol) wurde als Aminoverbindung der Formel III L-Asparaginsäure-(2-adamantylamid)-tert-butylester eingesetzt. Ausbeute: 410 mg.
ES(+)-MS: 659.4 $(M+H)^+$

Beispiel 25

((S)-2-(4,4-Dimethyl-3-(4-(3-phenylureido)-benzyl)-2,5-dioxoimidazolidin-1-yl)-2-(2-methylpropyl)-acetyl)-L-aspartyl-(2-adamantylamid)

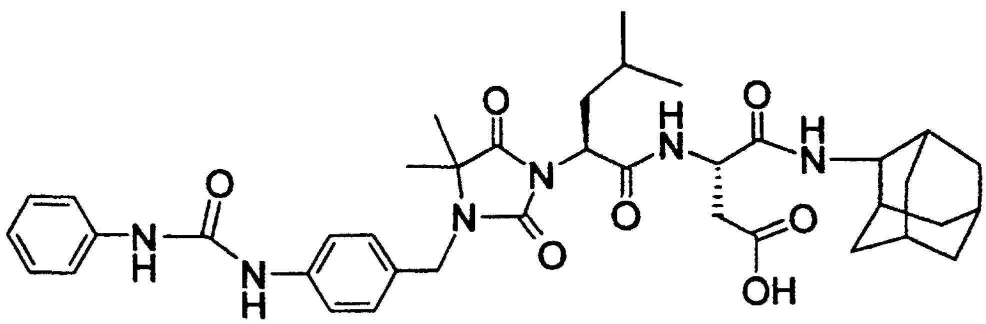

Die Verbindung wurde nach dem Verfahren gemäß Schema 1, Schritte A, B, D (Methode 1), E, F, G, H (Methode 2), J (Methode 1) hergestellt. Im Schritt H (Ansatzgröße 1.28 mmol) wurde als Aminoverbindung der Formel III L-Asparaginsäure-(2-adamantylamid)-tert-butylester eingesetzt. Ausbeute: 576 mg.
ES(+)-MS: 715.5 $(M+H)^+$

Beispiel 26

(R)-3-(2-(3-(4-(3-(2-Methylphenyl)-ureido)-benzyl)-2,5-dioxoimidazolidin-1-yl)-acetylamino)-3-methyl-propionsäure

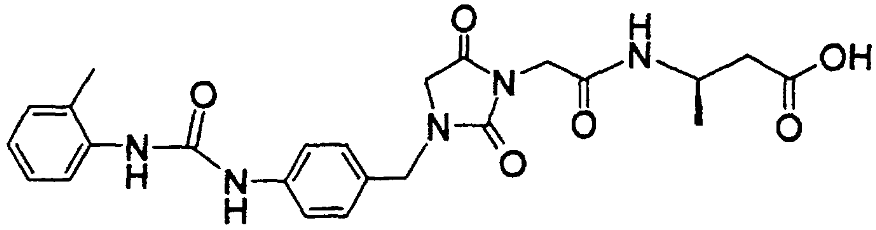

Die Verbindung wurde nach dem Verfahren gemäß Schema 1, Schritte C, D (Methode 1), E, F, G, H (Methode 2), J (Methode 1) hergestellt. Im Schritt H (Ansatzgröße 1.5 mmol) wurde als Aminoverbindung der Formel III der (R)-3-Amino-3-methyl-propionsäure-tert-butylester eingesetzt. Ausbeute: 7 mg.
ES(+)-MS: 482.3 $(M+H)^+$

Beispiel 27

((S)-2-(4,4-Dimethyl-3-(4-(3-(2-methylphenyl)-ureido)-benzyl)-2,5-dioxoimidazolidin-1-yl)-2-(2-methylpropyl)-acetyl)-L-asparaginsäure

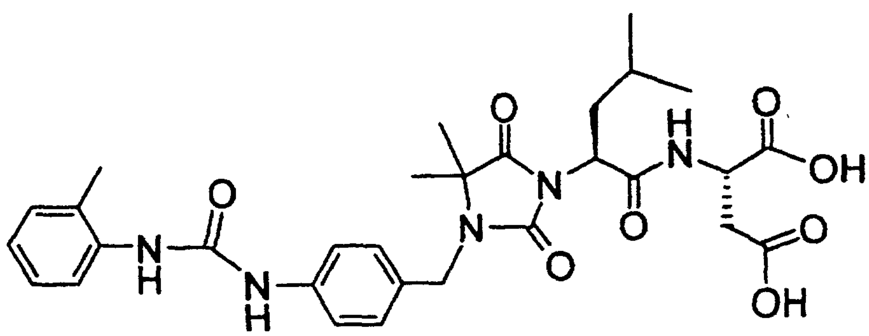

Die Verbindung wurde nach dem Verfahren gemäß Schema 1, Schritte A, B, D (Methode 1), E, F, G, H (Methode 2), J (Methode 1) hergestellt. Im Schritt H (Ansatzgröße 4.2 mmol) wurde als Aminoverbindung der Formel III L-Asparaginsäure-di-tert-butylester-hydrochlorid eingesetzt. Ausbeute: 692 mg.
ES(+)-MS: 596.4 $(M+H)^+$

Beispiel 28

(2-(4,4-Dimethyl-3-(4-(3-(2-methylphenyl)-ureido)-benzyl)-2,5-dioxoimidazolidin-1-yl)-acetyl)-N-methyl-L-asparaginsäure

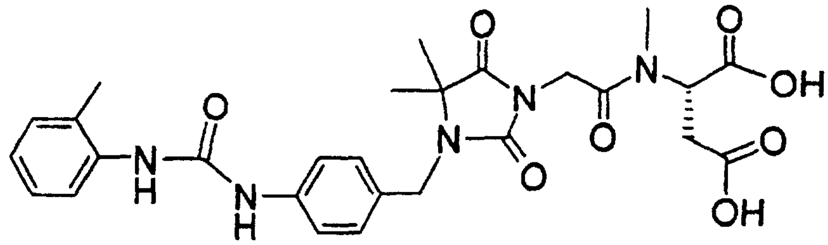

Die Verbindung wurde nach dem Verfahren gemäß Schema 1, Schritte C, D (Methode 1), E, F, G, H (Methode 2), J (Methode 1) hergestellt. Im Schritt H (Ansatzgröße 4.7 mmol) wurde als Aminoverbindung der Formel III N-Methyl-L-asparaginsäure-di-tert-butylester-hydrochlorid eingesetzt. Ausbeute: 628 mg.
ES(+)-MS: 554.3 $(M+H)^+$

Beispiel 29

(S)-3-(2-(3-(4-(3-(2-Methylphenyl)-ureido)-benzyl)-2,5-dioxoimidazolidin-1-yl)-acetylamino)-3-phenyl-propionsäure

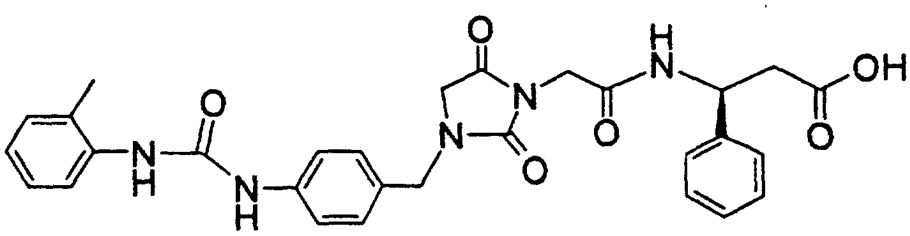

Die Verbindung wurde nach dem Verfahren gemäß Schema 1, Schritte C, D (Methode 1), E, F, G, H (Methode 2), J (Methode 2) hergestellt. Im Schritt H (Ansatzgröße 1.5 mmol) wurde als Aminoverbindung der Formel III (S)-3-Amino-3-phenyl-propionsäure-ethylester eingesetzt. Ausbeute: 59 mg.
ES(+)-MS: 544.3 $(M+H)^+$

Beispiel 30

(R)-3-(2-(3-(4-(3-(2-Chlorphenyl)-ureido)-benzyl)-2,5-dioxoimidazolidin-1-yl)-acetylamino)-3-methyl-propionsäure

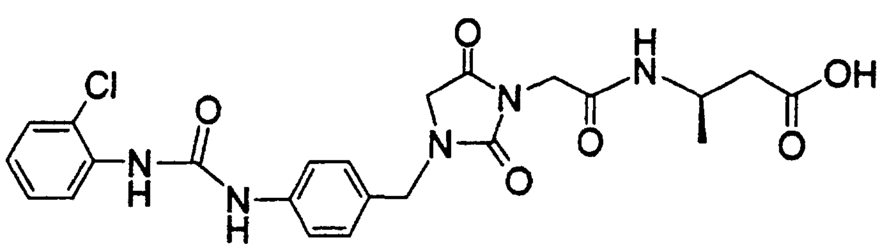

Die Verbindung wurde nach dem Verfahren gemäß Schema 1, Schritte C, D (Methode 1), E, F, G, H (Methode 2), J (Methode 1) hergestellt. Im Schritt H (Ansatzgröße 1.44 mmol) wurde als Aminoverbindung der Formel III der (R)-3-Amino-3-methyl-propionsäure-tert-butylester eingesetzt. Ausbeute: 448 mg.
ES(+)-MS: 502.3 $(M+H)^+$

Beispiele 31 - 46

Die Verbindungen wurden nach dem Verfahren gemäß Schema 1, Schritte C, D (Methode 1), E, F, G, H (Methode 2) hergestellt. Im Schritt H (Ansatzgröße 0.21 - 0.23 mmol) wurde als Aminoverbindung der Formel III im Falle der Beispiele 31 - 38 der (R)-3-Amino-3-methyl-propionsäure-tert-butylester, im Falle der Beispiele 39 - 46 der (S)-3-Amino-3-phenyl-propionsäure-ethylester eingesetzt. Schritt J erfolgte im Falle der Beispiele 31 - 38 nach Methode 1 (mit TFA), im Falle der Beispiele 39 - 46 nach Methode 2 (mit Lithiumhydroxid). Ausbeuten: 30-87 mg. Die hergestellten Verbindungen der Formel Ib sind in Tabelle 1 aufgeführt.

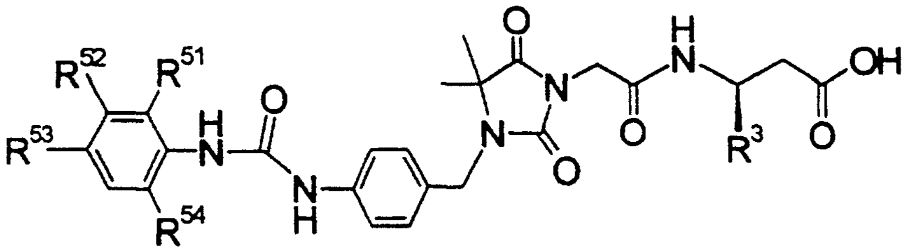

(Ib)

Tabelle 1:

| Beispiele der Formel Ib | | | | | | |
|---|---|---|---|---|---|---|
| Beispiel Nr. | $R^3$ | $R^{51}$ | $R^{52}$ | $R^{53}$ | $R^{54}$ | ES-(+)-MS $(M+H)^+$ |
| 31 | Me | Me | H | Me | Me | 538.4 |
| 32 | Me | iPr | H | H | H | 538.4 |
| 33 | Me | Me | H | H | Et | 538.4 |
| 34 | Me | Me | H | H | Me | 524.4 |
| 35 | Me | Me | H | Me | H | 524.4 |
| 36 | Me | Me | Me | H | H | 524.4 |
| 37 | Me | Et | H | H | H | 524.4 |
| 38 | Me | $CO_2Me$ | H | H | H | 554.3 |
| 39 | Ph | Me | H | Me | Me | 600.4 |
| 40 | Ph | iPr | H | H | H | 600.4 |
| 41 | Ph | Me | H | H | Et | 600.3 |
| 42 | Ph | Me | H | H | Me | 586.3 |
| 43 | Ph | Me | H | Me | H | 586.3 |
| 44 | Ph | Me | Me | H | H | 586.3 |
| 45 | Ph | Et | H | H | H | 586.3 |
| 46 | Ph | $CO_2H$ | H | H | H | 602.3 |

Beispiel 47

((S)-2-(4,4-Dimethyl-3-(4-(3-(2-methylphenyl)-ureido)-benzyl)-2,5-dioxoimidazolidin-1-yl)-2-(2-methylpropyl)-acetyl) -L-aspartyl-L-phenylglycin

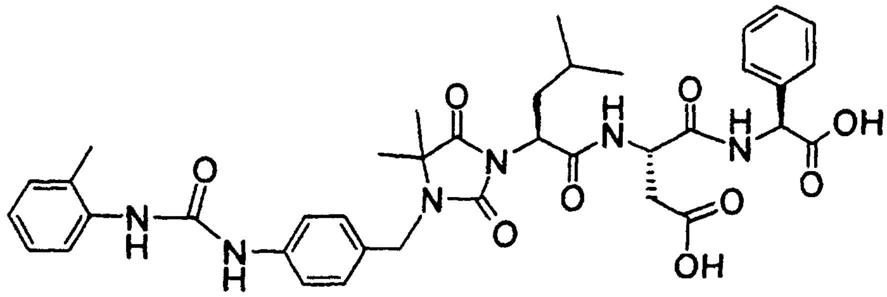

Die Verbindung wurde nach dem Verfahren gemäß Schema 1, Schritte A, B, D (Methode 1), E, F, G, H (Methode 1), J (Methode 1) hergestellt. Im Schritt H (Ansatzgröße 1.04 mmol) wurde als Aminoverbindung der Formel III H-Asp(OtBu)-Phg-OtBu (Hydrochlorid) eingesetzt. Ausbeute: 350 mg.
ES(+)-MS: 729.4 $(M+H)^+$

Beispiele 48 - 69

Die Verbindungen wurden nach dem Verfahren gemäß dem Schema 4 hergestellt durch Kupplung von Hydantoincarbonsäuren der Formel IIa mit H-Asp-Phg-OtBu, das über die freie COOH-Gruppe der Asp-Einheit an Wang-Polystyrolharz geknüpft war. Als Aminosäureester der Formel $H_2N-CH(R^{41})-COOtBu$ in Schema 4 wurde L-Phenylglycin-tert-butylester eingesetzt. Die hergestellten Verbindungen der Formel Ic sind in Tabelle 2 aufgeführt.

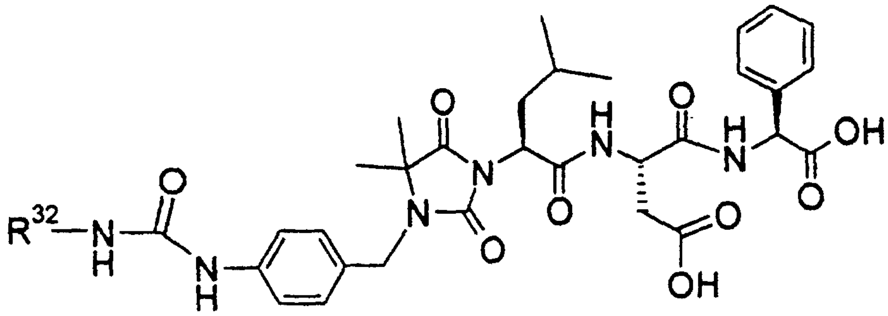

(Ic)

Tabelle 2

| Beispiele der Formel Ic | | |
|---|---|---|
| Beispiel Nr. | $R^{32}$ | ES-(+)-MS $(M+H)^+$ |
| 48 | 3-Fluorphenyl | 733.4 |
| 49 | 4-Fluorphenyl | 733.4 |
| 50 | 4-Methylphenyl | 729.4 |
| 51 | 3-Methylphenyl | 729.4 |
| 52 | n-Propyl | 681.4 |
| 53 | 4-Isopropylphenyl | 757.4 |
| 54 | 3,5-Bistrifluormethylphenyl | 851.4 |

Tabelle 2 (fortgesetzt)

| Beispiele der Formel Ic | | |
|---|---|---|
| Beispiel Nr. | $R^{32}$ | ES-(+)-MS $(M+H)^+$ |
| 55 | 4-Trifluormethoxyphenyl | 799.4 |
| 56 | 2-Trifluormethoxyphenyl | 799.4 |
| 57 | 2-Nitrophenyl | 760.4 |
| 58 | Benzyl | 729.5 |
| 59 | Phenyl | 715.3 |
| 60 | 4-Methoxyphenyl | 745.4 |
| 61 | 2-Methoxyphenyl | 745.4 |
| 62 | 2-Chlorphenyl | 749.4 |
| 63 | Isopropyl | 681.4 |
| 64 | 3-Methoxyphenyl | 745.4 |
| 65 | tert-Butyl | 695.4 |
| 66 | Cyclohexyl | 721.4 |
| 67 | 2-Fluorphenyl | 733.4 |
| 68 | 2-Trifluormethylphenyl | 783.3 |
| 69 | 4-Trifluormethylphenyl | 783.3 |

Beispiele 70-87

Die Verbindungen wurden nach dem Verfahren gemäß dem Schema 5, Variante A, hergestellt durch Kupplung von Hydantoincarbonsäuren der Formel IIa mit 3-Amino-3-(3,4-ethylendioxyphenyl)-propionsäure, die über die freie COOH-Gruppe an das Harz geknüpft war. Die hergestellten Verbindungen der Formel Id sind in Tabelle 3 aufgeführt.

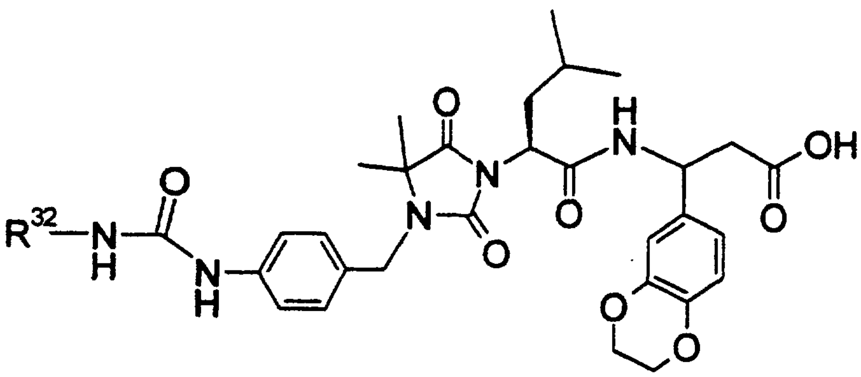

(Id)

Tabelle 3:

| Beispiele der Formel Id | | |
|---|---|---|
| Beispiel Nr. | $R^{32}$ | ES-(+)-MS $(M+H)^+$ |
| 70 | 3-Fluorphenyl | 690.3 |
| 71 | 4-Fluorphenyl | 690.3 |
| 72 | 4-Methylphenyl | 686.4 |
| 73 | 3-Methylphenyl | 686.4 |
| 74 | n-Propyl | 638.4 |
| 75 | 4-Isopropylphenyl | 714.4 |
| 76 | 3,5-Bistrifluormethylphenyl | 808.3 |
| 77 | 4-Trifluormethoxyphenyl | 756.3 |
| 78 | 2-Trifluormethoxyphenyl | 756.3 |

Tabelle 3: (fortgesetzt)

| Beispiele der Formel Id | | |
|---|---|---|
| Beispiel Nr. | $R^{32}$ | ES-(+)-MS $(M+H)^+$ |
| 79 | 2-Nitrophenyl | 717.3 |
| 80 | Benzyl | 686.4 |
| 81 | 2-Methylphenyl | 690.4 |
| 82 | 2-Trifluormethylphenyl | 740.3 |
| 83 | Ethyl | 624.4 |
| 84 | 4-Trifluormethylphenyl | 740.3 |
| 85 | 4-Methoxyphenyl | 702.4 |
| 86 | 2-Methoxyphenyl | 702.4 |
| 87 | 2-Chlorphenyl | 706.3 |

Beispiel 88

(R)-3-((S)-2-(4,4-Dimethyl-3-(4-(3-phenylureido)-benzyl)-2,5-dioxoimidazolidin-1-yl)-2-(2-methylpropyl)-acetylamino)-3-methyl-propionsäure-Natriumsalz

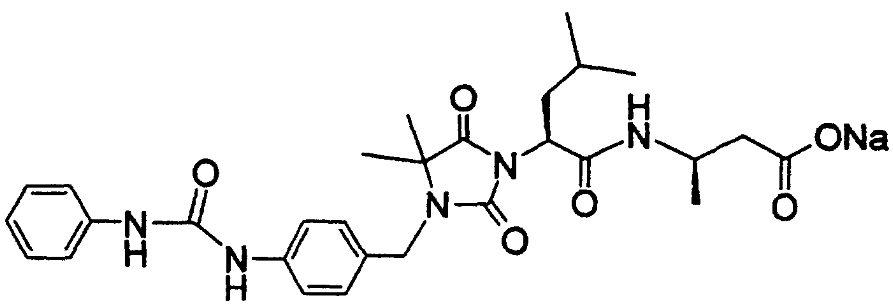

Zu einer Lösung von 1 g (1.81 mmol) (R)-3-((S)-2-(4,4-Dimethyl-3-(4-(3-phenylureido)-benzyl)-2,5-dioxoimidazolidin-1-yl)-2-(2-methylpropyl)-acetylamino)-3-methyl-propionsäure in 20 ml THF und 50 ml Wasser wurde 1 Äquivalent 1 N Natronlauge gegeben. Nach 30 Minuten bei Raumtemperatur wurde der Großteil des THF im Vakuum entfernt und der Rückstand gefriergetrocknet. Nach Chromatographie über Sephadex LH20 (Laufmittel: Wasser) erhielt man 930 mg des Titel-Salzes.
ES(+)-MS: 552.5 $(M+H)^+$, 574.4 (Natriumsalz)

Beispiel 89

(R)-3-((S)-2-(4,4-Dimethyl-3-(4-(3-phenylureido)-benzyl)-2,5-dioxoimidazolidin-1-yl)-2-methyl-acetylamino)-3-methyl-propionsäure

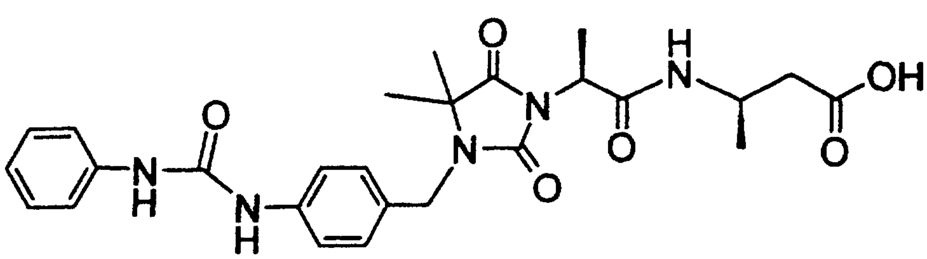

Die Verbindung wurde nach dem Verfahren gemäß Schema 2 hergestellt (Schritt J nach Methode 1). Im Schritt H (Ansatzgröße 5.2 mmol) wurde als Aminoverbindung der Formel III der (R)-3-Amino-3-methyl-propionsäure-tert-butylester eingesetzt.
Ausbeute: 1.86 g.
ES(+)-MS: 510.4 $(M+H)^+$

Beispiel 90

(R)-3-((S)-2-(4,4-Dimethyl-3-(4-(3-(2-methylphenyl)-ureido)-benzyl)-2,5-dioxoimidazolidin-1-yl)-2-methyl-acetylamino)-3-methyl-propionsäure

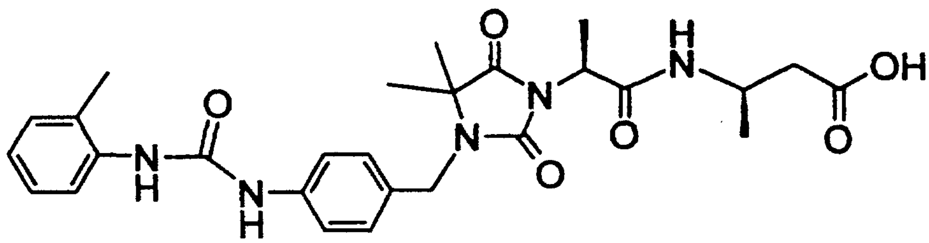

Die Verbindung wurde nach dem Verfahren gemäß Schema 2 hergestellt (Schritt J nach Methode 1). Im Schritt H (Ansatzgröße 11.9 mmol) wurde als Aminoverbindung der Formel III der (R)-3-Amino-3-methyl-propionsäure-tert-butylester eingesetzt.
Ausbeute: 4.3 g.
FAB(+)-MS: 524.3 $(M+H)^+$

Beispiel 91

3-(2-(4,4-Dimethyl-3-(4-(3-(2-methylphenyl)-ureido)-benzyl)-2,5-dioxoimidazolidin-1-yl)-acetylamino)-3,3-dimethyl-propionsäure

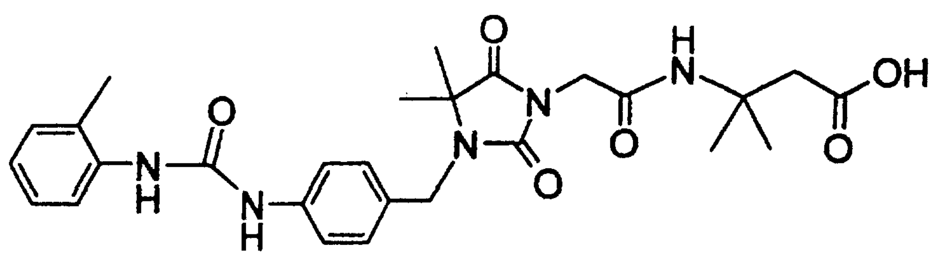

Die Verbindung wurde nach dem Verfahren gemäß Schema 1, Schritte C, D (Methode 1), E, F, G, H (Methode 2), J (Methode 2) hergestellt. Im Schritt H (Ansatzgröße 0.9 mmol) wurde als Aminoverbindung der Formel III der 3-Amino-3,3-dimethyl-propionsäure-methylester eingesetzt. Ausbeute: 53 mg.
ES(+)-MS: 524.4 $(M+H)^+$

Beispiel 92

(R)-3-((S)-2-(4,4-Dimethyl-3-(4-(3-phenylureido)-benzyl)-2,5-dioxoimidazolidin-1-yl)-2-cyclopropylmethyl-acetylamino)-3-methyl-propionsäure

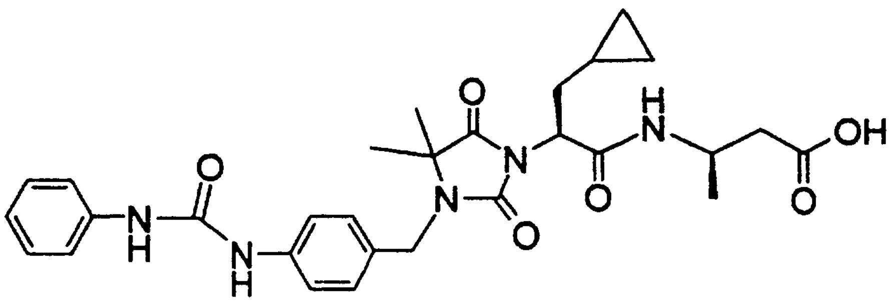

Die Verbindung wurde nach dem Verfahren gemäß Schema 2 hergestellt (Schritt J nach Methode 1). Im Schritt H (Ansatzgröße 1.29 mmol) wurde als Aminoverbindung der Formel III der (R)-3-Amino-3-methyl-propionsäure-tert-butylester eingesetzt.
Ausbeute: 493 mg.
ES(+)-MS: 550.5 $(M+H)^+$

Beispiel 93

(S)-3-(2-(4,4-Dimethyl-3-(4-(3-(2-methylphenyl)-ureido)-benzyl)-2,5-dioxoimidazolidin-1-yl)-acetylamino)-3-(3,4-methylendioxy-phenyl)-propionsäure

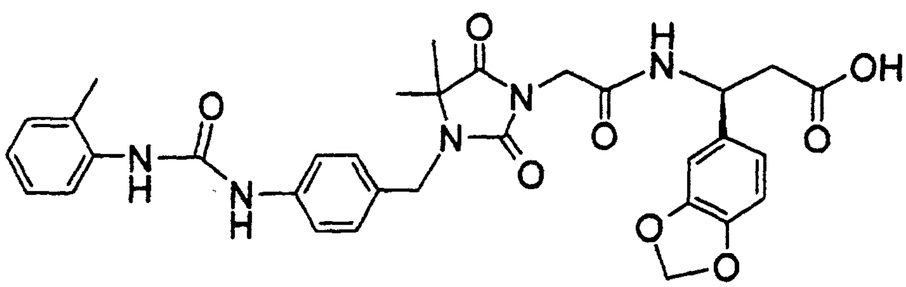

Die Verbindung wurde nach dem Verfahren gemäß Schema 1, Schritte C, D (Methode 1), E, F, G, H (Methode 2), J (Methode 1) hergestellt. Im Schritt H (Ansatzgröße 4 mmol) wurde als Aminoverbindung der Formel III der (S)-3-Amino-3-(3,4-methylendioxyphenyl)-propionsäure-tert-butylester eingesetzt. Ausbeute: 1.08 g.
FAB(+)-MS: 61.6.2 $(M+H)^+$

Beispiel 94

(S)-2-Benzyloxycarbonylamino-3-((4,4-dimethyl-3-(4-(3-(2-methylphenyl)-ureido)-benzyl)-2,5-dioxoimidazolidin-1-yl)-acetylamino)-propionsäure

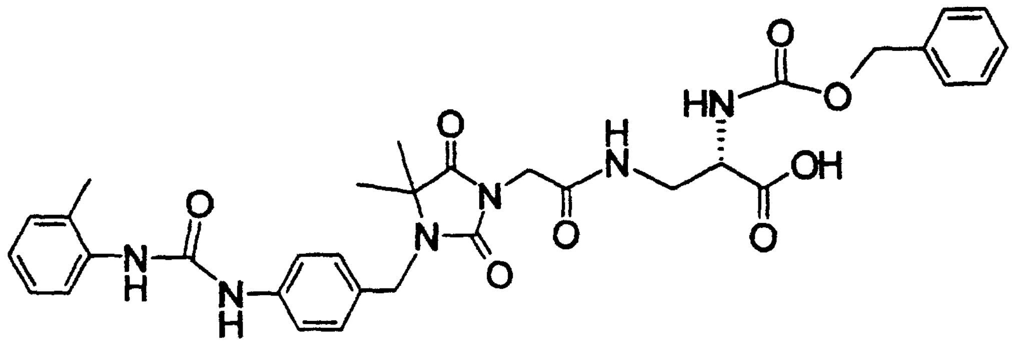

Die Verbindung wurde nach dem Verfahren gemäß Schema 1, Schritte C, D (Methode 1), E, F, G, H (Methode 2), J (Methode 1) hergestellt. Im Schritt H (Ansatzgröße 1.89 mmol) wurde als Aminoverbindung der Formel III der (S)-3-Amino-2-benzyloxycarbonylamino-propionsäure-tert-butylester eingesetzt.
Ausbeute: 410 mg.
FAB(+)-MS: 645.2 $(M+H)^+$

Beispiele 95 - 116

Die Ester der Beispiele 95, 96, 98 - 102 und 104 - 116 wurden aus den entsprechenden Carbonsäuren (Verbindungen der Formel I mit E = $R^{10}CO$, $R^{10}$ = Hydroxy) durch Veresterung der COOH-Gruppe nach dem folgenden allgemeinen Verfahren hergestellt: Man gab zu einer Lösung der Carbonsäure in absolutem DCM (7 - 10 ml pro mmol Carbonsäure) 6 Äquivalente des entsprechenden absoluten Alkohols und anschließend 0.8 Äquivalente DMAP und 1.1 Äquivalente DCC und ließ das Reaktiongemisch über Nacht bei Raumtemperatur stehen. Nach Filtration wurde das Lösungsmittel im Vakuum entfernt und der Rückstand chromatographisch gereinigt. Die Ester der Beispiele 97 und 103 wurden direkt bei der Herstellung der Carbonsäuren der Beispiele 19 und 11 erhalten (als Zwischenprodukte nach Schritt H). Die hergestellten Ester der Formel Ie sind in Tabelle 4 aufgeführt.

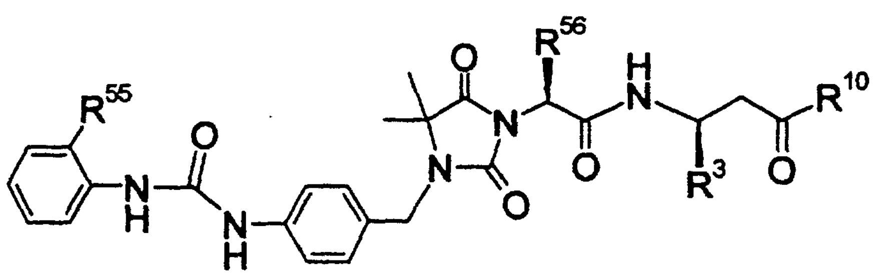

(Ie)

Tabelle 4:

| Beispiele der Formel Ie | | | | | |
|---|---|---|---|---|---|
| Beispiel Nr. | $R^{55}$ | $R^{56}$ | $R^3$ | $R^{10}$ | ES-(+)- oder FAB-(+)-MS $(M+H)^+$ |
| 95 | H | iBu | Me | OiPr | 594.4 |
| 96 | H | iBu | Me | OEt | 580.3 |
| 97 | H | iBu | Ph | OEt | 642.3 |

Tabelle 4: (fortgesetzt)

| Beispiele der Formel Ie | | | | | |
|---|---|---|---|---|---|
| Beispiel Nr. | $R^{55}$ | $R^{56}$ | $R^3$ | $R^{10}$ | ES-(+)- oder FAB-(+)-MS $(M+H)^+$ |
| 98 | H | iBu | Ph | OiPr | 656.5 |
| 99 | H | iBu | Ph | OiBu | 670.5 |
| 100 | H | iBu | Me | OiBu | 608.5 |
| 101 | H | iBu | Me | OMe | 566.4 |
| 102 | Me | H | Ph | OiPr | 614.4 |
| 103 | Me | H | Ph | OEt | 600.4 |
| 104 | Me | H | Me | OEt | 538.4 |
| 105 | Me | H | Me | OiPr | 552.4 |
| 106 | H | Me | Me | OiPr | 552.4 |
| 107 | H | Me | Me | OEt | 538.4 |
| 108 | Me | Me | Me | OEt | 552.4 |
| 109 | Me | Me | Me | OiPr | 566.5 |
| 110 | Me | H | Me | OiBu | 566.3 |
| 111 | H | Cyclopropyl-CH2- | Me | OEt | 578.6 |
| 112 | H | Cyclopropyl-CH2- | Me | OiPr | 592.6 |
| 113 | Me | H | Me | OMe | 524.5 |
| 114 | Me | H | 3,4-Methylen-dioxyphenyl | OiPr | 658.3 |
| 115 | Me | H | Me | OnPr | 552.2 |
| 116 | Me | H | Me | OnBu | 566.5 |

Beispiel 117

(2-(4,4-Dimethyl-3-(4-(3-(2-methylphenyl)-ureido)-benzyl)-2,5-dioxoimidazolidin-1-yl)-acetyl)-L-aspartyl-(2-adamantylamid)-isopropylester

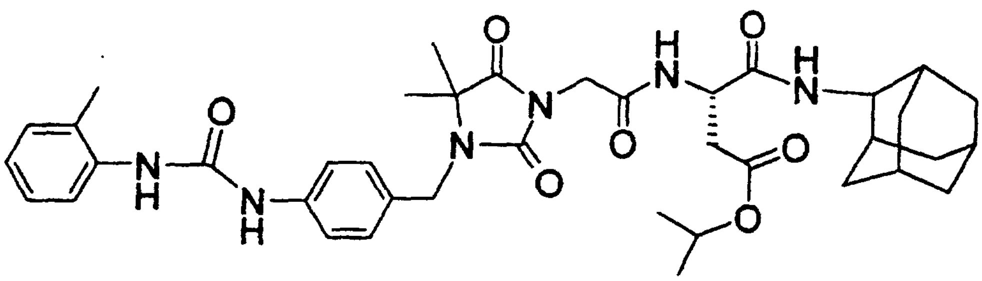

Die Verbindung wurde aus (2-(4,4-Dimethyl-3-(4-(3-(2-methylphenyl)-ureido)-benzyl)-2,5-dioxoimidazolidin-1-yl)-acetyl)-L-aspartyl-(2-adamantylamid) und Isopropanol wie für die Beispiele 95, 96, 98 - 102 und 104 - 116 beschrieben hergestellt. Ansatzgröße: 0.371 mmol der Ausgangs-Aspartylverbindung. Ausbeute: 210 mg.
ES(+)-MS: 715.4 $(M+H)^+$

Beispiel 118

(S)-2-Benzyloxycarbonylamino-3-((4,4-dimethy(-3-(4-(3-(2-methylphenyl)-ureido)-benzyl)-2,5-dioxoimidazolidin-1-yl)-acetylamino)-propionsäure-isopropylester

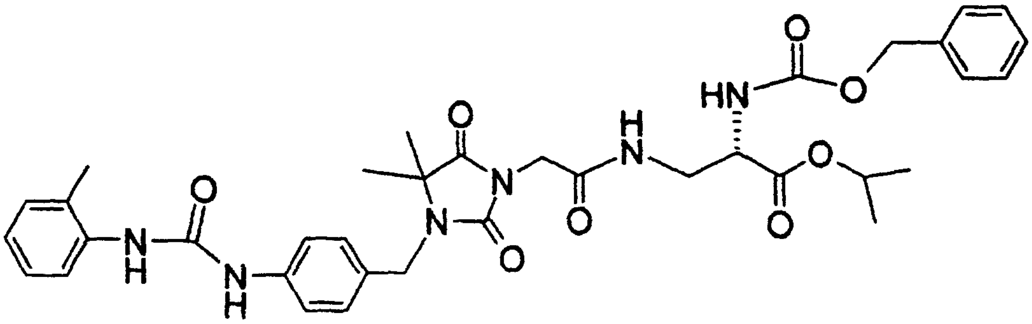

Die Verbindung wurde aus (S)-2-Benzyloxycarbonylamino-3-((4,4-dimethyl-3-(4-(3-(2-methylphenyl)-ureido)-benzyl)-2,5-dioxoimidazolidin-1-yl)-acetylamino)-propionsäure und Isopropanol wie für die Beispiele 95, 96, 98-102 und 104 - 116 beschrieben hergestellt. Ansatzgröße: 0.465 mmol der Ausgangs-Propionsäure.
Ausbeute: 233 mg.
FAB(+)-MS: 687.3 $(M+H)^+$

Beispiele 119 - 124

Die Synthese erfolgte analog N. M. Nielsen, H. Bundgaard, Journal of Pharmaceutical Sciences, 1988, 77 (4), 285, durch Umsetzung von (R)-3-(2-(4,4-Dimethyl-3-(4-(3-(2-methylphenyl)-ureido)-benzyl)-2,5-dioxoimidazolidin-1-yl)-acetylamino)-3-methyl-propionsäure mit dem jeweiligen Chloracetamid (Beispiele 119, 120, 122) oder mit Chlormethylpivalat (Beispiel 121) oder mit (1-Chlorethyl)-ethylcarbonat (Beispiel 123) oder mit Brommethylacetat (Beispiel 124). Die Reaktionen wurden bei 80 °C durchgeführt. Die Reinigung der Substanzen erfolgte mittels präparativer HPLC über Sephadex LH20 (Laufmittel: Acetonitril/Wasser). Ansatzgröße: 1.374 mmol der Ausgangs-Propionsäure. Die hergestellten Verbindungen der Formel If sind in Tabelle 5 aufgeführt.

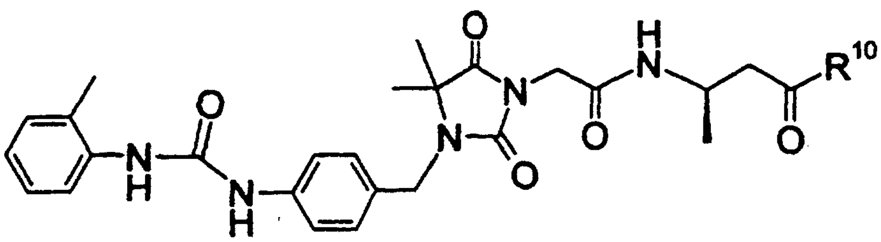

(If)

Tabelle 5:

| Beispiele der Formel If | | | |
|---|---|---|---|
| Beispiel Nr. | $R^{10}$ | Ausbeute | ES-(+)- oder FAB-(+)-MS $(M+H)^+$ |
| 119 | O-$CH_2$-CO-$NMe_2$ | 280 mg | 595.5 |
| 120 | O-$CH_2$-CO-$NEt_2$ | 435 mg | 623.3 |
| 121 | O-$CH_2$-O-CO-tBu | 291 mg | 624.1 |
| 122 | O-$CH_2$-CO-$NH_2$ | 374 mg | 567.5 |
| 123 | O-CH(Me)-O-CO-OEt | 133 mg | 626.5 |
| 124 | O-$CH_2$-O-CO-Me | 276 mg | 582.5 |

Beispiele 125-129

Die Beispiele 125, 127, 128 und 129 wurden nach dem Verfahren gemäß Schema 1, Schritte A, B, D (Methode 1), E, F, G, H (Methode 2) hergestellt. Im Schritt H wurden als Aminoverbindung der Formel III (R)-3-Amino-3-methyl-propanol (Beispiele 125 und 129) oder (S)-3-Amino-3-phenyl-propanol (Beispiel 128) oder (S)-3-Amino-3-(4-methoxyphenyl)-propanol (Beispiel 127) eingesetzt. Das Beispiel 126 wurde nach dem Verfahren gemäß Schema 1, Schritte C, D (Methode 1), E, F, G, H (Methode 2) hergestellt. Im Schritt H wurde als Aminoverbindung der Formel III (S)-3-Amino-3-phenyl-propanol eingesetzt. Die hergestellten Verbindungen der Formel Ig sind in Tabelle 6 aufgeführt.

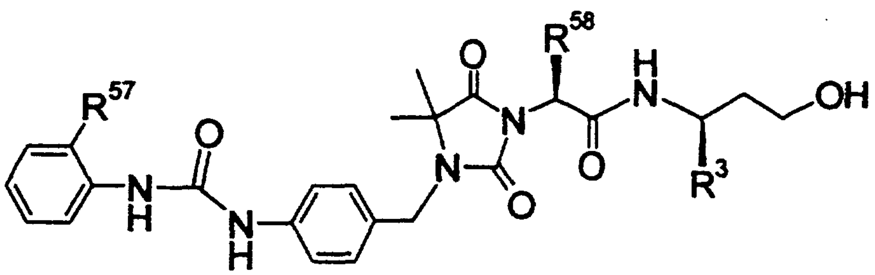

(Ig)

Tabelle 6:

| Beispiele der Formel Ig | | | | |
|---|---|---|---|---|
| Beispiel Nr. | $R^{57}$ | $R^{58}$ | $R^3$ | ES-(+)-oder FAB-(+)-MS $(M+H)^+$ |
| 125 | H | iBu | Me | 538.4 |
| 126 | Me | H | Ph | 558.3 |
| 127 | H | iBu | 4-Methoxyphenyl | 630.3 |
| 128 | H | iBu | Ph | 600.2 |
| 129 | Me | iBu | Me | 552.2 |

Die bei der Herstellung der Verbindungen der Beispiele 125 - 129 eingesetzten 3-Amino-propanole wurden wie folgt hergestellt.

(S)-3-Amino-3-phenyl-propanol

Man gab zu einer Suspension von 3.5 g (15.2 mmol) (S)-3-Amino-3-phenylpropionsäure-ethylester-hydrochlorid in 150 ml absolutem THF unter Eiskühlung portionsweise 1.45 g (38.1 mmol) Lithiumaluminiumhydrid und ließ 1 Stunde bei Raumtemperatur nachrühren. Anschließend wurden unter Eiskühlung 5 ml Wasser vorsichtig zugetropft. Der Niederschlag wurde abfiltriert und das Filtrat im Vakuum eingeengt. Der Rückstand wurde in DCM aufgenommen und die Lösung mit Wasser extrahiert. Die organische Phase wurde über Natriumsulfat getrocknet. Nach Filtration und Entfernen des Lösungsmittels im Vakuum erhielt man 1.84 g (S)-3-Amino-3-phenyl-propanol.

(R)-3-Amino-3-methyl-propanol und (S)-3-Amino-3-(4-methoxyphenyl)-propanol

Man gab zu einer Lösung von Aluminiumtrichlorid in absolutem Diethylether (ca. 3 ml pro mmol an Aluminiumtrichlorid) portionsweise 1 Äquivalent Lithiumaluminiumhydrid und erhitzte das Gemisch 30 Minuten unter Rückfluß. Es wurden 0.4 Äquivalente (R)-3-Amino-3-methyl-propionsäure-tert-butylester bzw. (S)-3-Amino-3-(4-methoxyphenyl)-propionsäure-tert-butylester langsam zugetropft und das Reaktionsgemisch 1 Stunde unter Rückfluß erhitzt. Anschließend wurden unter Eiskühlung vorsichtig Wasser (0.072 ml pro mmol an Lithiumaluminiumhydrid) und eine Lösung von Kaliumhydroxid in Wasser (pro mmol Lithiumaluminiumhydrid 1.688 g Kaliumhydroxid in 2.8 ml Wasser) zugetropft. Man ließ das Gemisch über Nacht bei Raumtemperatur stehen, dekantierte die Etherphase ab und verrührte den Rückstand mehrmals mit Diethylether und DCM. Die vereinigten organischen Phasen wurden über Natriumsulfat getrocknet. Nach Filtration und Entfernen des Lösungsmittels im Vakuum erhielt man den entsprechenden Aminoalkohol.

Beispiel 130

(R)-3-((S)-2-(4,4-Dimethyl-3-(4-(3-phenylureido)-benzyl)-2,5-dioxoimidazolidin-1-yl)-2-(2-methylpropyl)-acetylamino)-3-methyl-propanal

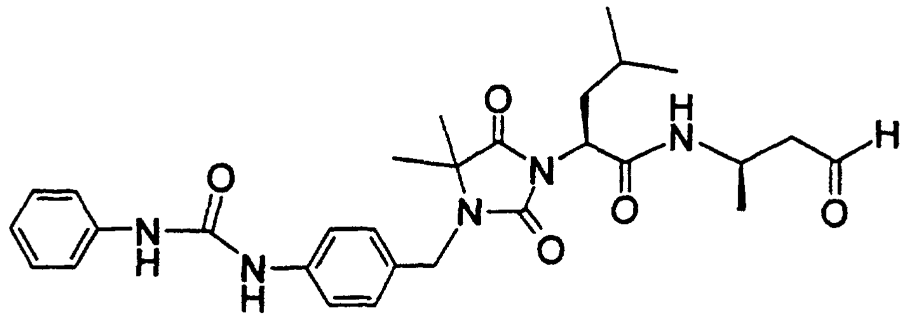

56.5 mg (R)-3-((S)-2-(4,4-Dimethyl-3-(4-(3-phenylureido)-benzyl)-2,5-dioxoimidazolidin-1-yl)-2-(2-methylpropyl)-acetylamino)-3-methyl-propanol wurden mit 10.8 mg Kaliumbromid in einem Gemisch aus 3 ml Ethylacetat, 1 ml Toluol und 1 ml Wasser gelöst. Nach Zugabe einer katalytischen Menge 4-Acetamido-2,2,6,6-tetramethyl-piperidin-1-oxyl (= 4-Acetamido-TEMPO) wurde bei 0 °C eine Mischung von 0.5 ml Natriumhypochloritlösung (13%ig), 0.5 ml gesättigter Natriumhydrogencarbonatlösung und 1 ml Wasser trofpenweise zugefügt. Das Gemisch wurde 25 Minuten bei 0 °C gerührt. Nach vollständigem Umsatz wurde die Mischung mit Ethylacetat versetzt, die organische Phase mit Natriumthiosulfatlösung gewaschen und über Natriumsulfat getrocknet. Nach Filtration wurde das Lösungsmittel am Rotationsverdampfer entfernt und der Rückstand durch Reversed Phase-HPLC gereinigt (Wasser/Acetonitril). Ausbeute: 15 mg.

Beispiel 131

(R)-3-((S)-2-(4,4-Dimethyl-3-(4-(3-phenylureido)-benzyl)-2,5-dioxoimidazolidin-1-yl)-2-(2-methylpropyl)-acetylamino)-3-methyl-propionamid

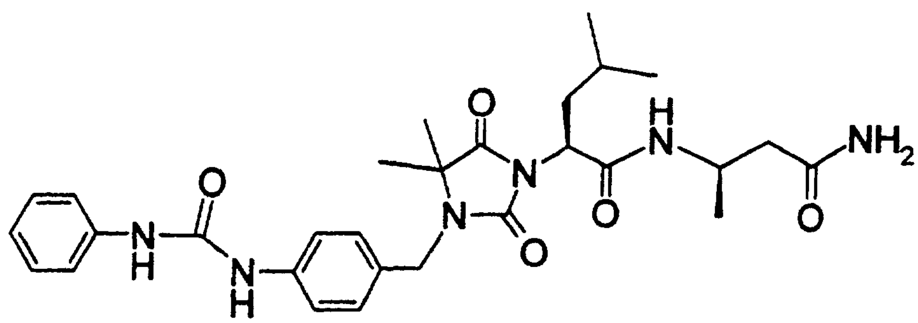

Die Verbindung wurde aus 0.5 g (R)-3-((S)-2-(4,4-Dimethyl-3-(4-(3-phenylureido)-benzyl)-2,5-dioxoimidazolidin-1-yl)-2-(2-methylpropyl)-acetylamino)-3-methylpropionsäure und Rink-Amidharz nach dem oben beschriebenen allgemeinen Verfahren zur Herstellung von unsubstituierten Carbonsäureamiden an der Festphase hergestellt. Ausbeute: 349 mg.
ES(+)-MS: 551.3 $(M+H)^+$

Beispiel 132

(S)-3-((S)-2-(4,4-Dimethyl-3-(4-(3-phenylureido)-benzyl)-2,5-dioxoimidazolidin-1-yl)-2-(2-methylpropyl)-acetylamino)-3-phenyl-propionamid

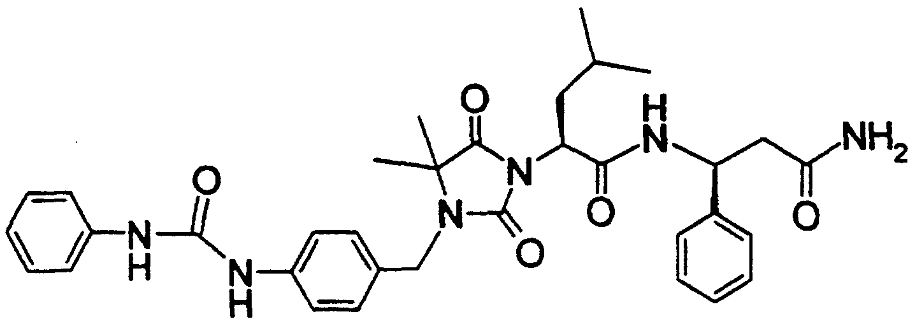

Die Verbindung wurde analog Beispiel 131 aus (S)-3-((S)-2-(4,4-Dimethyl-3-(4-(3-phenylureido)-benzyl)-2,5-dioxoimidazolidin-1-yl)-2-(2-methylpropyl)-acetylamino)-3-phenyl-propionsäure hergestellt.
ES(+)-MS: 613.3 $(M+H)^+$

Beispiel 133

((S)-2-(4,4-Dimethyl-3-(4-(3-(2-methylphenyl)-ureido)-benzyl)-2,5-dioxoimidazolidin-1-yl)-2-(2-methylpropyl)-acetyl) -L-aspartyl-L-valyl-L-prolin

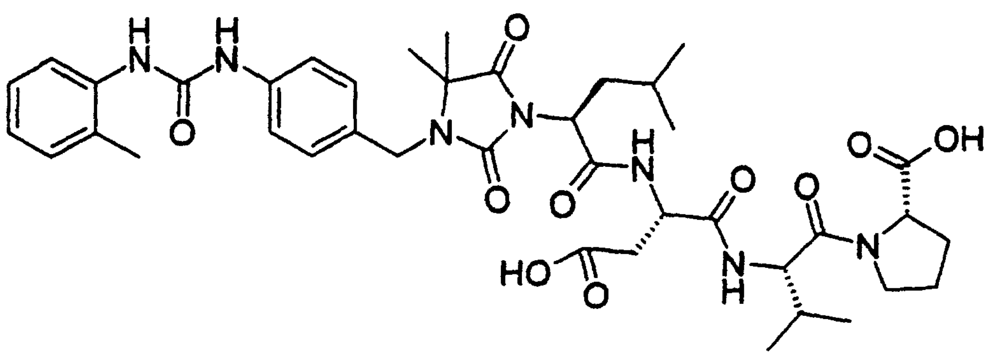

Die Verbindung wurde analog dem oben beschriebenen allgemeinen Verfahren zur Herstellung von Verbindungen der Formel I, die eine Peptideinheit enthalten, durch Festphasensynthese hergestellt. Zum Aufbau der Tripeptideinheit Asp-Val-Pro wurden zunächst 6 g 2-Chlortritylchlorid-Polystyrolharz mit 4 g Fmoc-Pro-OH beladen. Nach Abspaltung der Fmoc-Schutzgruppe wurden im zweiten Kupplungsschritt 3.1 g Fmoc-Val-OH und nach erneuter Abspaltung der Fmoc-Gruppe im dritten Kupplungsschritt 3.4 g Fmoc-Asp(OtBu)-OH eingesetzt. Man erhielt 11 g des mit Fmoc-Asp(OtBu)-Val-Pro beladenen Harzes. 4 g dieses Harzes wurden nach Abspaltung der Fmoc-Gruppe mit 2.7 g (S)-2-(4,4-Dimethyl-3-(4-(3-(2-methylphenyl)-ureido)-benzyt)-2,5-dioxoimidazolidin-1-yl)-2-(2-methylpropyl)-essigsäure, 1.8 g TOTU, 0.75 g HOBT und 0.72 g DIPEA in 25 ml DMF gekuppelt. Nach Waschen des Harzes wurde mit TFA/DCM die Verbindung vom Harz abgespalten (und gleichzeitig die tert-Butylester-Schutzgruppe gespalten). Die Abspaltlösung wurde eingeengt und der Rückstand mit Diethylether kristallisiert.
Ausbeute 750 mg.
ES(+)-MS: 792.5 $(M+H)^+$

Beispiel 134

(2-(4,4-Dimethyl-3-(4-(3-(2-methylphenyl)-ureido)-benzyl)-2,5-dioxoimidazolidin-1-yl)-acetyl)-L-aspartyl-(2-adamantylamid)

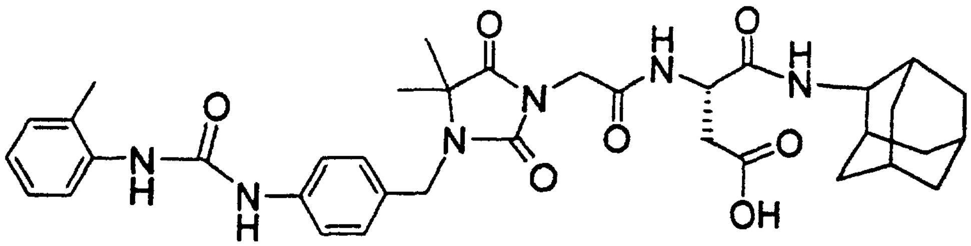

Die Verbindung wurde nach dem Verfahren gemäß Schema 1, Schritte C, D (Methode 1), E, F, G, H (Methode 2), J (Methode 1) hergestellt. Im Schritt H (Ansatzgröße 1.41 mmol) wurde als Aminoverbindung L-Asparaginsäure-(2-adamantylamid)-tert-butylester eingesetzt. Ausbeute: 504 mg.
ES(+)-MS: 673.4 $(M+H)^+$

Beispiele 135 - 158

Die Harnstoffe der Beispiele 135 - 158 wurden nach dem Verfahren gemäß Schema 5, Variante B, hergestellt. Wie oben beschrieben wurden die entsprechenden 3-(4-(N-Fmoc-Amino)-benzyl)-hydantoincarbonsäuren mit 3-Amino-3-(3,4-methylendioxyphenyl)-propionsäure, die über die freie COOH-Gruppe an das Harz geknüpft war, gekuppelt, dann wurde die Fmoc-Schutzgruppe abgespalten und die Aminogruppe durch Umsetzung mit dem entsprechenden Isocyanat bzw. mit Di-(Nsuccinimidyl)-carbonat und dem entsprechenden Amin derivatisiert. Die hergestellten Verbindungen der Formel Ih sind in Tabelle 7 aufgeführt.

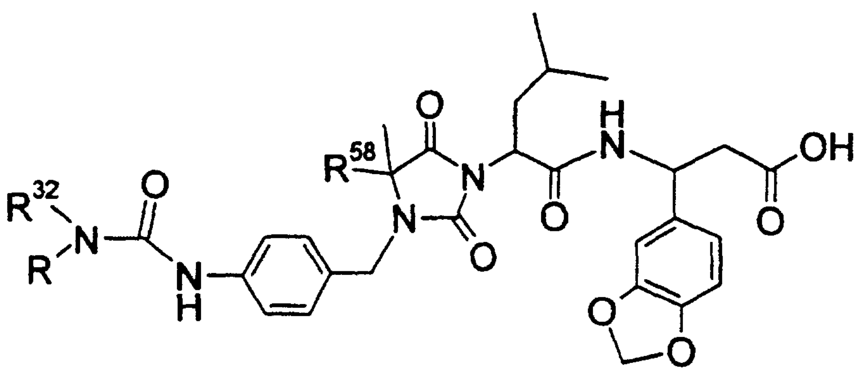
(Ih)

Tabelle 7

| Beispiele der Formel Ih | | | | |
|---|---|---|---|---|
| Beispiel Nr. | $R^{32}$ | R | $R^{58}$ | ES-(+)- oder FAB-(+)-MS $(M+H)^+$ |
| 135 | 2-Methylphenyl | H | Me | 672 |
| 136 | 2-Methoxybenzyl | H | Ph | 764 |
| 137 | 2-Methylphenyl | Me | Ph | 748 |
| 138 | 2-Trifluormethylphenyl | H | Me | 726 |
| 139 | Ethyl | H | Me | 610 |
| 140 | 4-Trifluormethylphenyl | H | Me | 726 |

Tabelle 7 (fortgesetzt)

| Beispiele der Formel Ih | | | | |
|---|---|---|---|---|
| Beispiel Nr. | $R^{32}$ | R | $R^{58}$ | ES-(+)- oder FAB-(+)-MS $(M+H)^+$ |
| 141 | Cyclohexyl | H | Me | 664 |
| 142 | 3-Methylphenyl | H | Me | 672 |
| 143 | 4-Fluorphenyl | H | Me | 676 |
| 144 | 4-Methylphenyl | H | Me | 672 |
| 145 | n-Propyl | H | Me | 624 |
| 146 | 4-Isopropylphenyl | H | Me | 700 |
| 147 | 3,5-Bistrifluormethylphenyl | H | Me | 794 |
| 148 | 4-Trifluormethoxyphenyl | H | Me | 742 |
| 149 | 2-Trifluormethoxyphenyl | H | Me | 742 |
| 150 | 2-Nitrophenyl | H | Me | 703 |
| 151 | 4-Methoxyphenyl | H | Me | 688 |
| 152 | 2-Methoxyphenyl | H | Me | 688 |
| 153 | 2-Chlorphenyl | H | Me | 692 |
| 154 | Isopropyl | H | Me | 624 |
| 155 | 3-Methoxyphenyl | H | Me | 688 |
| 156 | tert-Butyl | H | Me | 638 |
| 157 | Benzyl | H | Me | 672 |
| 158 | Phenyl | H | Me | 658 |

Beispiele 159 -166

Die Thioharnstoffe der Beispiele 159 -166 wurden nach dem Verfahren gemäß Schema 5, Variante B, hergestellt. Wie oben beschrieben wurde die entsprechende 3-(4-(N-Fmoc-Amino)-benzyl)-hydantoincarbonsäure mit 3-Amino-3-(3,4-methylendioxyphenyl)-propionsäure, die über die freie COOH-Gruppe an das Harz geknüpft war, gekuppelt, dann wurde die Fmoc-Schutzgruppe abgespalten und die Aminogruppe durch Umsetzung mit dem entsprechenden Isothiocyanat derivatisiert. Die hergestellten Verbindungen der Formel Ik sind in Tabelle 8 aufgeführt.

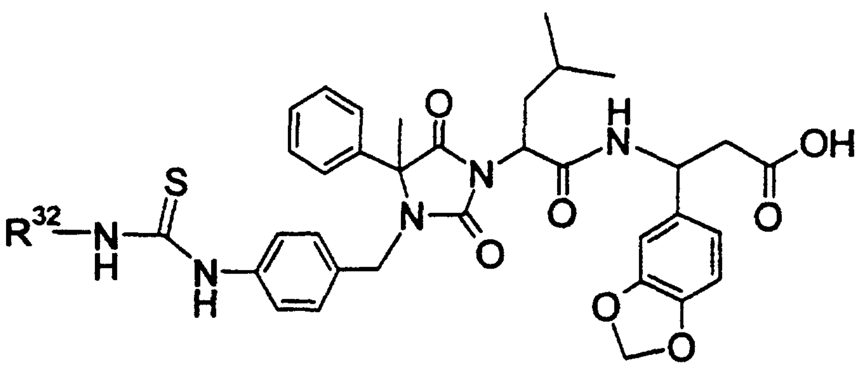

(Ik)

Tabelle 8

| Beispiele der Formel Ik | | |
|---|---|---|
| Beispiel Nr. | $R^{32}$ | ES-(+)- oder FAB-(+)-MS $(M+H)^+$ |
| 159 | 2-Methylphenyl | 750 |
| 160 | 4-Methylphenyl | 750 |
| 161 | Benzyl | 750 |
| 162 | 2-Iodphenyl | 862 |

Tabelle 8 (fortgesetzt)

| Beispiele der Formel Ik | | |
|---|---|---|
| Beispiel Nr. | $R^{32}$ | ES-(+)- oder FAB-(+)-MS $(M+H)^+$ |
| 163 | 2-Methoxyphenyl | 766 |
| 164 | tert-Butyl | 716 |
| 165 | 2-Tetrahydrofurylmethyl | 744 |
| 166 | 3-Methoxyphenyl | 766 |

Beispiele 167 - 182

Die Verbindungen der Beispiele 167-182 wurden nach dem Verfahren gemäß Schema 5, Variante B, hergestellt. Wie oben beschrieben wurde die entsprechende 3-(4-(N-Fmoc-Amino)-benzyl)-hydantoincarbonsäure mit 3-Amino-3-(3,4-methylendioxyphenyl)-propionsäure, die über die freie COOH-Gruppe an das Harz geknüpft war, gekuppelt, dann wurde die Fmoc-Schutzgruppe abgespalten und die Aminogruppe wie beschrieben in ein Amid überführt. Die hergestellten Verbindungen der Formel Im sind in Tabelle 9 aufgeführt.

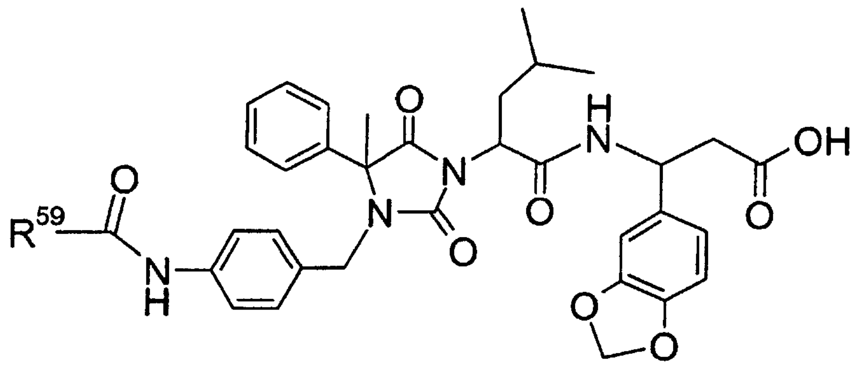

(Im)

Tabelle 9:

| Beispiele der Formel Im | | |
|---|---|---|
| Beispiel Nr. | $R^{59}$ | ES-(+)- oder FAB-(+)-MS $(M+H)^+$ |
| 169 | Phenyl | 705 |
| 170 | 2-Methylbenzyl | 733 |
| 171 | 2-Methylphenyl | 719 |
| 172 | 2-Chlorphenyl | 740 |
| 173 | 2-Fluorphenyl | 723 |
| 174 | 2-Nitrophenyl | 750 |
| 175 | 2-Trifluormethylbenzyl | 787 |
| 176 | 2-Iodphenyl | 831 |
| 177 | 2-Methoxyphenyl | 735 |
| 178 | 2-Bromphenyl | 784 |
| 179 | 2-Brombenzyl | 798 |
| 180 | 2-Fluorbenzyl | 737 |
| 181 | 2-Nitrobenzyl | 764 |
| 182 | 2-Chlorbenzyl | 754 |

Beispiele 184 - 188

Die Verbindungen wurden nach dem Verfahren gemäß Schema 1, Schritte A, B, D (Methode 1), E, F, G, H

(Methode 2) hergestellt. Im Schritt H (Ansatzgröße 0.5 mmol) wurde im Falle der Beispiele 184, 185, 186 und 188 als Aminoverbindung der Formel III der entsprechende (S)-3-Amino-3-aryl-propionsäure-tert-butylester eingesetzt, im Falle des Beispiels 187 der (S)-3-Amino-3-pentafluorphenyl-propionsäure-ethylester. Im Falle der Beispiele 184, 185, 186 und 188 wurde Schritt J nach Methode 1 mit TFA durchgeführt, im Falle des Beispiels 187 analog Methode 2 mit Lithiumhydroxid wie im Beispiel 183 beschrieben. Das im Beispiel 187 erhaltene Produkt enthielt Lithiumtrifluoracetat. Die hergestellten (S)-3-((S)-(2-(4,4-Dimethyl-3-(4-(3-(2-methylphenyl)-ureido)-benzyl)-2,5-dioxoimidazolidin-1-yl)-2-(2-methylpropyl)-acetylamino)-3-aryl-propionsäuren der Formel In sind in Tabelle 10 aufgeführt.

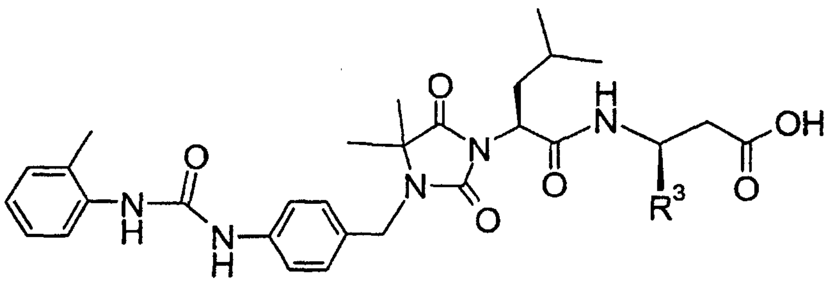

(In)

Tabelle 10:

| Beispiele der Formel In | | | |
|---|---|---|---|
| Beispiel Nr. | $R^3$ | Ausbeute | ES-(+)-MS $(M+H)^+$ |
| 184 | 2-Naphthyl | 85 mg | 678.3 |
| 185 | 4-Biphenylyl | 140 mg | 704.3 |
| 186 | 1-Naphthyl | 100 mg | 678.3 |
| 187 | Pentafluorphenyl | 580 mg | 724.5 |
| 188 | 2,4-Dimethoxyphenyl | 320 mg | 688.5 |

Beispiel 189

(S)-3-((RS)-2-((RS)-4-Methyl-4-phenyl-3-(4-(3-(2-methylphenyl)-ureido)-benzyl)-2,5-dioxoimidazolidin-1-yl)-2-(2-methylpropyl)-acetylamino)-3-(2,4-dimethoxyphenyl)-propionsäure

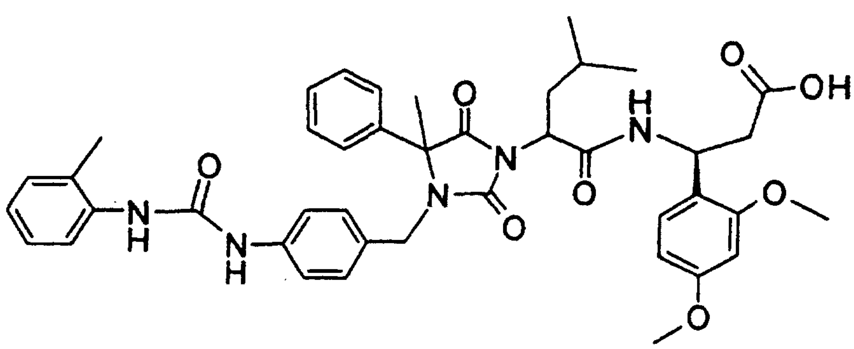

Die Verbindung wurde nach dem Verfahren gemäß Schema 1, Schritte C, D (Methode 1), E, F, G, H (Methode 2), J (Methode 1) hergestellt. Im Schritt H (Ansatzgröße 0.5 mmol) wurde als Aminoverbindung der Formel III der (S)-3-Amino-3-(2,4-dimethoxyphenyl)-propionsäure-tert-butylester eingesetzt. Ausbeute: 320 mg.
ES(+)-MS: 750.5 $(M+H)^+$

Beispiele 190 - 194

Die Verbindungen wurde nach dem Verfahren gemäß Schema 1, Schritte C, D (Methode 1), E, F, G, H (Methode 2) hergestellt. Im Schritt H (Ansatzgröße 0.25 mmol) wurde der entsprechende (RS)-3-Amino-3-aryl-propionsäure-ethylester eingesetzt. Die Spaltung der Esterschutzgruppe in Schritt J erfolgte analog Methode 2 mit Lithiumhydroxid wie im Beispiel 183 beschrieben. Die hergestellten (RS)-3-((RS)-(2-((RS)-4-Methyl-4-phenyl-3-(4-(3-(2-methylphenyl)-ureido)-benzyl)-2,5-dioxoimidazolidin-1-yl)-2-(2-methylpropyl)-acetylamino)-3-aryl-propionsäuren der Formel Ip sind in Tabelle 11 aufgeführt.

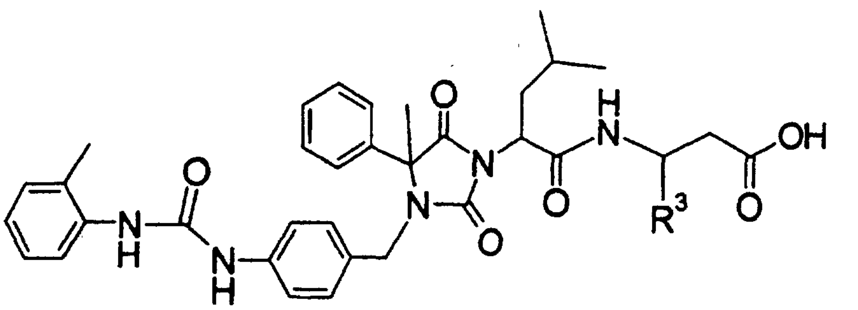

(Ip)

Tabelle 11:

| Beispiele der Formel Ip | | | |
|---|---|---|---|
| Beispiel Nr. | $R^3$ | Ausbeute | ES-(+)-MS $(M+H)^+$ |
| 190 | 3,4-Dimethoxyphenyl | 145 mg | 750.4 |
| 191 | 4-tert-Butylphenyl | 161 mg | 752.4 |
| 192 | 4-Fluorphenyl | 163 mg | 714.3 |
| 193 | 4-Methoxyphenyl | 159 mg | 720.5 |
| 194 | 4-Isobutylphenyl | 159 mg | 746.5 |

Beispiel 195

(RS)-2-Butylsulfonylamino-3-((RS)-2-((RS)-4-methyl-4-phenyl-3-(4-(3-(2-methylphenyl)-ureido)-benzyl)-2,5-dioxoimidazolidin-1-yl)-2-(2-methylpropyl)-acetylamino)-propionsäure

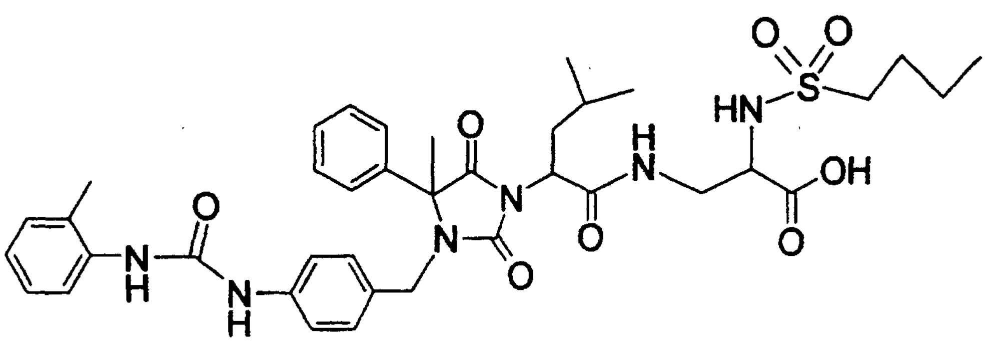

Die Verbindung wurde nach dem Verfahren gemäß Schema 1, Schritte C, D (Methode 1), E, F, G, H (Methode 2) hergestellt. Im Schritt H (Ansatzgröße 0.25 mmol) wurde als Aminoverbindung der Formel III der (RS)-3-Amino-2-(n-butylsulfonylamino)-propionsäure-ethylester eingesetzt. Die Spaltung der Esterschutzgruppe in Schritt J erfolgte analog Methode 2 mit Lithiumhydroxid wie im Beispiel 183 beschrieben. Ausbeute: 259 mg (enthielt Lithiumtrifluoracetat).
ES(+)-MS: 749.4 $(M+H)^+$

Beispiel 196

(RS)-3-((S)-2-(4,4-Dimethyl-3-(4-(3-(2-methylphenyl)-ureido)-benzyl)-2,5-dioxoimidazolidin-1-yl)-2-(2-methylpropyl)-acetylamino)-3-phenyl-propionsäure

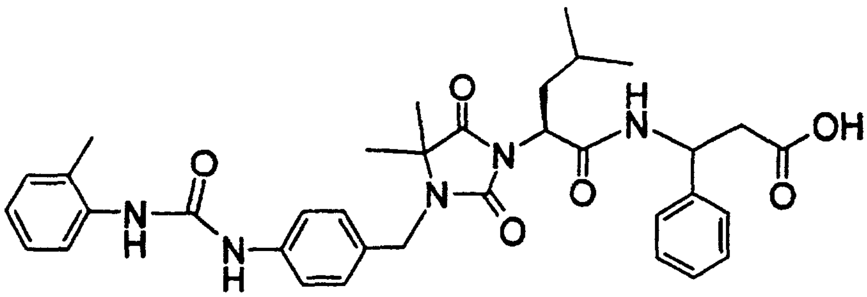

Die Verbindung wurde analog dem Verfahren gemäß Schema 5 durch Kupplung von Harz-gebundener (RS)-3-Amino-3-phenyl-propionsäure mit der entsprechenden, nach dem Verfahren gemäß Schema 1 hergestellten Hydantoincarbonsäure der Formel IIa hergestellt (Ansatzgröße der Kupplung: 0.05 mmol Verbindung der Formel IIa). Ausbeute: 4.2 mg
ES(+)-MS: 628.1 $(M+H)^+$

Beispiele 197 - 218

Die Verbindungen wurde analog dem Verfahren gemäß Schema 5 durch Kupplung der entsprechenden Harz-gebundenen 3-substituierten (RS)-3-Amino-propionsäure mit der entsprechenden, nach dem Verfahren gemäß Schema 1 hergestellten Hydantoincarbonsäure der Formel IIa hergestellt (Ansatzgröße der Kupplung: 0.05 mmol Verbindung der Formel IIa). Die hergestellten 3-substituierten (RS)-3-((RS)-(2-((RS)-4-Methyl-4-phenyl-3-(4-(3-(2-methylphenyl)-ureido)-benzyl)-2,5-dioxoimidazolidin-1-yl)-2-(2-methylpropyl)-acetylamino)-propionsäuren der Formel Iq sind in Tabelle 12 aufgeführt.

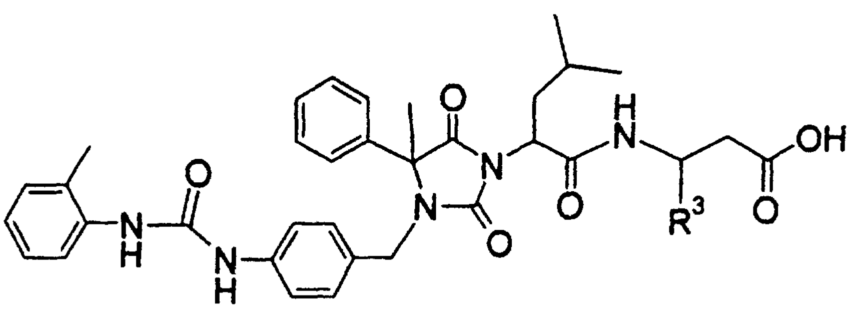

(Iq)

Tabelle 12:

| Beispiele der Formel Iq | | | |
|---|---|---|---|
| Beispiel Nr. | $R^3$ | Ausbeute | ES-(+)-MS $(M+H)^+$ |
| 197 | 2,3,5,6-Tetrafluorphenyl | 15.1 mg | 762.3 |
| 198 | 3-Methoxyphenyl | 9.7 mg | 720.3 |
| 199 | 3,4-Ethylendioxyphenyl | 9.8 mg | 748.3 |

Tabelle 12: (fortgesetzt)

| Beispiele der Formel Iq | | | |
|---|---|---|---|
| Beispiel Nr. | $R^3$ | Ausbeute | ES-(+)-MS $(M+H)^+$ |
| 200 | 4-Trifluormethoxyphenyl | 15.6 mg | 774.3 |
| 201 | 2,3-Dimethoxyphenyl | 10.0 mg | 750.5 |
| 202 | 2-Chlorphenyl | 14.6 mg | 724.3 |
| 203 | 3-Methylphenyl | 19.7 mg | 704.3 |
| 204 | 3,4-Difluorphenyl | 15.0 mg | 726.3 |
| 205 | 2,6-Difluorphenyl | 16.1 mg | 726.4 |
| 206 | tert-Butyl | 6.1 mg | 669.1 |
| 207 | 3-Fluorphenyl | 11.3mg | 708.2 |
| 208 | 2,4,4-Trimethylpentyl | 4.3 mg | 668.3 |
| 209 | 4-Chlorphenyl | 6.4 mg | 724.3 |
| 210 | 4-Dimethylamino-1-naphthyl | 0.8 mg | 783.4 |
| 211 | Bicyclo[2.2.1]hept-2-en-5-yl | 0.6 mg | 706.4 |
| 212 | n-Octyl | 0.5 mg | 726.0 |
| 213 | 4-Methoxy-2,3-dimethylphenyl | 4.3 mg | 765.2 $(M+NH_3)^+$ |
| 214 | 2-Fluorphenyl | 1.1 mg | 725.1 $(M+NH_3)^+$ |
| 215 | 2,3-Dichlorphenyl | 12.8 mg | 758.3 |
| 216 | 4-Fluorphenyl | 1.7 mg | 708.3 |
| 217 | 2-Chlor-5-nitrophenyl | 13.1 mg | 746.4 |
| 218 | 4-(n-Butyl)phenyl | 17.9 mg | 746.4 |

Beispiel 219

((RS)-2-((RS)-4-Methyl-4-phenyl-3-(4-(3-(2-methylphenyl)-ureido)-benzyl)-2,5-dioxoimidazolidin-1-yl)-2-(2-methylpropyl)-acetylamino)-L-aspartyl-L-phenylglycin-tert-butylester

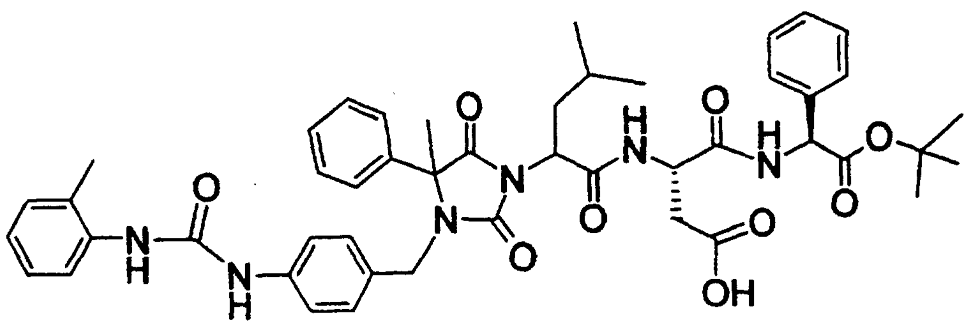

Die Verbindung wurde analog dem Verfahren gemäß Schema 4 durch Festphasensynthese hergestellt. Aspartyl-phenylglycin-tert-butylester, der an Chlortritylchlorid-Polystyrolharz gebunden war, wurde mit der entsprechenden, nach dem Verfahren gemäß Schema 1 hergestellten Hydantoincarbonsäure der Formel IIa gekuppelt (Ansatzgröße der Kupplung: 0.05 mmol Verbindung der Formel IIa). Die Abspaltung vom Harz erfolgte mit einer 10%igen Lösung von TFA in DCM für 20 Minuten. Ausbeute: 4.7 mg
ES(+)-MS: 846.9 $(M+H)^+$

Beispiel 220

(R)-3-(2-(4,4-Pentamethylen-3-(4-(3-(2-methylphenyl)-ureido)-benzyl)-2,5-dioxoimidazolidin-1-yl)-acetylamino)-3-methyl-propionsäure

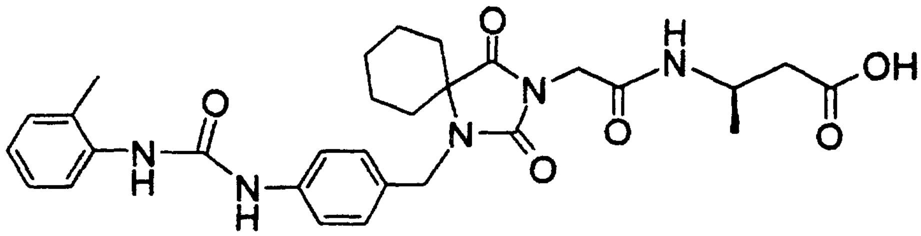

Die Verbindung kann nach dem Verfahren gemäß Schema 1, Schritte C, D (Methode 1), E, F, G, H (Methode 2), J (Methode 1) hergestellt werden. Im Schritt H wird als Aminoverbindung der Formel III der (R)-3-Amino-3-methyl-propionsäure-tert-butylester eingesetzt.

Beispiel 221

(R)-3-((S)-2-((S)4-(4-(Amino-imino-methyl)-phenyl)-4-methyl-3-(4-(3-(2-methylphenyl)-ureido)-benzyl)-2,5-dioxoimidazolidin-1-yl)-2-(2-methylpropyl)-acetylamino)-3-methyl-propionsäure

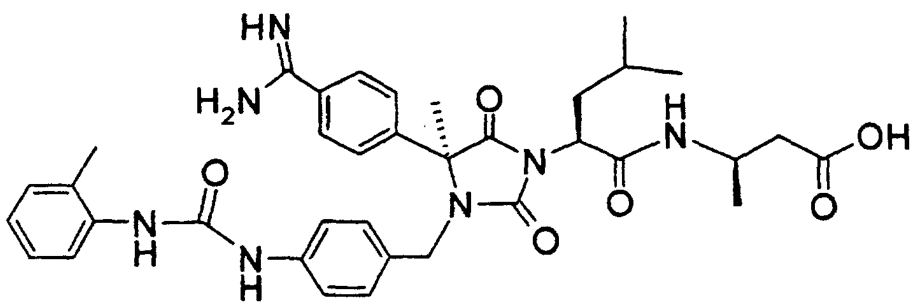

Die Verbindung kann nach dem Verfahren gemäß Schema 1, Schritte A, B, D (Methode 1), E, F, G, H (Methode 2), J (Methode 1) hergestellt werden. Im Schritt H wird als Aminoverbindung der Formel III (R)-3-Amino-3-methyl-propionsäure-tert-butylester eingesetzt.

Untersuchung der biologischen Aktivität

A) U937/VCAM-1 Zelladhäsionstest

Als Testmethode für die Wirksamkeit der Verbindungen der Formel I auf die Interaktion zwischen VCAM-1 und VLA-4 wird der im folgenden beschriebene Assay verwendet, der für diese Interaktion spezifisch ist. Die zellulären Bindungspartner, d. h. die VLA-4-Integrine, werden in ihrer natürlichen Form als Oberflächenmoleküle auf humanen U937-Zellen (ATCC CRL 1593), die zur Gruppe der Leukozyten gehören, angeboten. Als spezifische Bindungspartner werden gentechnisch hergestellte rekombinante lösliche Fusionsproteine, bestehend aus der extrazytoplasmatischen Domäne von humanen VCAM-1 und der konstanten Region eines humanen Immunglobulins der Subklasse IgG1, verwendet.

Assay zur Messung der Adhäsion von U937-Zellen (ATCC CRL 1593) an hVCAM-1(1-3)-IgG

1. Herstellung von humanem VCAM-1(1-3)-IgG und humanem CD4-IgG

Eingesetzt wurde ein genetisches Konstrukt zur Expression der extrazellulären Domäne des humanen VCAM-1, verbunden mit der genetischen Sequenz der schweren Kette des humanen Immunglobulins IgG1 (Hinge, CH2 und CH3 Regionen) (von Dr. Brian Seed, Massachusetts General Hospital, Boston, USA; vgl. Damle und Aruffo, Proc. Natl. Acad. Sci. USA 1991, 88, 6403-6407). Das lösliche Fusionsprotein hVCAM-1(1-3)-IgG enthielt die drei aminoterminalen extrazellulären Immunglobulin-ähnlichen Domänen des humanen VCAM-1 (Damle und Aruffo, Proc. Natl. Acad. Sci. USA 1991, 88, 6403-6407). CD4-IgG (Zettlmeissl et al., DNA and Cell Biology 1990, 9, 347) diente als Fusionsprotein für negative Kontrollen. Die rekombinanten Proteine wurden als lösliche Proteine nach DEAE/Dextranvermittelter DNA-Transfektion in COS-Zellen (ATCC CRL1651) gemäß Standardprozeduren exprimiert (Ausubel et al., Current protocols in molecular biology, John Wiley & Sons, Inc., 1994).

2. Assay zur Messung der Adhäsion von U937-Zellen an hVCAM-1 (1-3)-IgG

2.1 96 well-Mikrotitertestplatten (Nunc Maxisorb) wurden mit 100 µl/well einer Ziege-anti-human-IgG-Antikörperlösung (10 µg/ml in 50 mM Tris, pH 9.5) 1 Stunde bei Raumtemperatur inkubiert. Nach Entfernen der Antikörperlösung wurde einmal mit PBS gewaschen.

2.2 150 µl/well eines Blockierungspuffers (1 % BSA in PBS) wurden 0.5 Stunden bei Raumtemperatur auf den Platten inkubiert. Nach Entfernen des Blockierungspuffers wurde einmal mit PBS gewaschen.

2.3 100 µl pro well eines Zellkulturüberstandes von transfektierten COS-Zellen wurden für 1.5 Stunden bei Raumtemperatur auf den Platten inkubiert. Die COS-Zellen waren mit einem Plasmid transfiziert, welches für die drei N-terminalen Immunglobulin-ähnlichen Domänen des VCAM-1, gekoppelt an den Fc-Teil von humanem $IgG_1$ (hVCAM-1(1-3)-IgG), codiert. Der Gehalt an hVCAM-1(1-3)-IgG betrug ca. 0.5 - 1 µg/ml. Nach Entfernen des Kulturüberstandes wurde einmal mit PBS gewaschen.

2.4 Die Platten wurden mit 100 µl/well Fc-Rezeptor-Blockpuffer (1 mg/ml Y-Globulin, 100 mM NaCl, 100 µM $MgCl_2$, 100 µM $MnCl_2$, 100 µM $CaCl_2$, 1 mg/ml BSA in 50 mM HEPES, pH 7.5) für 20 Minuten bei Raumtemperatur inkubiert. Nach Entfernen des Fc-Rezeptor-Blockpuffers wurde einmal mit PBS gewaschen.

2.5 20 µl Bindungspuffer (100 mM NaCl, 100 µM $MgCl_2$, 100 µM $MnCl_2$, 100 µM $CaCl_2$, 1 mg/ml BSA in 50 mM HEPES, pH 7.5) wurden vorgelegt, die zu testenden Substanzen in 10 µl Bindungspuffer zugegeben und für 20 Minuten inkubiert. Als Kontrollen dienten Antikörper gegen VCAM-1 (BBT, Nr. BBA6) und gegen VLA-4 (Immunotech, Nr. 0764).

2.6 U937-Zellen wurden 20 Minuten in Fc-Rezeptor-Blockpuffer inkubiert und anschließend in einer Konzentration von 1 x $10^6$/ml und in einer Menge von 100 µl pro well zupipettiert (Endvolumen 125 µl/well).

2.7 Die Platten wurden in einem 45°-Winkel in Stoppuffer (100 mM NaCl, 100 µM $MgCl_2$, 100 µM $MnCl_2$, 100 µM $CaCl_2$ in 25 mM Tris, pH 7.5) langsam eingetaucht und ausgeschlagen. Der Vorgang wurde wiederholt.

2.8 Anschließend wurden 50 µl/well einer Färbelösung (16.7 µg/ml Hoechst Farbstoff 33258, 4 % Formaldehyd, 0.5 % Triton-X-100 in PBS) 15 Minuten auf den Platten inkubiert.

2.9 Die Platten wurden ausgeschlagen und in einem 45°-Winkel in Stop-Puffer (100 mM NaCl, 100 µM $MgCl_2$, 100 µM $MnCl_2$, 100 µM $CaCl_2$ in 25 mM Tris, pH 7.5) langsam eingetaucht. Der Vorgang wurde wiederholt. Anschließend wurden die Platten mit der enthaltenen Flüssigkeit (Stop-Puffer) in einem Cytofluorimeter (Millipore) gemessen (Sensitivität: 5, Filter: Anregungswellenlänge: 360 nm, Emissionswellenlänge: 460 nm).

Die Intensität des von den angefärbten U937-Zellen emittierten Lichts ist ein Maß für die Zahl der an der Platte verbliebenen, an das hVCAM-1(1-3)-IgG adhärierten U937-Zellen und somit ein Maß für die Fähigkeit der zugesetzten Testsubstanz, diese Adhäsion zu hemmen. Aus der Hemmung der Adhäsion bei verschiedenen Konzentrationen der Testsubstanz wurde die Konzentration $IC_{50}$ berechnet, die zu einer Hemmung der Adhäsion um 50 % führt.

3. Ergebnisse

Testergebnisse, die mit Verbindungen der Formel I erhalten wurden, sind in Tabelle 13 aufgeführt.

Tabelle 13

| Ergebnisse des U937/VCAM-1 Zelladhäsionstests | | | |
|---|---|---|---|
| Beispiel Nr. | IC $_{50}$ (nM) | Beispiel Nr. | IC $_{50}$ (nM) |
| 1 | 4 | 5 | 5 |
| 6 | 30 | 7 | 2.5 |
| 8 | 3.8 | 9 | 20 |
| 10 | 600 | 11 | 10 |
| 15 | 30 | 16 | 55 |
| 17 | 1.5 | 19 | 3 |
| 20 | 160 | 21 | 520 |
| 22 | 4 | 23 | 16 |
| 24 | 6 | 25 | 6 |
| 26 | 2900 | 27 | 27 |
| 28 | 110 | 29 | 890 |
| 30 | 580 | 34 | 490 |
| 38 | 400 | 41 | 1470 |
| 42 | 470 | 43 | 740 |
| 47 | 0.85 | 49 | 13 |
| 58 | 450 | 61 | 24.5 |
| 62 | 5 | 67 | 2.3 |
| 68 | 300 | 90 | 82 |
| 91 | 210 | 92 | 40 |
| 93 | 7 | 94 | 22 |
| 133 | 1.5 | 184 | 4 |
| 185 | 11 | 186 | 2.9 |
| 187 | 2.5 | 188 | 1.6 |
| 189 | 50 | 190 | 8 |
| 191 | 122 | 192 | 50 |
| 193 | 15 | 194 | 450 |
| 195 | 23 | 196 | 25 |
| 201 | 25 | 205 | 95 |
| 214 | 17 | 216 | 50 |
| 217 | 40 | 219 | 175 |

B) Leukozytenadhäsion an der Ratte

In dem Modell der Leukozytenadhäsion wird die Beeinflussung der Adhäsion von Leukozyten durch die Verbindungen der Formel I in Venolen der Ratte untersucht. Die Leukozytenadhäsion an das Endothel von postkapillaren Venolen wird als wichtiger Schritt bei Entzündungsreaktionen angesehen (J. M. Harlan, Blood 1985, 65, 513 - 525). Bei der Rekrutierung von Leukozyten aus dem Blut in entzündete Bereiche läuft eine wohlkoordinierte dynamische Sequenz von Ereignissen ab, in der chemotaktische Cytokine und zelluläre Adhäsionsmoleküle eine aktive Rolle spielen. Es wurde gefunden, daß VCAM-1/VLA-4-Wechselwirkungen eine entscheidende Rolle spielen bei der Adhäsion und Emigration von Leukozyten und der gesteigerten Permeabilität von Gefäßen für Makromoleküle, die durch verschiedene Mediatorsubstanzen und Cytokine induziert werden (D. Seiffge, Int. J. Microcirc. 1995, 15, 301 - 308). In dem vorliegenden Modell wird durch lokale oder systemische Injektion von Endotoxinen, zum Beispiel Zymosan, Bakterientoxinen wie Lipopolysacchariden (LPS) oder Freund's Adjuvanz, eine generalisierte Entzündung bzw. rheumatoide Arthritis hervorgerufen, die zu einer Adhäsion der Leukozyten und ihrer Emigration in erkrankte Organbereiche führt. Bestimmt wird die durch das Endotoxin hervorgerufene gesteigerte Adhäsion an das Endothel der Venolen.

Zur Bestimmung der Leukozytenadhäsion wurde ein Kamera-Umkehrmikroskop (Fa. Zeiss) verwendet, daß

mit einem Videosystem ausgerüstet war. Männlichen Sprague-Dawley Ratten (Körpergewicht ca. 250 g) wurde unter einer leichten Halothan-Prämedikation Zymosan oder Bakterienendotoxin injiziert. Die Kontrolltiere erhielten ein gleiches Volumen 0.9%ige Kochsalzlösung. Anschließend wurde den Tieren die Testsubstanz subkutan oder oral als Einmaldosis oder als Mehrfachdosis verabreicht. Für die Durchführung der Messung wurden die Ratten durch eine intramuskuläre Injektion von 1.25 g/kg Urethan betäubt. Man ließ sie durch einen Trachealtubus spontan atmen. Die Körpertemperatur wurde mittels einer regulierbaren Heizdecke bei 37 °C gehalten. Auf einem thermostatisierten (37 °C) Fenster des Mikroskoptisches wurde durch eine Bauchöffnung das Dünndarmgekröse vorsichtig freigelegt und bei 37 °C mit flüssigem Paraffin bedeckt. Mit drei stumpfen Nadeln und Knetmasse wurde der Ileocekalbereich des Gekröses in Position gehalten. Nach einer 30minütigen Äquilibrierungszeit, während der sich das Gewebe stabilisieren konnte, wurde in postkapillaren Venolen von 20 - 30 μm Durchmesser und ca. 100 μm Länge die Leukozytenadhäsion bestimmt durch Zählung in 2 - 3 Segmenten der Venolen in Abständen von 10 Minuten über 1 Stunde. Ein Leukozyt wurde als an das Endothel adhärent angesehen, wenn er für mehr als 30 Sekunden stationär war. Nach dem Experiment wurde die systemische Leukozytenzahl und der Fibrinogengehalt des Blutes bestimmt. Die Hemmung der Leukozytenadhäsion durch die Testsubstanz wird angegeben durch die Verringerung (in %) der Zahl der adhärenten Leukozyten in den behandelten Tieren im Vergleich zu der Zahl in den Kontrolltieren.

C) Hypersensitivität vom verzögerten Typ an der Maus

In dem Modell der Hypersensitivität vom verzögerten Typ (DTH; delayed-type hypersensitivity) wird die antiallergische bzw. entzündungshemmende Wirkung der Verbindungen der Formel I untersucht. DTH ist eine entzündliche Reaktion der Haut, die durch eine Sensibilisierung mit antigenen Substanzen ausgelöst wird. Um die entsprechende Entzündungsreaktion und die Leukozyten-Rekrutierung in den entzündeten Bereichen in vivo zu ermitteln, werden die Substanzen in dem folgenden DTH-Modell an der Maus getestet (siehe auch T. B. Issekutz, J. Immunol. 1991, 147, 4178 - 4184).

Gruppen von weiblichen BALB/c-Mäusen (Körpergewicht ca. 20 g) wurden an einem rasierten Teil der Haut epikutan mit 150 μl einer 3%igen Lösung von Oxazolon sensibilisiert, das eine starke entzündliche DTH-Reaktion induziert. 6 Tage später wurde die Reaktion durch Gabe von 20 μl einer 1%igen Oxazolonlösung am rechten Ohr der Tiere ausgelöst. Die Testsubstanzen wurden jeweils 44 Stunden vor der Auslösung der Reaktion, 20 Stunden vor der Auslösung und 4 Stunden nach der Auslösung subkutan oder oral verabreicht. Direkt vor der Auslösung der Reaktion und 24 Stunden nach der Auslösung wurde mit einem Mitutoyo Engineering-Mikrometer die durch die entzündliche Schwellung des Ohres veränderte Ohrdicke am rechten Ohr gemessen. Die Differenz zwischen diesen beiden Messungen wurde für jedes Tier der Gruppe ermittelt. Die Mittelwerte der Differenzen einer mit der Testsubstanz behandelten Tiergruppe einerseits und einer unbehandelten Kontrollgruppe andererseits werden verglichen. Angegeben wird die prozentuale Inhibition der Ohrschwellung.

D) Anti-asthmatische Wirkung am Meerschweinchen

Die Beeinflussung der Lungenfunktion und die anti-asthmatische Wirkung der Verbindungen der Formel I kann in einem Modell am Meerschweinchen bestimmt werden, das sich an die von G. Moacevic, Arch. Toxicol. 1975, 34,1, beschriebene Methode anlehnt. Dazu werden die technischen Vorbereitungen für die Untersuchung entsprechend den von Moacevic beschriebenen Einzelheiten durchgeführt. Eingesetzt werden männliche Albino-Meerschweinchen mit einem Körpergewicht von 300 - 500 g. Die Tiere werden in einen Plethysmograph (Fa. FMI) gesetzt und drei Ausgangswerte der Parameter Atemfrequenz und Atemamplitude werden aufgenommen. In diesem Modell ist eine asthmatische Atmung charakterisiert durch die Abnahme der Atemamplitude (= Verringerung des Atemvolumens aufgrund der Bronchokonstriktion) und die Zunahme der Atemfrequenz (= Reflexreaktion). Dieser Zustand ist in Asthmapatienten als Dyspnoe bekannt.

Die Albino-Meerschweinchen werden 22 Tage vor dem Beginn der Studie sensibilisiert mit 1 ml pro Tier einer 0.1%igen Ovalbuminlösung an zwei aufeinander folgenden Tagen. Der experimentelle Asthma-Anfall wird durch Inhalation einer 0.3%igen Ovalbuminlösung für 1 Minute ausgelöst. Nach einer Erholungsphase von 40 - 60 Minuten inhalieren die Tiere die Testsubstanz als wäßrige Lösung. Sofort danach wird für 1 Minute 0.3%ige Ovalbuminlösung verabreicht. In der folgenden Erholungsphase von 30 Minuten atmen die Tiere normale Luft. Dieses Verfahren wird zweimal wiederholt. Wenn die Asthma-Anfälle lebensbedrohlich werden, wird den Tieren Sauerstoff verabreicht.

**Patentansprüche**

1. Verbindung der Formel I,

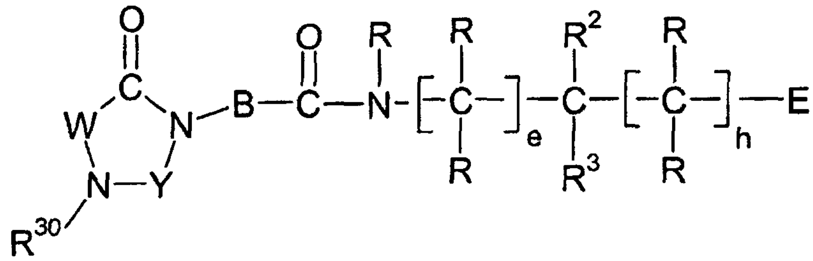

(I)

worin

W für einen zweiwertigen Rest aus der Reihe $R^1$-A-C($R^{13}$) und

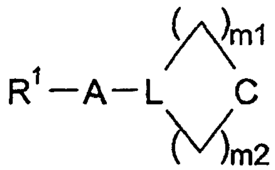

steht, worin die Ringsysteme

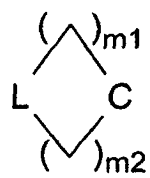

ein oder zwei gleiche oder verschiedene Heteroatome aus der Reihe N und O enthalten können, gesättigt oder einfach ungesättigt sein können und durch 1 oder 2 gleiche oder verschiedene Substituenten $R^{13}$ und/oder durch ein oder zwei doppelt gebundene Sauerstoffatome substituiert sein können, und worin L für C($R^{13}$) oder N steht und worin m1 und m2 unabhängig voneinander für eine der Zahlen 0, 1, 2, 3 und 4 stehen, die Summe m1 + m2 aber für eine der Zahlen 1, 2, 3 und 4 steht;

Y für eine Carbonylgruppe oder Thiocarbonylgruppe steht;

A für eine direkte Bindung, einen der zweiwertigen Reste ($C_1$-$C_6$)-Alkylen, ($C_5$-C6)-Cycloalkylen, Phenylen, Phenylen-($C_1$-$C_4$)-alkyl oder für einen zweiwertigen Rest eines 5-gliedrigen oder 6-gliedrigen gesättigten oder ungesättigten Heterocyclus, der ein oder zwei Stickstoffatome enthalten kann und einfach oder zweifach durch ($C_1$-$C_6$)-Alkyl oder doppelt gebundenen Sauerstoff oder Schwefel substituiert sein kann, steht, wobei in dem Rest Phenylenalkyl der Rest $R^1$ an die Phenylengruppe gebunden ist;

B für einen zweiwertigen Methylenrest oder Ethylenrest steht, wobei der Methylenrest und der Ethylenrest unsubstituiert sind oder substituiert sind durch einen oder zwei gleiche oder verschiedene Reste aus der Reihe ($C_1$-$C_8$)-Alkyl, ($C_2$-$C_8$)-Alkenyl, ($C_2$-$C_8$)-Alkinyl, ($C_3$-$C_6$)-Cycloalkyl, ($C_3$-$C_6$)-Cycloalkyl-($C_1$-$C_6$)-alkyl, gegebenenfalls substituiertes ($C_6$-$C_{10}$)-Aryl, im Arylrest gegebenenfalls substituiertes ($C_6$-$C_{10}$)-Aryl-($C_1$-$C_6$)-alkyl, gegebenenfalls substituiertes Heteroaryl, im Heteroarylrest gegebenenfalls substituiertes Heteroaryl-($C_1$-$C_6$)-alkyl;

E für $R^{10}$CO, HO-$CH_2$ oder $R^8$CO-O-$CH_2$ steht;

R für Wasserstoff oder ($C_1$-$C_8$)-Alkyl steht, wobei alle Reste R unabhängig voneinander sind und die Reste R gleich oder verschieden sein können;

$R^1$ für Wasserstoff, ($C_1$-$C_{10}$)-Alkyl, das gegebenenfalls durch Fluor einfach oder mehrfach substituiert sein kann, im Arylrest gegebenenfalls substituiertes $R^{21}$-(($C_6$-$C_{10}$)-Aryl), im Arylrest gegebenenfalls substituiertes ($R^{21}$-(($C_6$-$C_{10}$)-Aryl))-($C_1$-$C_6$)-alkyl, den Rest Het-, Het-($C_1$-$C_6$)-alkyl oder für einen der Reste X-NH-C(=NH)-$R^{20}$-, $X^1$-NH-$R^{20}$-, $R^{22}$N($R^{21}$)-C(O)-, O= und S= steht;

x für Wasserstoff, ($C_1$-$C_6$)-Alkyl, ($C_1$-$C_6$)-Alkylcarbonyl, ($C_1$-$C_6$)-Alkoxycarbonyl, ($C_1$-$C_8$)-Alkylcarbonyloxy-($C_1$-$C_6$)-alkoxycarbonyl, gegebenenfalls substituiertes ($C_6$-$C_{10}$)-Arylcarbonyl, gegebenenfalls sub-

stituiertes $(C_6-C_{10})$-Aryloxycarbonyl, $(C_6-C_{14})$-Aryl-$(C_1-C_6)$-alkoxycarbonyl, das im Arylrest auch substituiert sein kann, Hydroxy, $(C_1-C_6)$-Alkoxy oder Amino steht;

$X^1$ eine der Bedeutungen von X hat oder für R'-NH-C(=N-R") steht, worin R' und R" unabhängig voneinander die Bedeutungen von X haben;

R2 für Wasserstoff oder $(C_1-C_8)$-Alkyl steht;

R3 für Wasserstoff, $(C_1-C_8)$-Alkyl, das gegebenenfalls durch 1 bis 6 Fluoratome substituiert sein kann, gegebenenfalls substituiertes $(C_6-C_{12})$-Aryl, im Arylrest gegebenenfalls substituiertes $(C_6-C_{12})$-Aryl-$(C_1-C_6)$-alkyl, gegebenenfalls substituiertes Heteroaryl, im Heteroarylrest gegebenenfalls substituiertes Heteroaryl-$(C_1-C_6)$-alkyl, $(C_3-C_8)$-Cycloalkyl, $(C_3-C_8)$-Cycloalkyl-$(C_1-C_6)$-alkyl, $(C_6-C_{12})$-Bicycloalkyl, $(C_6-C_{12})$-Bicycloalkyl-$(C_1-C_6)$-alkyl, $(C_6-C_{12})$-Tricycloalkyl, $(C_6-C_{12})$-Tricycloalkyl-$(C_1-C_6)$-alkyl, $(C_2-C_8)$-Alkenyl, $(C_2-C_8)$-Alkinyl, $R^{11}$NH, $COOR^{21}$, $CON(CH_3)R^4$, $CONHR^4$, $CON(CH_3)R^{15}$ oder $CONHR^{15}$ steht;

$R^4$ für $(C_1-C_8)$-Alkyl steht, das unsubstituiert ist oder einfach oder zweifach substituiert ist durch gleiche oder verschiedene Reste aus der Reihe Hydroxy, $(C_1-C_8)$-Alkoxy, $R^5$, gegebenenfalls substituiertes $(C_3-C_8)$-Cycloalkyl, Hydroxycarbonyl, Aminocarbonyl, $(C_6-C_{10})$-Aryl-$(C_1-C_4)$-alkoxycarbonyl, das im Arylrest auch substituiert sein kann, $(C_1-C_6)$-Alkoxycarbonyl, $R^6$-CO, $R^7$-CO, Tetrazolyl, Trifluormethyl;

$R^5$ für gegebenenfalls substituiertes $(C_6-C_{12})$-Aryl, im Arylrest gegebenenfalls substituiertes $(C_6-C_{12})$-Aryl-$(C_1-C_8)$-alkyl oder einen Rest eines gegebenenfalls substituierten monocyclischen oder bicyclischen, 5-gliedrigen bis 12-gliedrigen heterocyclischen Ringes, der aromatisch, teilweise gesättigt oder vollständig gesättigt sein kann und der ein, zwei oder drei gleiche oder verschiedene Heteroatome aus der Reihe Stickstoff, Sauerstoff und Schwefel enthalten kann, steht;

$R^6$ für den Rest einer natürlichen oder unnatürlichen Aminosäure, Iminosäure, gegebenenfalls N-$(C_1-C_8)$-alkylierten oder N-($(C_6-C_{12})$-Aryl-$(C_1-C_8)$-alkylierten) Azaaminosäure, die im Arylrest auch substituiert sein kann, oder den Rest eines Dipeptids oder Tripeptids steht, sowie für deren Ester und Amide, worin freie funktionelle Gruppen durch in der Peptidchemie übliche Schutzgruppen geschützt sein können und worin die Stickstoffatome in den Amidbindungen in der Gruppe $R^6$-CO einen Rest R als Substituenten tragen können;

$R^7$ für den Rest eines über ein Stickstoffatom gebundenen 5-gliedrigen bis 7-gliedrigen, gesättigten monocyclischen oder bicyclischen Heterocyclus steht, der ein, zwei, drei oder vier gleiche oder verschiedene zusätzliche Ring-Heteroatome aus der Reihe Sauerstoff, Stickstoff und Schwefel enthalten kann und der an Kohlenstoffatomen und an zusätzlichen Ring-Stickstoffatomen gegebenenfalls substituiert sein kann, worin zusätzliche Ring-Stickstoffatome gleiche oder verschiedene Reste aus der Reihe Wasserstoff, $R^h$, HCO, $R^h$CO, $R^h$O-CO, HO-CO-$(C_1-C_4)$-Alkyl und $R^h$O-CO-$(C_1-C_4)$-Alkyl als Substituenten tragen können und $R^h$ für $(C_1-C_6)$-Alkyl, $(C_3-C_8)$-Cycloalkyl, $(C_3-C_8)$-Cycloalkyl-$(C_1-C_8)$-alkyl, gegebenenfalls substituiertes $(C_6-C_{10})$-Aryl oder im Arylrest gegebenenfalls substituiertes $(C_6-C_{10})$-Aryl-$(C_1-C_4)$-alkyl steht;

$R^8$ für Wasserstoff, $(C_1-C_6)$-Alkyl oder im Phenylrest gegebenenfalls substituiertes Phenyl-$(C_1-C_4)$-alkyl steht;

$R^{10}$ für Hydroxy, $(C_1-C_8)$-Alkoxy, $(C_6-C_{10})$-Aryl-$(C_1-C_6)$-alkoxy, das im Arylrest auch substituiert sein kann, gegebenenfalls substituiertes $(C_6-C_{10})$-Aryloxy, $(C_1-C_6)$-Alkylcarbonyloxy-$(C_1-C_6)$-alkoxy, $(C_1-C_6)$-Alkoxycarbonyloxy-$(C_1-C_6)$-alkoxy, Amino, Mono- oder Di-($(C_1-C_6)$-alkyl)-amino, Aminocarbonyl-$(C_1-C_6)$-alkoxy oder (Mono- oder Di-($(C_1-C_6)$-alkyl)-amino)-carbonyl-$(C_1-C_6)$-alkoxy steht;

$R^{11}$ für Wasserstoff, $R^{12a}$, $R^{12a}$-CO, $R^{12a}$-O-CO, $R^{12b}$-CO oder $R^{12a}$-$S(O)_2$ steht;

$R^{12a}$ für $(C_1-C_{10})$-Alkyl, $(C_2-C_8)$-Alkenyl, $(C_2-C_8)$-Alkinyl, $(C_5-C_{10})$-Cycloalkyl, $(C_5-C_{10})$-Cycloalkyl-$(C_1-C_8)$-alkyl, gegebenenfalls substituiertes $(C_6-C_{14})$-Aryl, im Arylrest gegebenenfalls substituiertes $(C_6-C_{14})$-Aryl-$(C_1-C_8)$-alkyl, gegebenenfalls substituiertes Heteroaryl, im Heteroarylrest gegebenenfalls substituiertes Heteroaryl-$(C_1-C_8)$-alkyl oder den Rest $R^{15}$ steht;

$R^{12b}$ für Amino, Di-($(C_1-C_{10})$-alkyl)-amino oder $R^{12a}$-NH steht;

$R^{13}$ für Wasserstoff oder $(C_1-C_6)$-Alkyl steht;

$R^{15}$ für $R^{16}$-$(C_1-C_6)$-alkyl oder für $R^{16}$ steht;

$R^{16}$ für einen 6-gliedrigen bis 14-gliedrigen bicyclischen oder tricyclischen Rest steht, der gesättigt oder teilweise ungesättigt ist und der auch ein, zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe $(C_1-C_4)$-Alkyl und Oxo substituiert sein kann;

$R^{20}$ für eine direkte Bindung oder $(C_1-C_2)$-Alkylen steht;

$R^{21}$ für Wasserstoff, $(C_1-C_6)$-Alkyl, $(C_3-C_6)$-Cycloalkyl, $(C_3-C_6)$-Cycloalkyl-$(C_1-C_4)$-alkyl, gegebenenfalls substituiertes $(C_6-C_{10})$-Aryl, im Arylrest gegebenenfalls substituiertes $(C_6-C_{10})$-Aryl-$(C_1-C_4)$-alkyl, den

Rest Het- oder Het-($C_1$-$C_4$)-alkyl steht, wobei Alkylreste durch Fluor einfach oder mehrfach substituiert sein können und die Reste $R^{21}$ bei mehrfachem Auftreten gleich oder verschieden sein können;

$R^{22}$ für einen der Reste $R^{21}$-, $R^{21}N(R^{21})$- oder $R^{21}N(R^{21})$-C(=N($R^{21}$))- steht;

$R^{30}$ für einen der Reste $R^{32}$(R)N-CO-N(R)-$R^{31}$, $R^{32}$(R)N-CS-N(R)-$R^{31}$, $R^{32}$-CO-N(R)-$R^{31}$ oder $R^{32}$(R)N-CO-$R^{31}$ steht, wobei $R^{30}$ nicht für $R^{32}$-CO-N(R)-$R^{31}$ stehen kann, wenn gleichzeitig W für $R^1$-A-C($R^{13}$) steht, A für eine direkte Bindung steht und $R^1$ und $R^{13}$ für Wasserstoff stehen;

$R^{31}$ für den zweiwertigen Rest -$R^{33}$-$R^{34}$-$R^{35}$-$R^{36}$- steht, wobei $R^{36}$ an das Stickstoffatom im Imidazolidinring in der Formel I gebunden ist;

$R^{32}$ für Wasserstoff, ($C_1$-$C_6$)-Alkyl, das gegebenenfalls durch 1 bis 6 Fluoratome substituiert sein kann, ($C_2$-$C_6$)-Alkenyl, ($C_2$-$C_6$)-Alkinyl, ($C_5$-$C_6$)-Cycloalkyl, ($C_5$-$C_6$)-Cycloalkyl-($C_1$-$C_6$)-alkyl, gegebenenfalls substituiertes ($C_6$-$C_{10}$)-Aryl, im Arylrest gegebenenfalls substituiertes ($C_6$-$C_{10}$)-Aryl-($C_1$-$C_6$)-alkyl, gegebenenfalls substituiertes Heteroaryl oder im Heteroarylrest gegebenenfalls substituiertes Heteroaryl-($C_1$-$C_6$)-alkyl steht;

$R^{33}$ für eine direkte Bindung oder einen zweiwertigen ($C_1$-$C_4$)-Alkylenrest steht;

$R^{34}$ für einen zweiwertigen Rest aus der Reihe ($C_1$-$C_6$)-Alkylen, ($C_5$-$C_6$)-Cycloalkylen, gegebenenfalls substituiertes ($C_6$-$C_{10}$)-Arylen und gegebenenfalls substituiertes Heteroarylen steht;

$R^{35}$ für eine direkte Bindung oder einen zweiwertigen ($C_1$-$C_4$)-Alkylenrest steht;

$R^{36}$ für eine direkte Bindung, die Gruppe -CO- oder die Gruppe -S(O)$_n$- steht;

Het für einen Rest eines monocyclischen oder polycyclischen, 5-gliedrigen bis 12-gliedrigen, aromatischen oder nicht aromatischen Ringes steht, der 1 oder 2 gleiche oder verschiedene Heteroatome aus der Reihe N und O als Ringglieder enthält und gegebenenfalls durch einen oder mehrere, gleiche oder verschiedene Substituenten substituiert sein kann;

e und h unabhängig voneinander für 0 oder 1 stehen;

n für 1 oder 2 steht;

in allen ihren stereoisomeren Formen und Mischungen davon in allen Verhältnissen, und ihre physiologisch verträglichen Salze.

**2.** Verbindung der Formel I gemäß Anspruch 1, worin

W für den zweiwertigen Rest $R^1$-A-C($R^{13}$) steht;

Y für eine Carbonylgruppe steht;

A für eine direkte Bindung, einen der zweiwertigen Reste ($C_1$-$C_6$)-Alkylen, Phenylen, Phenylen-($C_1$-$C_2$)-alkyl oder für einen zweiwertigen Rest eines 5gliedrigen oder 6-gliedrigen gesättigten oder ungesättigten Heterocyclus, der ein oder zwei Stickstoffatome enthalten kann und einfach oder zweifach durch ($C_1$-$C_6$)-Alkyl oder doppelt gebundenen Sauerstoff oder Schwefel substituiert sein kann, steht, wobei in dem Rest Phenylenalkyl der Rest $R^1$ an die Phenylengruppe gebunden ist;

B für einen zweiwertigen Methylenrest oder Ethylenrest steht, wobei der Methylenrest und der Ethylenrest unsubstituiert sind oder substituiert sind durch einen Rest aus der Reihe ($C_1$-$C_8$)-Alkyl, ($C_2$-$C_8$)-Alkenyl, ($C_2$-$C_8$)-Alkinyl, ($C_3$-$C_6$)-Cycloalkyl, ($C_3$-$C_6$)-Cycloalkyl-($C_1$-$C_6$)-alkyl, gegebenenfalls substituiertes ($C_6$-$C_{10}$)-Aryl, im Arylrest gegebenenfalls substituiertes ($C_6$-$C_{10}$)-Aryl-($C_1$-$C_6$)-alkyl, gegebenenfalls substituiertes Heteroaryl, im Heteroarylrest gegebenenfalls substituiertes Heteroaryl-($C_1$-$C_6$)-alkyl;

E für $R^{10}$CO, HO-$CH_2$ oder $R^8$CO-O-$CH_2$ steht;

R für Wasserstoff oder ($C_1$-$C_8$)-Alkyl steht, wobei alle Reste R unabhängig voneinander sind und die Reste R gleich oder verschieden sein können;

$R^1$ für Wasserstoff, ($C_1$-$C_{10}$)-Alkyl, das gegebenenfalls durch Fluor einfach oder mehrfach substituiert sein kann, im Arylrest gegebenenfalls substituiertes $R^{21}$-(($C_6$-$C_{10}$)-Aryl), im Arylrest gegebenenfalls substituiertes ($R^{21}$-(($C_6$-$C_{10}$)-Aryl))-($C_1$-$C_6$)-alkyl, den Rest Het-, Het-($C_1$-$C_4$)-alkyl oder für einen der Reste X-NH-C(=NH)-$R^{20}$-, $X^1$-NH-$R^{20}$- und O= steht;

X für Wasserstoff, ($C_1$-$C_6$)-Alkyl, ($C_1$-$C_6$)-Alkylcarbonyl, ($C_1$-$C_6$)-Alkoxycarbonyl, ($C_1$-$C_6$)-Alkylcarbonyloxy-($C_1$-$C_6$)-alkoxycarbonyl, gegebenenfalls substituiertes ($C_6$-$C_{10}$)-Arylcarbonyl, gegebenenfalls substituiertes ($C_6$-$C_{10}$)-Aryloxycarbonyl, ($C_6$-$C_{14}$)-Aryl-($C_1$-$C_6$)-alkoxycarbonyl, das im Arylrest auch substituiert sein kann, Hydroxy, ($C_1$-$C_6$)-Alkoxy oder Amino steht;

$X^1$ eine der Bedeutungen von X hat oder für R'-NH-C(=N-R") steht, worin R' und R" unabhängig voneinander die Bedeutungen von X haben;

$R^2$ für Wasserstoff oder ($C_1$-$C_6$)-Alkyl steht;

$R^3$ für Wasserstoff, ($C_1$-$C_8$)-Alkyl, das gegebenenfalls durch 1 bis 6 Fluoratome substituiert sein kann, gegebenenfalls substituiertes ($C_6$-$C_{10}$)-Aryl, im Arylrest gegebenenfalls substituiertes ($C_6$-$C_{10}$)

-Aryl-($C_1$-$C_4$)-alkyl, gegebenenfalls substituiertes Heteroaryl, im Heteroarylrest gegebenenfalls substituiertes Heteroaryl-($C_1$-$C_4$)-alkyl, ($C_3$-$C_8$)-Cycloalkyl, ($C_3$-$C_8$)-Cycloalkyl-($C_1$-$C_4$)-alkyl, ($C_6$-$C_{12}$)-Bicycloalkyl, ($C_6$-$C_{12}$)-Bicycloalkyl-($C_1$-$C_4$)-alkyl, ($C_6$-$C_{12}$)-Tricycloalkyl, ($C_6$-$C_{12}$)-Tricycloalkyl-($C_1$-$C_4$)-alkyl, ($C_2$-$C_8$)-Alkenyl, ($C_2$-$C_8$)-Alkinyl, $R^{11}$NH, $COOR^{21}$, $CON(CH_3)R^4$, $CONHR^4$, $CON(CH_3)R^{15}$ oder $CONHR^{15}$ steht;

$R^4$ für ($C_1$-$C_6$)-Alkyl steht, das unsubstituiert ist oder einfach oder zweifach substituiert ist durch gleiche oder verschiedene Reste aus der Reihe Hydroxy, ($C_1$-$C_8$)-Alkoxy, $R^5$, gegebenenfalls substituiertes ($C_3$-$C_8$)-Cycloalkyl, Hydroxycarbonyl, Aminocarbonyl, ($C_6$-$C_{10}$)-Aryl-($C_1$-$C_4$)-alkoxycarbonyl, das im Arylrest auch substituiert sein kann, ($C_1$-$C_6$)-Alkoxycarbonyl, $R^6$-CO, $R^7$-CO, Tetrazolyl, Trifluormethyl;

$R^5$ für gegebenenfalls substituiertes ($C_6$-$C_{10}$)-Aryl, im Arylrest gegebenenfalls substituiertes ($C_6$-$C_{10}$) -Aryl-($C_1$-$C_4$)-alkyl oder einen Rest eines gegebenenfalls substituierten monocyclischen oder bicyclischen, 5-gliedrigen bis 12-gliedrigen heterocyclischen Ringes, der aromatisch, teilweise gesättigt oder vollständig gesättigt sein kann und der ein, zwei oder drei gleiche oder verschiedene Heteroatome aus der Reihe Stickstoff, Sauerstoff und Schwefel enthalten kann, steht;

$R^6$ für einen Rest einer natürlichen oder unnatürlichen Aminosäure oder den Rest eines Dipeptids oder Tripeptids steht, sowie für deren Ester und Amide, worin freie funktionelle Gruppen durch in der Peptidchemie übliche Schutzgruppen geschützt sein können und worin die Stickstoffatome in den Amidbindungen in der Gruppe $R^6$-CO einen Rest R als Substituenten tragen können;

$R^7$ für den Rest eines über ein Stickstoffatom gebundenen 5-gliedrigen bis 7-gliedrigen, gesättigten monocyclischen Heterocyclus steht, der ein oder zwei gleiche oder verschiedene zusätzliche Ring-Heteroatome aus der Reihe Sauerstoff, Stickstoff und Schwefel enthalten kann und der an Kohlenstoffatomen und an zusätzlichen Ring-Stickstoffatomen gegebenenfalls substituiert sein kann, worin zusätzliche Ring-Stickstoffatome gleiche oder verschiedene Reste aus der Reihe Wasserstoff, $R^h$, HCO, $R^h$CO, $R^h$O-CO, HO-CO-($C_1$-$C_4$)-Alkyl und $R^h$O-CO-($C_1$-$C_4$)-Alkyl als Substituenten tragen können und $R^h$ für ($C_1$-$C_4$)-Alkyl, gegebenenfalls substituiertes ($C_6$-$C_{10}$)-Aryl oder im Arylrest gegebenenfalls substituiertes ($C_6$-$C_{10}$)-Aryl-($C_1$-$C_4$)-alkyl steht;

$R^8$ für Wasserstoff, ($C_1$-$C_6$)-Alkyl oder im Phenylrest gegebenenfalls substituiertes Phenyl-($C_1$-$C_4$)-alkyl steht;

$R^{10}$ für Hydroxy, ($C_1$-$C_8$)-Alkoxy, ($C_6$-$C_{10}$)-Aryl-($C_1$-$C_6$)-alkoxy, das im Arylrest auch substituiert sein kann, gegebenenfalls substituiertes ($C_6$-$C_{10}$)-Aryloxy, ($C_1$-$C_6$)-Alkylcarbonyloxy-($C_1$-$C_6$)-alkoxy, ($C_1$-$C_6$)-Alkoxycarbonyloxy-($C_1$-$C_6$)-alkoxy, Amino, Mono- oder Di-(($C_1$-$C_6$)-alkyl)-amino, Aminocarbonyl-($C_1$-$C_6$)-alkoxy oder (Mono- oder Di-(($C_1$-$C_6$)-alkyl)-amino)-carbonyl-($C_1$-$C_6$)-alkoxy steht;

$R^{11}$ für Wasserstoff, $R^{12a}$, $R^{12a}$-CO, $R^{12a}$-O-CO, $R^{12b}$-CO oder $R^{12a}$-$S(O)_2$ steht;

$R^{12a}$ für ($C_1$-$C_8$)-Alkyl, ($C_2$-$C_8$)-Alkenyl, ($C_2$-$C_8$)-Alkinyl, ($C_5$-$C_6$)-Cycloalkyl, ($C_5$-$C_6$)Cycloalkyl-($C_1$-$C_4$)-alkyl, gegebenenfalls substituiertes ($C_6$-$C_{10}$)-Aryl, im Arylrest gegebenenfalls substituiertes ($C_6$-$C_{10}$) -Aryl-($C_1$-$C_4$)-alkyl, gegebenenfalls substituiertes Heteroaryl, im Heteroarylrest gegebenenfalls substituiertes Heteroaryl-($C_1$-$C_4$)-alkyl oder den Rest $R^{15}$ steht;

$R^{12b}$ für Amino, Di-(($C_1$-$C_8$)-alkyl)-amino oder $R^{12a}$-NH steht;

$R^{13}$ für Wasserstoff oder ($C_1$-$C_6$)-Alkyl steht;

$R^{15}$ für $R^{16}$-($C_1$-$C_6$)-alkyl oder für $R^{16}$ steht;

$R^{16}$ für einen 6-gliedrigen bis 12-gliedrigen bicyclischen oder tricyclischen Rest steht, der gesättigt oder teilweise ungesättigt ist und der auch ein, zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe ($C_1$-$C_4$)-Alkyl und Oxo substituiert sein kann;

$R^{20}$ für eine direkte Bindung oder Methylen steht;

$R^{21}$ für Wasserstoff, ($C_1$-$C_6$)-Alkyl, gegebenenfalls substituiertes ($C_6$-$C_{10}$)-Aryl, im Arylrest gegebenenfalls substituiertes ($C_6$-$C_{10}$)-Aryl-($C_1$-$C_2$)-alkyl, den Rest Het- oder Het-($C_1$-$C_2$)-alkyl steht, wobei Alkylreste einfach bis vierfach durch Fluor substituiert sein können und die Reste $R^{21}$ bei mehrfachem Auftreten gleich oder verschieden sein können;

$R^{30}$ für einen der Reste $R^{32}$(R)N-CO-N(R)-$R^{31}$ oder $R^{32}$(R)N-CS-N(R)-$R^{31}$ steht;

$R^{31}$ für einen zweiwertigen Rest aus der Reihe ($C_1$-$C_6$)-Alkylen, gegebenenfalls substituiertes ($C_6$-$C_{10}$)-Arylen, im Arylenrest gegebenenfalls substituiertes ($C_6$-$C_{10}$)-Arylen-($C_1$-$C_4$)-alkyl, ($C_5$-$C_6$)-Cycloalkylen, ($C_5$-$C_6$)-Cycloalkylen-($C_1$-$C_4$)-alkyl, gegebenenfalls substituiertes Heteroarylen oder im Heteroarylenrest gegebenenfalls substituiertes Heteroarylen-($C_1$-$C_4$)-alkyl steht, wobei im Falle des Arylenalkylrestes, des Cycloalkylenalkylrestes und des Heteroarylenalkylrestes die Alkylgruppe an das Stickstoffatom im Imidazolidinring in der Formel I gebunden ist;

$R^{32}$ für Wasserstoff, $(C_1\text{-}C_6)$-Alkyl, das gegebenenfalls durch 1 bis 6 Fluoratome substituiert sein kann, $(C_2\text{-}C_6)$-Alkenyl, $(C_2\text{-}C_6)$-Alkinyl, $(C_5\text{-}C_6)$-Cycloalkyl, $(C_5\text{-}C_6)$-Cycloalkyl-$(C_1\text{-}C_4)$-alkyl, gegebenenfalls substituiertes $(C_6\text{-}C_{10})$-Aryl, im Arylrest gegebenenfalls substituiertes $(C_6\text{-}C_{10})$-Aryl-$(C_1\text{-}C_4)$-alkyl, gegebenenfalls substituiertes Heteroaryl oder im Heteroarylrest gegebenenfalls substituiertes Heteroaryl-$(C_1\text{-}C_4)$-alkyl steht;

Het für einen Rest eines monocyclischen oder polycyclischen, 5-gliedrigen bis 10-gliedrigen, aromatischen oder nicht aromatischen Ringes steht, der 1 oder 2 gleiche oder verschiedene Heteroatome aus der Reihe N und O als Ringglieder enthält und gegebenenfalls durch einen oder mehrere, gleiche oder verschiedene Substituenten substituiert sein kann;

e und h unabhängig voneinander für 0 oder 1 stehen;

in allen ihren stereoisomeren Formen und Mischungen davon in allen Verhältnissen, und ihre physiologisch verträglichen Salze.

**3.** Verbindung der Formel I gemäß Anspruch 1 und/oder 2, worin W eine andere Bedeutung als $CH_2$ hat, in allen ihren stereoisomeren Formen und Mischungen davon in allen Verhältnissen, und ihre physiologisch verträglichen Salze.

**4.** Verbindung der Formel I gemäß einem oder mehreren der Ansprüche 1 bis 3, worin W für den zweiwertigen Rest $((C_1\text{-}C_4)\text{-Alkyl})_2C<$ steht, in allen ihren stereoisomeren Formen und Mischungen davon in allen Verhältnissen, und ihre physiologisch verträglichen Salze.

**5.** Verbindung der Formel I gemäß einem oder mehreren der Ansprüche 1 bis 4, worin B für unsubstituiertes Methylen steht oder für Methylen steht, das durch $(C_1\text{-}C_6)$-Alkyl oder $(C_3\text{-}C_6)$-Cycloalkyl-$(C_1\text{-}C_2)$-alkyl substituiert ist, in allen ihren stereoisomeren Formen und Mischungen davon in allen Verhältnissen, und ihre physiologisch verträglichen Salze.

**6.** Verbindung der Formel I gemäß einem oder mehreren der Ansprüche 1 bis 5, worin $R^{30}$ für einen der Reste $R^{32}$(R)N-CO-N(R)-$R^{31}$ und $R^{32}$(R)N-CS-N(R)-$R^{31}$ steht, in allen ihren stereoisomeren Formen und Mischungen davon in allen Verhältnissen, und ihre physiologisch verträglichen Salze.

**7.** Verbindung der Formel I gemäß einem oder mehreren der Ansprüche 1 bis 6, worin $R^{31}$ für im Arylrest gegebenenfalls substituiertes $(C_6\text{-}C_{10})$-Arylen-$(C_1\text{-}C_2)$-alkyl steht, in dem die Alkylgruppe an das Stickstoffatom im Imidazolidinring gebunden ist, in allen ihren stereoisomeren Formen und Mischungen davon in allen Verhältnissen, und ihre physiologisch verträglichen Salze.

**8.** Verbindung der Formel I gemäß einem oder mehreren der Ansprüche 1 bis 7, worin $R^{32}$ für gegebenenfalls substituiertes $(C_6\text{-}C_{10})$-Aryl steht, in allen ihren stereoisomeren Formen und Mischungen davon in allen Verhältnissen, und ihre physiologisch verträglichen Salze.

**9.** Verbindung der Formel I gemäß einem oder mehreren der Ansprüche 1 bis 8, worin $R^3$ für $(C_1\text{-}C_4)$-Alkyl, das gegebenenfalls durch 1 bis 6 Fluoratome substituiert sein kann, steht, e für 0 steht und h für 1 steht, in allen ihren stereoisomeren Formen und Mischungen davon in allen Verhältnissen, und ihre physiologisch verträglichen Salze.

**10.** Verbindung der Formel I gemäß einem oder mehreren der Ansprüche 1 bis 8, worin $R^3$ für unsubstituiertes oder substituiertes $(C_6\text{-}C_{10})$-Aryl steht, e für 0 steht und h für 1 steht, in allen ihren stereoisomeren Formen und Mischungen davon in allen Verhältnissen, und ihre physiologisch verträglichen Salze.

**11.** Verbindung der Formel I gemäß einem oder mehreren der Ansprüche 1 bis 10, worin $R^{30}$ für den Rest $R^{32}$NH-CO-NH-$R^{31}$ steht und darin $R^{31}$ für den zweiwertigen Rest -(1,4-Phenylen)-$CH_2$- steht, in dem die Methylengruppe an das Stickstoffatom im Imidazolidinring gebunden ist, und $R^{32}$ für unsubstituiertes oder substituiertes Phenyl steht, in allen ihren stereoisomeren Formen und Mischungen davon in allen Verhältnissen, und ihre physiologisch verträglichen Salze.

**12.** Verbindung der Formel I gemäß einem oder mehreren der Ansprüche 1 bis 11, worin W eine andere Bedeutung als $CH_2$ hat, B für unsubstituiertes Methylen steht oder für Methylen steht, das durch $(C_1\text{-}C_6)$-Alkyl oder $(C_3\text{-}C_6)$-Cycloalkyl-$(C_1\text{-}C_2)$-alkyl substituiert ist, $R^{30}$ für den Rest $R^{32}$NH-CO-NH-$R^{31}$ steht, $R^{31}$ für den zweiwertigen Rest -(1,4-Phenylen)-$CH_2$- steht, in dem die Methylengruppe an das Stickstoffatom im Imidazolidinring gebunden ist,

$R^{32}$ für unsubstituiertes oder substituiertes Phenyl steht, und die Gruppe -NR-$[C(R)(R)]_e$-$C(R^2)(R^3)$-$[C(R)(R)]_h$-E in der Formel I für die Gruppe -NH-$CH(R^3)$-$CH_2$-E steht, in allen ihren stereoisomeren Formen und Mischungen davon in allen Verhältnissen, und ihre physiologisch verträglichen Salze.

**13.** (R)-3-((S)-2-(4,4-Dimethyl-3-(4-(3-phenylureido)-benzyl)-2,5-dioxoimidazolidin-1-yl)-2-(2-methylpropyl)-acetylamino)-3-methyl-propionsäure der Formel

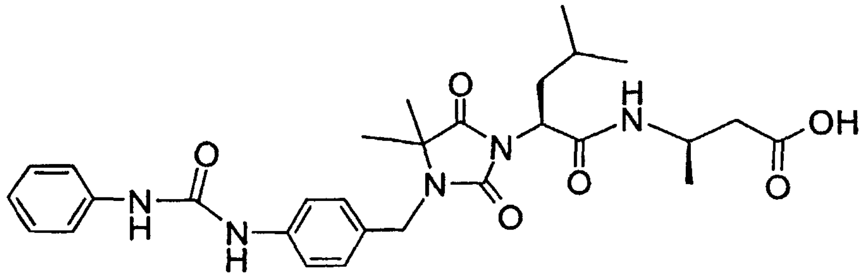

und ihre physiologisch verträglichen Salze.

**14.** (S)-3-((S)-2-(4,4-Dimethyl-3-(4-(3-(2-methylphenyl)-ureido)-benzyl)-2,5-dioxoimidazolidin-1-yl)-2-(2-methylpropyl)-acetylamino)-3-phenyl-propionsäure der Formel

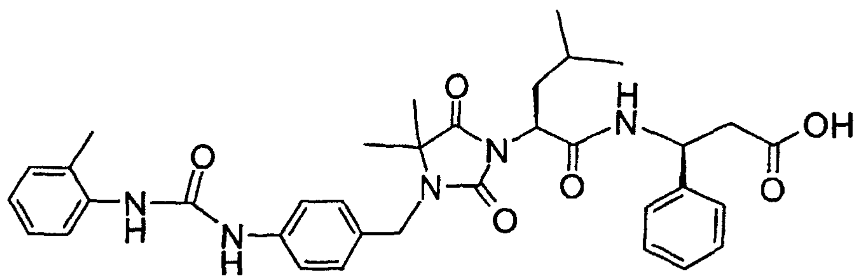

und ihre physiologisch verträglichen Salze.

**15.** (R)-3-((S)-2-(4,4-Dimethyl-3-(4-(3-phenylureido)-benzyl)-2,5-dioxoimidazolidin-1-yl)-2-(2-methylpropyl)-acetylamino)-3-methyl-propionsäure-Natriumsalz der Formel

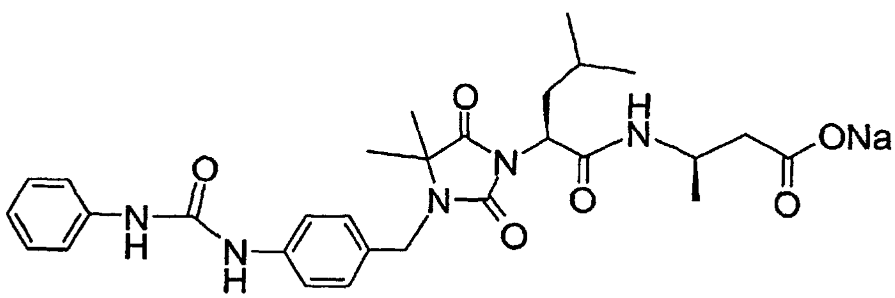

**16.** (R)-3-((S)-2-(4,4-Dimethyl-3-(4-(3-phenylureido)-benzyl)-2,5-dioxoimidazolidin-1-yl)-2-cyclopropylmethyl-acetylamino)-3-methyl-propionsäure der Formel

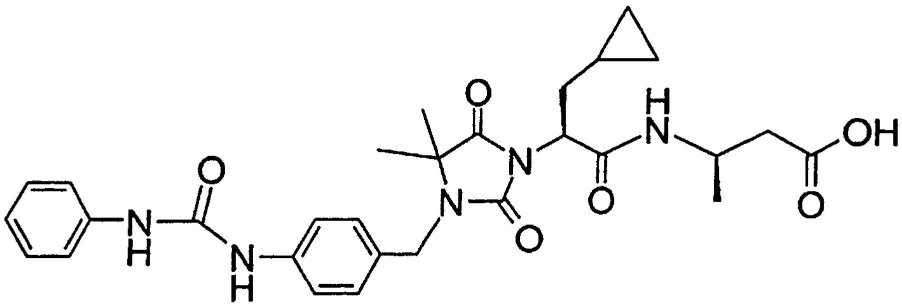

und ihre physiologisch verträglichen Salze.

**17.** Verbindung der Formel

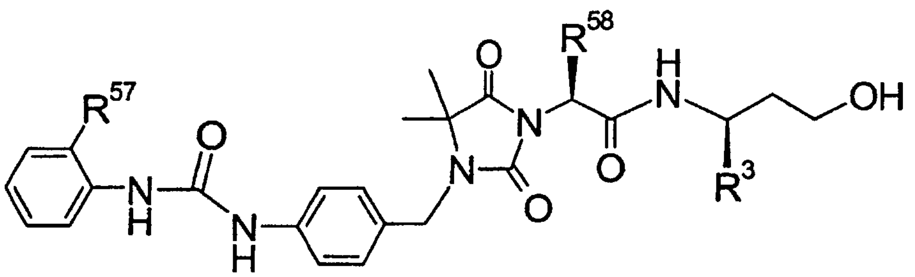

in der $R^3$ für Methyl, $R^{57}$ für Wasserstoff und $R^{58}$ für Isobutyl steht.

**18.** Verfahren zur Herstellung einer Verbindung der Formel I gemäß einem oder mehreren der Ansprüche 1 bis 17, **dadurch gekennzeichnet, daß** man eine Fragmentkondensation einer Verbindung der Formel II

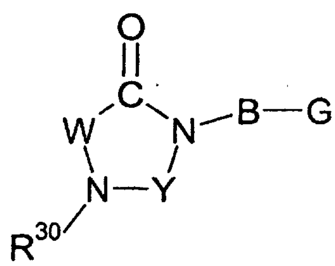

(II)

mit einer Verbindung der Formel III,

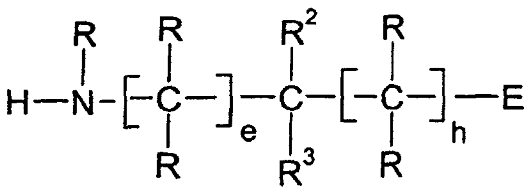

(III)

durchführt, wobei in den Formeln II und III die Gruppen W, Y, B, E, R, $R^2$, $R^3$, $R^{30}$ sowie e und h wie in den Ansprüchen 1 bis 17 angegeben definiert sind oder auch funktionelle Gruppen in geschützter Form oder in Form von Vorstufen enthalten sein können, und wobei G für Hydroxycarbonyl, $(C_1-C_6)$-Alkoxycarbonyl oder aktivierte Carbonsäurederivate steht.

**19.** Verbindung der Formel I gemäß einem oder mehreren der Ansprüche 1 bis 17 und/oder ein physiologisch verträgliches Salz davon zur Verwendung als Arzneimittel.

**20.** Pharmazeutisches Präparat, **dadurch gekennzeichnet, daß** es eine oder mehrere Verbindungen der Formel I gemäß einem oder mehreren der Ansprüche 1 bis 17 und/oder ihre physiologisch verträglichen Salze und einen pharmazeutisch einwandfreien Träger enthält.

**21.** Pharmazeutisches Präparat, **dadurch gekennzeichnet, daß** es eine Verbindung gemäß einem der Ansprüche 13 bis 17 und/oder ein physiologisch verträgliches Salz davon und einen pharmazeutisch einwandfreien Träger enthält.

**22.** Verbindung der Formel I gemäß einem oder mehreren der Ansprüche 1 bis 17 und/oder ein physiologisch verträgliches Salz davon zur Verwendung als Entzündungshemmstoff.

**23.** Verbindung der Formel I gemäß einem oder mehreren der Ansprüche 1 bis 17 und/oder ein physiologisch verträgliches Salz davon zur Verwendung in der Therapie oder Prophylaxe der Arthritis, der rheumatoiden Arthritis, der Polyarthritis, der inflammatorischen bowel disease, des systemischen Lupus erythematosus, der Multiplen Sklerose oder von inflammatorischen Erkrankungen des zentralen Nervensystems.

**24.** Verbindung der Formel I gemäß einem oder mehreren der Ansprüche 1 bis 17 und/oder ein physiologisch verträgliches Salz davon zur Verwendung in der Therapie oder Prophylaxe von Asthma oder Allergien.

**25.** Verbindung der Formel I gemäß einem oder mehreren der Ansprüche 1 bis 17 und/oder ein physiologisch verträgliches Salz zur Verwendung in der Therapie oder Prophylaxe von cardiovaskulären Erkrankungen, der Arteriosklerose, von Restenosen, von Diabetes, der Schädigung von Organtransplantaten, von Immunerkrankungen, von Autoimmunerkrankungen, von Tumorwachstum oder Tumormetastasierung oder der Malaria.

**26.** Verbindung der Formel I gemäß einem oder mehreren der Ansprüche 1 bis 17 und/oder ein physiologisch verträgliches Salz davon zur Verwendung als Hemmstoffe der Adhäsion und/oder Migration von Leukozyten oder zur Hemmung des VLA-4-Rezeptors.

## Claims

**1.** A compound of the formula I

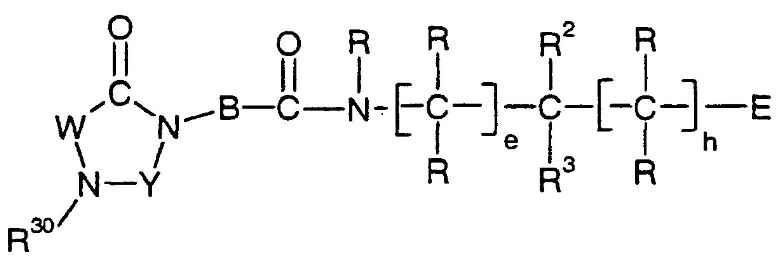

(I)

wherein

w is a divalent radical from the group consisting of $R^1$-A-C($R^{13}$) and

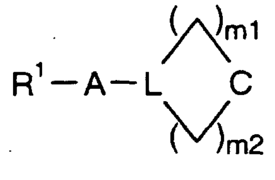

wherein the ring systems

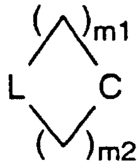

can contain one or two identical or different heteroatoms from the group consisting of N and O, can be saturated or monounsaturated and can be substituted by 1 or 2 identical or different substituents $R^{13}$ and/or by one or two doubly bonded oxygen atoms, and wherein L is $C(R^{13})$ or N and wherein m1 and m2 independently of one another are one of the numbers 0, 1, 2, 3 and 4, the sum m1 + m2, however, is one of the numbers 1, 2, 3 and 4;

Y is a carbonyl group or thiocarbonyl group;

A is a direct bond, one of the divalent radicals $(C_1-C_6)$-alkylene, $(C_5-C_6)$-cycloalkylene, phenylene, phenylene-$(C_1-C_4)$-alkyl or a divalent radical of a 5-membered or 6-membered, saturated or unsaturated heterocycle which can contain one or two nitrogen atoms and can be monosubstituted or disubstituted by $(C_1-C_6)$-alkyl or doubly bonded oxygen or sulfur, where in the radical phenylenealkyl the radical $R^1$ is bonded to the phenylene group;

B is a divalent methylene radical or ethylene radical where the methylene radical and the ethylene radical are unsubstituted or are substituted by one or two identical or different radicals from the group consisting of $(C_1-C_8)$-alkyl, $(C_2-C_8)$-alkenyl, $(C_2-C_8)$-alkynyl, $(C_3-C_6)$-cycloalkyl, $(C_3-C_6)$-cycloalkyl-$(C_1-C_6)$-alkyl, optionally substituted $(C_6-C_{10})$-aryl, $(C_6-C_{10})$-aryl-$(C_1-C_6)$-alkyl optionally substituted in the aryl radical, optionally substituted heteroaryl and heteroaryl-$(C_1-C_6)$-alkyl optionally substituted in the heteroaryl radical;

E is $R^{10}CO$, HO-$CH_2$ or $R^8CO$-O-$CH_2$;

R is hydrogen or $(C_1-C_8)$-alkyl, where all radicals R are independent of one another and the radicals R can be identical or different;

$R^1$ is hydrogen, $(C_1-C_{10})$-alkyl, which can optionally be monosubstituted or polysubstituted by fluorine, $R^{21}$-($(C_6-C_{10})$-aryl) optionally substituted in the aryl radical, ($R^{21}$-( $(C_6-C_{10})$-aryl))-$(C_1-C_6)$-alkyl optionally substituted in the aryl radical, the radical Het-, Het-$(C_1-C_6)$-alkyl or one of the radicals X-NH-C (=NH)-$R^{20}$-, $X^1$-NH-$R^{20}$-, $R^{22}N(R^{21})$-C(O)-, O= and S=;

X is hydrogen, $(C_1-C_6)$-alkyl, $(C_1-C_6)$-alkylcarbonyl, $(C_1-C_6)$-alkoxycarbonyl, $(C_1-C_8)$-alkylcarbonyloxy-$(C_1-C_6)$-alkoxycarbonyl, optionally substituted $(C_6-C_{10})$-arylcarbonyl, optionally substituted $(C_6-C_{10})$-aryloxycarbonyl, $(C_6-C_{14})$-aryl-$(C_1-C_6)$-alkoxycarbonyl which can also be substituted in the aryl radical, hydroxyl, $(C_1-C_6)$-alkoxy, or amino;

$X^1$ has one of the meanings of X or is R'-NH-C(=N-R''), where R' and R'' independently of one another have the meanings of X;

$R^2$ is hydrogen or $(C_1-C_8)$-alkyl;

$R^3$ is hydrogen, $(C_1-C_8)$-alkyl, which can optionally be substituted by 1 to 6 fluorine atoms, optionally substituted $(C_6-C_{12})$-aryl, $(C_6-C_{12})$-aryl-$(C_1-C_6)$-alkyl optionally substituted in the aryl radical, optionally substituted heteroaryl, heteroaryl-$(C_1-C_6)$-alkyl optionally substituted in the heteroaryl radical, $(C_3-C_8)$-cycloalkyl, $(C_3-C_8)$-cycloalkyl-$(C_1-C_6)$-alkyl, $(C_6-C_{12})$-bicycloalkyl, $(C_6-C_{12})$-bicycloalkyl-$(C_1-C_6)$-alkyl, $(C_6-C_{12})$-tricycloalkyl, $(C_6-C_{12})$-tricycloalkyl-$(C_1-C_6)$-alkyl, $(C_2-C_8)$-alkenyl, $(C_2-C_8)$-alkynyl, $R^{11}NH$, $COOR^{21}$, $CON(CH_3)R^4$, $CONHR^4$, $CON(CH_3)R^{15}$ or $CONHR^{15}$;

$R^4$ is $(C_1-C_8)$-alkyl which is unsubstituted or monosubstituted or disubstituted by identical or different radicals from the group consisting of hydroxyl, $(C_1-C_8)$-alkoxy, $R^5$, optionally substituted $(C_3-C_8)$-cycloalkyl, hydroxycarbonyl, aminocarbonyl, $(C_6-C_{10})$-aryl-$(C_1-C_4)$-alkoxycarbonyl which can also be substituted in the aryl radical, $(C_1-C_6)$-alkoxycarbonyl, $R^6$-CO, $R^7$-CO, tetrazolyl and trifluoromethyl;

$R^5$ is optionally substituted $(C_6-C_{12})$-aryl, $(C_6-C_{12})$-aryl-$(C_1-C_8)$-alkyl optionally substituted in the aryl radical or a radical of an optionally substituted monocyclic or bicyclic, 5-membered to 12-membered heterocyclic ring, which can be aromatic, partially saturated or completely saturated and which can contain one, two or three identical or different heteroatoms from the group consisting of nitrogen, oxygen and sulfur;

$R^6$ is the radical of a natural or unnatural amino acid, imino acid, optionally N-$(C_1-C_8)$-alkylated or N-($(C_6-C_{12})$-aryl-$(C_1-C_8)$-alkylated) azaamino acid, which can also be substituted in the aryl radical, or

the radical of a dipeptide or tripeptide, as well as their esters and amides, wherein free functional groups can be protected by protective groups customary in peptide chemistry and wherein the nitrogen atoms in the amide bonds in the group $R^6$-CO can carry a radical R as a substituent;

$R^7$ is the radical of a 5-membered to 7-membered, saturated monocyclic or bicyclic heterocycle bonded via a nitrogen atom, which can contain one, two, three or four identical or different additional ring heteroatoms from the group consisting of oxygen, nitrogen and sulfur and which can optionally be substituted on carbon atoms and on additional ring nitrogen atoms, wherein additional ring nitrogen atoms can carry identical or different radicals from the group consisting of hydrogen, $R^h$, HCO, $R^h$CO, $R^h$O-CO, HO-CO-($C_1$-$C_4$)-alkyl and $R^h$O-CO-($C_1$-$C_4$)-alkyl as substituents and $R^h$ is ($C_1$-$C_6$)-alkyl, ($C_3$-$C_8$)-cycloalkyl, ($C_3$-$C_8$)-cycloalkyl-($C_1$-$C_8$)-alkyl, optionally substituted ($C_6$-$C_{10}$)-aryl or ($C_6$-$C_{10}$)-aryl-($C_1$-$C_4$)-alkyl optionally substituted in the aryl radical;

$R^8$ is hydrogen, ($C_1$-$C_6$)-alkyl or phenyl-($C_1$-$C_4$)-alkyl optionally substituted in the phenyl radical;

$R^{10}$ is hydroxyl, ($C_1$-$C_8$)-alkoxy, ($C_6$-$C_{10}$)-aryl-($C_1$-$C_6$)-alkoxy which can also be substituted in the aryl radical, optionally substituted ($C_6$-$C_{10}$)-aryloxy, ($C_1$-$C_6$)-alkylcarbonyloxy-($C_1$-$C_6$)-alkoxy, ($C_1$-$C_6$)-alkoxycarbonyloxy-($C_1$-$C_6$)-alkoxy, amino, mono- or di-(($C_1$-$C_6$)-alkyl)-amino, aminocarbonyl-($C_1$-$C_6$)-alkoxy or (mono- or di- (($C_1$-$C_6$)-alkyl)-amino)-carbonyl-($C_1$-$C_6$)-alkoxy;

$R^{11}$ is hydrogen, $R^{12a}$, $R^{12a}$-CO, $R^{12a}$-O-CO, $R^{12b}$-CO or $R^{12a}$-$S(O)_2$;

$R^{12a}$ is ($C_1$-$C_{10}$)-alkyl, ($C_2$-$C_8$)-alkenyl, ($C_2$-$C_8$)-alkynyl, ($C_5$-$C_{10}$)-cycloalkyl, ($C_5$-$C_{10}$)-cycloalkyl-($C_1$-$C_8$)-alkyl, optionally substituted ($C_6$-$C_{14}$)-aryl, ($C_6$-$C_{14}$)-aryl-($C_1$-$C_8$)-alkyl optionally substituted in the aryl radical, optionally substituted heteroaryl, heteroaryl-($C_1$-$C_8$)-alkyl optionally substituted in the heteroaryl radical, or the radical $R^{15}$;

$R^{12b}$ is amino, di-(($C_1$-$C_{10}$)-alkyl)-amino or $R^{12a}$-NH;

$R^{13}$ is hydrogen or ($C_1$-$C_6$)-alkyl;

$R^{15}$ is $R^{16}$-($C_1$-$C_6$)-alkyl or $R^{16}$;

$R^{16}$ is a 6-membered to 14-membered, bicyclic or tricyclic radical which is saturated or partially unsaturated and which can also contain one, two, three or four identical or different heteroatoms from the group consisting of nitrogen, oxygen and sulfur and which can also be substituted by one or more identical or different substituents from the group consisting of ($C_1$-$C_4$)-alkyl and oxo;

$R^{20}$ is a direct bond or ($C_1$-$C_2$)-alkylene;

$R^{21}$ is hydrogen, ($C_1$-$C_6$)-alkyl, ($C_3$-$C_6$)-cycloalkyl, ($C_3$-$C_6$)-cycloalkyl-($C_1$-$C_4$)-alkyl, optionally substituted ($C_6$-$C_{10}$)-aryl, ($C_6$-$C_{10}$)-aryl-($C_1$-$C_4$)-alkyl optionally substituted in the aryl radical, the radical Het- or Het-($C_1$-$C_4$)-alkyl, where alkyl radicals can be monosubstituted or polysubstituted by fluorine and the radicals $R^{21}$, if they occur a number of times, can be identical or different;

$R^{22}$ is one of the radicals $R^{21}$-, $R^{21}N(R^{21})$- or $R^{21}N(R^{21})$-C(=N($R^{21}$))-;

$R^{30}$ is one of the radicals $R^{32}$(R)N-CO-N(R)-$R^{31}$, $R^{32}$(R)N-CS-N(R)-$R^{31}$, $R^{32}$-CO-N(R)-$R^{31}$ or $R^{32}$(R)N-CO-$R^{31}$, where $R^{30}$ cannot be $R^{32}$-CO-N(R)-$R^{31}$ if at the same time W is $R^1$-A-C($R^{13}$), A is a direct bond and $R^1$ and $R^{13}$ are hydrogen;

$R^{31}$ is the divalent radical -$R^{33}$-$R^{34}$-$R^{35}$-$R^{36}$-, where $R^{36}$ is bonded to the nitrogen atom in the imidazolidine ring in the formula I;

$R^{32}$ is hydrogen, ($C_1$-$C_6$)-alkyl which can optionally be substituted by 1 to 6 fluorine atoms, ($C_2$-$C_6$)-alkenyl, ($C_2$-$C_6$)-alkynyl, ($C_5$-$C_6$)-cycloalkyl, ($C_5$-$C_6$)-cycloalkyl-($C_1$-$C_6$)-alkyl, optionally substituted ($C_6$-$C_{10}$)-aryl, ($C_6$-$C_{10}$)-aryl-($C_1$-$C_6$)-alkyl optionally substituted in the aryl radical, optionally substituted heteroaryl or heteroaryl-($C_1$-$C_6$)-alkyl optionally substituted in the heteroaryl radical;

$R^{33}$ is a direct bond or a divalent ($C_1$-$C_4$)-alkylene radical;

$R^{34}$ is a divalent radical from the group consisting of ($C_1$-$C_6$)-alkylene, ($C_5$-$C_6$)-cycloalkylene, optionally substituted ($C_6$-$C_{10}$)-arylene and optionally substituted heteroarylene;

$R^{35}$ is a direct bond or a divalent ($C_1$-$C_4$)-alkylene radical;

$R^{36}$ is a direct bond, the group -CO- or the group -$S(O)_n$-;

Het is a radical of a monocyclic or polycyclic, 5-membered to 12-membered, aromatic or nonaromatic ring which contains 1 or 2 identical or different heteroatoms from the group consisting of N and O as ring members and which can optionally be substituted by one or more, identical or different substituents;

e and h independently of one another are 0 or 1;

n is 1 or 2;

in all its stereoisomeric forms and mixtures thereof in all ratios, or its physiologically tolerable salts.

**2.** A compound of the formula I as claimed in claim 1, wherein

W is the divalent radical $R^1$-A-C($R^{13}$);

Y is a carbonyl group;

A is a direct bond, one of the divalent radicals ($C_1$-$C_6$)-alkylene, phenylene, phenylene-($C_1$-$C_2$)-alkyl or a divalent radical of a 5-membered or 6-membered, saturated or unsaturated heterocycle which can contain one or two nitrogen atoms and can be monosubstituted or disubstituted by ($C_1$-$C_6$)-alkyl or doubly bonded oxygen or sulfur, where in the radical phenylenealkyl the radical $R^1$ is bonded to the phenylene group;

B is a divalent methylene radical or ethylene radical where the methylene radical and the ethylene radical are unsubstituted or are substituted by a radical from the group consisting of ($C_1$-$C_8$)-alkyl, ($C_2$-$C_8$)-alkenyl, ($C_2$-$C_8$)-alkynyl, ($C_3$-$C_6$)-cycloalkyl, ($C_3$-$C_6$)-cycloalkyl-($C_1$-$C_6$)-alkyl, optionally substituted ($C_6$-$C_{10}$)-aryl, ($C_6$-$C_{10}$)-aryl-($C_1$-$C_6$)-alkyl optionally substituted in the aryl radical, optionally substituted heteroaryl and heteroaryl-($C_1$-$C_6$)-alkyl optionally substituted in the heteroaryl radical;

E is $R^{10}$CO, HO-$CH_2$ or $R^8$CO-O-$CH_2$;

R is hydrogen or ($C_1$-$C_8$)-alkyl where all radicals R are independent of one another and the radicals R can be identical or different;

$R^1$ is hydrogen, ($C_1$-$C_{10}$)-alkyl, which can optionally be monosubstituted or polysubstituted by fluorine, $R^{21}$-(($C_6$-$C_{10}$)-aryl) optionally substituted in the aryl radical, ($R^{21}$-(($C_6$-$C_{10}$)-aryl))-($C_1$-$C_6$)-alkyl optionally substituted in the aryl radical, the radical Het-, Het-($C_1$-$C_4$)-alkyl or one of the radicals X-NH-C(=NH)-$R^{20}$-, $X^1$-NH-$R^{20}$-, and O=;

X is hydrogen, ($C_1$-$C_6$)-alkyl, ($C_1$-$C_6$)-alkylcarbonyl, ($C_1$-$C_6$)-alkoxycarbonyl, ($C_1$-$C_6$)-alkylcarbonyloxy-($C_1$-$C_6$)-alkoxycarbonyl, optionally substituted ($C_6$-$C_{10}$)-arylcarbonyl, optionally substituted ($C_6$-$C_{10}$)-aryloxycarbonyl, ($C_6$-$C_{14}$)-aryl-($C_1$-$C_6$)-alkoxycarbonyl which can also be substituted in the aryl radical, hydroxyl, ($C_1$-$C_6$)-alkoxy or amino;

$X^1$ has one of the meanings of X or is R'-NH-C(=N-R''), where R' and R'' independently of one another have the meanings of X;

$R^2$ is hydrogen or ($C_1$-$C_6$)-alkyl;

$R^3$ is hydrogen, ($C_1$-$C_8$)-alkyl, which can optionally be substituted by 1 to 6 fluorine atoms, optionally substituted ($C_6$-$C_{10}$)-aryl, ($C_6$-$C_{10}$)-aryl-($C_1$-$C_4$)-alkyl optionally substituted in the aryl radical, optionally substituted heteroaryl, heteroaryl-($C_1$-$C_4$)-alkyl optionally substituted in the heteroaryl radical, ($C_3$-$C_8$)-cycloalkyl, ($C_3$-$C_8$)-cycloalkyl-($C_1$-$C_4$)-alkyl, ($C_6$-$C_{12}$)-bicycloalkyl, ($C_6$-$C_{12}$)-bicycloalkyl-($C_1$-$C_4$)-alkyl, ($C_6$-$C_{12}$)-tricycloalkyl, ($C_6$-$C_{12}$)-tricycloalkyl-($C_1$-$C_4$)-alkyl, ($C_2$-$C_8$)-alkenyl, ($C_2$-$C_8$)-alkynyl, $R^{11}$NH, COOR$^{21}$, CON($CH_3$)$R^4$, CONHR$^4$, CON($CH_3$)$R^{15}$ or CONHR$^{15}$;

$R^4$ is ($C_1$-$C_6$)-alkyl which is unsubstituted or monosubstituted or disubstituted by identical or different radicals from the group consisting of hydroxyl, ($C_1$-$C_8$)-alkoxy, $R^5$, optionally substituted ($C_3$-$C_8$)-cycloalkyl, hydroxycarbonyl, aminocarbonyl, ($C_6$-$C_{10}$)-aryl-($C_1$-$C_4$)-alkoxycarbonyl, which can also be substituted in the aryl radical, ($C_1$-$C_6$)-alkoxycarbonyl, $R^6$-CO, $R^7$-CO, tetrazolyl and trifluoromethyl;

$R^5$ is optionally substituted ($C_6$-$C_{10}$)-aryl, ($C_6$-$C_{10}$)-aryl-($C_1$-$C_4$)-alkyl optionally substituted in the aryl radical or a radical of an optionally substituted monocyclic or bicyclic, 5-membered to 12-membered heterocyclic ring, which can be aromatic, partially saturated or completely saturated and which can contain one, two or three identical or different heteroatoms from the group consisting of nitrogen, oxygen and sulfur;

$R^6$ is a radical of a natural or unnatural amino acid or the radical of a dipeptide or tripeptide, as well as their esters and amides, wherein free functional groups can be protected by protective groups customary in peptide chemistry and wherein the nitrogen atoms in the amide bonds in the group $R^6$-CO can carry a radical R as a substituent;

$R^7$ is the radical of a 5-membered to 7-membered, saturated monocyclic heterocycle bonded via a nitrogen atom, which can contain one or two identical or different additional ring heteroatoms from the group consisting of oxygen, nitrogen and sulfur and which can optionally be substituted on carbon atoms and on additional ring nitrogen atoms, wherein additional ring nitrogen atoms can carry identical or different radicals from the group consisting of hydrogen, $R^h$, HCO, $R^h$CO, $R^h$O-CO, HO-CO-($C_1$-$C_4$)-alkyl and $R^h$O-CO-($C_1$-$C_4$)-alkyl as substituents and $R^h$ is ($C_1$-$C_4$)-alkyl, optionally substituted ($C_6$-$C_{10}$)-aryl or ($C_6$-$C_{10}$)-aryl-($C_1$-$C_4$)-alkyl optionally substituted in the aryl radical;

$R^8$ is hydrogen, ($C_1$-$C_6$)-alkyl or phenyl-($C_1$-$C_4$)-alkyl optionally substituted in the phenyl radical;

$R^{10}$ is hydroxyl, ($C_1$-$C_8$)-alkoxy, ($C_6$-$C_{10}$)-aryl-($C_1$-$C_6$)-alkoxy which can also be substituted in the aryl radical, optionally substituted ($C_6$-$C_{10}$)-aryloxy, ($C_1$-$C_6$)-alkylcarbonyloxy-($C_1$-$C_6$)-alkoxy, ($C_1$-$C_6$)-alkoxycarbonyloxy-($C_1$-$C_6$)-alkoxy, amino, mono- or di-(($C_1$-$C_6$)-alkyl)-amino, aminocarbonyl-($C_1$-$C_6$)-alkoxy or (mono- or di-(($C_1$-$C_6$)-alkyl)-amino)-carbonyl-($C_1$-$C_6$)-alkoxy;

$R^{11}$ is hydrogen, $R^{12a}$, $R^{12a}$-CO, $R^{12a}$-O-CO, $R^{12b}$-CO, or $R^{12a}$-S(O)$_2$;

$R^{12a}$ is $(C_1-C_8)$-alkyl, $(C_2-C_8)$-alkenyl, $(C_2-C_8)$-alkynyl, $(C_5-C_6)$-cycloalkyl, $(C_5-C_6)$-cycloalkyl-$(C_1-C_4)$-alkyl, optionally substituted $(C_6-C_{10})$-aryl, ($C_6$-C10)-aryl-$(C_1-C_4)$-alkyl optionally substituted in the aryl radical, optionally substituted heteroaryl, heteroaryl-$(C_1-C_4)$-alkyl optionally substituted in the heteroaryl radical, or the radical $R^{15}$;
$R^{12b}$ is amino, di-($(C_1-C_8)$-alkyl)-amino or $R^{12a}$-NH;
$R^{13}$ is hydrogen or $(C_1-C_6)$-alkyl;
$R^{15}$ is $R^{16}$-$(C_1-C_6)$-alkyl or $R^{16}$;
$R^{16}$ is a 6-membered to 12-membered, bicyclic or tricyclic radical which is saturated or partially unsaturated and which can also contain one, two, three or four identical or different heteroatoms from the group consisting of nitrogen, oxygen and sulfur and which can also be substituted by one or more identical or different substituents from the group consisting of $(C_1-C_4)$-alkyl and oxo;
$R^{20}$ is a direct bond or methylene;
$R^{21}$ is hydrogen, $(C_1-C_6)$-alkyl, optionally substituted $(C_6-C_{10})$-aryl, $(C_6-C_{10})$-aryl-$(C_1-C_2)$-alkyl optionally substituted in the aryl radical, the radical Het- or Het-$(C_1-C_2)$-alkyl, where alkyl radicals can be monosubstituted to tetrasubstituted by fluorine and the radicals $R^{21}$, if they occur a number of times, can be identical or different;
$R^{30}$ is one of the radicals $R^{32}$(R)N-CO-N(R)-$R^{31}$ or $R^{32}$(R)N-CS-N(R)-$R^{31}$;
$R^{31}$ is a divalent radical from the group consisting of $(C_1-C_6)$-alkylene, optionally substituted $(C_6-C_{10})$-arylene, $(C_6-C_{10})$-arylene-$(C_1-C_4)$-alkyl optionally substituted in the arylene radical, $(C_5-C_6)$-cycloalkylene, $(C_5-C_6)$-cycloalkylene-$(C_1-C_4)$-alkyl, optionally substituted heteroarylene or heteroarylene-$(C_1-C_4)$-alkyl optionally substituted in the heteroarylene radical, where in the case of the arylenealkyl radical, of the cycloalkylenealkyl radical and of the heteroarylenealkyl radical the alkyl group is bonded to the nitrogen atom in the imidazolidine ring in the formula I;
$R^{32}$ is hydrogen, $(C_1-C_6)$-alkyl which can optionally be substituted by 1 to 6 fluorine atoms, $(C_2-C_6)$-alkenyl, $(C_2-C_6)$-alkynyl, $(C_5-C_6)$-cycloalkyl, $(C_5-C_6)$-cycloalkyl-$(C_1-C_4)$-alkyl, optionally substituted $(C_5-C_{10})$-aryl, $(C_6-C_{10})$-aryl-$(C_1-C_4)$-alkyl optionally substituted in the aryl radical, optionally substituted heteroaryl or heteroaryl-$(C_1-C_4)$-alkyl optionally substituted in the heteroaryl radical;
Het is a radical of a monocyclic or polycyclic, 5-membered to 10-membered, aromatic or nonaromatic ring which contains 1 or 2 identical or different heteroatoms from the group consisting of N and O as ring members and which can optionally be substituted by one or more, identical or different substituents;
e and h independently of one another are 0 or 1;

in all its stereoisomeric forms and mixtures thereof in all ratios, or its physiologically tolerable salts.

**3.** A compound of the formula I as claimed in claim 1 and/or 2, wherein W has a meaning other than $CH_2$, in all its stereoisomeric forms and mixtures thereof in all ratios, or its physiologically acceptable salts.

**4.** A compound of the formula I as claimed in one or more of claims 1 to 3, wherein W is the divalent radical $((C_1-C_4)$-alkyl$)_2C<$, in all its stereoisomeric forms and mixtures thereof in all ratios, or its physiologically tolerable salts.

**5.** A compound of the formula I as claimed in one or more of claims 1 to 4, wherein B is unsubstituted methylene or methylene which is substituted by $(C_1-C_6)$-alkyl or $(C_3-C_6)$-cycloalkyl-$(C_1-C_2)$-alkyl, in all its stereoisomeric forms and mixtures thereof in all ratios, or its physiologically tolerable salts.

**6.** A compound of the formula I as claimed in one or more of claims 1 to 5, wherein $R^{30}$ is one of the radicals $R^{32}$(R)N-CO-N(R)-$R^{31}$ and $R^{32}$(R)N-CS-N(R)-$R^{31}$, in all its stereoisomeric forms and mixtures thereof in all ratios, or its physiologically tolerable salts.

**7.** A compound of the formula I as claimed in one or more of claims 1 to 6, wherein $R^{31}$ is $(C_6-C_{10})$-arylene-$(C_1-C_2)$-alkyl which is optionally substituted in the aryl radical and in which the alkyl group is bonded to the nitrogen atom in the imidazolidine ring, in all its stereoisomeric forms and mixtures thereof in all ratios, or its physiologically tolerable salts.

**8.** A compound of the formula I as claimed in one or more of claims 1 to 7, wherein $R^{32}$ is optionally substituted $(C_6-C_{10})$-aryl, in all its stereoisomeric forms and mixtures thereof in all ratios, or its physiologically tolerable salts.

**9.** A compound of the formula I as claimed in one or more of claims 1 to 8, wherein $R^3$ is $(C_1-C_4)$-alkyl which can optionally be substituted by 1 to 6 fluorine atoms, e is 0 and h is 1, in all its stereoisomeric forms and mixtures

thereof in all ratios, or its physiologically tolerable salts.

**10.** A compound of the formula I as claimed in one or more of claims 1 to 8, wherein $R^3$ is unsubstituted or substituted $(C_6\text{-}C_{10})$-aryl, e is 0 and h is 1, in all its stereoisomeric forms and mixtures thereof in all ratios, or its physiologically tolerable salts.

**11.** A compound of the formula I as claimed in one or more of claims 1 to 10, wherein $R^{30}$ is the radical $R^{32}$NH-CO-NH-$R^{31}$ and therein $R^{31}$ is the divalent radical -(1,4-phenylene)-$CH_2$-, in which the methylene group is bonded to the nitrogen atom in the imidazoline ring, and $R^{32}$ is unsubstituted or substituted phenyl, in all its stereoisomeric forms and mixtures thereof in all ratios, or its physiologically tolerable salts.

**12.** A compound of the formula I as claimed in one or more of claims 1 to 11, wherein W has a meaning other than $CH_2$, B is unsubstituted methylene or is methylene which is substituted by $(C_1\text{-}C_6)$-alkyl or $(C_3\text{-}C_6)$-cycloalkyl-$(C_1\text{-}C_2)$-alkyl, $R^{30}$ is the radical $R^{32}$NH-CO-NH-$R^{31}$, $R^{31}$ is the divalent radical -(1,4-phenylene)-$CH_2$-, in which the methylene group is bonded to the nitrogen atom in the imidazolidine ring, $R^{32}$ is unsubstituted or substituted phenyl, and the group -NR-$[C(R)(R)]_e$-$C(R^2)(R^3)$-$[C(R)(R)]_h$-E in the formula I is the group -NH-CH($R^3$) -$CH_2$-E , in all its stereoisomeric forms and mixtures thereof in all ratios, or its physiologically tolerable salts.

**13.** (R)-3-((S)-2-(4,4-Dimethyl-3-(4-(3-phenylureido)-benzyl)-2,5-dioxoimidazolidin-1-yl)-2-(2-methylpropyl)-acetylamino)-3-methylpropionic acid of the formula

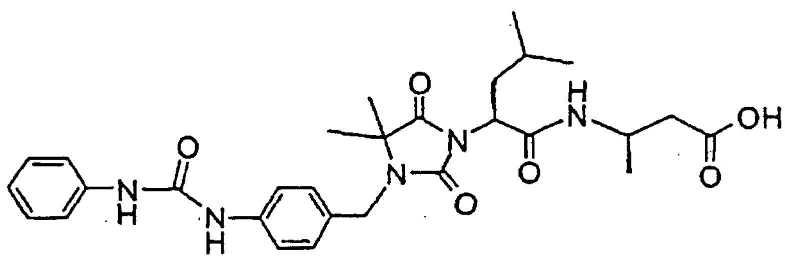

or its physiologically tolerable salts.

**14.** (S)-3-((S)-2-(4,4-Dimethyl-3-(4-(3-(2-methylphenyl)-ureido)-benzyl)-2,5-dioxoimidazolidin-1-yl)-2- (2-methylpropyl)-acetylamino)-3-phenylpropionic acid of the formula

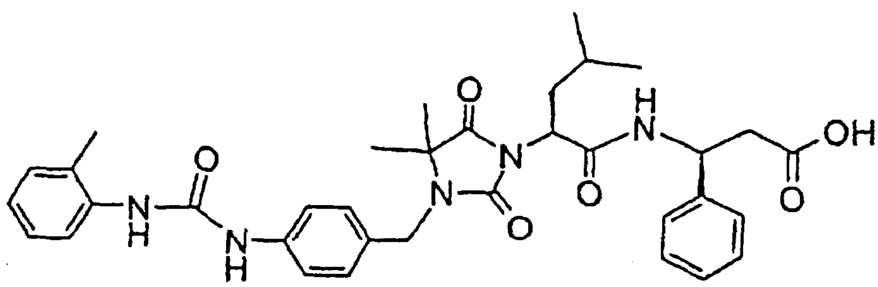

or its physiologically tolerable salts.

**15.** (R)-3-((S)-2-(4,4-Dimethyl-3-(4-(3-phenylureido)-benzyl)-2,5-dioxoimidazolidin-l-yl)-2-(2-methylpropyl)-acetylamino)-3-methylpropionic acid sodium salt of the formula

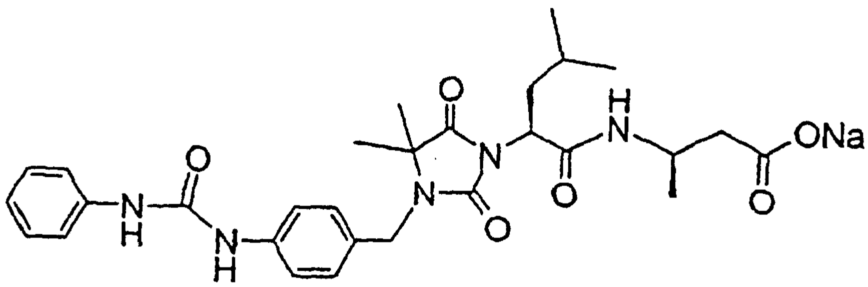

**16.** (R)-3-((S)-2-(4,4-Dimethyl-3-(4-(3-phenylureido)-benzyl)-2,5-dioxoimidazolidin-1-yl)-2-cyclopropylmethyl-acetylamino)-3-methylpropionic acid of the formula

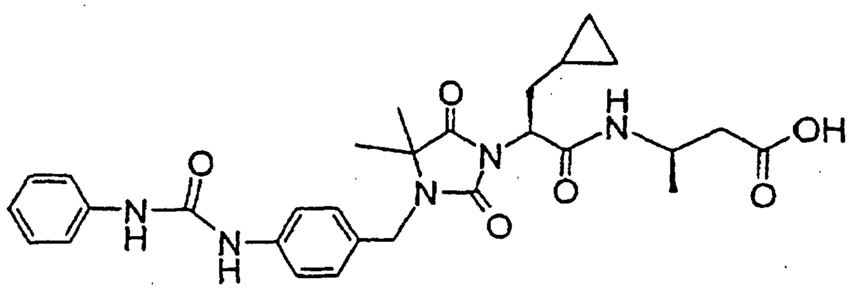

or its physiologically tolerable salts.

**17.** A compound of the formula

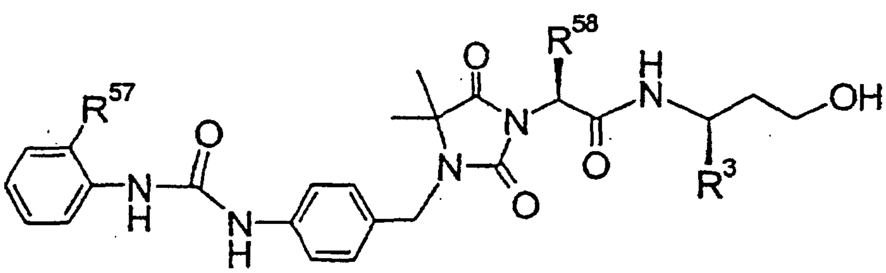

in which $R^3$ is methyl, $R^{57}$ is hydrogen and $R^{58}$ is isobutyl.

**18.** A process for the preparation of a compound of the formula I as claimed in one or more of claims 1 to 17, **characterized in that** it comprises carrying out a fragment condensation of a compound of the formula II

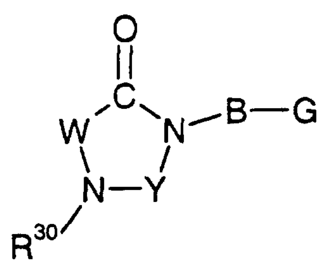

(II)

with a compound of the formula III,

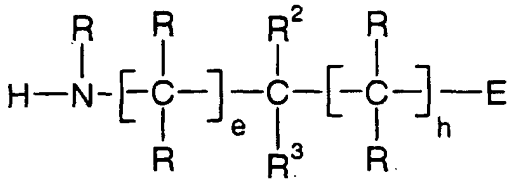

(III)

where, in the formulae II and III, the groups W, Y, B, E, R, $R^2$, $R^3$ and $R^{30}$ as well as e and h are defined as indicated in claims 1 to 17 or alternatively functional groups can be contained in protected form or in the form of precursors, and where G is hydroxycarbonyl, ($C_1$-$C_6$)-alkoxycarbonyl or activated carboxylic acid derivatives.

**19.** A compound of the formula I as claimed in one or more of claims 1 to 17 and/or a physiologically tolerable salt thereof for use as a pharmaceutical.

**20.** A pharmaceutical preparation, **characterized in that** it contains one or more compounds of the formula I as claimed in one or more of claims 1 to 17 and/or its physiologically tolerable salts and a pharmaceutically innocuous excipient.

**21.** A pharmaceutical preparation, **characterized in that** it contains a compound as claimed in any of claims 13 to 17 and/or a physiologically tolerable salt thereof and a pharmaceutically innocuous excipient.

**22.** A compound of the formula I as claimed in one or more of claims 1 to 17 and/or a physiologically tolerable salt thereof for use as an antiinflammatory.

**23.** A compound of the formula I as claimed in one or more of claims 1 to 17 and/or a physiologically tolerable salt thereof for use in the therapy or prophylaxis of arthritis, of rheumatoid arthritis, of polyarthritis, of inflammatory bowel disease, of systemic lupus erythematosus, of multiple sclerosis or of inflammatory disorders of the central nervous system.

**24.** A compound of the formula I as claimed in one or more of claims 1 to 17 and/or a physiologically tolerable salt thereof for use in the therapy or prophylaxis of asthma or allergies.

**25.** A compound of the formula I as claimed in one or more of claims 1 to 17 and/or a physiologically tolerable salt for use in the therapy or prophylaxis of cardiovascular disorders, arteriosclerosis, of restenoses, of diabetes, of damage to organ transplants, of immune disorders, of autoimmune disorders, of tumor growth or formation of tumor metastases or of malaria.

**26.** A compound of the formula I as claimed in one or more of claims 1 to 17 and/or a physiologically tolerable salt thereof for use as inhibitors of the adhesion and/or migration of leucocytes or for the inhibition of the VLA-4 receptor.

## Revendications

**1.** Composé de formule I

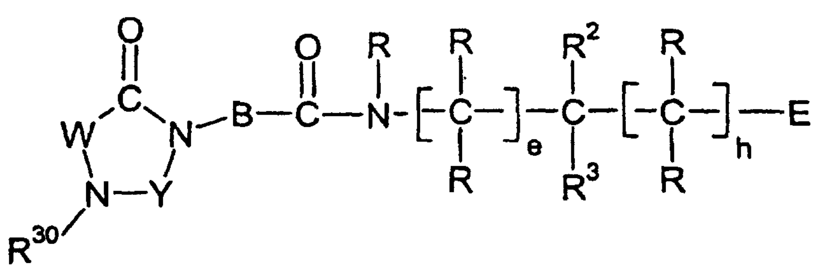

(I)

dans laquelle

w représente un reste divalent de la série de $R^1$-A-C($R^{13}$) et

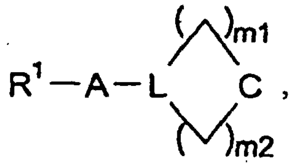

où le système cyclique

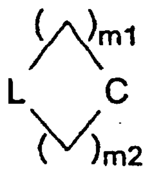

peut contenir un ou deux hétéroatomes identiques ou différents de la série de N et O, peut être saturé ou contenir une insaturation et peut être substitué par 1 ou 2 substituants $R^{13}$ identiques ou différents et/ou par 1 ou 2 atomes d'oxygène doublement liés, L représente C($R^{13}$) ou N et m1 et m2 représentent, indépendamment l'un de l'autre, l'un des chiffres 0, 1, 2, 3 et 4, la somme m1 + m2 devant cependant représenter l'un des chiffres 1, 2, 3 et 4;

Y représente un groupe carbonyle ou un groupe thiocarbonyle;

A représente une liaison directe, l'un des restes divalents alkylène en $C_1$-$C_6$, cycloalkylène en $C_5$-$C_6$, phénylène, phénylène-(alkyle en $C_1$-$C_4$) ou un reste divalent d'un hétérocycle de 5 ou 6 chaînons saturé ou insaturé pouvant contenir 1 ou 2 atomes d'azote et pouvant être substitué une ou deux fois par alkyle en $C_1$-$C_6$ ou par de l'oxygène ou du soufre doublement lié, et, dans le cas du reste phénylène-alkyle, le reste R' est lié au groupe phénylène;

B représente un reste divalent méthylène ou éthylène, le reste méthylène et le reste éthylène étant non substitués ou substitués par un ou deux restes identiques ou différents de la série des restes alkyle en $C_1$-$C_8$, alcényle en $C_2$-$C_8$, alcynyle en $C_2$-$C_8$, cycloalkyle en $C_3$-$C_6$, (cycloalkyl en $C_3$-$C_6$)-(alkyle en $C_1$-$C_6$), aryle en $C_6$-$C_{10}$ éventuellement substitué, (aryl en $C_6$-$C_{10}$)-(alkyle en $C_1$-$C_6$) éventuellement substitué sur le reste aryle, hétéroaryle éventuellement substitué, hétéroaryl-(alkyle en $C_1$-$C_6$) éventuellement substitué sur le reste hétéroaryle;

E représente $R^{10}$CO, HO-$CH_2$ ou $R^8$CO-O-$CH_2$;

R représente un atome d'hydrogène ou un reste alkyle en $C_1$-$C_8$, tous les restes R étant indépendants les uns des autres et les restes R pouvant être identiques ou différents;

$R^1$ représente un atome d'hydrogène, un reste alkyle en $C_1$-$C_{10}$ pouvant éventuellement être substitué une ou plusieurs fois par du fluor, un reste $R^{21}$-(aryle en $C_1$-$C_{10}$) éventuellement substitué sur le reste aryle, un reste ($R^{21}$-(aryl en $C_6$-$C_{10}$))-(alkyle en $C_1$-$C_6$) éventuellement substitué sur le reste aryle, un reste Het, Het-(alkyle en $C_1$-$C_6$) ou l'un des restes X-NH-C(=NH)-$R^{20}$-, $X^1$-NH-$R^{20}$-, $R^{22}$N($R^{21}$)-C(O)-, O= et S=;

X représente un atome d'hydrogène ou un reste alkyle en $C_1$-$C_6$, (alkyl en $C_1$-$C_6$)-carbonyle, (alcoxy en $C_1$-$C_6$)-carbonyle, (alkyl en $C_1$-$C_8$)-carbonyloxy-(alcoxy en $C_1$-$C_6$)-carbonyle, (aryl en $C_6$-$C_{10}$)-carbonyle éventuellement substitué, (aryl en $C_6$-$C_{10}$)-oxycarbonyle éventuellement substitué, (aryl en $C_6$-$C_{14}$)-(alcoxy en $C_1$-$C_6$)-carbonyle pouvant aussi être substitué sur le reste aryle, hydroxy, alcoxy en $C_1$-$C_6$ ou amino;

$X^1$ a l'une des significations de X ou représente un reste R'-NH-C(=N-R") dans lequel R' et R" ont indépendamment l'un de l'autre les significations de X;

$R^2$ représente un atome d'hydrogène ou un reste alkyle en $C_1$-$C_8$;

$R^3$ représente un atome d'hydrogène ou un reste alkyle en $C_1$-$C_8$ pouvant éventuellement être substitué par 1 à 6 atomes de fluor, aryle en $C_6$-$C_{12}$ éventuellement substitué, (aryle en $C_6$-$C_{12}$)-(alkyle en $C_1$-$C_6$) éventuellement substitué sur le reste aryle, hétéroaryle éventuellement substitué, hétéroaryl-(alkyle en $C_1$-$C_6$) éventuellement substitué sur le reste hétéroaryle, cycloalkyle en $C_3$-$C_8$, (cycloalkyl en $C_3$-$C_8$)-(alkyle en $C_1$-$C_6$), bicycloalkyle en $C_6$-$C_{12}$, (bicycloalkyl en $C_6$-$C_{12}$)-(alkyle en $C_1$-$C_6$), tricycloalkyle en $C_6$-$C_{12}$, (tricycloalkyl en $C_6$-$C_{12}$)-(alkyle en $C_1$-$C_6$), alcényle en $C_2$-$C_8$, alcynyle en $C_2$-$C_8$, $R^{11}$NH,

$COOR^{21}$, $CON(CH_3)R^4$, $CONHR^4$, $CON(CH_3)R^{15}$ ou $CONHR^{15}$;

$R^4$ représente un reste alkyle en $C_1$-$C_8$ non substitué ou substitué une ou deux fois par des restes identiques ou différents de la série des restes hydroxy, alcoxy en $C_1$-$C_8$, $R^3$, cycloalkyle en $C_3$-$C_8$ éventuellement substitué, hydroxycarbonyle, aminocarbonyle, (aryl en $C_6$-$C_{10}$)-(alcoxy en $C_1$-$C_4$)-carbonyle pouvant aussi être substitué sur le reste aryle, (alcoxy en $C_1$-$C_6$)-carbonyle, $R^6$-CO, $R^7$-CO, tétrazolyle, trifluorométhyle;

$R^5$ représente un reste aryle en $C_6$-$C_{12}$ éventuellement substitué, (aryl en $C_6$-$C_{12}$)-(alkyle en $C_1$-$C_8$) éventuellement substitué sur le reste aryle, ou un reste d'un système bétérocyclique de 5 à 12 chaînons, monocyclique ou bicyclique éventuellement substitué, qui peut être aromatique, partiellement saturé ou entièrement saturé et qui peut contenir 1, 2 ou 3 hétéroatomes identiques ou différents de la série de l'azote, de l'oxygène et du soufre;

$R^6$ représente le reste d'un aminoacide naturel ou non naturel, d'un iminoacide, d'un aza-aminoacide éventuellement N-substitué par alkyle en $C_1$-$C_8$ ou par (aryl en $C_6$-$C_{12}$)-(alkyle en $C_1$-$C_8$) pouvant aussi être substitué sur le reste aryle, ou le reste d'un dipeptide ou d'un tripeptide, et leurs esters et amides, dans lequel les groupes fonctionnels libres peuvent être protégés par des groupes protecteurs classiques dans la chimie des peptides et dans lequel les atomes d'azote des liaisons amide se trouvant dans le groupe $R^6$-CO peuvent porter un reste R comme substituant;

$R^7$ représente le reste d'un hétérocycle monocyclique ou bicyclique saturé de 5 à 7 chaînons lié par l'intermédiaire d'un atome d'azote, qui peut contenir 1, 2, 3 ou 4 autres hétéroatomes cycliques identiques ou différents de la série de l'oxygène, de l'azote et du soufre et qui peut éventuellement être substitué sur les atomes de carbone et sur les autres atomes d'azotes cycliques, les autres atomes d'azote cycliques pouvant porter des restes identiques ou différents de la série de l'atome d'hydrogène et des restes $R^h$, HCO, $R^h$CO, $R^h$O-CO, HO-CO-(alkyle en $C_1$-$C_4$) et $R^h$O-CO-(alkyle en $C_1$-$C_4$) comme substituants et $R^h$ représente un reste alkyle en $C_1$-$C_6$, cycloalkyle en $C_3$-$C_8$, (cycloalkyl en $C_3$-$C_8$)-(alkyle en $C_1$-$C_8$), aryle en $C_6$-$C_{10}$ éventuellement substitué ou (aryl en $C_6$-$C_{10}$)-(alkyle en $C_1$-$C_4$) éventuellement substitué sur le reste aryle;

$R^8$ représente un atome d'hydrogène ou un reste alkyle en $C_1$-$C_6$ ou phényl-(alkyle en $C_1$-$C_4$) éventuellement substitué sur le reste phényle;

$R^{10}$ représente un reste hydroxy, alcoxy en $C_1$-$C_8$, (aryl en $C_6$-$C_{10}$)-(alcoxy en $C_1$-$C_6$) pouvant aussi être substitué sur le reste aryle, aryloxy en $C_6$-$C_{10}$ éventuellement substitué, (alkyl en $C_1$-$C_6$)-carbonyloxy (alcoxy en $C_1$-$C_6$), (alcoxy en $C_1$-$C_6$)-carbonyloxy-(alcoxy en $C_1$-$C_6$), amino, mono- ou di(alkyl en $C_1$-$C_6$) amino, aminocarbonyl-(alcoxy en $C_1$-$C_6$) ou (mono- ou di(alkyl en $C_1$-$C_6$)amino)-carbonyl-(alcoxy en $C_1$-$C_6$);

$R^{11}$ représente un atome d'hydrogène, $R^{12a}$, $R^{12a}$-CO, $R^{12a}$-O-CO, $R^{12b}$-CO ou $R^{12a}$-$S(O)_2$;

$R^{12a}$ représente un reste alkyle en $C_1$-$C_{10}$, alcényle en $C_2$-$C_8$, alcynyle en $C_2$-$C_8$, cycloalkyle en $C_5$-$C_{10}$, (cycloalkyl en $C_5$-$C_{10}$)-(alkyle en $C_1$-$C_8$), aryle en $C_6$-$C_{14}$ éventuellement substitué, (aryl en $C_6$-$C_{14}$)-(alkyle en $C_1$-$C_8$) éventuellement substitué sur le reste aryle, hétéroaryle éventuellement substitué ou hétéroaryl-(alkyle en $C_1$-$C_8$) éventuellement substitué sur le reste hétéroaryle ou le reste $R^{15}$;

$R^{12b}$ représente un reste amino, di(alkyl en $C_1$-$C_{10}$)amino ou $R^{12a}$-NH;

$R^{13}$ représente un atome d'hydrogène ou un reste alkyle en $C_1$-$C_6$;

$R^{15}$ représente un reste $R^{16}$-(alkyle en $C_1$-$C_6$) ou $R^{16}$;

$R^{16}$ représente un reste bicyclique ou tricyclique de 6 à 14 chaînons, saturé ou partiellement insaturé, qui peut aussi contenir 1, 2, 3 ou 4 hétéroatomes identiques ou différents de la série de l'azote, de l'oxygène et du soufre et qui peut aussi être substitué par un ou plusieurs substituants identiques ou différents de la série des restes alkyle en $C_1$-$C_4$ et oxo;

$R^{20}$ représente une liaison directe ou un reste alkylène en $C_1$-$C_2$;

$R^{21}$ représente un atome d'hydrogène ou un reste alkyle en $C_1$-$C_6$, cycloalkyle en $C_3$-$C_6$, (cycloalkyl en $C_3$-$C_6$) -(alkyle en $C_1$-$C_4$), aryle en $C_6$-$C_{10}$ éventuellement substitué, (aryl en $C_6$-$C_{10}$)-(alkyle en $C_1$-$C_4$) éventuellement substitué sur le reste aryle, le reste Het ou Het(alkyle en $C_1$-$C_4$), les restes alkyle pouvant être substitués une ou plusieurs fois par du fluor et les restes $R^{21}$ pouvant être identiques ou différents s'ils apparaissent plusieurs fois;

$R^{22}$ représente l'un des restes $R^{21}$, $R^{21}N(R^{21})$ ou $R^{21}N(R^{21})$-C(=N($R^{21}$));

$R^{30}$ représente l'un des restes $R^{32}$(R)N-CO-N(R)-$R^{31}$, $R^{32}$(R)N-CS-N(R)-$R^{31}$, $R^{32}$-CO-N(R)-$R^{31}$ ou $R^{32}$(R) N-CO-$R^{31}$, $R^{30}$ ne pouvant représenter le reste $R^{32}$-CO-N(R)-$R^{31}$ lorsque, en même temps, W représente un reste $R^1$-A-C($R^{13}$), A représente une liaison directe et $R^1$ et $R^{13}$ sont des atomes d'hydrogène;

$R^{31}$ représente le reste divalent -$R^{33}$-$R^{34}$-$R^{35}$-$R^{36}$-, où $R^{36}$ est lié à l'atome d'azote du cycle imidazolidine de la formule I;

$R^{32}$ représente un atome d'hydrogène ou un reste alkyle en $C_1$-$C_6$ pouvant éventuellement être substitué par

1 à 6 atomes de fluor, alcényle en $C_2$-$C_6$, alcynyle en $C_2$-$C_6$, cycloalkyle en $C_5$-$C_6$, (cycloalkyl en $C_5$-$C_6$) -(alkyle en $C_1$-$C_6$), aryle en $C_6$-$C_{10}$ éventuellement substitué, (aryl en $C_6$-$C_{10}$)-(alkyle en $C_1$-$C_6$) éventuellement substitué sur le reste aryle, hétéroaryle éventuellement substitué, ou hétéroaryl-(alkyle en $C_1$-$C_6$) éventuellement substitué sur le reste hétéroaryle;

$R^{33}$ représente une liaison directe ou un reste alkylène en $C_1$-$C_4$ divalent;

$R^{34}$ représente un reste divalent de la série des restes alkylène en $C_1$-$C_6$, cycloalkylène en $C_5$-$C_6$, arylène en $C_6$-$C_{10}$ éventuellement substitué et hétéroarylène éventuellement substitué;

$R^{35}$ représente une liaison directe ou un reste alkylène en $C_1$-$C_4$ divalent;

$R^{36}$ représente une liaison directe, le groupe -CO- ou le groupe -$S(O)_n$-;

Het représente un reste d'un cycle de 5 à 12 chaînons monocyclique ou polycyclique, aromatique ou non aromatique, qui contient 1 ou 2 hétéroatomes identiques ou différents de la série de N et O comme chaînons cycliques et qui peut éventuellement être substitué par un ou plusieurs substituants identiques ou différents;

e et h représentent indépendamment l'un de l'autre 0 ou 1;

n représente 1 ou 2;

sous toutes ses formes stéréo-isomères et leurs mélanges en toutes proportions, et ses sels physiologiquement acceptables.

**2.** Composé de formule I selon la revendication 1, dans lequel

W représente le reste divalent $R^1$-A-C($R^{13}$);

Y représente un groupe carbonyle;

A représente une liaison directe, l'un des restes divalents alkylène en $C_1$-$C_6$, phénylène, phénylène-(alkyle en $C_1$-$C_2$) ou un reste divalent d'un hétérocycle de 5 ou 6 chaînons saturé ou insaturé pouvant contenir 1 ou 2 atomes d'azote et pouvant être substitué une ou deux fois par alkyle en $C_1$-$C_6$ ou par de l'oxygène ou du soufre doublement lié, et, dans le cas du reste phénylène-alkyle, le reste $R^1$ est lié au groupe phénylène;

B représente un reste divalent méthylène ou éthylène, le reste méthylène et le reste éthylène étant non substitués ou substitués par un reste de la série des restes alkyle en $C_1$-$C_8$, alcényle en $C_2$-$C_8$, alcynyle en $C_2$-$C_8$, cycloalkyle en $C_3$-$C_6$, (cycloalkyl en $C_3$-$C_6$)-(alkyle en $C_1$-$C_6$), aryle en $C_6$-$C_{10}$ éventuellement substitué, (aryl en $C_6$-$C_{10}$)-(alkyle en $C_1$-$C_6$) éventuellement substitué sur le reste aryle, hétéroaryle éventuellement substitué, hétéroaryl-(alkyle en $C_1$-$C_6$) éventuellement substitué sur le reste hétéroaryle;

E représente $R^{10}$CO, HO-$CH_2$ ou $R^8$CO-O-$CH_2$;

R représente un atome d'hydrogène ou un reste alkyle en $C_1$-$C_8$, tous les restes R étant indépendants les uns des autres et les restes R pouvant être identiques ou différents;

$R^1$ représente un atome d'hydrogène, un reste alkyle en $C_1$-$C_{10}$ pouvant éventuellement être substitué une ou plusieurs fois par du fluor, un reste $R^{21}$-(aryle en $C_1$-$C_{10}$) éventuellement substitué sur le reste aryle, un reste ($R^{21}$-(aryl en $C_6$-$C_{10}$))-(alkyle en $C_1$-$C_6$) éventuellement substitué sur le reste aryle, un reste Het, Het-(alkyle en $C_1$-$C_6$) ou l'un des restes X-NH-C(=NH)-$R^{20}$-, $X^1$-NH-$R^{20}$- et O=;

X représente un atome d'hydrogène ou un reste alkyle en $C_1$-$C_6$, (alkyl en $C_1$-$C_6$)-carbonyle, (alcoxy en $C_1$-$C_6$)-carbonyle, (alkyl en $C_1$-$C_6$)-carbonyloxy-(alcoxy en $C_1$-$C_6$)-carbonyle, (aryl en $C_6$-$C_{10}$)-carbonyle éventuellement substitué, (aryl en $C_6$-$C_{10}$)-oxycarbonyle éventuellement substitué, (aryl en $C_6$-$C_{14}$)-(alcoxy en $C_1$-$C_6$)-carbonyle pouvant aussi être substitué sur le reste aryle, hydroxy, alcoxy en $C_1$-$C_6$ ou amino;

$X^1$ a l'une des significations de X ou représente un reste R'-NH-C(=N-R") dans lequel R' et R" ont indépendamment l'un de l'autre les significations de X;

$R^2$ représente un atome d'hydrogène ou un reste alkyle en $C_1$-$C_6$;

$R^3$ représente un atome d'hydrogène ou un reste alkyle en $C_1$-$C_8$ pouvant éventuellement être substitué par 1 à 6 atomes de fluor, aryle en $C_6$-$C_{10}$ éventuellement substitué, (aryle en $C_6$-$C_{10}$)-(alkyle en $C_1$-$C_4$) éventuellement substitué sur le reste aryle, hétéroaryle éventuellement substitué, hétéroaryl-(alkyle en $C_1$-$C_4$) éventuellement substitué sur le reste hétéroaryle, cycloalkyle en $C_3$-$C_8$, (cycloalkyl en $C_3$-$C_8$) -(alkyle en $C_1$-$C_4$), bicycloalkyle en $C_6$-$C_{12}$, (bicycloalkyl en $C_6$-$C_{12}$)-(alkyle en $C_1$-$C_4$), tricycloalkyle en $C_6$-$C_{12}$, (tricycloalkyl en $C_6$-$C_{12}$)-(alkyle en $C_1$-$C_6$), alcényle en $C_2$-$C_8$, alcynyle en $C_2$-$C_8$, $R^{11}$NH, $COOR^{21}$, $CON(CH_3)R^4$, $CONHR^4$, $CON(CH_3)R^{15}$ ou $CONHR^{15}$;

$R^4$ représente un reste alkyle en $C_1$-$C_6$ non substitué ou substitué une ou deux fois par des restes identiques ou différents de la série des restes hydroxy, alcoxy en $C_1$-$C_8$, $R^5$, cycloalkyle en $C_3$-$C_8$ éventuellement substitué, hydroxycarbonyle, aminocarbonyle, (aryl en $C_6$-$C_{10}$)-(alcoxy en $C_1$-$C_4$)-carbonyle pouvant aus-

si être substitué sur le reste aryle, (alcoxy en $C_1$-$C_6$)-carbonyle, $R^6$-CO, $R^7$-CO, tétrazolyle, trifluorométhyle;

$R^5$ représente un reste aryle en $C_6$-$C_{10}$ éventuellement substitué, (aryl en $C_6$-$C_{10}$)-(alkyle en $C_1$-$C_4$) éventuellement substitué sur le reste aryle, ou un reste d'un système hétérocyclique de 5 à 12 chaînons, monocyclique ou bicyclique éventuellement substitué, qui peut être aromatique, partiellement saturé ou entièrement saturé et qui peut contenir 1, 2 ou 3 hétéroatomes identiques ou différents de la série de l'azote, de l'oxygène et du soufre;

$R^6$ représente le reste d'un aminoacide naturel ou non naturel ou le reste d'un dipeptide ou d'un tripeptide et leurs esters et amides, dans lequel les groupes fonctionnels libres peuvent être protégés par des groupes protecteurs classiques dans la chimie des peptides et dans lequel les atomes d'azote des liaisons amide se trouvant dans le groupe $R^6$-CO peuvent porter un reste R comme substituant;

$R^7$ représente le reste d'un hétérocycle monocyclique saturé de 5 à 7 chaînons lié par l'intermédiaire d'un atome d'azote, qui peut contenir 1 ou 2 autres hétéroatomes cycliques identiques ou différents de la série de l'oxygène, de l'azote et du soufre et qui peut éventuellement être substitué sur les atomes de carbone et sur les autres atomes d'azotes cycliques, les autres atomes d'azote cycliques pouvant porter des restes identiques ou différents de la série de l'atome d'hydrogène et des restes $R^h$, HCO, $R^h$CO, $R^h$O-CO, HO-CO-(alkyle en $C_1$-$C_4$) et $R^h$O-CO-(alkyle en $C_1$-$C_4$) comme substituants et $R^h$ représente un reste alkyle en $C_1$-$C_4$, aryle en $C_6$-$C_{10}$ éventuellement substitué ou (aryl en $C_6$-$C_{10}$)-(alkyle en $C_1$-$C_4$) éventuellement substitué sur le reste aryle;

$R^8$ représente un atome d'hydrogène ou un reste alkyle en $C_1$-$C_6$ ou phényl-(alkyle en $C_1$-$C_4$) éventuellement substitué sur le reste phényle;

$R^{10}$ représente un reste hydroxy, alcoxy en $C_1$-$C_8$, (aryl en $C_6$-$C_{10}$)-(alcoxy en $C_1$-$C_6$) pouvant aussi être substitué sur le reste aryle, aryloxy en $C_6$-$C_{10}$ éventuellement substitué, (alkyl en $C_1$-$C_6$)-carbonyloxy-(alcoxy en $C_1$-$C_6$), (alcoxy en $C_1$-$C_6$)-carbonyloxy-(alcoxy en $C_1$-$C_6$), amino, mono- ou di(alkyl en $C_1$-$C_6$) amino, aminocarbonyl-(alcoxy en $C_1$-$C_6$) ou (mono- ou di(alkyl en $C_1$-$C_6$)amino)-carbonyl-(alcoxy en $C_1$-$C_6$);

$R^{11}$ représente un atome d'hydrogène, $R^{12a}$, $R^{12a}$-CO, $R^{12a}$-O-CO, $R^{12b}$-CO ou $R^{12a}$-$S(O)_2$;

$R^{12a}$ représente un reste alkyle en $C_1$-$C_8$, alcényle en $C_2$-$C_8$, alcynyle en $C_2$-$C_8$, cycloalkyle en $C_5$-$C_6$, (cycloalkyl en $C_5$-$C_6$)-(alkyle en $C_1$-$C_4$), aryle en $C_6$-$C_{10}$ éventuellement substitué, (aryl en $C_6$-$C_{10}$)-(alkyle en $C_1$-$C_4$) éventuellement substitué sur le reste aryle, hétéroaryle éventuellement substitué ou hétéroaryl-(alkyle en $C_1$-$C_4$) éventuellement substitué sur le reste hétéroaryle ou le reste $R^{15}$;

$R^{12b}$ représente un reste amino, di(alkyl en $C_1$-$C_8$)amino ou $R^{12a}$-NH;

$R^{13}$ représente un atome d'hydrogène ou un reste alkyle en $C_1$-$C_6$;

$R^{15}$ représente un reste $R^{16}$-(alkyle en $C_1$-$C_6$) ou $R^{16}$;

$R^{16}$ représente un reste bicyclique ou tricyclique de 6 à 12 chaînons, saturé ou partiellement insaturé, qui peut aussi contenir 1, 2, 3 ou 4 hétéroatomes identiques ou différents de la série de l'azote, de l'oxygène et du soufre et qui peut aussi être substitué par un ou plusieurs substituants identiques ou différents de la série des restes alkyle en $C_1$-$C_4$ et oxo;

$R^{20}$ représente une liaison directe ou un reste méthylène;

$R^{21}$ représente un atome d'hydrogène ou un reste alkyle en $C_1$-$C_6$, aryle en $C_6$-$C_{10}$ éventuellement substitué, (aryl en $C_6$-$C_{10}$)-(alkyle en $C_1$-$C_2$) éventuellement substitué sur le reste aryle, le reste Het ou Het(alkyle en $C_1$-$C_2$), les restes alkyle pouvant être substitués une à quatre fois par du fluor et les restes $R^{21}$ pouvant être identiques ou différents s'ils apparaissent plusieurs fois;

$R^{30}$ représente l'un des restes $R^{32}$(R)N-CO-N(R)-$R^{31}$ et $R^{32}$(R)N-CS-N(R)-$R^{31}$;

$R^{31}$ représente un reste divalent de la série des restes alkylène en $C_1$-$C_6$, arylène en $C_6$-$C_{10}$ éventuellement substitué, (arylène en $C_6$-$C_{10}$)-(alkyle en $C_1$-$C_4$) éventuellement substitué sur le reste arylène, cycloalkylène en $C_5$-$C_6$, (cycloalkylène en $C_5$-$C_6$)-(alkyle en $C_1$-$C_4$), hétéroarylène éventuellement substitué ou hétéroarylène-(alkyle en $C_1$-$C_4$) éventuellement substitué sur le reste hétéroarylène, et, dans le cas du reste arylène-alkyle, du reste cycloalkylène-alkyle et du reste hétéroarylène-alkyle, le groupe alkyle est lié à l'atome d'azote du cycle imidazolidine de la formule I;

$R^{32}$ représente un atome d'hydrogène ou un reste alkyle en $C_1$-$C_6$ pouvant éventuellement être substitué par 1 à 6 atomes de fluor, alcényle en $C_2$-$C_6$, alcynyle en $C_2$-$C_6$, cycloalkyle en $C_5$-$C_6$, (cycloalkyl en $C_5$-$C_6$) -(alkyle en $C_1$-$C_4$), aryle en $C_6$-$C_{10}$ éventuellement substitué, (aryl en $C_6$-$C_{10}$)-(alkyle en $C_1$-$C_4$) éventuellement substitué sur le reste aryle, hétéroaryle éventuellement substitué, ou hétéroaryl-(alkyle en $C_1$-$C_4$) éventuellement substitué sur le reste hétéroaryle;

Het représente un reste d'un cycle de 5 à 10 chaînons monocyclique ou polycyclique, aromatique ou non aromatique, qui contient 1 ou 2 hétéroatomes identiques ou différents de la série de N et O comme chaînons cycliques et qui peut éventuellement être substitué par un ou plusieurs substituants identiques

ou différents;
e et h représentent indépendamment l'un de l'autre 0 ou 1;

sous toutes ses formes stéréo-isomères et leurs mélanges en toutes proportions, et ses sels physiologiquement acceptables.

**3.** Composé de formule I selon la revendication 1 et/ou 2, dans lequel W a une signification différente de $CH_2$, sous toutes ses formes stéréo-isomères et leurs mélanges en toutes proportions, et ses sels physiologiquement acceptables.

**4.** Composé de formule I selon l'une ou plusieurs des revendications 1 à 3, dans lequel W représente le reste divalent (alkyl en $C_1$-$C_4$)$_2$C<, sous toutes ses formes stéréo-isomères et leurs mélanges en toutes proportions, et ses sels physiologiquement acceptables.

**5.** Composé de formule I selon l'une ou plusieurs des revendications 1 à 4, dans lequel B représente un reste méthylène non substitué ou un reste méthylène substitué par alkyle en $C_1$-$C_6$ ou (cycloalkyl en $C_3$-$C_6$)-(alkyle en $C_1$-$C_2$), sous toutes ses formes stéréo-isomères et leurs mélanges en toutes proportions, et ses sels physiologiquement acceptables.

**6.** Composé de formule I selon l'une ou plusieurs des revendications 1 à 5, dans lequel $R^{30}$ représente l'un des restes $R^{32}$(R)N-CO-N(R)-$R^{31}$ et $R^{32}$(R)N-CS-N(R)-$R^{31}$, sous toutes ses formes stéréo-isomères et leurs mélanges en toutes proportions, et ses sels physiologiquement acceptables.

**7.** Composé de formule I selon l'une ou plusieurs des revendications 1 à 6, dans lequel $R^{31}$ représente un reste (arylène en $C_6$-$C_{10}$)-(alkyle en $C_1$-$C_2$) éventuellement substitué sur le reste arylène, dans lequel le groupe alkyle est lié à l'atome d'azote du cycle imidazolidine, sous toutes ses formes stéréo-isomères et leurs mélanges en toutes proportions, et ses sels physiologiquement acceptables.

**8.** Composé de formule I selon l'une ou plusieurs des revendications 1 à 7, dans lequel $R^{32}$ représente un reste aryle en $C_6$-$C_{10}$ éventuellement substitué, sous toutes ses formes stéréo-isomères et leurs mélanges en toutes proportions, et ses sels physiologiquement acceptables.

**9.** Composé de formule I selon l'une ou plusieurs des revendications 1 à 8, dans lequel $R^3$ représente un reste alkyle en $C_1$-$C_4$ pouvant éventuellement être substitué par 1 à 6 atomes de fluor, e est 0 et h est 1, sous toutes ses formes stéréo-isomères et leurs mélanges en toutes proportions, et ses sels physiologiquement acceptables.

**10.** Composé de formule I selon l'une ou plusieurs des revendications 1 à 8, dans lequel $R^3$ représente un reste aryle en $C_6$-$C_{10}$ non substitué ou substitué, e est 0 et h est 1, sous toutes ses formes stéréo-isomères et leurs mélanges en toutes proportions, et ses sels physiologiquement acceptables.

**11.** Composé de formule I selon l'une ou plusieurs des revendications 1 à 10, dans lequel $R^{30}$ représente le reste $R^{32}$NH-CO-NH-$R^{31}$ et $R^{31}$ représente le reste divalent -(1,4-phénylène)-$CH_2$- dans lequel le groupe méthylène est lié à l'atome d'azote du cycle imidazolidine, et $R^{32}$ représente un reste phényle non substitué ou substitué, sous toutes ses formes stéréo-isomères et leurs mélanges en toutes proportions, et ses sels physiologiquement acceptables.

**12.** Composé de formule I selon l'une ou plusieurs des revendications 1 à 11, dans lequel W a une signification différente de $CH_2$, B représente un reste méthylène non substitué ou un reste méthylène substitué par alkyle en $C_1$-$C_6$ ou (cycloalkyl en $C_3$-$C_6$)-(alkyle en $C_1$-$C_2$), $R^{30}$ représente le reste $R^{32}$NH-CO-NH-$R^{31}$, $R^{31}$ représente le reste divalent -(1,4-phénylène)-$CH_2$- dans lequel le groupe méthylène est lié à l'atome d'azote du cycle imidazolidine, $R^{32}$ représente un reste phényle non substitué ou substitué, et le groupe -NR-[C(R)(R)]$_e$-C($R^2$)($R^3$)-[C(R)(R)]$_h$-E de la formule I représente le groupe -NH-CH($R^3$)-$CH_2$-E, sous toutes ses formes stéréo-isomères et leurs mélanges en toutes proportions, et ses sels physiologiquement acceptables.

**13.** Acide (R)-3-((S)-2-(4,4-diméthyl-3-(4-(3-phényluréido)benzyl)-2,5-dioxoimidazolidin-1-yl)-2-(2-méthylpropyl)acétylamino)-3-méthylpropionique de formule

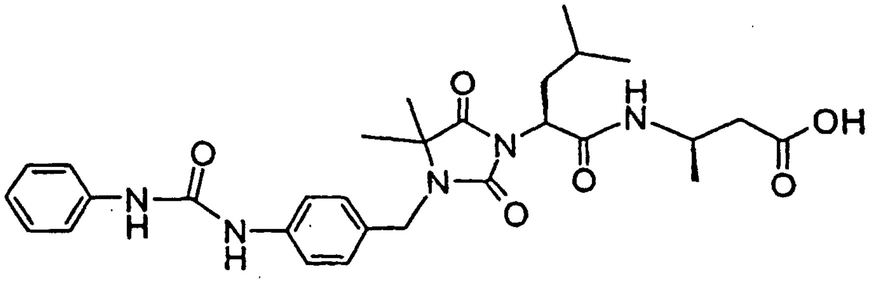

et ses sels physiologiquement acceptables.

**14.** Acide (S)-3-((S)-2-(4,4-diméthyl-3-(4-(3-(2-méthylphényl)uréido)-benzyl)-2,5-dioxoimidazolidin-1-yl)-2-(2-méthylpropyl)acétylamino)-3-phénylpropionique de formule

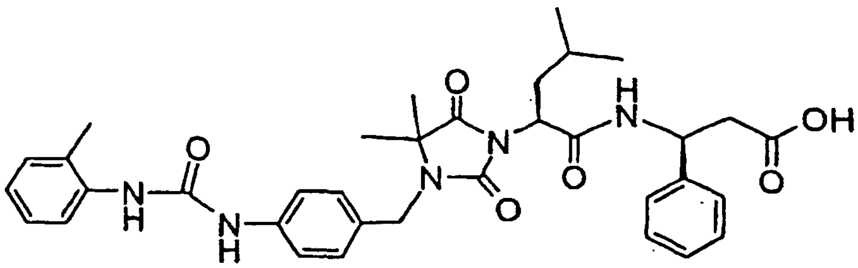

et ses sels physiologiquement acceptables.

**15.** Sel de sodium de l'acide (R)-3-((S)-2-(4,4-diméthyl-3-(4-(3-phényluréido)benzyl)-2,5-dioxoimidazolidin-1-yl)-2-(2-méthylpropyl)acétylamino)-3-méthylpropionique de formule

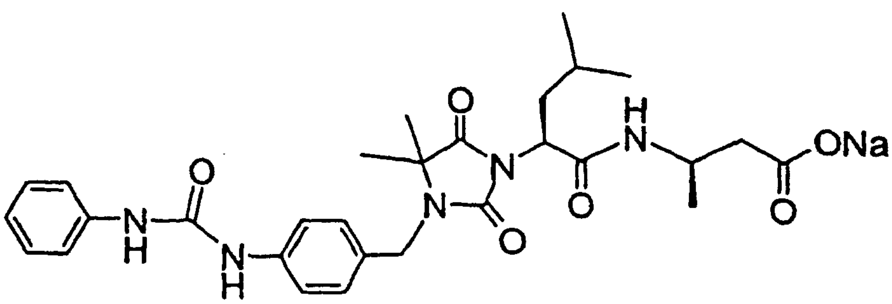

**16.** Acide (R)-3-((S)-2-(4,4-diméthyl-3-(4-(3-phényluréido)benzyl)-2,5-dioxoimidazolidin-1-yl)-2-(cyclopropylméthyl) acétylamino)-3-méthylpropionique de formule

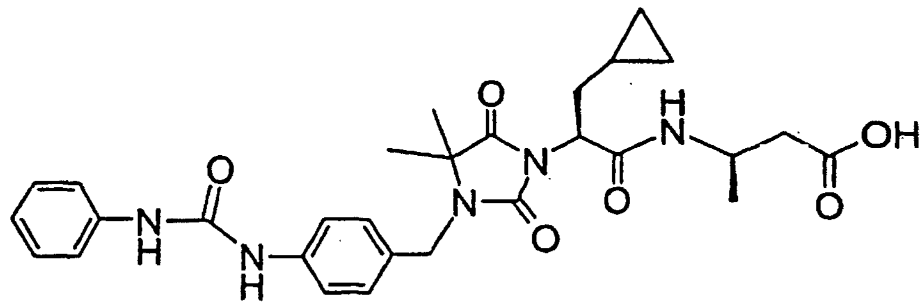

et ses sels physiologiquement acceptables.

**17.** Composé de formule

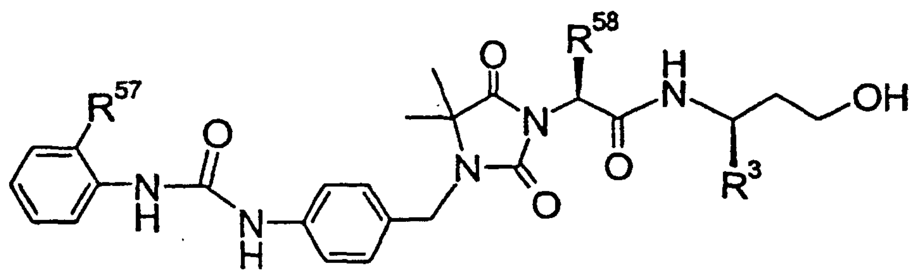

dans laquelle $R^3$ représente un reste méthyle, $R^{57}$ un atome d'hydrogène et $R^{58}$ un reste isobutyle.

**18.** Procédé de préparation d'un composé de formule I selon l'une ou plusieurs des revendications 1 à 17, **caractérisé en ce que** l'on effectue une condensation de fragments d'un composé de formule II

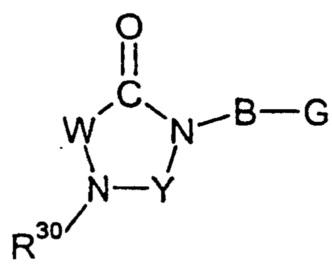

(II)

avec un composé de formule III

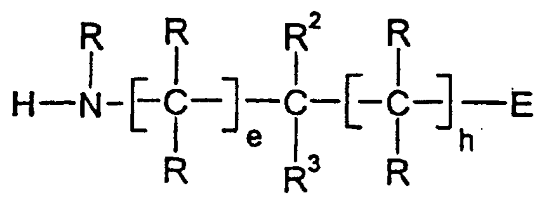

(III)

où, dans les formules II et III, les groupes W, Y, B, E, R, $R^2$, $R^3$, $R^{30}$ et e et h ont la définition indiquée dans les revendications 1 à 17 ou des groupes fonctionnels sous une forme protégée ou sous forme de précurseurs peuvent aussi être contenus, et G représente un groupe hydroxycarbonyle, (alcoxy en $C_1$-$C_6$)carbonyle ou des dérivés activés d'acides carboxyliques.

**19.** Composé de formule I selon l'une ou plusieurs des revendications 1 à 17 et/ou un de ses sels physiologiquement acceptables à utiliser comme médicament.

**20.** Composition pharmaceutique **caractérisée en ce qu'**elle contient un ou plusieurs composés de formule I selon l'une ou plusieurs des revendications 1 à 17 et/ou leurs sels physiologiquement acceptables et un support pharmaceutiquement acceptable.

**21.** Composition pharmaceutique **caractérisée en ce qu'**elle contient un composé selon l'une des revendications 13 à 17 et/ou un de ses sels physiologiquement acceptables et un support pharmaceutiquement acceptable.

**22.** Composé de formule I selon l'une ou plusieurs des revendications 1 à 17 et/ou un de ses sels physiologiquement acceptables, à utiliser comme anti-inflammatoire.

**23.** Composé de formule I selon l'une ou plusieurs des revendications 1 à 17 et/ou un de ses sels physiologiquement acceptables, à utiliser dans la thérapie ou la prophylaxie de l'arthrite, de l'arthrite rhumatoïde, de la polyarthrite, des maladies intestinales inflammatoires, du lupus érythémateux aigu disséminé, de la sclérose en plaques ou de maladies inflammatoires du système nerveux central.

**24.** Composé de formule I selon l'une ou plusieurs des revendications 1 à 17 et/ou un de ses sels physiologiquement acceptables, à utiliser dans la thérapie ou la prophylaxie de l'asthme ou d'allergies.

**25.** Composé de formule I selon l'une ou plusieurs des revendications 1 à 17 et/ou un de ses sels physiologiquement acceptables, à utiliser dans la thérapie ou la prophylaxie de maladies cardio-vasculaires, de l'artériosclérose, de resténoses, du diabète, de lésions de greffes d'organes, de maladies immunitaires, de maladies auto-immunes, de la croissance tumorale ou de la formation de métastases tumorales, ou du paludisme.

**26.** Composé de formule I selon l'une ou plusieurs des revendications 1 à 17 et/ou un de ses sels physiologiquement acceptables, à utiliser comme inhibiteur de l'adhésion et/ou de la migration de leucocytes ou pour inhiber le récepteur de VLA-4.